(19) Europäisches Patentamt / European Patent Office / Office européen des brevets

(11) EP 4 650 001 A2

(12) EUROPEAN PATENT APPLICATION

(43) Date of publication:
**19.11.2025 Bulletin 2025/47**

(21) Application number: **25205474.7**

(22) Date of filing: **13.05.2022**

(51) International Patent Classification (IPC):
***A61P 25/00*** *(2006.01)*

(52) Cooperative Patent Classification (CPC):
**C07D 487/04; A61P 25/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.05.2021 GB 202106872**

(62) Document number(s) of the earlier application(s) in accordance with Art. 76 EPC:
**22728261.3 / 4 337 664**

(71) Applicant: **Neurosterix Pharma Sàrl**
**1202 Geneva (CH)**

(72) Inventors:
- **PAPARIN, Jean-Laurent**
  **1202 Geneva (CH)**
- **ROCHER, Jean-Philippe**
  **1202 Geneva (CH)**
- **FINN, Terry**
  **1202 Geneva (CH)**
- **DUVEY, Guillaume**
  **74300 Thiez (FR)**

(74) Representative: **Reddie & Grose LLP**
**The White Chapel Building**
**10 Whitechapel High Street**
**London E1 8QS (GB)**

Remarks:
This application was filed on 29-09-2025 as a divisional application to the application mentioned under INID code 62.

(54) **FUSED HETEROCYCLIC DERIVATIVES AS NEGATIVE ALLOSTERIC MODULATORS OF MGLU7 RECEPTOR**

(57)

INVENTION

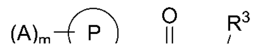

The present invention relates to novel compounds of Formula (I), wherein P, Q, A, B, m, n, $R^1$, $R^2$ and $R^3$ are defined as in Formula (I); which are negative allosteric modulators of the metabotropic glutamate receptor subtype 7 (mGlu7) and which are useful for the treatment or prevention of neurological, ear and psychiatric disorders associated with glutamate dysfunction and diseases in which the mGlu7 subtype of metabotropic receptors is involved. The invention is also directed to pharmaceutical compositions comprising such compounds, to processes to prepare such compounds and such compositions, and to the use of such compounds for the prevention or treatment of neurological, ear and psychiatric disorders and diseases in which mGlu7 is involved.

EP 4 650 001 A2

**Description**

SUMMARY OF THE INVENTION

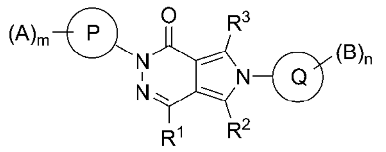

(I)

The present invention relates to novel compounds of Formula (I), wherein P, Q, A, B, m, n, $R^1$, $R^2$ and $R^3$ are defined as in Formula (I); which are negative allosteric modulators of the metabotropic glutamate receptor subtype 7 (mGlu7) and which are useful for the treatment or prevention of neurological, ear and psychiatric disorders associated with glutamate dysfunction and diseases in which the mGlu7 subtype of metabotropic receptors is involved. The invention is also directed to pharmaceutical compositions comprising such compounds, to processes of preparing such compounds and such compositions, and to the use of such compounds for the prevention or treatment of neurological, ear and psychiatric disorders and diseases in which mGlu7 is involved.

BACKGROUND OF THE INVENTION

Glutamate is the primary amino-acid transmitter in the mammalian central nervous system (CNS). Glutamate is associated with numerous physiological functions learning and memory, sensory perception, development of synaptic plasticity, motor control, respiration, and regulation of cardiovascular function. Furthermore, glutamate is at the centre of several different neurological and psychiatric diseases, where there is an imbalance in glutamatergic neurotransmission.

Glutamate mediates synaptic neurotransmission through the activation of ionotropic glutamate receptor channels (iGluRs), the NMDA, AMPA and kainate receptors which are responsible for fast excitatory transmission (Nakanishi et al. (1998) Brain Res. Rev., 26:230-235).

In addition, glutamate activates metabotropic glutamate receptors (mGluRs) which have a modulatory role that contributes to the fine-tuning of synaptic efficacy (Niswender & Conn (2010) Ann. Rev. Pharmacol. Toxicol. 50:295-322). As opposed to iGluRs, mGluRs do not mediate but rather "modulate" synaptic transmission acting at different levels of the tripartite synapse formed by the junction of axon terminals, dendritic spines, and astrocytes. The mGluRs are seven-transmembrane domain-containing G protein-coupled receptors (GPCRs) belonging to family 3 GPCRs along with the calcium-sensing, $GABA_B$, and pheromone receptors. Glutamate activates the mGluRs through binding to a site on the large extracellular amino-terminal domain of the receptor, herein called the orthosteric binding site. This activation induces a conformational change of the rest of the receptor which results in the activation of the G-protein and subsequently to a large variety of intracellular signalling pathways. The mGluR family is composed of eight members. They are classified into three groups (group I comprising mGlu1 and mGlu5; group II comprising mGlu2 and mGlu3; group III comprising mGlu4, mGlu6, mGlu7, and mGlu8) according to sequence homology, pharmacological profile, and nature of intracellular signalling cascades activated (Schoepp et al. (1999) Neuropharmacology, 38:1431-1476).

Among mGlu receptors, the mGlu7 subtype is the most widely distributed and is present pre-synaptically at a broad range of synapses that are postulated to be critical for both normal CNS functions and a range of psychiatric and neurological disorders (Ohishi et al. (1995) J. Comp. Neurol. 360(4):555-570; Kinzie et al. (1995) Neuroscience, 69(1):167-176; Corti et al. (1998) Eur. J. Neurosci, 10(12):3629-3641). mGlu7 is negatively coupled to adenylate cyclase via activation of G$\alpha$i-protein, and its activation as a pre-synaptic autoreceptor leads to inhibition of glutamate and GABA release in the synapse (Dalezios et al. (2002) Cereb. Cortex, 12(9):961-974; Cartmell and Schoepp (2000) J. Neurochem., 75:889-907; Somogyi et al. (2003) Eur. J. Neurosci. 17(12):2503-2520) therefore shaping the synaptic responses at glutamatergic synapses as well as being a key regulator of inhibitory GABAergic transmission with the final goal of fine tuning the overall excitability of the brain.

Previously, most available pharmacological tools targeting mGluRs were orthosteric ligands which cross react with several members of the family as they are structural analogs of glutamate (Schoepp et al. (1999) Neuropharmacology, 38:1431-1476). However, with new screening methods, it has become possible to identify molecules selective to individual mGluRs that act through allosteric mechanisms, modulating the receptor by binding to a site different from the highly conserved orthosteric binding site. These types of molecules have been discovered for several mGluRs (reviewed in Hellyer et al. (2017) Curr. Opin. Pharmacol. 32:49-55; Stansley & Conn (2019) Trends Pharmacol. Sci. 40(4):240-52; Dogra & Conn, (2022) Mol. 101(5):275-285). Several small molecules targeting mGlu7 receptors have been identified in recent years (reviewed in Vasquez-Villa & Trabanco (2019) Med. Chem. Comm. 10:193-9). AMN082 was described as

being a potent, selective and systemically active mGlu7 allosteric agonist (Mitsukawa et al. (2005) Proc. Natl. Acad. Sci. USA, 102:18712-18717). 7-Hydroxy-3-(4-iodophenoxy)-4H-chromen-4-one (XAP044), an allosteric antagonist of mGlu7 was also recently described (Gee et al. (2014) J. Biol. Chem. 18;289(16):10975-10987), acting via a binding pocket localized in the receptor's extracellular Venus flytrap domain. Finally, several classes of compounds have been described, such as isoxazolopyridinone derivatives, phenylbenzamide derivatives, dihydrobenzoxazolone derivatives, tetrahydrophthalazinone derivatives and pharmacologically characterized as selective mGlu7 negative allosteric modulators (Suzuki et al. (2007) J. Pharmacol. Exp. Ther., 323:147-156; Kalinichev et al. (2013) J. Pharmacol. Exp. Ther. 344(3):624-636; Reed et al. (2017) ACS Med. Chem. Lett. (12):1326-1330 and Duvey et al (2019) WO2019063569).

Specifically, modulators of the mGlu7, and preferably antagonists, inverse agonists, and negative allosteric modulators (NAMs), are reported to hold potential for the treatment of neurological, psychiatric, mood disorders as well as pain and otic disorders, based on experimental studies on laboratory animals, deemed relevant to clinical syndromes. Combined expression of mGlu7 in brain regions and pharmacological manipulations of mGlu7 in genetically modified mice and wild-type animals reveal an important role for mGlu7 in numerous CNS disorders, including depression, schizophrenia, anxiety, obsessive compulsive disorders and associated symptoms (reviewed by Pallazo et al. (2016) Curr. Neuropharmacol. 14(5): 504-513), and in particular in acute and chronic stress-related disorders (reviewed by Peterlik et al. (2016) Curr Neuropharmacol. 14(5):514-539).

mGlu7 has been shown to be located on limbic system nuclei such as the amygdala, hippocampus and the locus coerulus, regions that are known to be critical for the manifestation of anxiolysis and antidepressant actions (Kinoshita et al. (1998) J. Comp. Neurol., 393(3):332-352; Makoff et al. (1996) Brain Res. Mol. Brain Res., 40(1):165-170; Kinzie et al. (1995) Neuroscience, 69(1):167-176). Moreover, studies in several behavioral models (light-dark box test, elevated plus maze, staircase test, forced swim test and tail suspension test) have shown that mGlu7 knockout animals exhibit an anxiolytic and anti-depressant phenotype but also some deficits in amygdala-dependent behaviors (fear response and conditioned taste aversion) (Cryan et al. (2003) Eur. J. Neuroscience, 17:2409-2417). Therefore, a pharmacological agent aiming at modulating mGlu7 activity may represent a novel therapeutic approach for the treatment of neurological and psychiatric disorders such as anxiety and depression.

Activation of mGlu7 using the allosteric agonist AMN082 increase plasma levels of the stress hormones corticosterone and ACTH (Mitsukawa et al. (2005) PNAS, 102(51):18712-18717). This effect is totally absent in mGlu7 knock-out mice. Those results are in accordance with previous genetic studies showing that mGlu7 is an important regulator of stress response *in vivo* (Mitsukawa et al. (2006) Neuropsychopharm., 31(6):1112-1122). In this paper, Mitsukawa *et al.* demonstrated that mGlu7 ablation causes dysregulation of the HPA axis and increases hippocampal BDNF protein levels, indicating that this receptor might be implicated in stress-related psychiatric disorders such as anxiety, depression, post-traumatic stress syndrome, behaviours induced by innate fear such as acquisition and extinction of conditioned fear or conditioned taste aversion. These data also confirmed previous observations where mGlu7-deficient mice showed marked reduction in fear-mediated freezing responses during electric foot-shocks and impairment in the ability to associate between a taste stimulus and a malaise-evoking LiCl injection (conditioned taste aversion, CTA) (Masugi et al. (1999) J. Neurosc., 19(3):955-963). These mice also demonstrated a deficit in the acquisition and extinction learning of conditioned responses compared to wild type animals (Goddyn et al. (2008) Neurobiol. Learn. Mem., 90(1): 103-111).

Contradictory effects observed with the allosteric agonist AMN082 may be explained by the rapid and long-lasting mGlu7 receptor internalization, coinciding with functional antagonism, and its scarce selectivity in vivo suggests a potential off-target involvement (Sukoff Rizzo et al. (2011) J. Pharmacol. Exp. Ther., 338(1):345-352; Pelkey et al. (2007) Neuropharmacology 52(1):108-117).

The recent discovery of several negative allosteric modulators has contributed to better understanding of functional role of mGlu7 in neural functioning. 6-(4-Methoxyphenyl)-5-methyl-3-pyridin-4-ylisoxazolo[4,5-c]pyridin-4(5H)-one (MMPIP) administered in vivo has demonstrated anxiolytic, anti-depressant like properties, as well as improved cognitive performance in rodent models (Palazzo et al. (2015) Pain, 156(6):1060-1073). 7-Hydroxy-3-(4-iodophenoxy)-4H-chromen-4-one (XAP044) was shown to produce anti-stress, anti-depressant and anxiolytic-like effects and to reduce freezing in a fear-conditioning paradigm (Gee et al. (2014) J. Biol. Chem. 289(16):10975-10987). Furthermore, (*S*)-6-(2,4-dimethylphenyl)-2-ethyl-6,7-dihydrobenzo[*d*]oxazol-4(5*H*)-one (ADX71743) demonstrated anxiolytic-like effects in the elevated plus maze and marble burying tests, as well as reducing amphetamine-induced hyperactivity without altering baseline locomotor activity (Kalinichev et al. (2013) J. Pharmacol. Exp. Ther. 344(3):624-636). Taken together, these data indicate that inhibiting mGlu7 with a modulator would be useful for the treatment of mood disorders related to anxiety, depression and PTSD.

In addition, mGlu7 receptors have also been implicated in pathways affected during pain. Given its high and wide expression both in the peripheral and central nervous systems, mGlu7 was found to play a role in regulating pain behaviour. The role of mGlu7 in pain was also recently demonstrated using AMN082 injection directly into the central nucleus of the amygdala (CeA) or in the periaqueductal gray (PAG). Under normal conditions, activation of amygdala mGlu7 facilitates pain responses, as shown by a decrease in the spinal withdrawal reflex thresholds and increased audible

and ultrasonic vocalizations evoked by brief compression of the knee (Palazzo et al. (2008) Neuropharmacol., 55(4):537-545). In a similar manner, activation of PAG mGlu7 decreased thermoceptive thresholds measured using the tail flick latency in rats (Marabese et al. (2007) J. Neurophysiol., 98:43-53). In rodent models of pain, AMN082 inhibited hyperalgesia (Dolan et al. (2009) Behav. Pharmacol. 20(7):596-604); Osikowicz et al. (2008) Pain 139(1):117-126). In addition, the mGlu7 negative allosteric modulator ADX71743 was shown to reduce visceral pain in a stress-sensitive model of visceral hypersensitivity (Moloney et al. (2015) Neurobiol. Stress 2:28-33). Altogether these data suggest that activation of mGlu7 receptors worsen pain perception and mGlu7 inhibition reduces it, therefore suggesting negative allosteric modulators of this receptor might be useful in the treatment of pain and pain-related disorders.

Genome-wide studies have also shown an association of the mGlu7 receptor with age-related hearing impairment (ARHI), also called presbycusis. This resulted in the identification of a highly significant and replicated single nucleotide polymorphism (SNP) located in GRM7, the gene coding for the mGlu7 receptor (Van Laer et al. (2010) Eur. J. Hum. Genet., 18(6):685-693; Friedman et al. (2009) Hum. Mol. Genet., 18(4):785-796; Newman et al. (2012) Hear Res. 294:125-132; Luo et al. (2013) PLoS One, 8(10):e77153; Haider et al. (2017) Front. Aging Neurosci. 9:346; Matyas et al. (2019) Pathol. Oncol. Res. 25(4):1645-52; Chang et al. (2018) J. Int. Adv. Otol. 14(2):170-175). GRM7 variants were also identified to be in association of noise-induced hearing loss, as reported by Lu et al. (BMC Med. Genet. (2018), 19(1):4) and tinnitus, as reported by Haider et al. (Front. Aging Neurosci. (2017), 9:346). Finally, mGlu7 expression, studied by immunohistochemistry, is located in the neurons of the spiral ganglion, in the inner and outer hair cells of the organ of Corti, and the hair cells of the vestibular apparatus formed by the sacculus, the utriculus and the crista ampullaris (Friedman et al. (2008) WO2008131439). These data suggest that mGlu7 receptor modulators are of potential use in the experimental treatment of otic disorders linked to the inner ear and auditory nervous system such as age-related hearing loss (presbycusis), noise-induced hearing loss, acute and chronic hearing loss, tinnitus, Meniere's disease and vestibular disorders.

Finally, on top of its wide distribution throughout the CNS, mGlu7 shows the highest degree of evolutionary conservation of all mGluRs (Flor et al. (1997) Neuropharmacol., 36:153-159), suggesting an important role for this receptor in CNS functioning. Moreover, it has a relatively low affinity for glutamate (Okamato et al. (1994) J. Biol. Chem., 269:1231-1236), thus it may remain inactive during normal transmission, only becoming active during times of excessive glutamate release (Ferraguti F. and Shigemoto R. (2006) Cell Tissue Res., 326:483-504). Taken together these data strongly highlight the potential of mGlu7 modulators in clinical indications such as neuroprotection (to treat stroke and head injury, ischemic damage and neurotoxicity).

Altogether, these pharmacological and genetic data strongly support the potential of mGlu7 modulators for the treatment of a wide range of disease and associated symptoms across psychiatric, neurological, neurodevelopmental, otic and pain disorders.

Pyrrolopyridazinones have been shown to be useful as immunosuppressants by Bantick et al. in the international publication WO9929695. B. A. Steams et al. in Bioorg. & Med. Chem. Let ((2004), 14, 1295-1298) have described 6-aryl-6H-pyrrolo[3,4-d]pyridazine derivatives showing high-affinity to the $\alpha_2\delta$ subunit of voltage gated calcium channels. However, none of the specifically disclosed compounds are structurally related to the compounds of the present invention.

SUMMARY OF THE INVENTION

The invention relates to compounds having metabotropic glutamate receptor 7 modulator activity. In its most general compound aspect, the present invention provides a compound according to Formula (I):

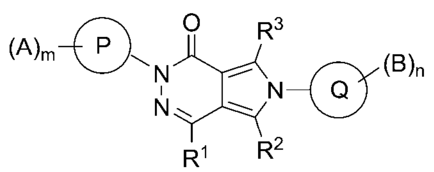
(I)

a pharmaceutically acceptable acid or base addition salt thereof, a stereochemically isomeric form thereof or an *N*-oxide form thereof, wherein:

$R^1$ is selected from the group of (for example the group consisting of) hydrogen, - $CH_3$ and $-CF_3$;

$R^2$ and $R^3$ are each independently selected from the group of (for example the group consisting of) hydrogen, $-(C_1-C_6)$alkyl, $-(C_1-C_6)$haloalkyl and $-CF_3$;

P represents a cycloalkyl, aryl or heteroaryl of formula:

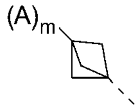
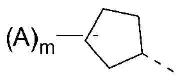
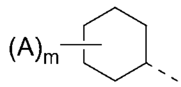

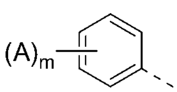
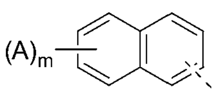
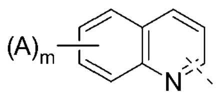
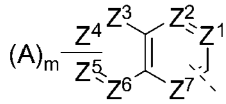

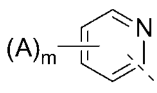
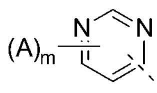
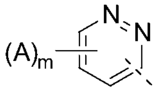
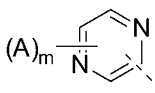
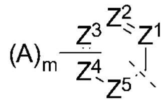

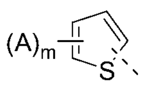
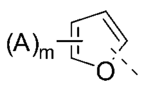
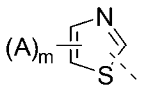
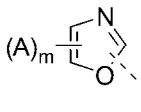
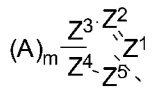

wherein each cycloalkyl, aryl or heteroaryl ring is optionally substituted with m radicals A, wherein m is an integer equal to zero, 1, 2, 3 or 4;

wherein $Z^1$, $Z^2$, $Z^3$, $Z^4$, $Z^5$, $Z^6$ and $Z^7$ are each independently selected from C, N, O or S; provided that at least one of $Z^1$, $Z^2$, $Z^3$, $Z^4$, $Z^5$, $Z^6$ and $Z^7$ is N;

the or each $(A)_m$ is independently selected from the group of (for example the group consisting of) hydrogen, halogen, -CN, -OH, $-NO_2$, $-CF_3$, -SH, $-NH_2$ and an optionally substituted radical selected from the group of (for example the group consisting of) $-(C_1-C_6)$alkyl, $-(C_1-C_6)$haloalkyl, $-(C_2-C_6)$alkynyl, $-(C_2-C_6)$alkenyl, $-(C_3-C_7)$cycloalkyl, $-(C_1-C_6)$alkylene-$(C_3-C_7)$cycloalkyl, $-(C_3-C_8)$cycloalkenyl, $-(C_1-C_6)$cyanoalkyl, $-(C_1-C_6)$alkylene-heteroaryl, $-(C_1-C_6)$alkylene-aryl, aryl, heteroaryl, $-(C_1-C_6)$alkylene-heterocycle, heterocycle, $-(C_0-C_6)$alkylene-$OR^4$, $-O-(C_2-C_6)$alkylene-$OR^4$, $-NR^4(C_2-C_6)$alkylene-$OR^5$, $-(C_3-C_6)$alkynylene-$OR^4$, $-(C_3-C_6)$alkynylene-$NR^4R^5$, $-(C_3-C_6)$alkenylene-$OR^4$, $-(C_3-C_6)$alkenylene-$NR^4R^5$, $-(C_0-C_6)$alkylene-S-$R^4$, $-O-(C_2-C_6)$alkylene-S-$R^4$, $-NR^4-(C_2-C_6)$alkylene-S-$R^5$, $-(C_0-C_6)$alkylene-S(=O)-$R^4$, $-O-(C_1-C_6)$alkylene-S(=O)-$R^4$, $-NR^4-(C_1-C_6)$alkylene-S(=O)-$R^5$, $-(C_0-C_6)$alkylene-$S(=O)_2-R^4$, - $O-(C_1-C_6)$alkylene-$S(=O)_2-R^4$, $-NR^4-(C_1-C_6)$alkylene-$S(=O)_2-R^5$, $-(C_0-C_6)$alkylene-$NR^4R^5$, $-O-(C_2-C_6)$alkylene-$NR^4R^5$, $-NR^4-(C_2-C_6)$alkylene-$NR^5R^6$, $-(C_0-C_6)$alkylene$S(=O)_2NR^4R^5$, $-O-(C_1-C_6)$alkylene-$S(=O)_2NR^4R^5$, $-NR^4-(C_1-C_6)$alkylene-$S(=O)_2NR^5R^6$, $-(C_0-C_6)$alkylene-$NR^4-S(=O)_2R^5$, $-O-(C_2-C_6)$alkylene-$NR^4-S(=O)_2R^5$, $-NR^4-(C_2-C_6)$alkylene-$NR^5-S(=O)_2R^6$, $-(C_0-C_6)$alkylene-C(=O)-$NR^4R^5$, $-O-(C_1-C_6)$alkylene-C(=O)-$NR^4R^5$, $-NR^4-(C_1-C_6)$alkylene-C(=O)-$NR^5R^6$, $-(C_0-C_6)$alkylene-$NR^4C(=O)-R^5$, - $O-(C_2-C_6)$alkylene-$NR^4C(=O)-R^5$, $-NR^4-(C_2-C_6)$alkylene-$NR^5C(=O)-R^6$, $-(C_0-C_6)$alkylene-OC(=O)-$R^4$, $-O-(C_2-C_6)$alkylene-OC(=O)-$R^4$, $-NR^4-(C_2-C_6)$alkylene-OC(=O)-$R^5$, $-(C_0-C_6)$alkylene-C(=O)-$OR^4$, $-O-(C_1-C_6)$alkylene-C(=O)-$OR^4$, $-NR^4-(C_1-C_6)$alkylene-C(=O)-$OR^5$, $-(C_0-C_6)$alkylene-C(=O)-$R^4$, $-O-(C_1-C_6)$alkylene-C(=O)-$R^4$, - $NR^4-(C_1-C_6)$alkylene-C(=O)-$R^5$, $-(C_0-C_6)$alkylene-$NR^4$-C(=O)-$OR^5$, -C(=O)-$(C_1-C_6)$alkylene-$NR^4$-C(=O)-$OR^5$, $-(C_0-C_6)$alkylene-O-C(=O)-$NR^4R^5$, $-(C_0-C_6)$alkylene-$NR^4$-C(=O)-$NR^5R^6$, $-O-(C_2-C_6)$alkylene-$NR^4$-C(=O)-$NR^5R^6$, $-NR^4-(C_2-C_6)$alkylene-$NR^5$-C(=O)-$NR^6R^7$, $-(C_0-C_6)$alkylene-$NR^4$-C(=S)-$NR^5R^6$ and $-(C_0-C_6)$alkylene-$NR^4$-C(=$NR^5$)-$NR^6R^7$

$R^4$, $R^5$, $R^6$ and $R^7$ are each independently hydrogen or an optionally substituted radical selected from the group of (for example the group consisting of) $-(C_1-C_6)$haloalkyl, $-(C_1-C_6)$alkyl, $-(C_1-C_6)$cyanoalkyl, $-(C_3-C_7)$cycloalkyl, $-(C_1-C_6)$alkylene-$(C_3-C_7)$cycloalkyl, heteroaryl, $-(C_1-C_6)$alkylene-heteroaryl, aryl, $-(C_1-C_6)$alkylene-heterocycle, heterocycle, $-(C_1-C_6)$alkylene-aryl, $-(C_0-C_6)$alkylene-O-$(C_0-C_6)$alkyl and - $(C_0-C_6)$alkylene-N-$((C_0-C_6)alkyl)_2$;

Q is aryl or heteroaryl which is optionally substituted with n radicals B, wherein n is an integer equal to zero, 1, 2, 3, 4 or 5;

the or each $(B)_n$ is independently selected from the group of (for example the group consisting of) hydrogen, halogen, -CN, -OH, $-NO_2$, $-CF_3$, -SH, $-NH_2$ and an optionally substituted radical selected from the group of (for example the group consisting of) $-(C_1-C_6)$alkyl, $-(C_1-C_6)$haloalkyl, $-(C_2-C_6)$alkynyl, $-(C_2-C_6)$alkenyl, $-(C_3-C_7)$cycloalkyl, $-(C_1-C_6)$alkylene-$(C_3-C_7)$cycloalkyl, $-(C_3-C_8)$cycloalkenyl, $-(C_1-C_6)$cyanoalkyl, $-(C_1-C_6)$alkylene-heteroaryl, $-(C_1-C_6)$alkylene-aryl, aryl, heteroaryl, $-(C_1-C_6)$alkylene-heterocycle, heterocycle, $-(C_0-C_6)$alkylene-$OR^8$, $-O-(C_2-C_6)$alkylene-$OR^8$, $-NR^8(C_2-C_6)$alkylene-$OR^9$, $-(C_3-C_6)$alkynylene-$OR^8$, $-(C_3-C_6)$alkynylene-$NR^8R^9$, $-(C_3-C_6)$alkenylene-$OR^8$,

$-(C_3-C_6)$alkenylene-$NR^8R^9$, $-(C_0-C_6)$alkylene-S-$R^8$, -O-$(C_2-C_6)$alkylene-S-$R^8$, -$NR^8$-$(C_2-C_6)$alkylen$_e$-S-$R^9$, $-(C_0-C_6)$alkylene-S(=O)-$R^8$, -O-$(C_1-C_6)$alkylene-S(=O)-$R^8$, -$NR^8$-$(C_1-C_6)$alkylene-S(=O)-$R^9$, $-(C_0-C_6)$alkylene-$S(=O)_2$-$R^8$, -O-$(C_1-C_6)$alkylene-$S(=O)_2$-$R^8$, -$NR^8$-$(C_1-C_6)$alkylene-$S(=O)_2$-$R^9$, $-(C_0-C_6)$alkylene-$NR^8R^9$, -O-$(C_2-C_6)$alkylene-$NR^8R^9$, -$NR^8$-$(C_2-C_6)$alkylene-$NR^9R^{10}$, $-(C_0-C_6)$alkylene$S(=O)_2NR^8R^9$, -O-$(C_1-C_6)$alkylene-$S(=O)_2NR^8R^9$, -$NR^8$-$(C_1-C_6)$alkylene$S(=O)_2NR^9R^{10}$, $-(C_0-C_6)$alkylene-$NR^8$-$S(=O)_2R^9$, -O-$(C_2-C_6)$alkylene-$NR^8$-$S(=O)_2R^9$, -$NR^8$-$(C_2-C_6)$alkylene-$NR^9$-$S(=O)_2R^{10}$, $-(C_0-C_6)$alkylene-C(=O)-$NR^8R^9$, -O-$(C_1-C_6)$alkylene-C(=O)-$NR^8R^9$, -$NR^8$-$(C_1-C_6)$alkylene-C(=O)-$NR^9R^{10}$, $-(C_0-C_6)$alkylene-$NR^8$C(=O)-$R^9$, -O-$(C_2-C_6)$alkylene-$NR^8$C(=O)-$R^9$, -$NR^8$-$(C_2-C_6)$alkylene-$NR^9$C(=O)-$R^{10}$, $-(C_0-C_6)$alkylene-OC(=O)-$R^8$, -O-$(C_2-C_6)$alkylene-OC(=O)-$R^8$, -$NR^8$-$(C_2-C_6)$alkylene-OC(=O)-$R^9$, $-(C_0-C_6)$alkylene-C(=O)-$OR^8$, -O-$(C_1-C_6)$alkylene-C(=O)-$OR^8$, -$NR^8$-$(C_1-C_6)$alkylene-C(=O)-$OR^9$, $-(C_0-C_6)$alkylene-C(=O)-$R^8$, -O-$(C_1-C_6)$alkylene-C(=O)-$R^8$, -$NR^8$-$(C_1-C_6)$alkylene-C(=O)-$R^9$, $-(C_0-C_6)$alkylene-$NR^8$-C(=O)-$OR^9$, $-(C_0-C_6)$alkylene-O-C(=O)-$NR^8R^9$, $-(C_0-C_6)$alkylene-$NR^8$-C(=O)-$NR^9R^{10}$, -O-$(C_2-C_6)$alkylene-$NR^8$-C(=O)-$NR^9R^{10}$, -$NR^8$-$(C_2-C_6)$alkylene-$NR^9$-C(=O)-$NR^{10}R^{11}$, $-(C_0-C_6)$alkylene-$NR^8$-C(=S)-$NR^9R^{10}$ and $-(C_0-C_6)$alkylene-$NR^8$-C(=$NR^9$)-$NR^{10}R^{11}$;

$R^8$, $R^9$, $R^{10}$ and $R^{11}$ are each independently hydrogen or an optionally substituted radical selected from the group of (for example the group consisting of) $-(C_1-C_6)$haloalkyl, $-(C_1-C_6)$alkyl, $-(C_1-C_6)$cyanoalkyl, $-(C_3-C_7)$cycloalkyl, $-(C_1-C_6)$alkylene-$(C_3-C_7)$cycloalkyl, heteroaryl, $-(C_1-C_6)$alkylene-heteroaryl, aryl, $-(C_1-C_6)$alkylene-heterocycle, heterocycle, $-(C_1-C_6)$alkylene-aryl, $-(C_0-C_6)$alkylene-O-$(C_0-C_6)$alkyl and - $(C_0-C_6)$alkylene-N-$((C_0-C_6)alkyl)_2$; whererin optionally any two radicals A are combined with the intervening atoms to form a 3 to 10 membered bicyclic heterocycle, aryl or heteroaryl ring, wherein each ring is optionally further substituted with 1 to 5 radicals independently selected from the group of (for example the group consisting of) halogen, -CN, nitro, $-(C_1-C_6)$alkyl, $-(C_0-C_6)$alkylene-O-$(C_0-C_6)$alkyl and $-(C_0-C_6)$alkylene-N-$((C_0-C_6)alkyl)_2$;

wherein optionally two of the substituents $R^4$, $R^5$, $R^6$ or $R^7$ are combined with the intervening atoms to form a 3 to 10 membered heterocycle, aryl or heteroaryl ring, wherein each ring is optionally further substituted with 1 to 5 radicals independently selected from the group of (for example the group consisting of) halogen, cyano, nitro, - $(C_1-C_6)$alkyl, $-(C_0-C_6)$alkylene-O-$(C_0-C_6)$alkyl and $-(C_0-C_6)$alkylene-N-$((C_0-C_6)alkyl)_2$;

wherein optionally two substituents from $R^8$, $R^9$, $R^{10}$ or $R^{11}$ are combined with the intervening atoms to form a 3 to 10 membered heterocycle, aryl or heteroaryl ring, wherein each ring is optionally further substituted with 1 to 5 radicals independently selected from the group of (for example the group consisting of) halogen, cyano, nitro, - $(C_1-C_6)$alkyl, $-(C_0-C_6)$alkylene-O-$(C_0-C_6)$alkyl and $-(C_0-C_6)$alkylene-N-$((Co-C_6)alkyl)_2$;

wherein optionally any two radicals B are combined with the intervening atoms to form a 3 to 10 membered bicyclic heterocycle, aryl or heteroaryl ring, wherein each ring is optionally further substituted with 1 to 5 radicals independently selected from the group of (for example the group consisting of) halogen, -CN, nitro, $-(C_1-C_6)$alkyl, $-(C_0-C_6)$alkylene-O-$(C_0-C_6)$alkyl and $-(C_0-C_6)$alkylene-N-$((C_0-C_6)alkyl)_2$.

It has now, surprisingly, been found that the compounds of general Formula (I) show potent activity and selectivity on the mGlu7 receptor. The compounds of the invention demonstrate advantageous properties over compounds of the prior art. Improvements have been observed in one or more of the following characteristics of the compounds of the invention: the potency on the target, the selectivity for the target, the bioavailability, the brain penetration, and the pharmacodynamics.

Preferably,

Q represents an aryl or heteroaryl group of formula:

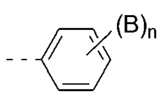
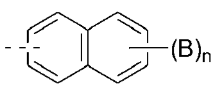
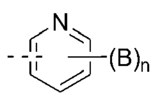
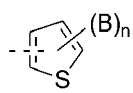
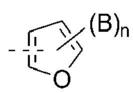
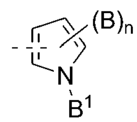

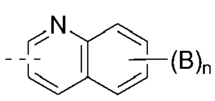
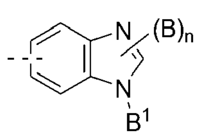
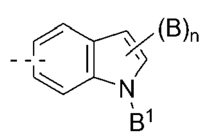
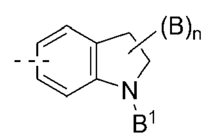
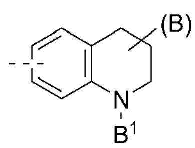

wherein each radical is optionally substituted with n radicals B, wherein n is an integer equal to zero, 1, 2, 3, 4 or 5. $B^1$ may be a radical B as described above. For example, $B^1$ may be hydrogen, -($C_1$-$C_6$)alkyl or -($C_3$-$C_7$)cycloalkyl.

For example, Q may represent an aryl or heteroaryl group of formula:

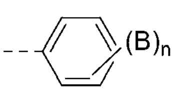
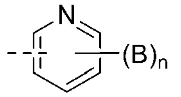
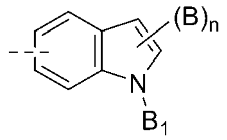
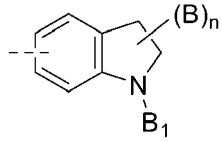
,

wherein each radical is optionally substituted with n radicals B, wherein n is an integer equal to zero, 1, 2, 3, 4 or 5. $B^1$ may be a radical B as described above. For example, $B^1$ may be hydrogen, -($C_1$-$C_6$)alkyl or -($C_3$-$C_7$)cycloalkyl.

For example, P may represent a cycloalkyl, aryl or heteroaryl of formula:

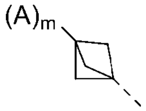
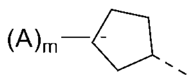
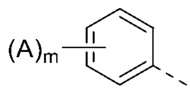

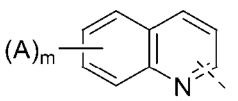
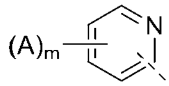
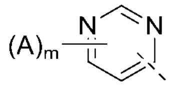
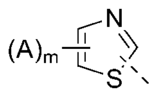

wherein each radical is optionally substituted with m radicals A, wherein m is an integer equal to zero, 1, 2, 3 or 4.

For example, P may be

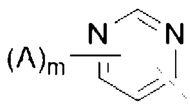

optionally substituted with m radicals A, wherein m is an integer equal to zero, 1, 2, 3, 4 or 5; and Q may be phenyl optionally substituted with n radicals B, wherein n is an integer equal to zero, 1, 2, 3, 4 or 5.

The cycloalkyl, heterocycle, aryl and heteroaryl ring systems of $(A)_m$ may be selected from the group of (for example the group consisting of) azetidinyl, benzimidazolyl, benzisothiazolyl benzisoxazolyl, benzofuryl, benzopyrazolyl, benzothiazolyl, benzothiophenyl, benzotriazolyl, benzoxazolyl, dihydrofuranyl, dihydrothienyl, dioxolanyl, 1,1-dioxothiomorpholinyl, furazanyl, furyl, imidazolidinyl, imidazolinyl, imidazolonyl, imidazolyl, imidazopyridazinyl, imidazopyridyl, indolyl, isoindolyl, isoquinolinyl, isothiazolinyl, isothiazolyl, isoxazolidinyl, isoxazolinyl, isoxazolyl, morpholinyl, naphthyl, naphthyridinyl, oxadiazolyl, oxazolidinyl, oxazolinyl, oxazolonyl, oxazolopyridazinyl, oxazolopyridyl, oxazolyl, oxetanyl, phenyl, piperazinonyl, piperazinyl, piperidinonyl, piperidinyl, phtalazinyl, pteridinyl, purinyl, pyranyl, pyrazinyl, pyrazolopyridinyl, pyrazolyl, pyridazinyl, pyridonyl, pyridyl, pyrimidyl, pyrrolidinonyl, pyrrolidinyl, pyrrolinyl, pyrrolyl, quinazolyl, quinolyl, quinoxalinyl, tetrahydrofuranyl, tetrahydropyranyl, tetrahydrothiopyranyl, tetrahydrotriazolopyridyl, tetrahydrotriazolopyrimidinyl, tetrazolyl, thiadiazolyl, thiazolidinyl, thiazolinyl, thiazolonyl, thiazolopyridazinyl, thiazolopyridyl, thiazolyl, thienyl, thiomorpholinyl, thionaphthyl, thiopyranyl, triazolinyl, triazinyl, triazolyl, cyclopropyl, cyclobutyl, cyclopentyl, cyclopentenyl, cyclohexyl, cyclohexenyl, cycloheptyl, cycloheptenyl, cyclooctyl and cyclooctenyl and each ring of said ring system is optionally substituted independently with 1 to 4 substituents $R^4$, $R^5$, $R^6$ or $R^7$.

The cycloalkyl, heterocycle, aryl and heteroaryl ring systems of $(B)_n$ may be selected from the group of (for example the group consisting of) azetidinyl, benzimidazolyl, benzisothiazolyl benzisoxazolyl, benzofuryl, benzopyrazolyl, benzothiazolyl, benzothiophenyl, benzotriazolyl, benzoxazolyl, dihydrofuranyl, dihydrothienyl, dioxolanyl, 1,1-dioxothiomorpholinyl, furazanyl, furyl, imidazolidinyl, imidazolinyl, imidazolonyl, imidazolyl, imidazopyridazinyl, imidazopyridyl, indolyl, isoindolyl, isoquinolinyl, isothiazolinyl, isothiazolyl, isoxazolidinyl, isoxazolinyl, isoxazolyl, morpholinyl, naphthyl, naphthyridinyl, oxadiazolyl, oxazolidinyl, oxazolinyl, oxazolonyl, oxazolopyridazinyl, oxazolopyridyl, oxazolyl, oxetanyl, phenyl, piperazinonyl, piperazinyl, piperidinonyl, piperidinyl, phtalazinyl, pteridinyl, purinyl, pyranyl, pyrazinyl, pyrazolopyridinyl, pyrazolyl, pyridazinyl, pyridonyl, pyridyl, pyrimidyl, pyrrolidinonyl, pyrrolidinyl, pyrrolinyl, pyrrolyl, quinazolyl, quinolyl, quinoxalinyl, tetrahydrofuranyl, tetrahydropyranyl, tetrahydrothiopyranyl, tetrahydrotriazolopyridyl, tetrahydrotriazolopyrimidinyl, tetrazolyl, thiadiazolyl, thiazolidinyl, thiazolinyl, thiazolonyl, thiazolopyridazinyl, thiazolopyridyl, thiazolyl, thienyl, thiomorpholinyl, thionaphthyl, thiopyranyl, triazolinyl, triazinyl, triazolyl, cyclopropyl, cyclobutyl, cyclopentyl, cyclopentenyl, cyclohexyl, cyclohexenyl, cycloheptyl, cycloheptenyl, cyclooctyl and cyclooctenyl and each ring of said ring

system is optionally substituted independently with 1 to 4 substituents $R^8$, $R^9$, $R^{10}$ or $R^{11}$.

The cycloalkyl, heterocycle, aryl and heteroaryl ring systems of $R^4$, $R^5$, $R^6$, $R^7$, $R^8$, $R^9$, $R^{10}$ or $R^{11}$ may be selected from the group of (for example the group consisting of) azetidinyl, benzimidazolyl, benzisothiazolyl benzisoxazolyl, benzofuryl, benzopyrazolyl, benzothiazolyl, benzothiophenyl, benzotriazolyl, benzoxazolyl, dihydrofuranyl, dihydrothienyl, dioxolanyl, 1,1-dioxo-thiomorpholinyl, furazanyl, furyl, imidazolidinyl, imidazolinyl, imidazolonyl, imidazolyl, imidazopyridazinyl, imidazopyridyl, indolyl, isoindolyl, isoquinolinyl, isothiazolinyl, isothiazolyl, isoxazolidinyl, isoxazolinyl, isoxazolyl, morpholinyl, naphthyl, naphthyridinyl, oxadiazolyl, oxazolidinyl, oxazolinyl, oxazolonyl, oxazolopyridazinyl, oxazolopyridyl, oxazolyl, oxetanyl, phenyl, piperazinonyl, piperazinyl, piperidinonyl, piperidinyl, phtalazinyl, pteridinyl, purinyl, pyranyl, pyrazinyl, pyrazolopyridinyl, pyrazolyl, pyridazinyl, pyridonyl, pyridyl, pyrimidyl, pyrrolidinonyl, pyrrolidinyl, pyrrolinyl, pyrrolyl, quinazolyl, quinolyl, quinoxalinyl, tetrahydrofuranyl, tetrahydropyranyl, tetrahydrothiopyranyl, tetrahydrotriazolopyridyl, tetrahydrotriazolopyrimidinyl, tetrazolyl, thiadiazolyl, thiazolidinyl, thiazolinyl, thiazolonyl, thiazolopyridazinyl, thiazolopyridyl, thiazolyl, thienyl, thiomorpholinyl, thionaphthyl, thiopyranyl, triazolinyl, triazinyl, triazolyl, cyclopropyl, cyclobutyl, cyclopentyl, cyclopentenyl, cyclohexyl, cyclohexenyl, cycloheptyl, cycloheptenyl, cyclooctyl and cyclooctenyl and each ring of said ring system is optionally substituted with 1-5 radicals independently selected from hydrogen, halogen, -CN, nitro, $-(C_1-C_6)$alkyl, $-(C_0-C_6)$alkylene-O-$(C_0-C_6)$alkyl and $-(C_0-C_6)$alkylene-N-$((C_0-C_6)$alkyl$)_2$.

For example, $R^2$ and $R^3$ may each be independently selected from the group of (for example the group consisting of) hydrogen, methyl and ethyl, for example methyl. For example, $R^2$ and $R^3$ may both be methyl.

The or each $(A)_m$ may be independently selected from the group of (for example the group consisting of) hydrogen, halogen, -CN, -OH, $-CF_3$ and an optionally substituted radical selected from the group of (for example the group consisting of) $-(C_1-C_6)$alkyl, - $(C_1-C_6)$haloalkyl, $-(C_3-C_7)$cycloalkyl, $-(C_1-C_6)$cyanoalkyl, aryl, heterocycle, $-(C_0-C_6)$alkylene-$OR^4$, -O-$(C_2-C_6)$alkylene-$OR^4$, $-(C_0-C_6)$alkylene-$S(=O)_2-R^4$, $-(C_0-C_6)$alkylene-$NR^4R^5$, $-(C_0-C_6)$alkylene-$S(=O)_2NR^4R^5$, $-(C_0-C_6)$alkylene-C(=O)-$NR^4R^5$, $-(C_0-C_6)$alkylene-$NR^4C(=O)-R^5$, $-(C_0-C_6)$alkylene-C(=O)-$OR^4$, $-(C_0-C_6)$alkylene-C(=O)-$R^4$ and -C(=O)-$(C_1-C_6)$alkylene-$NR^4$-C(=O)-$OR^5$. The optionally substituted radical may be optionally substituted with halogen, $-(C_1-C_6)$alkyl, OH, or CN.

$R^4$ and $R^5$ may each independently be hydrogen or $-(C_1-C_6)$alkyl.

The or each $(B)_n$ may be independently selected from the group of (for example the group consisting of) hydrogen, halogen, -CN, $-CF_3$, and an optionally substituted radical selected from the group of (for example the group consisting of) $-(C_1-C_6)$alkyl, $-(C_3-C_7)$cycloalkyl, aryl, heteroaryl, heterocycle, $-(C_0-C_6)$alkylene-$OR^8$, $-NR^8(C_2-C_6)$alkylene-$OR^9$, $-(C_0-C_6)$alkylene-$NR^8R^9$, $-(C_0-C_6)$alkylene-C(=O)-$OR^8$ and $-(C_0-C_6)$alkylene-C(=O)-$R^8$. The optionally substituted radical may be optionally substituted with halogen or $-(C_1-C_6)$alkyl. Optionally two $-(C_0-C_6)$alkylene-$OR^8$ radicals may be combined with the intervening atoms to form a 9 membered bicyclic heterocycle ring, for example a cyclic diethyl.

$R^8$ and $R^9$ may each be independently selected from the group of (for example the group consisting of) hydrogen, $-(C_1-C_6)$haloalkyl, $-(C_1-C_6)$alkyl, $-(C_3-C_7)$cycloalkyl and aryl.

For example, $R^2$ and $R^3$ may each be independently selected from the group of (for example the group consisting of) methyl and ethyl. For example, $R^2$ and $R^3$ may both be methyl.

The or each $(A)_m$ may be independently selected from the group of (for example the group consisting of) hydrogen, halogen, -CN, -OH, $-CF_3$ and an optionally substituted radical selected from the group of (for example the group consisting of) $-(C_1-C_6)$alkyl, - $(C_1-C_6)$haloalkyl, $-(C_3-C_7)$cycloalkyl, $-(C_1-C_6)$cyanoalkyl, aryl, heterocycle, $-(C_0-C_6)$alkylene-$OR^4$, -O-$(C_2-C_6)$alkylene-$OR^4$, $-(C_0-C_6)$alkylene-$S(=O)_2-R^4$, $-(C_0-C_6)$alkylene-$NR^4R^5$, $-(C_0-C_6)$alkylene-$S(=O)_2NR^4R^5$, $-(C_0-C_6)$alkylene-C(=O)-$NR^4R^5$, - $(C_0-C_6)$alkylene-$NR^4C(=O)-R^5$, $-(C_0-C_6)$alkylene-C(=O)-$OR^4$, $-(C_0-C_6)$alkylene-C(=O)-$R^4$ and -C(=O)-$(C_1-C_6)$alkylene-$NR^4$-C(=O)-$OR^5$.

$R^4$ and $R^5$ may each independently be hydrogen or $-(C_1-C_6)$alkyl.

The or each $(B)_n$ may be independently selected from the group of (for example the group consisting of) hydrogen, halogen and an optionally substituted radical selected from the group of (for example the group consisting of) $-(C_1-C_6)$alkyl, heterocycle, $-(C_0-C_6)$alkylene-$OR^8$, $-NR^8(C_2-C_6)$alkylene-$OR^9$, $-(C_0-C_6)$alkylene-$NR^8R^9$, $-(C_0-C_6)$alkylene-C(=O)-$OR^8$ and $-(C_0-C_6)$alkylene-C(=O)-$R^8$.

$R^8$ and $R^9$ may each independently be hydrogen or $-(C_1-C_6)$alkyl.

For example, $R^2$ and $R^3$ may each be independently selected from the group of (for example the group consisting of) hydrogen, methyl and ethyl. For example, $R^2$ and $R^3$ may both be methyl.

The or each $(A)_m$ may be independently selected from the group of (for example the group consisting of) hydrogen, halogen, -CN, -OH, $-CF_3$ and an optionally substituted radical selected from the group of (for example the group consisting of) $-(C_1-C_6)$alkyl, - $(C_3-C_7)$cycloalkyl, heterocycle and $-(C_0-C_6)$alkylene-$OR^4$.

$R^4$ may be selected from hydrogen or $-(C_1-C_6)$alkyl.

The or each $(B)_n$ may be independently selected from the group of (for example the group consisting of) hydrogen, halogen, -CN, $-CF_3$ and an optionally substituted radical selected from the group of (for example the group consisting of) $-(C_1-C_6)$alkyl, $-(C_3-C_7)$cycloalkyl, aryl, heteroaryl, heterocycle, $-(C_0-C_6)$alkylene-$OR^8$, $-(C_0-C_6)$alkylene-$NR^8R^9$, $-(C_0-C_6)$alkylene-C(=O)-$OR^8$ and $-(C_0-C_6)$alkylene-C(=O)-$R^8$.

$R^8$ and $R^9$ may each independently selected from the group of (for example the group consisting of) hydrogen,

-($C_1$-$C_6$)haloalkyl, -($C_1$-$C_6$)alkyl, -($C_3$-$C_7$)cycloalkyl and aryl, for example the -($C_1$-$C_6$)haloalkyl may be -$CF_3$. For example, the ($C_3$-$C_7$)cycloalkyl may be a -($C_3$)cycloalkyl.

For example, A may be hydrogen, halogen or -($C_1$-$C_6$)alkyl-OH and m may be 1. For example, B may be -($C_1$-$C_6$) alkyl, -($C_3$-$C_7$)cycloalkyl, heterocycle, -O-($C_3$-$C_7$)cycloalkyl, or -O-($C_1$-$C_6$)alkyl and n may be 1 or 2. For example, the heterocycle may be

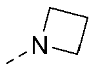.

Preferably, the compounds of Formula (I) are the compounds according to Formula (II):

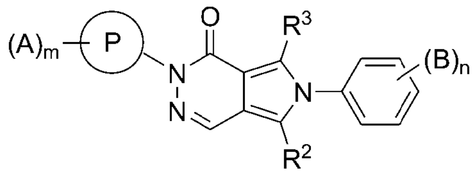

(II)

a pharmaceutically acceptable acid or base addition salt thereof, a stereochemically isomeric form thereof or an N-oxide form thereof, wherein $(A)_m$, P, $R^2$, $R^3$ and $(B)_n$ are as defined in any statement set out above.

The compounds of Formula (I) may be the compounds according to Formula (IIa)

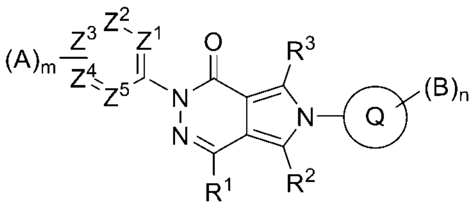

(IIa)

a pharmaceutically acceptable acid or base addition salt thereof, a stereochemically isomeric form thereof or an N-oxide form thereof wherein:

$Z^1$, $Z^2$, $Z^3$, $Z^4$ and $Z^5$ are each independently selected from C or N, and $(A)_m$, $R^1$, $R^2$, $R^3$, Q and $(B)_n$ are as defined in any statement set out above.

Preferably, the compounds of Formula (II) are according to Formula (III):

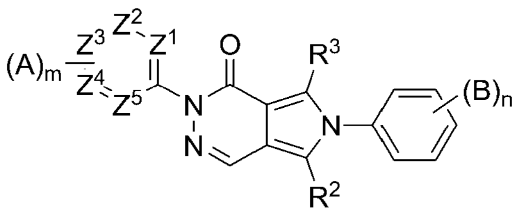

(III)

a pharmaceutically acceptable acid or base addition salt thereof, a stereochemically isomeric form thereof or an *N*-oxide form thereof wherein:

$Z^1$, $Z^2$, $Z^3$, $Z^4$ and $Z^5$ are each independently selected from C or N, and $(A)_m$, $R^2$, $R^3$ and $(B)_n$ are as defined in any statement set out above.

Preferably, the compounds of Formula (III) are the compounds according to Formula (IV):

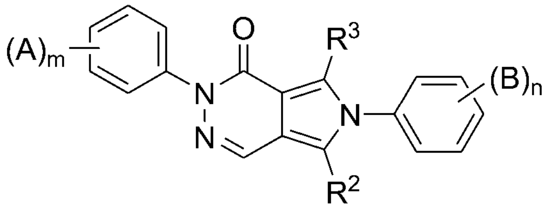

(IV)

a pharmaceutically acceptable acid or base addition salt thereof, a stereochemically isomeric form thereof or an *N*-oxide form thereof wherein $(A)_m$, $R^2$, $R^3$ and $(B)_n$ are as defined in any statement set out above.

Preferably, $R^2$ and $R^3$ are independently selected from the group of (for example the group consisting of) methyl and ethyl;

The or each $(A)_m$ is independently selected from the group of (for example the group consisting of) hydrogen, halogen, -CN, -OH, $-CF_3$ and an optionally substituted radical selected from the group of (for example the group consisting of) $-(C_1-C_6)$alkyl, $-(C_1-C_6)$haloalkyl, $-(C_3-C_7)$cycloalkyl, $-(C_1-C_6)$cyanoalkyl, aryl, heterocycle, $-(C_0-C_6)$alkylene-$OR^4$, $-O-(C_2-C_6)$alkylene-$OR^4$, $-(C_0-C_6)$alkylene-$S(=O)_2-R^4$, $-(C_0-C_6)$alkylene-$NR^4R^5$, $-(C_0-C_6)$alkylene-$S(=O)_2NR^4R^5$, $-(C_0-C_6)$alkylene-$C(=O)-NR^4R^5$, - $(C_0-C_6)$alkylene-$NR^4C(=O)-R^5$, $-(C_0-C_6)$alkylene-$C(=O)-OR^4$, $-(C_0-C_6)$alkylene-$C(=O)-R^4$ and $-C(=O)-(C_1-C_6)$alkylene-$NR^4-C(=O)-OR^5$;

$R^4$ and $R^5$ are each independently hydrogen or $-(C_1-C_6)$alkyl;

The or each $(B)_n$ is independently selected from the group of (for example the group consisting of) hydrogen, halogen and an optionally substituted radical selected from the group of (for example the group consisting of) $-(C_1-C_6)$alkyl, heterocycle, $-(C_0-C_6)$alkylene-$OR^8$, $-NR^8(C_2-C_6)$alkylene-$OR^9$, $-(C_0-C_6)$alkylene-$NR^8R^9$, $-(C_0-C_6)$alkylene-$C(=O)-OR^8$ and $-(C_0-C_6)$alkylene-$C(=O)-R^8$; and

$R^8$ and $R^9$ are each independently hydrogen or $-(C_1-C_6)$alkyl.

Preferably, one or both of $R^2$ and $R^3$ can be methyl.

Preferably, the compounds of Formula (III) are the compounds of Formula (V):

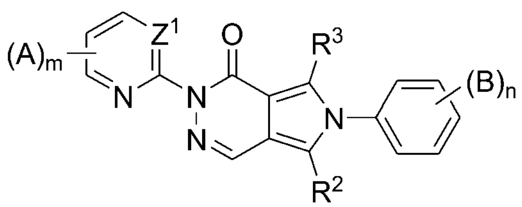

(V)

a pharmaceutically acceptable acid or base addition salt thereof, a stereochemically isomeric form thereof or an N-oxide form thereof wherein:

$Z^1$ is selected from C or N;

$R^2$ and $R^3$ are independently selected from the group of (for example the group consisting of) hydrogen, methyl and ethyl;

The or each $(A)_m$ is independently selected from the group of (for example the group consisting of) hydrogen, halogen, -CN, -OH, $-CF_3$ and an optionally substituted radical selected from the group of (for example the group consisting of) $-(C_1-C_6)$alkyl, $-(C_3-C_7)$cycloalkyl, heterocycle and $-(C_0-C_6)$alkylene-$OR^4$;

$R^4$ is selected from hydrogen or $-(C_1-C_6)$alkyl;

The or each $(B)_n$ is independently selected from the group of (for example the group consisting of) hydrogen, halogen, -CN, $-CF_3$ and an optionally substituted radical selected from the group of (for example the group consisting of) $-(C_1-C_6)$alkyl, $-(C_3-C_7)$cycloalkyl, aryl, heteroaryl, heterocycle, $-(C_0-C_6)$alkylene-$OR^8$, $-(C_0-C_6)$alkylene-$NR^8R^9$, $-(C_0-C_6)$alkylene-$C(=O)-OR^8$ and $-(C_0-C_6)$alkylene-$C(=O)-R^8$; and

$R^8$ and $R^9$ are each independently selected from the group of (for example the group consisting of) hydrogen, $-(C_1-C_6)$haloalkyl, $-(C_1-C_6)$alkyl, $-(C_3-C_7)$cycloalkyl and aryl.

Preferably, in the compounds defined by Formula (V), one or both of $R^2$ and $R^3$ can be methyl.

For example, A may be hydrogen, halogen or $-(C_1-C_6)$alkyl-OH and m may be 1. For example B may be $-(C_1-C_6)$alkyl, $-(C_3-C_7)$cycloalkyl, heterocycle, $-O-(C_3-C_7)$cycloalkyl, or $-O-(C_1-C_6)$alkyl and n may be 1 or 2. For example, the heterocycle may be

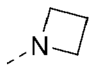 .

Particular preferred compounds of the invention are compounds as mentioned in the following list, as well as a pharmaceutically acceptable acid or base addition salt thereof, a stereochemically isomeric form thereof or an *N*-oxide form thereof:

5,7-Dimethyl-2-phenyl-6-(*p*-tolyl)-2,6-dihydro-1*H*-pyrrolo[3,4-*d*]pyridazin-1-one
2-(2-Methoxyphenyl)-5,7-dimethyl-6-(*p*-tolyl)-2,6-dihydro-1*H*-pyrrolo[3,4-*d*] pyridazin-1-one
6-(4-Chlorophenyl)-5,7-dimethyl-2-(pyridin-2-yl)-2,6-dihydro-1*H*-pyrrolo[3,4-*d*] pyridazin-1-one
5,7-Dimethyl-2,6-diphenyl-2,6-dihydro-1*H*-pyrrolo[3,4-*d*]pyridazin-1-one
2-(2-Methoxyphenyl)-5,7-dimethyl-6-phenyl-2,6-dihydro-1*H*-pyrrolo[3,4-*d*]pyridazin-1-one
5,7-Dimethyl-2-phenyl-6-(m-tolyl)-2,6-dihydro-1*H*-pyrrolo[3,4-*d*]pyridazin-1-one
5,7-Dimethyl-2-phenyl-6-(*o*-tolyl)-2,6-dihydro-1*H*-pyrrolo[3,4-*d*]pyridazin-1-one
6-(4-Chlorophenyl)-5,7-dimethyl-2-phenyl-2,6-dihydro-1*H*-pyrrolo[3,4-*d*]pyridazin-1-one
5,7-Dimethyl-6-phenyl-2-(pyridin-3-yl)-2,6-dihydro-1*H*-pyrrolo[3,4-*d*]pyridazin-1-one
2-(3-Fluorophenyl)-5,7-dimethyl-6-phenyl-2,6-dihydro-1*H*-pyrrolo[3,4-*d*]pyridazin-1-one
2-(4-Fluorophenyl)-5,7-dimethyl-6-phenyl-2,6-dihydro-1*H*-pyrrolo[3,4-*d*]pyridazin-1-one
2-(3-Methoxyphenyl)-5,7-dimethyl-6-phenyl-2,6-dihydro-1H-pyrrolo[3,4-*d*]pyridazin-1-one
2-(4-Methoxyphenyl)-5,7-dimethyl-6-phenyl-6-2,6-dihydro-1*H*-pyrrolo[3,4-*d*]pyridazin-1-one
5,7-Dimethyl-6-phenyl-2-(*m*-tolyl)-2,6-dihydro-1*H*-pyrrolo[3,4-*d*]pyridazin-1-one
5,7-Dimethyl-6-phenyl-2-(*p*-toly1)-2,6-dihydro-1*H*-pyrrolo[3,4-*d*]pyridazin-1-one
3-(5,7-Dimethyl-1-oxo-6-phenyl-1*H*-pyrrolo[3,4-*d*]pyridazin-2(6*H*)-yl)benzonitrile
4-(5,7-Dimethyl-1-oxo-6-phenyl-1*H*-pyrrolo[3,4-*d*]pyridazin-2(6*H*)-yl)benzonitrile
6-(2-Methoxyphenyl)-5,7-dimethyl-2-phenyl-2,6-dihydro-1*H*-pyrrolo[3,4-*d*]pyridazin-1-one
6-(2-Chlorophenyl)-5,7-dimethyl-2-phenyl-2,6-dihydro-1*H*-pyrrolo[3,4-*d*]pyridazin-1-one
2-(2-Chlorophenyl)-5,7-dimethyl-6-phenyl-2,6-dihydro-1*H*-pyrrolo[3,4-*d*]pyridazin-1-one
2-(2-Fluorophenyl)-5,7-dimethyl-6-phenyl-2,6-dihydro-1*H*-pyrrolo[3,4-*d*]pyridazin-1-one
5,7-Dimethyl-6-phenyl-2-(pyridin-2-yl)-2,6-dihydro-1*H*-pyrrolo[3,4-*d*]pyridazin-1-one
6-(4-(Dimethylamino)phenyl)-5,7-dimethyl-2-phenyl-2,6-dihydro-1*H*-pyrrolo[3,4-*d*] pyridazin-1-one
6-(4-Methoxyphenyl)-5,7-dimethyl-2-phenyl-2,6-dihydro-1*H*-pyrrolo[3,4-*d*]pyridazin-1-one
5,7-Dimethyl-2-phenyl-6-(pyridin-2-yl)-2,6-dihydro-1*H*-pyrrolo[3,4-*d*]pyridazin-1-one
6-(3-Methoxyphenyl)-5,7-dimethyl-2-phenyl-2,6-dlhydro-1*H*-pyrrolo[3,4-*d*]pyridazin-1-one
2-(4-Acetylphenyl)-5,7-dimethyl-6-phenyl-2,6-dihydro-1*H*-pyrrolo[3,4-*d*]pyridazin-1-one
4-(5,7-Dimethyl-1-oxo-6-phenyl-1*H*-pyrrolo[3,4-*d*]pyridazin-2(6*H*)-yl)benzene sulfonamide
6-(2-Fluorophenyl)-5,7-dimethyl-2-phenyl-2,6-dihydro-1*H*-pyrrolo[3,4-*d*]pyridazin-1-one
5,7-Dimethyl-2-(4-(methylsulfonyl)phenyl)-6-phenyl-2,6-dihydro-1*H*-pyrrolo[3,4-*d*] pyridazin-1-one
6-(4-Acetylphenyl)-5,7-dimethyl-2-phenyl-2,6-dihydro-1*H*-pyrrolo[3,4-*d*]pyridazin-1-one
6-(2,4-Dimethylphenyl)-5,7-dimethyl-2-phenyl-2,6-dihydro-1*H*-pyrrolo[3,4-*d*] pyridazin-1-one
5,7-Dimethyl-6-(3-morpholinophenyl)-2-phenyl-2,6-dihydro-1*H*-pyrrolo[3,4-*d*] pyridazin-1-one
6-(3-Bromophenyl)-5,7-dimethyl-2-phenyl-2,6-dihydro-1*H*-pyrrolo[3,4-*d*]pyridazin-1-one
6-(3-(Dimethylamino)phenyl)-5,7-dimethyl-2-phenyl-2,6-dihydro-1*H*-pyrrolo[3,4-*d*] pyridazin-1-one
6-(3-Acetylphenyl)-5,7-dimethyl-2-phenyl-2,6-dihydro-1*H*-pyrrolo[3,4-*d*]pyridazin-1-one
5,7-Dimethyl-6-phenyl-2-(pyridin-4-yl)-2,6-dihydro-1*H*-pyrrolo[3,4-*d*]pyridazin-1-one
2-(4-(Methoxymethyl)phenyl)-5,7-dimethyl-6-phenyl-2,6-dihydro-1*H*-pyrrolo[3,4-*d*] pyridazin-1-one
5,7-Dimethyl-2-(pyridin- 3-yl)-6-(p-tolyl)- 2,6-dihydro-1*H*-pyrrolo[3,4-*d*]pyridazin-1-one
5,7-Dimethyl-2-(pyridin-3-yl)-6-(m-tolyl)-2,6-dihydro-1*H*-pyrrolo[3,4-*d*]pyridazin-1-one
6-(3-Methoxyphenyl)-5,7-dimethyl-2-(pyridin-3-yl)-2,6-dihydro-1*H*-pyrrolo[3,4-*d*] pyridazin-1-one
6-(4-((2-Methoxyethyl)amino)phenyl)-5,7-dimethyl-2-phenyl-2,6-dihydro-1*H*-pyrrolo [3,4-*d*]pyridazin-1-one
6-(3-((2-Methoxyethyl)amino)phenyl)-5,7-dimethyl-2-phenyl-2,6-dihydro-1*H*-pyrrolo [3,4-*d*]pyridazin-1-one
6-(3-Methoxyphenyl)-5,7-dimethyl-2-(pyridin-2-yl)-2,6-dihydro-1*H*-pyrrolo[3,4-*d*] pyridazin-1-one
5,7-Dimethyl-2-(pyridin-2-yl)-6-(m-tolyl)-2,6-dihydro-1*H*-pyrrolo[3,4-*d*]pyridazin-1-one
6-(4-Methoxyphenyl)-5,7-dimethyl-2-(pyridin-3-yl)-2,6-dihydro-1*H*-pyrrolo[3,4-*d*] pyridazin-1-one
6-(4-Methoxyphenyl)-5,7-dimethyl-2-(pyridin-2-yl)-2,6-dihydro-1*H*-pyrrolo[3,4-*d*] pyridazin-1-one
5,7-Dimethyl-2-(pyridin-2-yl)-6-(p-tolyl)-2,6-dihydro-1*H*-pyrrolo[3,4-*d*]pyridazin-1-one
5,7-Dimethyl-6-(3-(2-oxopyrrolidin-1-yl)phenyl)-2-phenyl-2,6-dihydro-1*H*-pyrrolo [3,4-*d*]pyridazin-1-one
5,7-Dimethyl-2-(6-methylpyridin-3-yl)-6-phenyl-2,6-dihydro-1*H*-pyrrolo[3,4-*d*] pyridazin-1-one
6-(3-Hydroxyphenyl)-5,7-dimethyl-2-phenyl-2,6-dihydro-1*H*-pyrrolo[3,4-*d*]pyridazin-1-one

2-(6-Methoxypyridin-3-yl)-5,7-dimethyl-6-phenyl-2,6-dihydro-1*H*-pyrrolo[3,4-*d*] pyridazin-1-one
5,7-Dimethyl-2-(pyridin-2-yl)-6-(3-(trifluoromethyl)phenyl)-2,6-dihydro-1*H*-pyrrolo [3,4-*d*]pyridazin-1-one
3-(5,7-Dimethyl-1-oxo-2-(pyridin-2-yl)-1*H*-pyrrolo[3,4-*d*]pyridazin-6(2*H*)-yl) benzonitrile
6-(Benzo[*d*][1,3]dioxol-5-yl)-5,7-dimethyl-2-(pyridin-2-yl)-2,6-dihydro-1*H*-pyrrolo [3,4-*d*]pyridazin-1-one
5,7-Dimethyl-6-(5-methylpyridin-2-yl)-2-(pyridin-2-yl)-2,6-dihydro-1*H*-pyrrolo[3,4-*d*] pyridazin-1-one
4-(5,7-Dimethyl-1-oxo-6-phenyl-1*H*-pyrrolo[3,4-*d*]pyridazin-2(6*H*)-yl)benzamide 5,7-Dimethyl-6-phenyl-2-(pyrimidin-2-yl)-2,6-dihydro-1*H*-pyrrolo[3,4-*d*]pyridazin-1-one
5,7-Dimethyl-2-(pyridin-2-yl)-6-(3-(pyrrolidin-1-yl)phenyl)-2,6-dihydro-1*H*-pyrrolo [3,4-*d*]pyridazin-1-one
2-(4-Ethoxyphenyl)-5,7-dimethyl-6-phenyl-2,6-dihydro-1*H*-pyrrolo[3,4-*d*]pyridazin-1-one
6-(3-Methoxyphenyl)-5,7-dimethyl-2-(quinolin-2-yl)-2,6-dihydro-1*H*-pyrrolo[3,4-*d*] pyridazin-1-one
6-(2-Methoxypyridin-4-yl)-5,7-dimethyl-2-(pyridin-2-yl)-2,6-dihydro-1*H*-pyrrolo[3,4-d]pyridazin-1-one
6-(3-Methoxyphenyl)-4,5,7-trimethyl-2-(pyridin-2-yl)-2,6-dihydro-1*H*-pyrrolo[3,4-*d*] pyridazin-1-one
5,7-Dimethyl-6-(3-morpholinophenyl)-2-(pyridin-3-yl)-2,6-dihydro-1*H*-pyrrolo[3,4-*d*] pyridazin-1-one
*N*-(4-(6-(3-Methoxyphenyl)-5,7-dimethyl-1-oxo-1*H*-pyrrolo[3,4-*d*]pyridazin-2(6*H*)-yl) benzyl)acetamide
2-(4-(Hydroxymethyl)phenyl)-6-(3-methoxyphenyl)-5,7-dimethyl-2,6-dihydro-1*H*-pyrrolo[3,4-*d*]pyridazin-1-one
2-(4-Methoxyphenyl)-5,7-dimethyl-6-(3-morpholinophenyl)-2,6-dihydro-1*H*-pyrrolo [3,4-*d*]pyridazin-1-one
4-(5,7-Dimethyl-6-(3-morpholinophenyl)-1-oxo-1H-pyrrolo[3,4-*d*]pyridazin-2(6*H*)-yl) benzenesulfonamide
2-(4-(Methoxymethyl)phenyl)-5,7-dimethyl-6-(3-morpholinophenyl)-2,6-dihydro-1*H*-pyrrolo[3,4-*d*]pyridazin-1-one
6-(3-Methoxyphenyl)-5,7-dimethyl-2-(4-morpholinophenyl)-2,6-dihydro-1*H*-pyrrolo [3,4-*d*]pyridazin-1-one
2-([1,1'-Biphenyl]-4-yl)-6-(3-methoxyphenyl)-5,7-dimethyl-2,6-dihydro-1*H*-pyrrolo [3,4-*d*]pyridazin-1-one
6-(4-Chloro-3-methoxyphenyl)-5,7-dimethyl-2-(pyridin-2-yl)-2,6-dihydro-1*H*-pyrrolo [3,4-*d*]pyridazin-1-one
3-(6-(3-Methoxyphenyl)-5,7-dimethyl-1-oxo-1*H*-pyrrolo[3,4-*d*]pyridazin-2(6*H*-yl) benzamide
6-(3-Methoxyphenyl)-2-(6-methoxypyridin-2-yl)-5,7-dimethyl-2,6-dihydro-1*H*-pyrrolo [3,4-*d*]pyridazin-1-one
4-(6-(3-Methoxyphenyl)-5,7-dimethyl-1-oxo-1*H*-pyrrolo[3,4-*d*]pyridazin-2(6*H*)-yl)-*N,N*-dimethylbenzamide
6-([1,1'-Biphenyl]-3-yl)-5,7-dimethyl-2-(pyridin-2-yl)-2,6-dihydro-1*H*-pyrrolo[3,4-d] pyridazin-1-one
6-(3-Ethoxyphenyl)-5,7-dimethyl-2-(pyridin-2-yl)-2,6-dihydro-1*H*-pyrrolo[3,4-*d*] pyridazin-1-one
7-Ethyl-6-(3-methoxyphenyl)-5-methyl-2-(pyridin-2-yl)-2,6-dihydro-1*H*-pyrrolo[3,4-*d*]pyridazin-1-one
6-(3-Methoxy-4-methylphenyl)-5,7-dimethyl-2-(pyridin-2-yl)-2,6-dihydro-1*H*-pyrrolo [3,4-*d*]pyridazin-1-one
6-(1*H*-Indol-6-yl)-5,7-dimethyl-2-(pyridin-2-yl)-2,6-dihydro-1*H*-pyrrolo[3,4-*d*] pyridazin-1-one
2-(4-Methoxyphenyl)-5,7-dimethyl-6-(pyridin-2-yl)-2,6-dihydro-1*H*-pyrrolo[3,4-*d*] pyridazin-1-one
5,7-Dimethyl-6-(pyridin-2-yl)-2-m-tolyl-2,6-dihydro-1*H*-pyrrolo[3,4-*d*]pyridazin-1-one
6-(3-Methoxyphenyl)-2-(5-methoxypyridin-2-yl)-5,7-dimethyl-2,6-dihydro-1*H*-pyrrolo [3,4-*d*]pyridazin-1-one
5,7-Dimethyl-6-(3-morpholinophenyl)-2-(m-tolyl)-2,6-dihydro-1*H*-pyrrolo[3,4-*d*] pyridazin-1-one
6-(3-Ethylphenyl)-5,7-dimethyl-2-(pyridin-2-yl)-2,6-dihydro-1*H*-pyrrolo[3,4-*d*] pyridazin-1-one
6-(4-Ethylphenyl)-5,7-dimethyl-2-(pyridin-2-yl)-2,6-dihydro-1*H*-pyrrolo[3,4-*d*] pyridazin-1-one
4-(5,7-Dimethyl-1-oxo-6-phenyl-1*H*-pyrrolo[3,4-*d*]pyridazin-2(6*H*)-yl)-*N,N*-dimethyl benzenesulfonamide
2-(4-(Aminomethyl)phenyl)-6-(3-methoxyphenyl)-5,7-dimethyl-2,6-dihydro-1*H*-pyrrolo[3,4-*d*]pyridazin-1-one
7-Ethyl-6-(3-methoxyphenyl)-5-methyl-2-phenyl-2,6-dihydro-1*H*-pyrrolo[3,4-*d*] pyridazin-1-one
5,7-Dimethyl-6-(3-morpholinophenyl)-2-(pyridin-2-yl)-2,6-dihydro-1*H*-pyrrolo[3,4-*d*] pyridazin-1-one
6-(3-Methoxyphenyl)-5,7-dimethyl-2-(6-morpholinopyridin-3-yl)-2,6-dihydro-1*H*-pyrrolo[3,4-*d*]pyridazin-1-one
2-(4-(Hydroxymethyl)phenyl)-5,7-dimethyl-6-phenyl-2,6-dihydro-1*H*-pyrrolo[3,4-*d*] pyridazin-1-one
5,7-Dimethyl-2-(4-morpholinophenyl)-6-phenyl-2,6-dihydro-1*H*-pyrrolo[3,4-*d*] pyridazin-1-one
6-(3-Methoxyphenyl)-2-(5-methoxypyridin-3-yl)-5,7-dimethyl-2,6-dihydro-1*H*-pyrrolo [3,4-*d*]pyridazin-1-one
6-(3-Methoxyphenyl)-5,7-dimethyl-2-(pyrimidin-5-yl)-2,6-dihydro-1*H*-pyrrolo[3,4-*d*] pyridazin-1-one
5,7-Dimethyl-6-(l-methyl-1H-indol-6-yl)-2-(pyridin-2-yl)-2,6-dihydro-1*H*-pyrrolo [3,4-*d*]pyridazin-1-one
6-(3-Methoxyphenyl)-2-(4-methoxyphenyl)-5,7-dimethyl-2,6-dihydro-1*H*-pyrrolo[3,4-*d*]pyridazin-1-one
2-(4-(Hydroxymethyl)phenyl)-6-(4-methoxyphenyl)-5,7-dimethyl-2,6-dihydro-1*H*-pyrrolo[3,4-*d*]pyridazin-1-one
5,7-Dimethyl-2-(m-tolyl)-6-(p-tolyl)-2,6-dihydro-1*H*-pyrrolo[3,4-*d*]pyridazin-1-one
6-(3-(Diethylamino)phenyl)-5,7-dimethyl-2-(pyridin-2-yl)-2,6-dihydro-1*H*-pyrrolo [3,4-*d*]pyridazin-1-one
5,7-Dimethyl-2-phenyl-6-(3-(pyrrolidin-1-yl)phenyl)-2,6-dihydro-1*H*-pyrrolo[3,4-*d*] pyridazin-1-one
6-(3-(Azetidin-1-yl)phenyl)-5,7-dimethyl-2-(pyridin-2-yl)-2,6-dihydro-1*H*-pyrrolo[3,4-*d*]pyridazin-1-one
2,6-*bis*(4-Methoxyphenyl)-5,7-dimethyl-2,6-dihydro-1*H*-pyrrolo[3,4-*d*]pyridazin-1-one
*N*-(4-(5,7-Dimethyl-1-oxo-6-phenyl-1*H*-pyrrolo[3,4-*d*]pyridazin-2(6*H*)-yl)benzyl) acetamide
Methyl 2-methoxy-4-(6-(3-methoxyphenyl)-5,7-dimethyl-1-oxo-1*H*-pyrrolo[3,4-*d*] pyridazin-2(6*H*)-yl)benzoate
*N*-(4-(6-(4-Methoxyphenyl)-5,7-dimethyl-1-oxo-1*H*-pyrrolo[3,4-*d*]pyridazin-2(6*H*)-yl) benzyl)acetamide
6-(3-(Methoxymethyl)phenyl)-5,7-dimethyl-2-(pyridin-2-yl)-2,6-dihydro-1*H*-pyrrolo [3,4-*d*]pyridazin-1-one
6-(3-(Methoxymethyl)phenyl)-5,7-dimethyl-2-phenyl-2,6-dihydro-1*H*-pyrrolo[3,4-*d*] pyridazin-1-one
6-(3-Methoxyphenyl)-5,7-dimethyl-2-(4-((methylamino)methyl)phenyl)-2,6-dihydro-1*H*-pyrrolo[3,4-*d*]pyridazin-1-one
6-(3-(Dimethylamino)phenyl)-5,7-dimethyl-2-(pyridin-2-yl)-2,6-dihydro-1*H*-pyrrolo [3,4-*d*]pyridazin-1-one
5,7-Dimethyl-6-(3-(2-oxopyrrolidin-1-yl)phenyl)-2-(pyridin-2-yl)-2,6-dihydro-1*H*-pyrrolo[3,4-*d*]pyridazin-1-one
3-(6-(3-Methoxyphenyl)-5,7-dimethyl-1-oxo-1Hpyrrolo[3,4-*d*]pyridazin-2(6*H*)-yl)-*N,N*-dimethylbenzamide
4-(6-(3-Methoxyphenyl)-5,7-dimethyl-1-oxo-1H-pyrrolo[3,4-*d*]pyridazin-2(6*H*)-yl)-*N*-methylbenzamide
2-(3-(Hydroxymethyl)phenyl)-6-(3-methoxyphenyl)-5,7-dimethyl-2,6-dihydro-1*H*-pyrrolo[3,4-*d*]pyridazin-1-one
*N*-(3-(6-(3-Methoxyphenyl)-5,7-dimethyl-1-oxo-1*H*-pyrrolo[3,4-*d*]pyridazin-2(6*H*)-yl)benzyl)acetamide
2-(3-(Methoxymethyl)phenyl)-6-(3-methoxyphenyl)-5,7-dimethyl-2,6-dihydro-1*H*-pyrrolo[3,4-*d*]pyridazin-1-one
2-(3,4-Dimethoxyphenyl)-6-(3-methoxyphenyl)-5,7-dimethyl-2,6-dihydro-1*H*-pyrrolo [3,4-*d*]pyridazin-1-one
2-(4-(3-Hydroxypropyl)phenyl)-6-(3-methoxyphenyl)-5,7-dimethyl-2,6-dihydro-1*H*-pyrrolo[3,4-*d*]pyridazin-1-one
6-(3-Methoxyphenyl)-5,7-dimethyl-2-(4-(methylsulfonyl)phenyl)-2,6-dihydro-1*H*-pyrrolo[3,4-*d*]pyridazin-1-one
4-(6-(3-Methoxyphenyl)-5,7-dimethyl-1-oxo-1*H*-pyrrolo[3,4-*d*]pyridazin-2(6*H*)-yl) benzenesulfonamide
6-(3-Methoxyphenyl)-2-(2-methoxypyridin-4-yl)-5,7-dimethyl-2,6-dihydro-1*H*-pyrrolo [3,4-*d*]pyridazin-1-one
6-(3-Methoxyphenyl)-5,7-dimethyl-2-(5-(trifluoromethyl)pyridin-2-yl)-2,6-dihydro-1*H*-pyrrolo[3,4-*d*]pyridazin-1-one
6-(3-Methoxyphenyl)-2-(4-methoxypyridin-2-yl)-5,7-dimethyl-2,6-dihydro-1*H*-pyrrolo[3,4-*d*]pyridazin-1-one
3-(6-(3-Methoxyphenyl)-5,7-dimethyl-1-oxo-1*H*-pyrrolo[3,4-*d*]pyridazin-2(6*H*)-yl) benzonitrile
3-(6-(4-Methoxyphenyl)-5,7-dimethyl-1-oxo-1*H*-pyrrolo[3,4-*d*]pyridazin-2(6*H*)-yl) benzonitrile
4-(6-(3-Methoxyphenyl)-5,7-dimethyl-1-oxo-1*H*-pyrrolo[3,4-*d*]pyridazin-2(6*H*)-yl) benzonitrile
6-(3-Methoxyphenyl)-2-(6-methoxypyridin-3-yl)-5,7-dimethyl-2,6-dihydro-1*H*-pyrrolo [3,4-*d*]pyridazin-1-one
2-(4-(Methoxymethyl)phenyl)-6-(3-methoxyphenyl)-5,7-dimethyl-2,6-dihydro-1*H*-pyrrolo[3,4-*d*]pyridazin-1-one
2-(2-Hydroxypyridin-4-yl)-6-(3-methoxyphenyl)-5,7-dimethyl-2,6-dihydro-1*H*-pyrrolo [3,4-*d*]pyridazin-1-one
2,6-*bis*(3-Methoxyphenyl)-5,7-dimethyl-2,6-dihydro-1*H*-pyrrolo[3,4-*d*]pyridazin-1-one
2-(6-Hydroxypyridin-3-yl)-6-(3-methoxyphenyl)-5,7-dimethyl-2,6-dihydro-1*H*-pyrrolo [3,4-*d*]pyridazin-1-one
6-(4-Acetylphenyl)-5,7-dimethyl-2-(pyridin-2-yl)-2,6-dihydro-1*H*-pyrrolo[3,4-*d*] pyridazin-1-one
Ethyl 3-(5,7-dimethyl-1-oxo-2-(pyridin-2-yl)-1*H*-pyrrolo[3,4-*d*]pyridazin-6(2*H*)-yl) benzoate
Ethyl 3-(5,7-dimethyl-1-oxo-2-phenyl-1*H*-pyrrolo[3,4-*d*]pyridazin-6(2*H*)-yl)benzoate 2-(4-(2-Hydroxypropan-2-yl) phenyl)-6-(3-methoxyphenyl)-5,7-dimethyl-2,6-dihydro-1*H*-pyrrolo[3,4-*d*]pyridazin-1-one
6-(3-(Furan-2-yl)phenyl)-5,7-dimethyl-2-(pyridin-2-yl)-2,6-dihydro-1*H*-pyrrolo[3,4-*d*] pyridazin-1-one
6-(3-Cyclopropylphenyl)-5,7-dimethyl-2-(pyridin-2-yl)-2,6-dihydro-1*H*-pyrrolo[3,4-*d*] pyridazin-1-one
2-(4-(1-Hydroxyethyl)phenyl)-6-(3-methoxyphenyl)-5,7-dimethyl-2,6-dihydro-1*H*-pyrrolo[3,4-*d*]pyridazin-1-one
6-(3-Isopropoxyphenyl)-5,7-dimethyl-2-(pyridin-2-yl)-2,6-dihydro-1*H*-pyrrolo[3,4-*d*] pyridazin-1-one
5-Ethyl-6-(3-methoxyphenyl)-7-methyl-2-(pyridin-2-yl)-2,6-dihydro-1*H*-pyrrolo[3,4-*d*]pyridazin-1-one
6-(3-(Azetidin-1-yl)phenyl)-5,7-dimethyl-2-phenyl-2,6-dihydro-1*H*-pyrrolo[3,4-*d*] pyridazin-1-one
6-(3-(1*H*-Pyrrol-1-yl)phenyl)-5,7-dimethyl-2-(pyridin-2-yl)-2,6-dihydro-1*H*-pyrrolo [3,4-*d*]pyridazin-1-one
5,7-Dimethyl-6-(3-(piperidin-1-yl)phenyl)-2-(pyridin-2-yl)-2,6-dihydro-1*H*-pyrrolo [3,4-*d*]pyridazin-1-one
2-(3-Ethoxyphenyl)-6-(3-methoxyphenyl)-5,7-dimethyl-2,6-dihydro-1*H*-pyrrolo[3,4-*d*] pyridazin-1-one
2-(4-Hydroxyphenyl)-6-(3-methoxyphenyl)-5,7-dimethyl-2,6-dihydro-1*H*-pyrrolo[3,4-*d*]pyridazin-1-one
2-(3-(6-(3-Methoxyphenyl)-5,7-dimethyl-1-oxo-1*H*-pyrrolo[3,4-*d*]pyridazin-2(6*H*)-yl) phenyl)acetonitrile
2-(3-Acetylphenyl)-6-(3-methoxyphenyl)-5,7-dimethyl-2,6-dihydro-1*H*-pyrrolo[3,4-*d*] pyridazin-1-one
2-(2-Methoxyphenyl)-6-(3-methoxyphenyl)-5,7-dimethyl-2,6-dihydro-1*H*-pyrrolo[3,4-*d*]pyridazin-1-one
2-(2-Fluorophenyl)-6-(3-methoxyphenyl)-5,7-dimethyl-2,6-dihydro-1*H*-pyrrolo[3,4-*d*] pyridazin-1-one
2-(2-Chlorophenyl)-6-(3-methoxyphenyl)-5,7-dimethyl-2,6-dihydro-1*H*-pyrrolo[3,4-*d*] pyridazin-1-one
6-(3-(Azetidin-1-yl)phenyl)-5,7-dimethyl-2-(3-(methylsulfonyl)phenyl)-2,6-dihydro-1*H*-pyrrolo[3,4-*d*]pyridazin-1-one
3-(6-(3-(Azetidin-1-yl)phenyl)-5,7-dimethyl-1-oxo-1*H*-pyrrolo[3,4-*d*]pyridazin-2(6*H*)-yl)benzamide
6-(3-(3,3-Difluoroazetidin-1-yl)phenyl)-5,7-dimethyl-2-(pyridin-2-yl)-2,6-dihydro-1*H*-pyrrolo[3,4-*d*]pyridazin-1-one
6-(3-(Azetidin-1-yl)phenyl)-5,7-dimethyl-2-(6-methylpyridin-2-yl)-2,6-dihydro-1*H*-pyrrolo[3,4-*d*]pyridazin-1-one
6-(3-(Azetidin-1-yl)phenyl)-5,7-dimethyl-2-(5-methylpyridin-2-yl)-2,6-dihydro-1*H*-pyrrolo[3,4-*d*]pyridazin-1-one
6-(3-(Azetidin-1-yl)phenyl)-5,7-dimethyl-2-(pyridin-4-yl)-2,6-dihydro-1*H*-pyrrolo[3,4-*d*]pyridazin-1-one
6-(3-(Azetidin-1-yl)phenyl)-5,7-dimethyl-2-(6-methylpyridin-3-yl)-2,6-dihydro-1*H*-pyrrolo[3,4-*d*]pyridazin-1-one
6-(3-(Azetidin-1-yl)phenyl)-5,7-dimethyl-2-(5-methylpyridin-3-yl)-2,6-dihydro-1*H*-pyrrolo[3,4-*d*]pyridazin-1-one
6-(3-(Azetidin-1-yl)phenyl)-5,7-dimethyl-2-(3-methylpyridin-2-yl)-2,6-dihydro-1*H*-pyrrolo[3,4-*d*]pyridazin-1-one
6-(6-(3-(Azetidin-1-yl)phenyl)-5,7-dimethyl-1-oxo-1*H*-pyrrolo[3,4-*d*]pyridazin-2(6*H*)-yl)nicotinonitrile
6-(3-(Azetidin-1-yl)phenyl)-5,7-dimethyl-2-(4-methylpyridin-2-yl)-2,6-dihydro-1*H*-pyrrolo[3,4-*d*]pyridazin-1-one
6-(3-(Azetidin-1-yl)phenyl)-2-(4-(2-methoxyethoxy)phenyl)-5,7-dimethyl-2,6-dihydro-1*H*-pyrrolo[3,4-d]pyridazin-1-one
6-(3-(Azetidin-1-yl)phenyl)-2-(3-(hydroxymethyl)-4-methoxyphenyl)-5,7-dimethyl-2,6-dihydro-1*H*-pyrrolo[3,4-*d*]pyridazin-1-one

6-(3-(Azetidin-1-yl)phenyl)-5,7-dimethyl-2-(pyridin-3-yl)-2,6-dihydro-1*H*-pyrrolo[3,4-*d*]pyridazin-1-one
6-(3-(Azetidin-1-yl)phenyl)-2-(2,6-dimethylpyridin-4-yl)-5,7-dimethyl-2,6-dihydro-1*H* -pyrrolo[3,4-*d*]pyridazin-1-one
6-(3-(Azetidin-1-yl)phenyl)-2-(4-(hydroxymethyl)-3-methoxyphenyl)-5,7-dimethyl-2,6-dihydro-1*H*-pyrrolo[3,4-*d*]pyridazin-1-one
6-(3-(Azetidin-1-yl)phenyl)-5,7-dimethyl-2-(3-morpholinophenyl)-2,6-dihydro-1*H*-pyrrolo[3,4-*d*]pyridazin-1-one
6-(3-(Azetidin-1-yl)phenyl)-5,7-dimethyl-2-(4-(pyrrolidin-1-yl)phenyl)-2,6-dihydro-1*H*-pyrrolo[3,4-*d*]pyridazin-1-one
6-(3-(Azetidin-1-yl)phenyl)-2-(4-(2-hydroxyethyl)phenyl)-5,7-dimethyl-2,6-dihydro-1*H*-pyrrolo[3,4-*d*]pyridazin-1-one
6-(3-(Azetidin-1-yl)phenyl)-2-(4-(2-hydroxypropan-2-yl)phenyl)-5,7-dimethyl-2,6-dihydro-1*H*-pyrrolo[3,4-*d*]pyridazin-1-one
2-(4-Acetylphenyl)-6-(3-(azetidin-1-yl)phenyl)-5,7-dimethyl-2,6-dihydro-1*H*-pyrrolo [3,4-*d*]pyridazin-1-one
6-(3-Methoxyphenyl)-5,7-dimethyl-2-(5-methylpyridin-2-yl)-2,6-dihydro-1*H*-pyrrolo [3,4-*d*]pyridazin-1-one
6-(3-(Azetidin-1-yl)phenyl)-2-(5-fluoropyridin-2-yl)-5,7-dimethyl-2,6-dihydro-1*H*-pyrrolo[3,4-*d*]pyridazin-1-one
6-(3-(Azetidin-1-yl)phenyl)-5,7-dimethyl-2-(4-(piperazin-1-yl)phenyl)-2,6-dihydro-1*H* -pyrrolo[3,4-*d*]pyridazin-1-one
6-(3,5-Dimethoxyphenyl)-5,7-dimethyl-2-(pyridin-2-yl)-2,6-dihydro-1*H*-pyrrolo[3,4-*d*] pyridazin-1-one
6-(3-(Azetidin-1-yl)phenyl)-2-(2-methoxyphenyl)-5,7-dimethyl-2,6-dihydro-1*H*-pyrrolo[3,4-*d*]pyridazin-1-one
6-(3-(Azetidin-1-yl)phenyl)-2-(2-fluorophenyl)-5,7-dimethyl-2,6-dihydro-1*H*-pyrrolo [3,4-*d*]pyridazin-1-one
6-(3-(Azetidin-1-yl)phenyl)-5,7-dimethyl-2-(4-(3-oxopiperazin-1-yl)phenyl)-2,6-dihydro-1*H*-pyrrolo[3,4-*d*]pyridazin-1-one
6-(3-(3,3-Dimethylpyrrolidin-1-yl)phenyl)-5,7-dimethyl-2-(pyridin-2-yl)-2,6-dihydro-1*H*-pyrrolo[3,4-*d*]pyridazin-1-one
2-(4-(6-(3-(Azetidin-1-yl)phenyl)-5,7-dimethyl-1-oxo-1*H*-pyrrolo[3,4-*d*]pyridazin-2 (6*H*)-yl)phenyl)-*N*-methylacetamide
6-(3-(Azetidin-1-yl)phenyl)-2-(4-iodophenyl)-5,7-dimethyl-2,6-dihydro-1*H*-pyrrolo [3,4-*d*]pyridazin-1-one
6-(3-(Azetidin-1-yl)phenyl)-5,7-dimethyl-2-(2-methylpyridin-4-yl)-2,6-dihydro-1*H*-pyrrolo[3,4-*d*]pyridazin-1-one
6-(3-(Azetidin-1-yl)phenyl)-2-(5-chloropyridin-2-yl)-5,7-dimethyl-2,6-dihydro-1*H*-pyrrolo[3,4-*d*]pyridazin-1-one
*tert*-Butyl 2-(4-(6-(3-(azetidin-1-yl)phenyl)-5,7-dimethyl-1-oxo-1*H*-pyrrolo[3,4-*d*] pyridazin-2(6*H*)-yl)phenyl)-2-oxoethylcarbamate
2-(4-(6-(3-(Azetidin-1-yl)phenyl)-5,7-dimethyl-1-oxo-1*H*-pyrrolo[3,4-*d*]pyridazin-2 (6*H*)-yl)phenyl)acetamide
6-(4-Cyclopropylphenyl)-5,7-dimethyl-2-(pyridin-2-yl)-2,6-dihydro-1*H*-pyrrolo[3,4-*d*] pyridazin-1-one
5,7-Dimethyl-6-(3-phenoxyphenyl)-2-(pyridin-2-yl)-2,6-dihydro-1*H*-pyrrolo[3,4-d] pyridazin-1-one
6-(3-(Azetidin-1-yl)phenyl)-2-(pyridin-2-yl)-2,6-dihydro-1*H*-pyrrolo[3,4-*d*]pyridazin-1-one
6-(3-(Azetidin-1-yl)phenyl)-2-(4-(2-fluoroethyl)phenyl)-5,7-dimethyl-2,6-dihydro-1*H*-pyrrolo[3,4-*d*]pyridazin-1-one
6-(3-(Azetidin-1-yl)phenyl)-2-cyclopentyl-5,7-dimethyl-2,6-dihydro-1*H*-pyrrolo[3,4-*d*]pyridazin-1-one
6-(4-Fluoro-2-methoxyphenyl)-5,7-dimethyl-2-(pyridin-2-yl)-2,6-dihydro-1*H*-pyrrolo[3,4-*d*]pyridazin-1-one
6-(3-(Azetidin-1-yl)-4-fluorophenyl)-5,7-dimethyl-2-(pyridin-2-yl)-2,6-dihydro-1*H*-pyrrolo[3,4-*d*]pyridazin-1-one
6-(2-Methoxyphenyl)-5,7-dimethyl-2-(pyridin-2-yl)-2,6-dihydro-1*H*-pyrrolo[3,4-*d*]pyridazin-1-one
6-(2-Fluoro-5-methoxyphenyl)-5,7-dimethyl-2-(pyridin-2-yl)-2,6-dihydro-1*H*-pyrrolo[3,4-*d*]pyridazin-1-one
6-(3-(Azetidin-1-yl)-5-fluorophenyl)-5,7-dimethyl-2-(pyridin-2-yl)-2,6-dihydro-1*H*-pyrrolo[3,4-*d*]pyridazin-1-one
6-(5-(Azetidin-1-yl)-2-fluorophenyl)-5,7-dimethyl-2-(pyridin-2-yl)-2,6-dihydro-1*H*-pyrrolo[3,4-*d*]pyridazin-1-one
6-(2-Chloro-3-methoxyphenyl)-5,7-dimethyl-2-(pyridin-2-yl)-2,6-dihydro-1*H*-pyrrolo[3,4-*d*]pyridazin-1-one
6-(1-Cyclopropyl-1*H*-indol-4-yl)-5,7-dimethyl-2-(pyrimidin-2-yl)-2,6-dihydro-1*H*-pyrrolo[3,4-*d*]pyridazin-1-one
6-(3-(2*H*-1,2,3-Triazol-2-yl)phenyl)-5,7-dimethyl-2-(pyridin-2-yl)-2,6-dihydro-1*H*-pyrrolo[3,4-*d*]pyridazin-1-one
6-(3-Cyclopropoxy-2-methylphenyl)-5,7-dimethyl-2-(pyrimidin-2-yl)-2,6-dihydro-1*H*-pyrrolo[3,4-*d*]pyridazin-1-one
6-(2,4-Dimethylphenyl)-5,7-dimethyl-2-(pyridin-2-yl)-2,6-dihydro-1*H*-pyrrolo[3,4-*d*]pyridazin-1-one
6-(3-(Difluoromethoxy)phenyl)-5,7-dimethyl-2-(pyridin-2-yl)-2,6-dihydro-1*H*-pyrrolo[3,4-*d*]pyridazin-1-one
6-(2-Chloro-3-methoxyphenyl)-5,7-dimethyl-2-(pyrimidin-2-yl)-2,6-dihydro-1*H*-pyrrolo[3,4-*d*]pyridazin-1-one
6-(2-Chloro-3-methoxyphenyl)-5,7-dimethyl-2-(5-methylpyrimidin-2-yl)-2,6-dihydro-1*H*-pyrrolo[3,4-d]pyridazin-1-one
6-(3-Cyclopropoxy-2-methylphenyl)-5,7-dimethyl-2-(5-methylpyrimidin-2-yl)-2,6-dihydro-1*H*-pyrrolo[3,4-*d*]pyridazin-1-one
2-(5-*tert*-Butylpyridin-2-yl)-6-(3-methoxyphenyl)-5,7-dimethyl-2,6-dihydro-1*H*-pyrrolo[3,4-*d*]pyridazin-1-one
2-(5-Cyclopropylpyrimidin-2-yl)-6-(3-methoxy-2-methylphenyl)-5,7-dimethyl-2,6-dihydro-1*H*-pyrrolo[3,4-*d*]pyridazin-1-one
6-(3-(Azetidin-1-yl)-5-fluorophenyl)-2-(5-fluoropyrimidin-2-yl)-5,7-dimethyl-2,6-dihydro-1*H*-pyrrolo[3,4-*d*]pyridazin-1-one
6-(3-(Azetidin-1-yl)-5-fluorophenyl)-2-(3-fluoropyridin-2-yl)-5,7-dimethyl-2,6-dihydro-1*H*-pyrrolo[3,4-*d*]pyridazin-1-one

5,7-Dimethyl-6-(1-methylindolin-4-yl)-2-(pyridin-2-yl)-2,6-dihydro-1*H*-pyrrolo[3,4-*d*]pyridazin-1-one
6-(3-Cyclopropoxy-2-methylphenyl)-2-(3-fluoropyridin-2-yl)-5,7-dimethyl-2,6-dihydro-1*H*-pyrrolo[3,4-*d*]pyridazin-1-one
6-(3-Cyclopropoxy-2-methylphenyl)-2-(5-fluoropyrimidin-2-yl)-5,7-dimethyl-2,6-dihydro-1*H*-pyrrolo[3,4-*d*]pyridazin-1-one
6-(1-Cyclopropyl-1*H*-indol-4-yl)-2-(3-fluoropyridin-2-yl)-5,7-dimethyl-2,6-dihydro-1*H*-pyrrolo[3,4-*d*]pyridazin-1-one
6-(1-Cyclopropyl-1*H*-indol-4-yl)-2-(5-fluoropyrimidin-2-yl)-5,7-dimethyl-2,6-dihydro-1*H*-pyrrolo[3,4-*d*]pyridazin-1-one
6-(2-Chloro-3-cyclopropoxyphenyl)-5,7-dimethyl-2-(pyrimidin-2-yl)-2,6-dihydro-1*H*-pyrrolo[3,4-*d*]pyridazin-1-one
2-(5-(2-Hydroxypropan-2-yl)pyridin-2-yl)-6-(3-methoxyphenyl)-5,7-dimethyl-2,6-dihydro-1*H*-pyrrolo[3,4-*d*]pyridazin-1-one
6-(5-(Azetidin-1-yl)-2-fluorophenyl)-2-(3-fluoropyridin-2-yl)-5,7-dimethyl-2,6-dihydro-1*H*-pyrrolo[3,4-*d*]pyridazin-1-one
6-(5-(Azetidin-1-yl)-2-fluorophenyl)-2-(5-fluoropyrimidin-2-yl)-5,7-dimethyl-2,6-dihydro-1*H*-pyrrolo[3,4-*d*]pyridazin-1-one
6-(3-(Azetidin-1-yl)phenyl)-5,7-dimethyl-2-(4-morpholinophenyl)-2,6-dihydro-1*H*-pyrrolo[3,4-*d*]pyridazin-1-one
6-(3-Hydroxy-2-methylphenyl)-5,7-dimethyl-2-(4-morpholinophenyl)-2,6-dihydro-1*H*-pyrrolo[3,4-*d*]pyridazin-1-one
6-(3-Cyclopropoxy-2-methylphenyl)-5,7-dimethyl-2-(4-morpholinophenyl)-2,6-dihydro-1*H*-pyrrolo[3,4-*d*]pyridazin-1-one.
6-(3-(Azetidin-1-yl)phenyl)-2-(bicyclo[1.1.1]pentan-1-yl)-5,7-dimethyl-2,6-dihydro-1*H*-pyrrolo[3,4-*d*]pyridazin-1-one
6-(3-(Azetidin-1-yl)phenyl)-5,7-dimethyl-2-(pyrimidin-2-yl)-2,6-dihydro-1*H*-pyrrolo[3,4-*d*]pyridazin-1-one
6-(3-(Azetidin-1-yl)phenyl)-2-(5-methoxypyrimidin-2-yl)-5,7-dimethyl-2,6-dihydro-1*H*-pyrrolo[3,4-*d*]pyridazin-1-one
6-(3-(Azetidin-1-yl)phenyl)-2-(3-fluoropyridin-2-yl)-5,7-dimethyl-2,6-dihydro-1*H*-pyrrolo[3,4-*d*]pyridazin-1-one
6-(3-(Azetidin-1-yl)phenyl)-2-(5-fluoropyrimidin-2-yl)-5,7-dimethyl-2,6-dihydro-1*H*-pyrrolo[3,4-*d*]pyridazin-1-one
6-(3-(Azetidin-1-yl)phenyl)-5,7-dimethyl-2-(thiazol-2-yl)-2,6-dihydro-1*H*-pyrrolo[3,4-*d*]pyridazin-1-one
6-(3-(Azetidin-1-yl)phenyl)-5,7-dimethyl-2-(5-methylpyrimidin-2-yl)-2,6-dihydro-1*H*-pyrrolo[3,4-*d*]pyridazin-1-one
6-(3-(Azetidin-1-yl)phenyl)-2-(4-methoxypyridin-2-yl)-5,7-dimethyl-2,6-dihydro-1*H*-pyrrolo[3,4-*d*]pyridazin-1-one
6-(3-Methoxy-2-methylphenyl)-5,7-dimethyl-2-(pyridin-2-yl)-2,6-dihydro-1*H*-pyrrolo[3,4-*d*]pyridazin-1-one
6-(3-Methoxy-2-methylphenyl)-5,7-dimethyl-2-(pyrimidin-2-yl)-2,6-dihydro-1*H*-pyrrolo[3,4-*d*]pyridazin-1-one
6-(3-(Difluoromethoxy)-2-methylphenyl)-5,7-dimethyl-2-(pyridin-2-yl)-2,6-dihydro-1*H*-pyrrolo[3,4-*d*]pyridazin-1-one
6-(2-Fluoro-3-methoxy-phenyl)-5,7-dimethyl-3-(2-pyridyl)pyrrolo[3,4-*d*]pyridazin-4-one
6-(2-Fluoro-3-methoxy-phenyl)-5,7-dimethyl-3-pyrimidin-2-yl-pyrrolo[3,4-*d*]pyridazin-4-one
6-[3-(Cyclopropoxy)-2-fluoro-phenyl]-5,7-dimethyl-3-(2-pyridyl)pyrrolo[3,4-*d*]pyridazin-4-one
6-[3-(Cyclopropoxy)-2-fluoro-phenyl]-5,7-dimethyl-3-pyrimidin-2-yl-pyrrolo[3,4-*d*]pyridazin-4-one
6-(2-Cyclopropoxy-3-fluoropyridin-4-yl)-5-methyl-2-(pyrimidin-2-yl)-2,6-dihydro-1*H*-pyrrolo[3,4-*d*]pyridazin-1-one
6-(6-Methoxypyridin-3-yl)-5,7-dimethyl-2-(pyridin-2-yl)-2,6-dihydro-1*H*-pyrrolo[3,4-*d*]pyridazin-1-one
6-(2-Fluoro-6-methoxypyridin-3-yl)-5,7-dimethyl-2-(pyrimidin-2-yl)-2,6-dihydro-1*H*-pyrrolo[3,4-*d*]pyridazin-1-one
6-(2-Cyclopropoxy-3-methylpyridin-4-yl)-5-methyl-2-(pyrimidin-2-yl)-2,6-dihydro-1*H*-pyrrolo[3,4-*d*]pyridazin-1-one
6-(6-Methoxypyridin-3-yl)-5,7-dimethyl-2-(pyrimidin-2-yl)-2,6-dihydro-1*H*-pyrrolo[3,4-*d*]pyridazin-1-one
6-(6-Methoxy-2-methylpyridin-3-yl)-5,7-dimethyl-2-(pyrimidin-2-yl)-2,6-dihydro-1*H*-pyrrolo[3,4-*d*]pyridazin-1-one
and
6-(3-Cyclopropoxy-2-fluorophenyl)-5-methyl-2-(pyrimidin-2-yl)-2,6-dihydro-1*H*-pyrrolo[3,4-*d*]pyridazin-1-one.

The above list of compounds can also be represented by the following skeletal formulae :

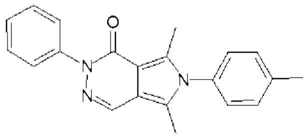
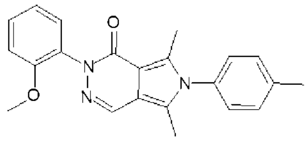
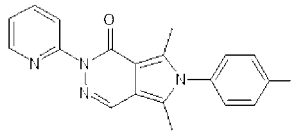

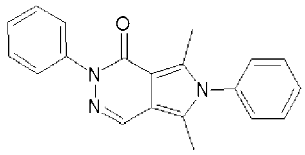
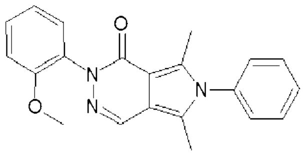
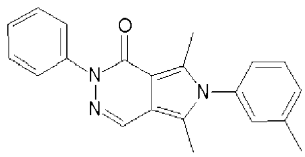

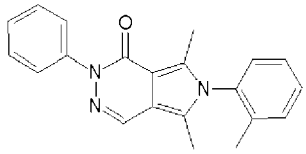
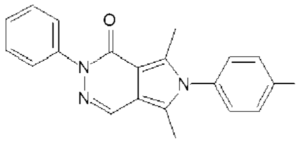
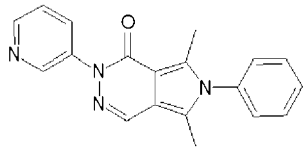
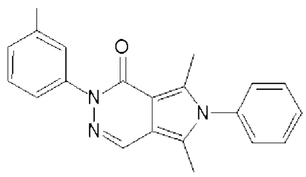
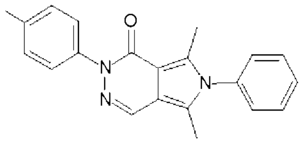
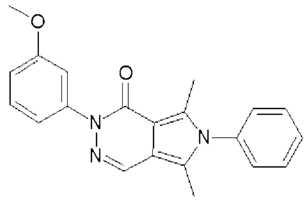
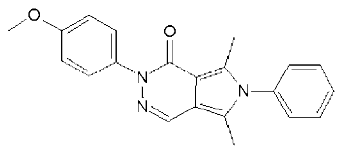
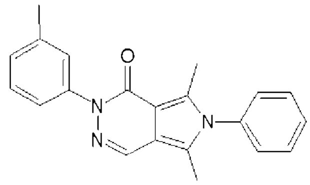
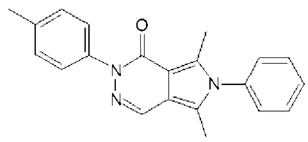
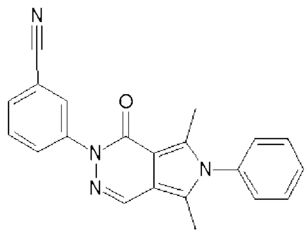
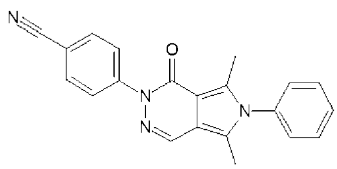
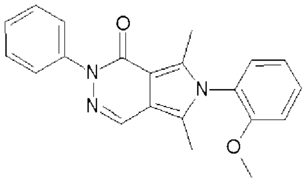
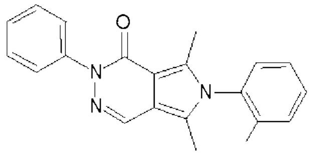
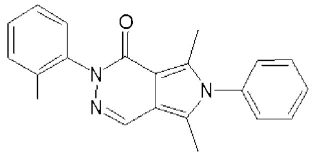
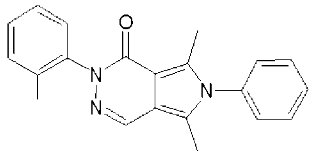
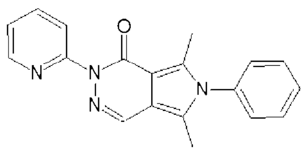
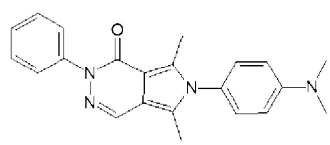
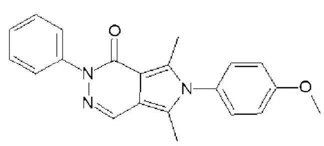
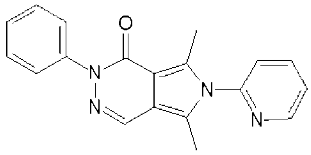
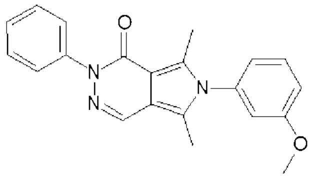
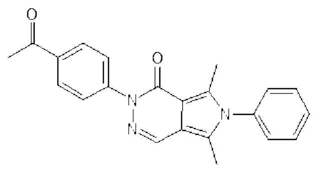
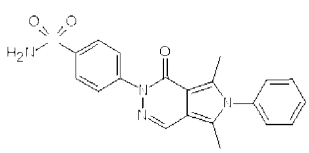
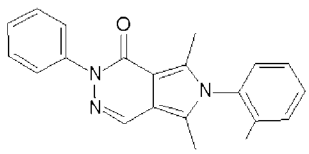
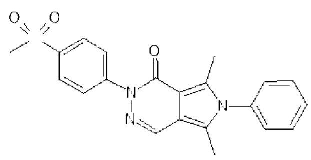

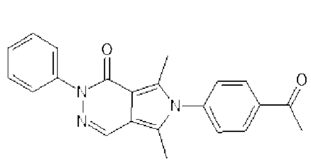
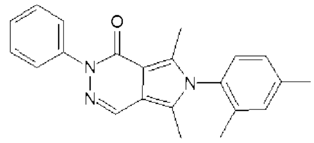
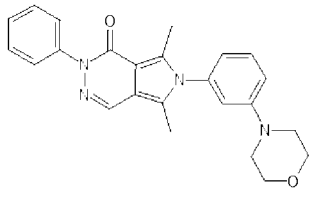

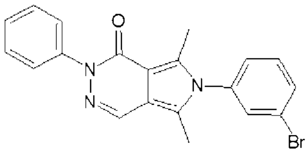
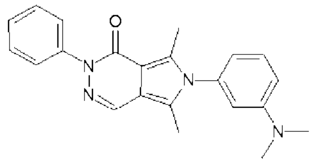
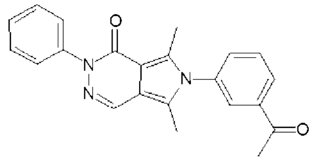

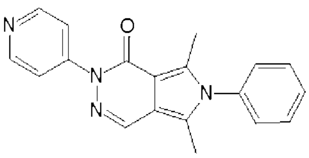
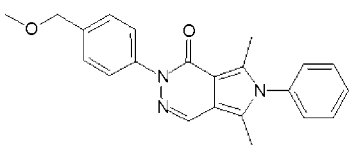
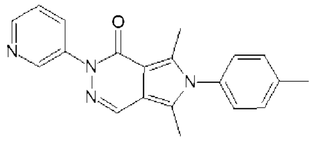

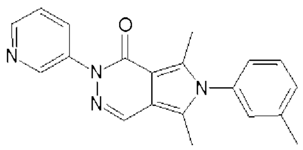
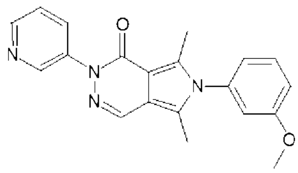
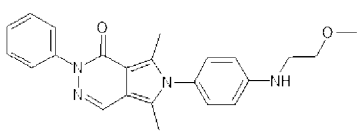

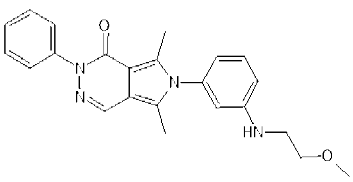
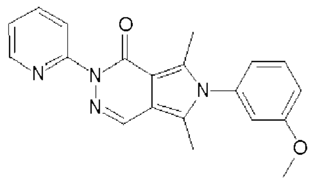
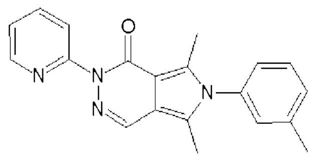

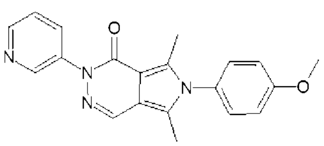
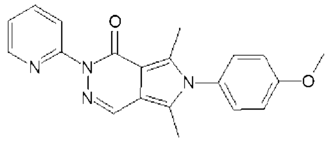
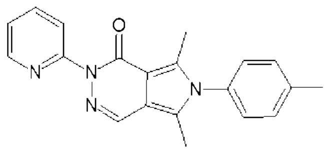

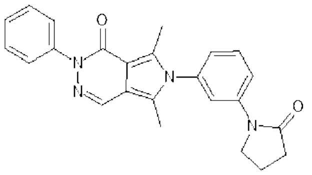
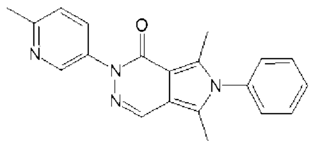
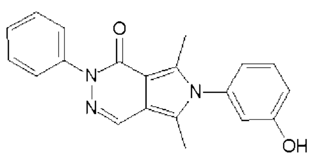

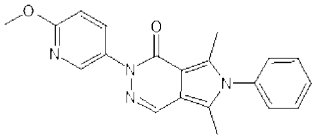
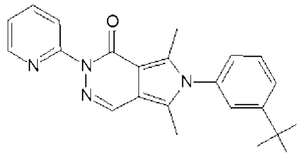
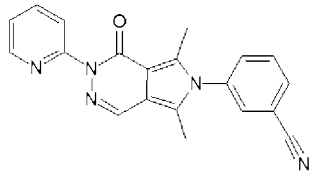

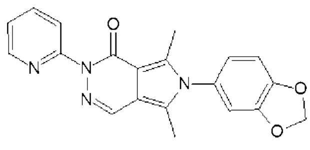
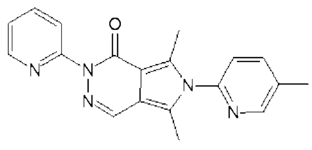
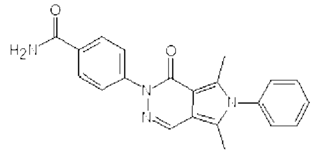

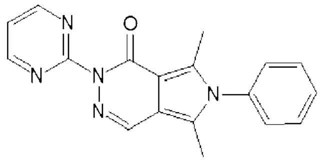
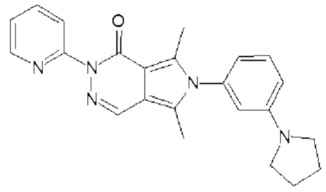
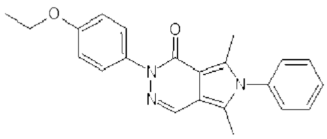

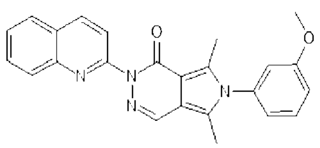
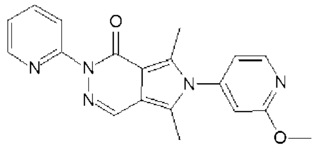
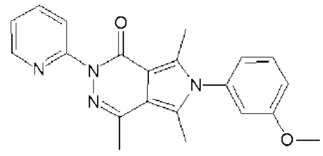

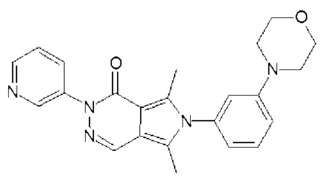
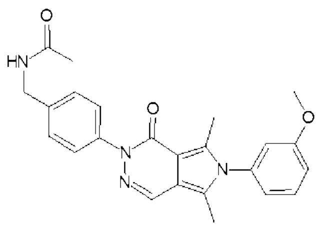
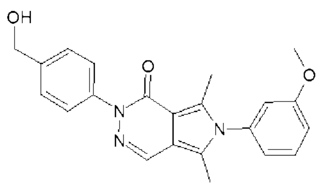

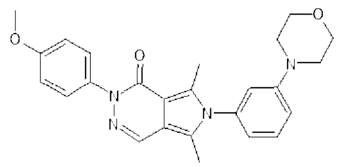
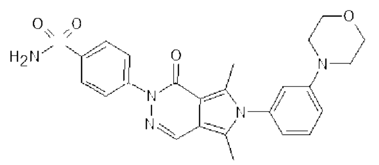
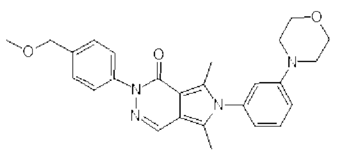

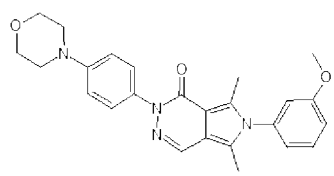
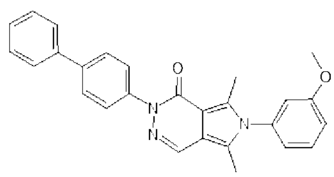
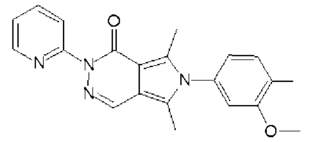

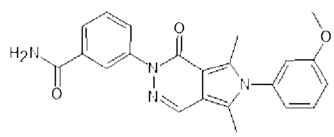
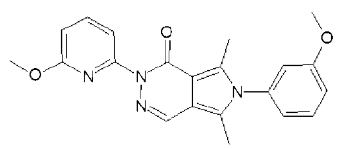
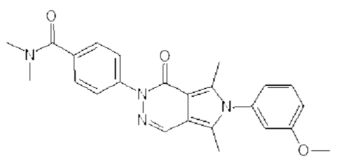

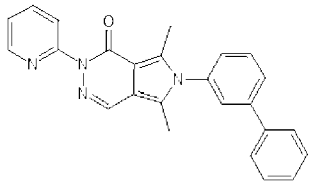
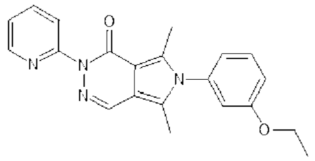
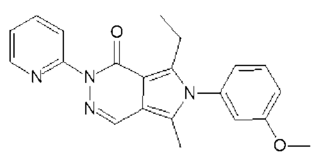

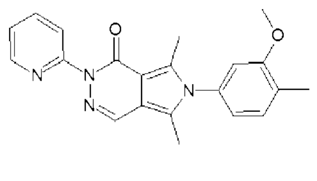
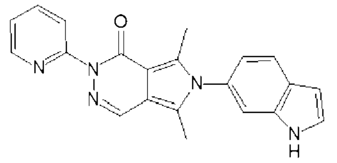
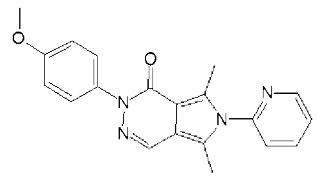

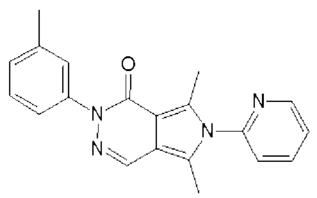
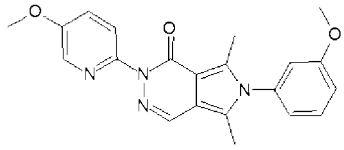
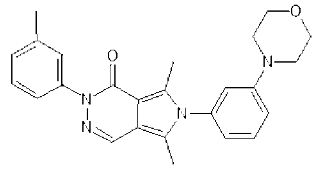

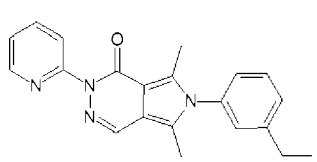
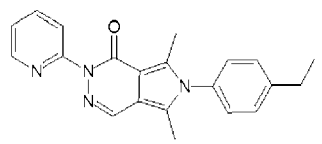
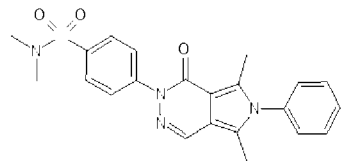

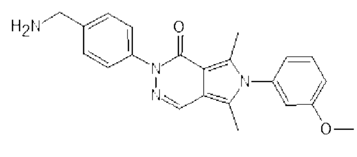
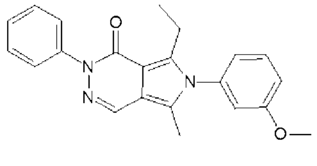
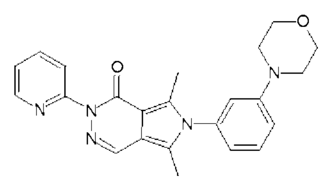

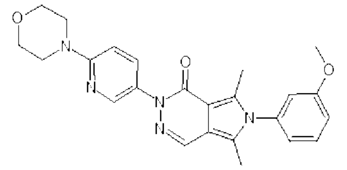
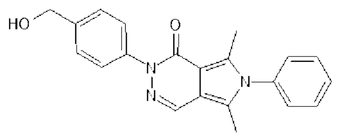
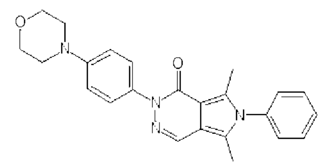

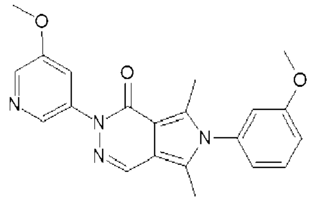
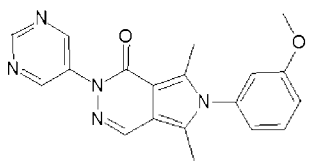
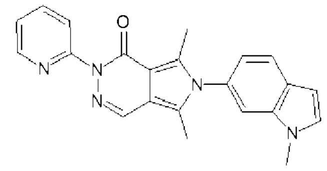

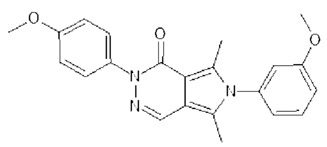
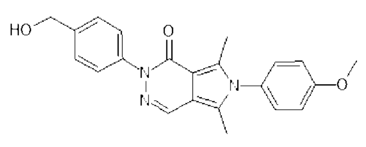
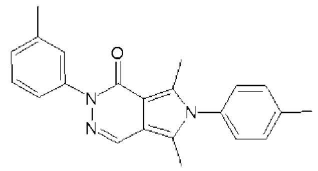

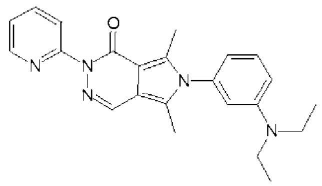
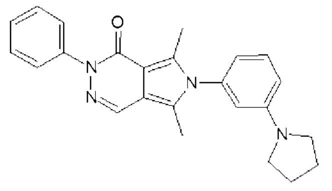
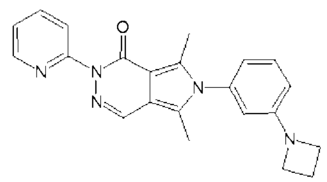

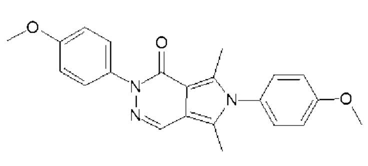
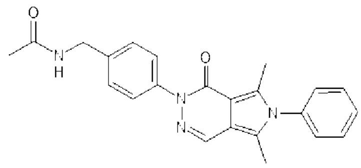
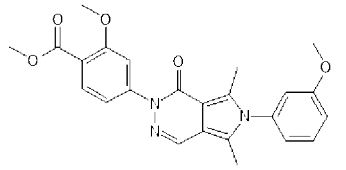

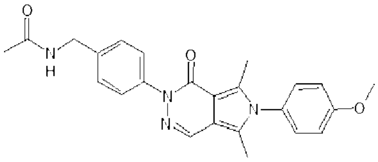
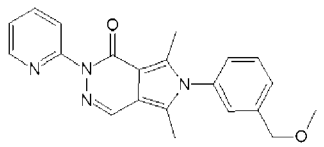
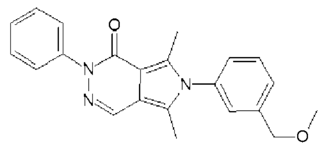

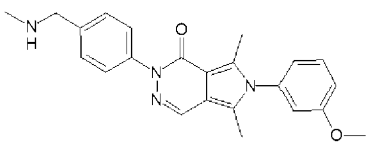
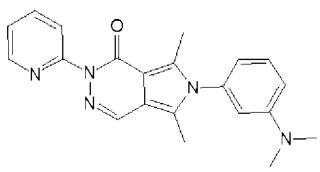
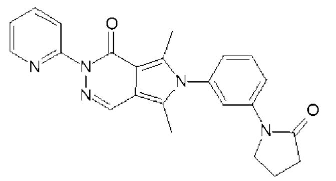

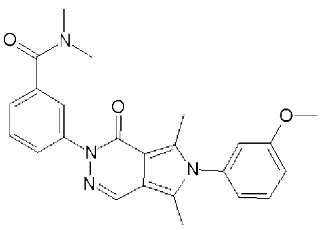
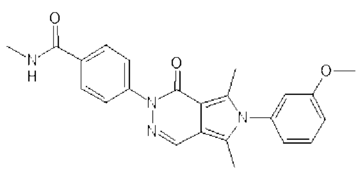
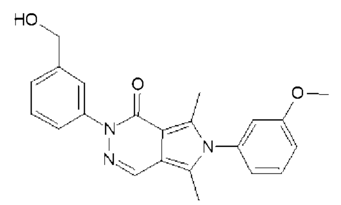

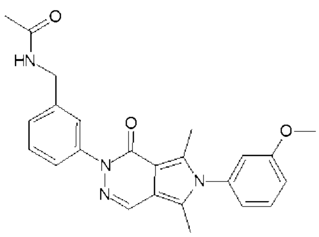
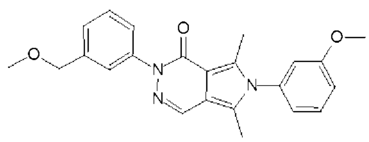
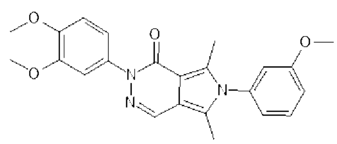

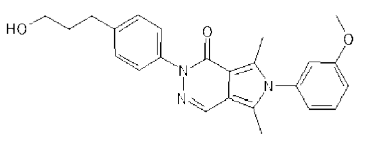
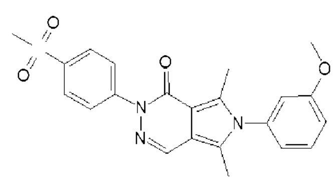
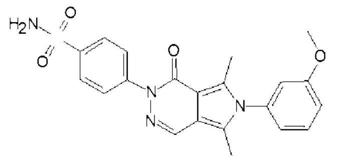

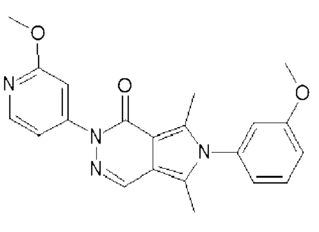
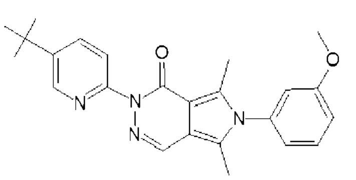
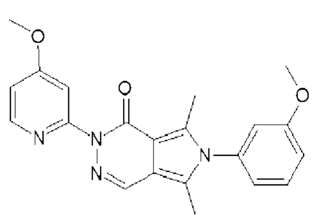

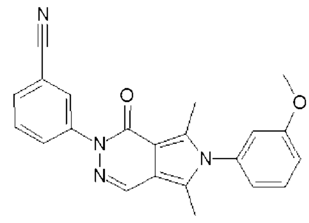
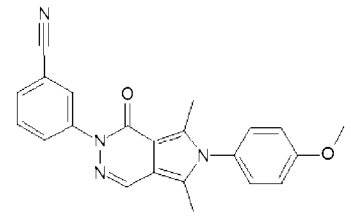
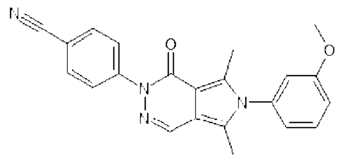

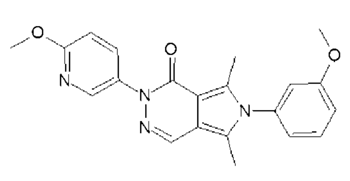
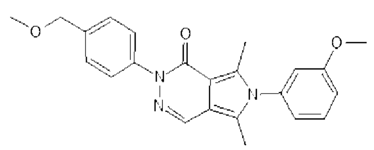
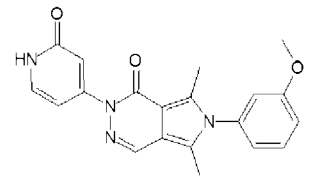

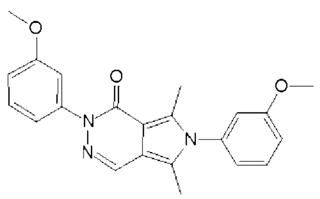
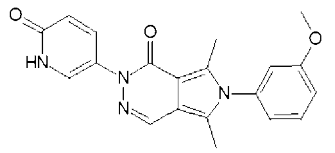
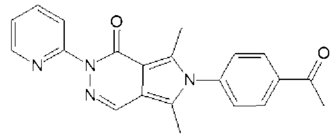

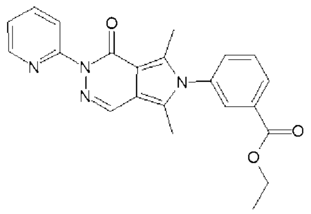
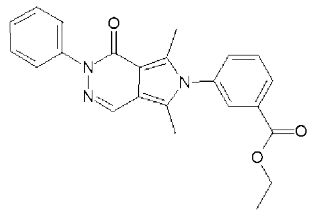
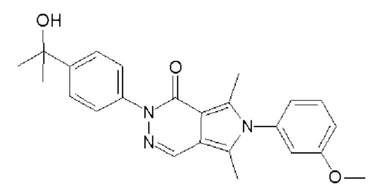

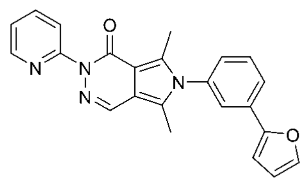
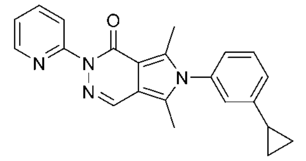
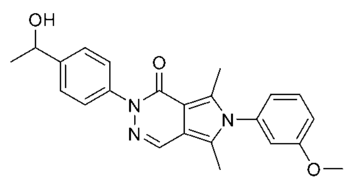

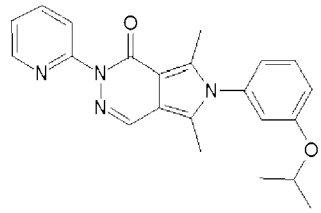
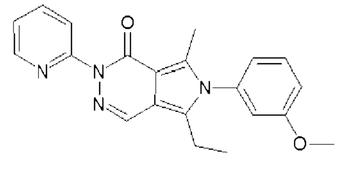
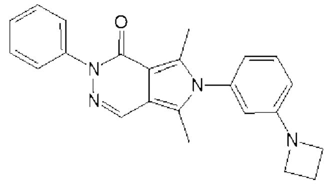

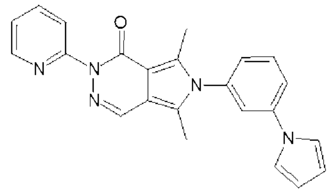
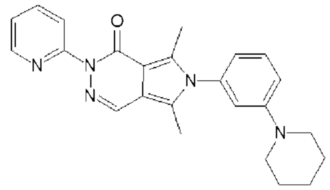
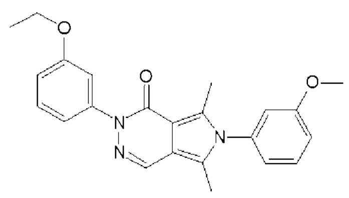

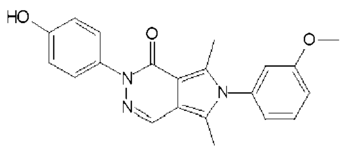
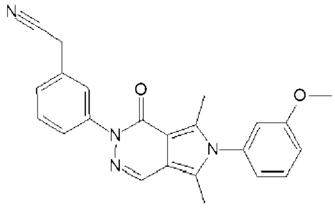
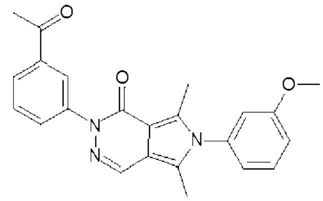

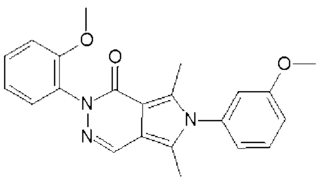
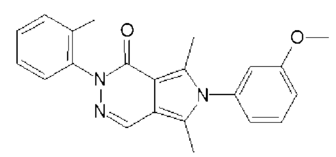
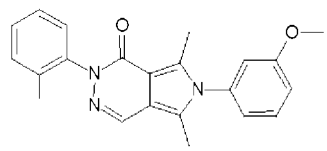

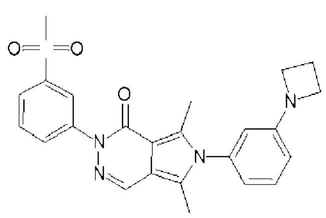
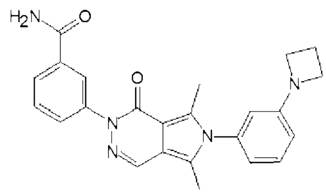
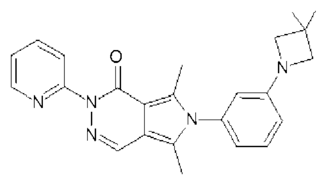

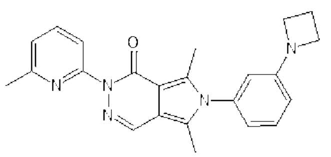
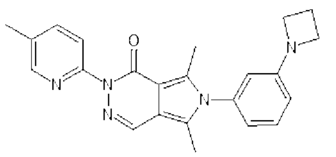
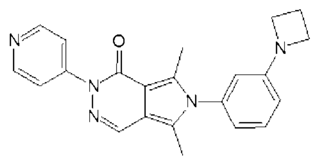

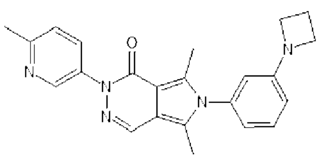
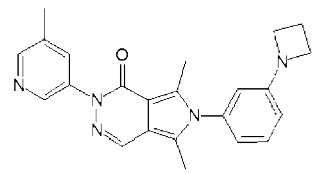
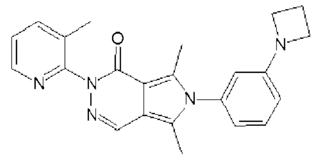

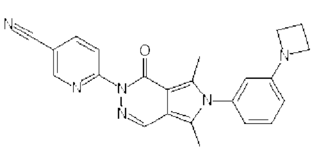
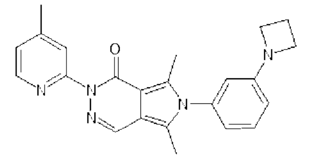
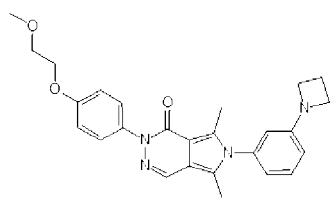

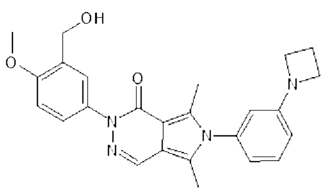
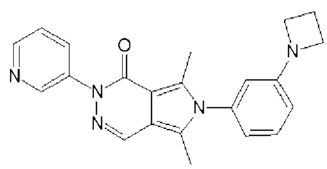
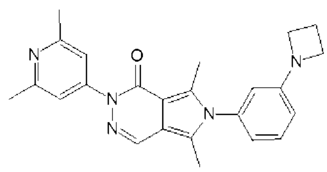

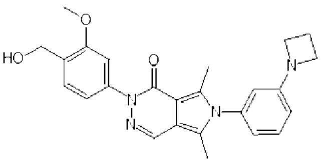
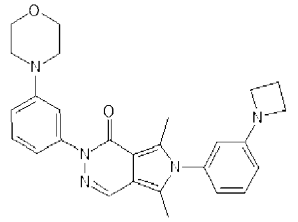
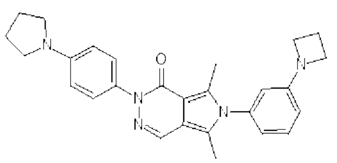
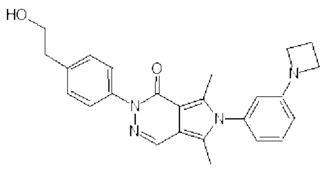
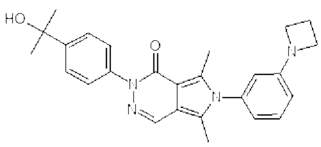
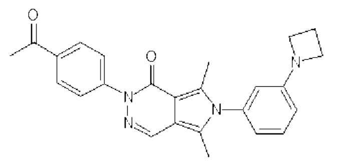
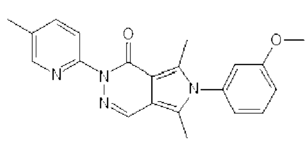
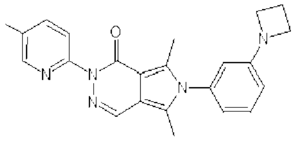
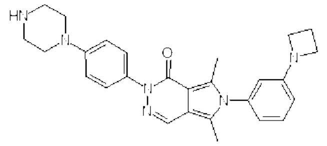
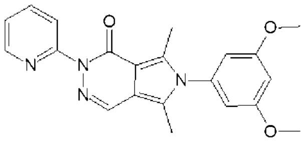
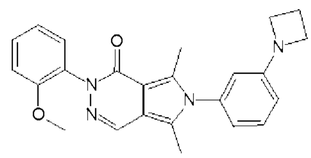
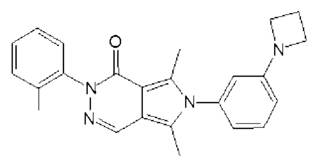
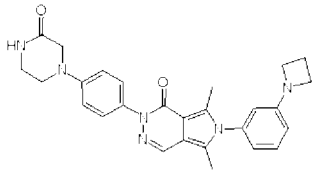
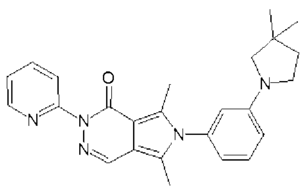
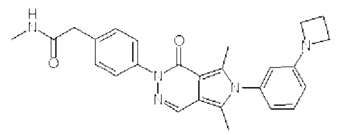
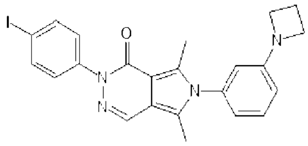
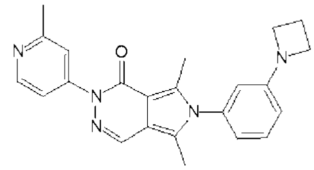
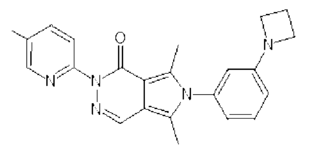
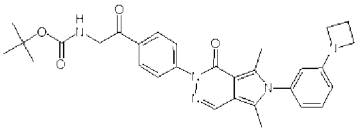
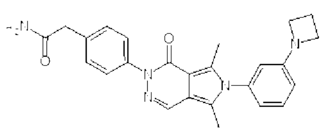
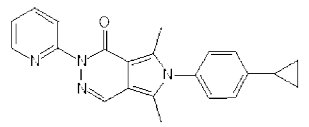
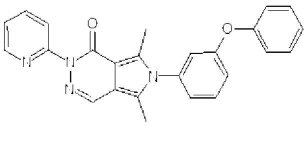
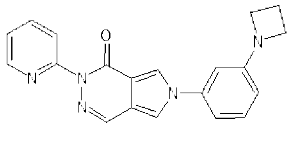
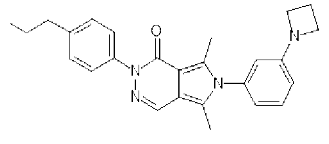

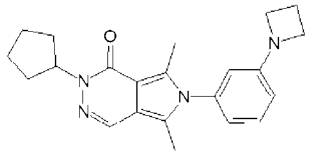
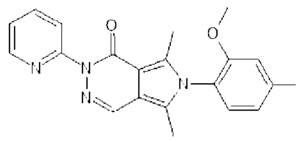
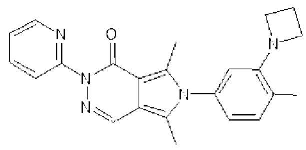

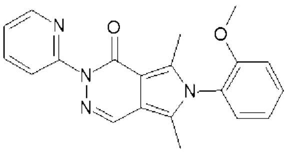
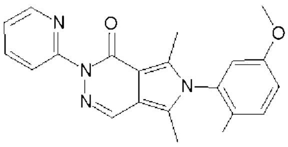
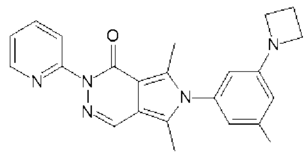

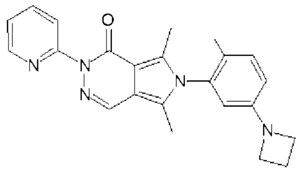
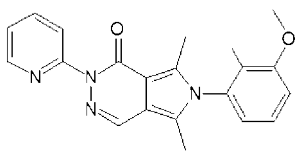
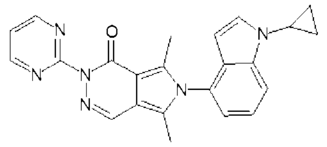

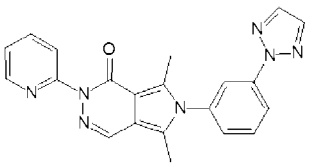
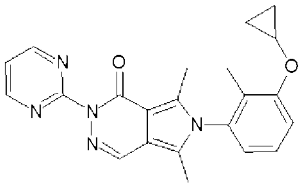
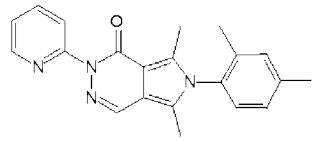

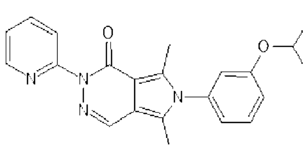
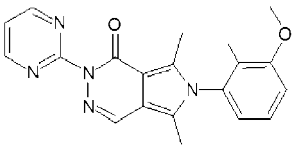
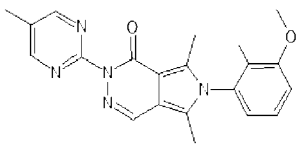

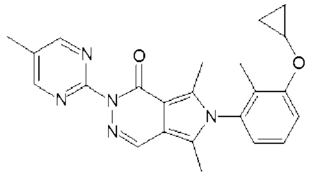
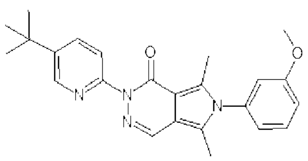
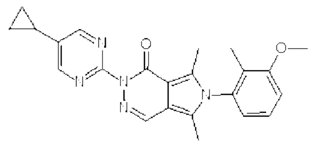

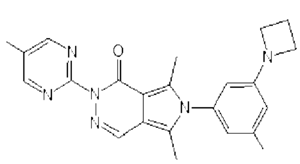
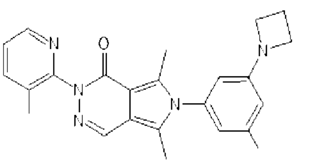
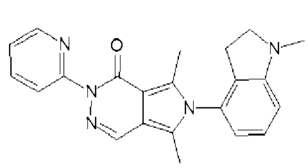

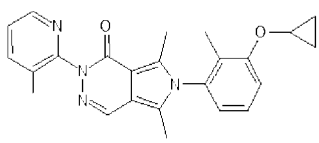
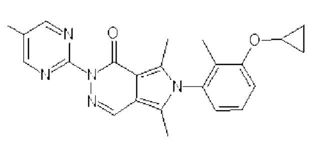
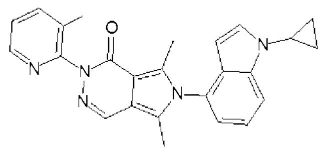

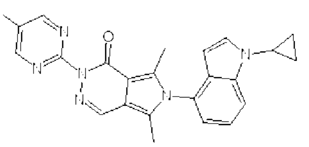
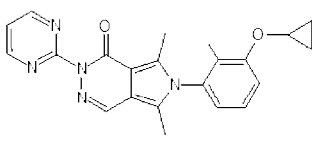
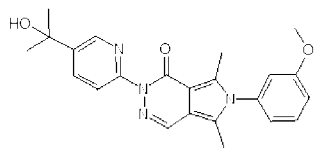

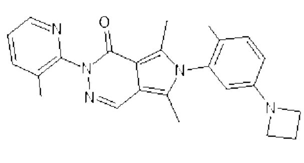 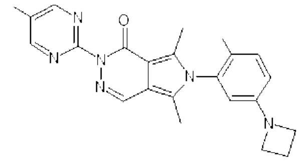 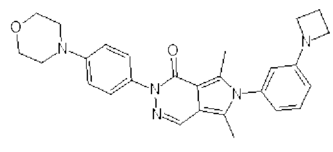

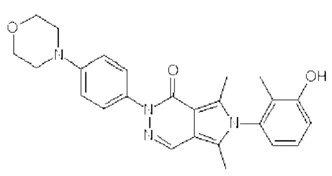 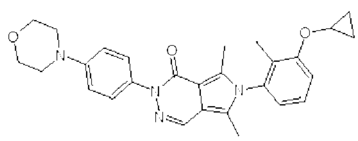 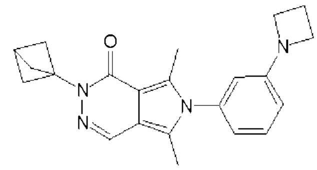

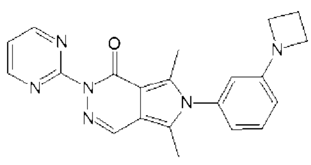 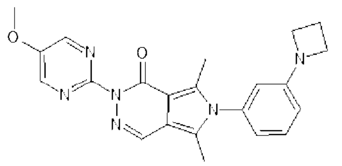 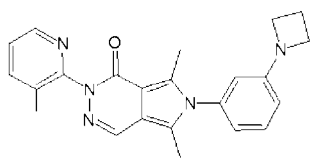

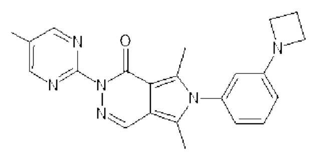 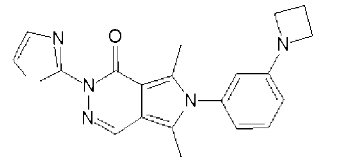 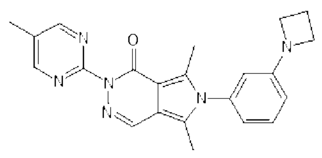

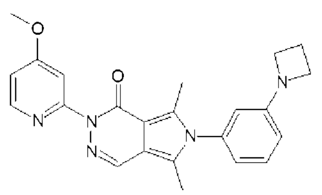 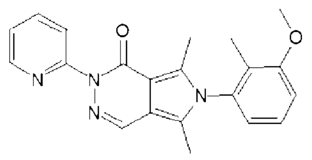 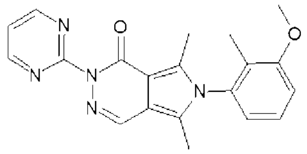

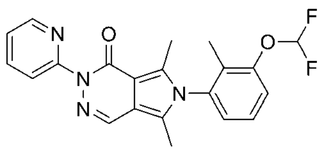 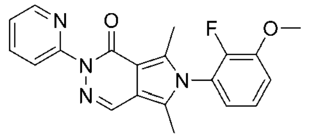 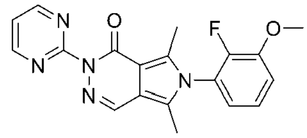

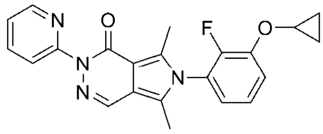 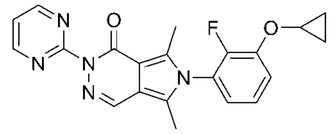 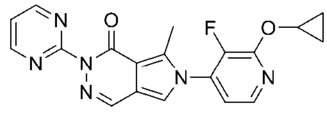

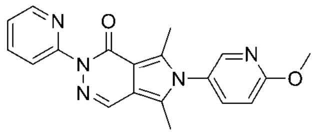 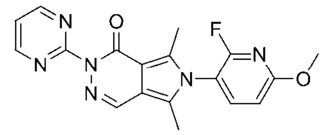 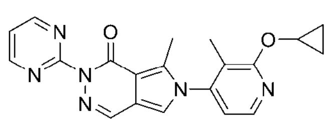

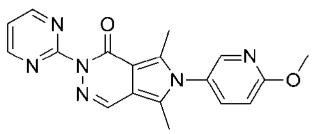 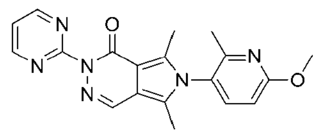 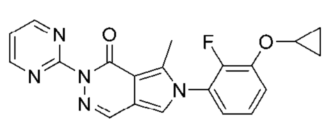 .

Preferably, the compound is one or more selected from the following list, as well as a pharmaceutically

acceptable acid or base addition salt thereof, a stereochemically isomeric form thereof or an *N*-oxide form thereof:

6-(3-(Azetidin-1-yl)phenyl)-5,7-dimethyl-2-(pyridin-2-yl)-2,6-dihydro-1*H*-pyrrolo [3,4-*d*]pyridazin-1-one
6-(3-(Azetidin-1-yl)-5-fluorophenyl)-5,7-dimethyl-2-(pyridin-2-yl)-2,6-dihydro-1*H*-pyrrolo[3,4-*d*]pyridazin-1-one
6-(5-(Azetidin-1-yl)-2-fluorophenyl)-5,7-dimethyl-2-(pyridin-2-yl)-2,6-dihydro-1*H*-pyrrolo[3,4-*d*]pyridazin-1-one
6-(1-Cyclopropyl-1*H*-indol-4-yl)-5,7-dimethyl-2-(pyrimidin-2-yl)-2,6-dihydro-1*H*-pyrrolo[3,4-*d*]pyridazin-1-one
6-(3-Cyclopropoxy-2-methylphenyl)-5,7-dimethyl-2-(pyrimidin-2-yl)-2,6-dihydro-1*H*-pyrrolo[3,4-*d*]pyridazin-1-one
6-(3-(Azetidin-1-yl)-5-fluorophenyl)-2-(5-fluoropyrimidin-2-yl)-5,7-dimethyl-2,6-dihydro-1*H*-pyrrolo[3,4-*d*]pyridazin-1-one
6-(3-(Azetidin-1-yl)-5-fluorophenyl)-2-(3-fluoropyridin-2-yl)-5,7-dimethyl-2,6-dihydro-1*H*-pyrrolo[3,4-*d*]pyridazin-1-one
6-(3-Cyclopropoxy-2-methylphenyl)-2-(5-fluoropyrimidin-2-yl)-5,7-dimethyl-2,6-dihydro-1*H*-pyrrolo[3,4-*d*]pyridazin-1-one
6-(1-Cyclopropyl-1*H*-indol-4-yl)-2-(5-fluoropyrimidin-2-yl)-5,7-dimethyl-2,6-dihydro-1*H*-pyrrolo[3,4-d]pyridazin-1-one
2-(5-(2-Hydroxypropan-2-yl)pyridin-2-yl)-6-(3-methoxyphenyl)-5,7-dimethyl-2,6-dihydro-1*H*-pyrrolo[3,4-d]pyridazin-1-one
6-(3-(Azetidin-1-yl)phenyl)-2-(3-fluoropyridin-2-yl)-5,7-dimethyl-2,6-dihydro-1*H*-pyrrolo[3,4-*d*]pyridazin-1-one
6-(3-(Azetidin-1-yl)phenyl)-2-(5-fluoropyrimidin-2-yl)-5,7-dimethyl-2,6-dihydro-1*H*-pyrrolo[3,4-*d*]pyridazin-1-one and
6-[3-(Cyclopropoxy)-2-fluoro-phenyl]-5,7-dimethyl-3-pyrimidin-2-yl-pyrrolo[3,4-*d*]pyridazin-4-one.

The above list of compounds can also be represented by the following skeletal formulae :

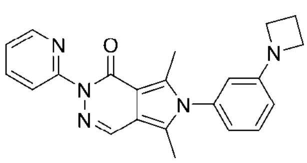
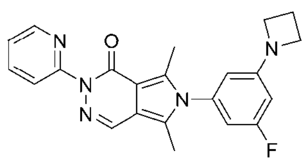
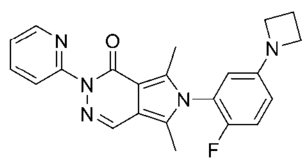
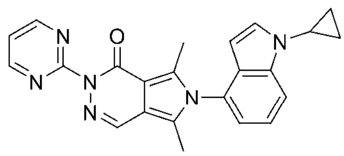
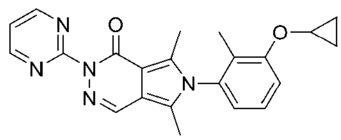
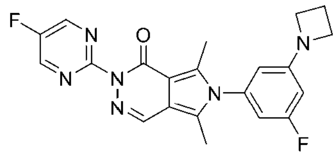
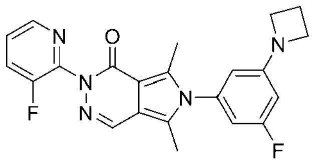
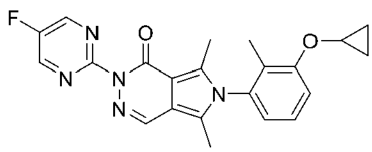
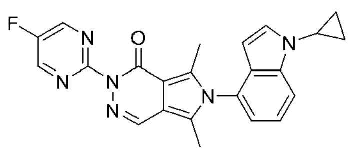
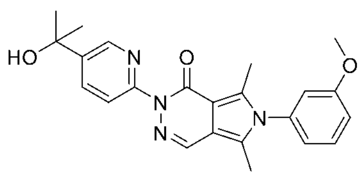
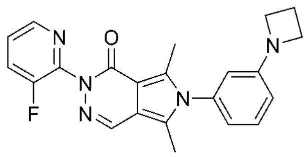
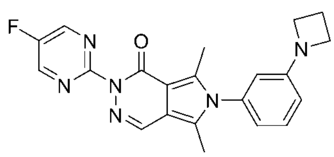
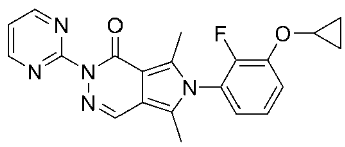
.

Preferably, the compound is one or more selected from the following list, as well as a pharmaceutically acceptable acid or base addition salt thereof, a stereochemically isomeric form thereof or an *N*-oxide form thereof:

6-(3-(Azetidin-1-yl)phenyl)-5,7-dimethyl-2-(pyridin-2-yl)-2,6-dihydro-1*H*-pyrrolo[3,4-*d*]pyridazin-1-one
6-(3-Cyclopropoxy-2-methylphenyl)-5,7-dimethyl-2-(pyrimidin-2-yl)-2,6-dihydro-1*H*-pyrrolo[3,4-*d*]pyridazin-1-one
6-(3-Cyclopropoxy-2-methylphenyl)-2-(5-fluoropyrimidin-2-yl)-5,7-dimethyl-2,6-dihydro-1*H*-pyrrolo[3,4-*d*]pyridazin-1-one and
6-[3-(Cyclopropoxy)-2-fluoro-phenyl]-5,7-dimethyl-3-pyrimidin-2-yl-pyrrolo[3,4-*d*]pyridazin-4-one.

The above list of compounds can also be represented by the following skeletal formulae:

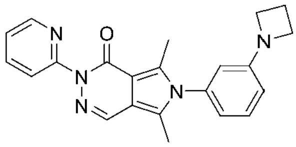
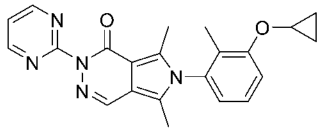
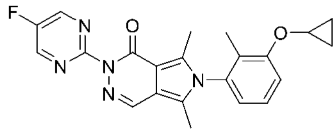

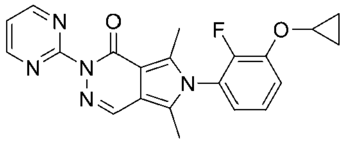
.

The compounds according to any statement above may exhibit metabotropic glutamate receptor 7 modulator activity.

The disclosed compounds also include all pharmaceutically acceptable isotopic variations, in which at least one atom is replaced by an atom having the same atomic number, but an atomic mass different from the atomic mass usually found in nature. Examples of isotopes suitable for inclusion in the disclosed compounds include, without limitation, isotopes of hydrogen, such as $^{2}H$ and $^{3}H$; isotopes of carbon, such as $^{11}C$, $^{13}C$ and $^{14}C$; isotopes of nitrogen, such as $^{15}N$; isotopes of oxygen, such as $^{17}O$ and $^{18}O$; isotopes of phosphorus, such as $^{31}P$, $^{32}P$ and $^{33}P$; isotopes of sulfur, such as $^{35}S$; isotopes of fluorine, such as $^{18}F$; isotopes of chlorine, such as $^{36}Cl$; and isotopes of iodine, such as $^{125}I$. The invention includes various isotopically labelled compounds as defined herein, for example those into which radioactive isotopes, such as $^{3}H$ and $^{14}C$, or those into which non-radioactive isotopes, such as $^{2}H$ and $^{13}C$ are present.

Such isotopically labelled compounds are useful in metabolic studies (with $^{14}C$), reaction kinetic studies (with for example $^{2}H$ or $^{3}H$), detection or imaging techniques, such as positron emission tomography (PET) or single-photon emission computed tomography (SPECT) including drug or substrate tissue distribution assays, or in radioactive treatment of patients. In particular, $^{11}C$, $^{18}F$, $^{15}O$ and $^{13}N$ or labelled compounds may be particularly desirable for PET studies for examining substrate receptor occupancy. Further, substitution with heavier isotopes, particularly deuterieum (e.g., $^{2}H$ or D) may afford certain therapeutic advantages resulting from greater metabolic stability, for example, increased in vivo half-life or reduced dosage requirements or an improvemet in therapeutic index. It is understood that deuterium in this context is regarded as a substituent of a compound of Formula (I) to (V). Isotopically-labelled compounds of Formula (I) to (V) can generally be prepared by conventional techniques known to those skilled in the art or by processes analogous to those described in the accompanying Examples using appropriate isotopically-labelled reagents in place of the non-labelled reagent previously employed.

In an aspect of the present invention there is provided a pharmaceutical composition comprising a compound according to any statement set out above. The pharmaceutical composition may further comprise a pharmaceutically acceptable carrier and/or excipient. The pharmaceutical composition may comprise a therapeutically effective amount of the compound according to any statement set out above.

In an aspect of the present invention there is provided a method of treating or preventing a condition in a mammal comprising administering to a mammal in need of such treatment or prevention, an effective amount of a compound/-composition according to any statement set out above.

The treatment or prevention may be affected or facilitated by the modulatory effect of a mGlu7 allosteric modulator such as a mGlu7 negative allosteric modulator.

The condition may be one or more of a central nervous system disorder or an otic disease or disorder or a pain disorder.

The central nervous system disorder may be post-traumatic stress disorder (PTSD).

The otic disease and disorder may be one or more of an inner ear impairment, age-related hearing impairment (presbycusis), Meniere's disease, sudden hearing loss, noise induced hearing loss, otitis media, autoimmune inner ear disease, acute tinnitus, chronic tinnitus, drug-induced hearing loss, hidden hearing loss, cisplatin-induced hearing loss, aminoglycosides-induced hearing loss, ototoxicity, central auditory processing disorder or vestibular disorder.

In a further aspect of the present invention, there is provided a method of treating, preventing, ameliorating,

controlling or reducing the risk of various neurological and psychiatric disorders associated with glutamate dysfunction in a mammal, comprising administering to a mammal in need of such treatment or prevention, an effective amount of a compound/composition according to any statement set out above. The treatment or prevention may be affected or facilitated by the modulatory effect of mGlu7 negative allosteric modulators.

Preferably, the methods are for the treatment or prevention of a condition in a human.

In a further aspect of the present invention, there is provided the compounds or compositions as set out in any statement above for use as a medicament.

In a further aspect of the present invention, there is provided the compounds or compositions as set out in any statement above for use in a method of treatment or prevention as defined in any statement set out above.

In a furher aspect of the present invention there is provided a use of a compound according to any statement set out above in the manufacture of a medicament for the treatment or prevention of a condition as defined in any statement set out above.

## DEFINITION OF TERMS

Listed below are definitions of various terms used in the specification and claims to describe the present invention.

For the avoidance of doubt it is to be understood that in this specification "($C_1$-$C_6$)" means a carbon radical having 1, 2, 3, 4, 5 or 6 carbon atoms. "($C_0$-$C_6$)" means a carbon radical having 0, 1, 2, 3, 4, 5 or 6 carbon atoms. In this specification "C" means a carbon atom, "N" means a nitrogen atom, "O" means an oxygen atom and "S" means a sulphur atom.

In the case where a subscript is the integer 0 (zero) the radical to which the subscript refers, indicates that the radical is absent, i.e. there is a direct bond between the radicals. In the case where a subscript is the integer 0 (zero) and the radical to which the subscript refers is alkyl, this indicates the radical is a hydrogen atom.

In this specification, unless stated otherwise, the term "bond" refers to a saturated covalent bond. When two or more bonds are adjacent to one another, they are assumed to be equal to one bond. For example, a radical -A-B-, wherein both A and B may be a bond, the radical is depicting a single bond.

In this specification, unless stated otherwise, the term "alkyl" includes both straight and branched chain alkyl radicals and may be methyl, ethyl, n-propyl, i-propyl, n-butyl, *i*-butyl, s-butyl, t-butyl, n-pentyl, i-pentyl, t-pentyl, neo-pentyl, n-hexyl, i-hexyl or t-hexyl. The term "($C_0$-$C_3$)alkyl" refers to an alkyl radical having 0, 1, 2 or 3 carbon atoms and may be methyl, ethyl, n-propyl or i-propyl.

In this specification, unless stated otherwise, the term "alkylene" includes both straight and branched difunctional saturated hydrocarbon radicals and may be methylene ($-CH_2-$), ethylene ($-CH_2-CH_2-$), n-propylene ($-CH_2-CH_2-CH_2-$), i-propylene ($-CH-(CH_3)-CH_2-$), n-butylene ($-CH_2-CH_2-CH_2-CH_2-$), i-butylene ($-CH_2-CH-(CH_3)-CH_2-$), t-butylene ($-CH_2-C-(CH_3)-CH_2-$), n-pentylene ($-CH_2-CH_2-CH_2-CH_2-CH_2-$), i-pentylene ($-CH_2-CH(CH_3)-CH_2-CH_2-$), neo-pentylene ($-CH_2-C(CH_3)_2-CH_2-$), n-hexylene ($-CH_2-CH_2-CH_2-CH_2-CH_2-CH_2-$), i-hexylene ($-CH_2-CH-(CH_3)-CH_2-CH_2-CH_2-$) or neo-hexylene ($-CH_2-C(CH_3)_2-CH_2-CH_2-$). The term "O-($C_1$-$C_6$)alkylene-aryl" refers to a an alkyl chain having 0, 1, 2, 3, 4, 5 or 6 carbon atoms between an oxygen atom and an aryl group.

In this specification, unless stated otherwise, the term "cycloalkyl" refers to an optionally substituted carbocycle containing no heteroatoms, including mono-, bi-, and tricyclic saturated carbocycles, as well as fused ring systems. Such fused ring systems can include one ring that is partially or fully unsaturated such as a benzene ring to form fused ring systems such as benzo- fused carbocycles. Cycloalkyl includes such fused ring systems as spirofused ring systems. Examples of cycloalkyl include cyclopropyl, cyclobutyl, cyclopentyl, cyclohexyl, bicyclo[1.1.1]pentanyl, decahydronaphthalene, adamantane, indanyl, fluorenyl and 1,2,3,4-tetrahydronaphthalene and the like. The term "($C_3$-$C_7$)cycloalkyl" may be cyclopropyl, cyclobutyl, cyclopentyl, cyclohexyl, cycloheptyl and the like.

In this specification, unless stated otherwise, the term "alkenyl" includes both straight and branched chain alkenyl radicals. The term "($C_2$-$C_6$)alkenyl" refers to an alkenyl radical having 2 to 6 carbon atoms and one or two double bonds, and may be, but is not limited to vinyl, allyl, propenyl, i-propenyl, butenyl, i-butenyl, crotyl, pentenyl, i-pentenyl or hexenyl.

In this specification, unless stated otherwise, the term "alkenylene" includes both straight and branched chain disubstituted alkenyl radicals. The term "($C_2$-$C_6$)alkenylene" refers to an alkenylene radical having 2 to 6 carbon atoms and one or two double bonds, and may be, but is not limited to vinylene, allylene, propenylene, i-propenylene, butenylene, i-butenylene, crotylene, pentenylene, i-pentenylene or hexenylene.

In this specification, unless stated otherwise, the term "alkynyl" includes both straight and branched chain alkynyl radicals. The term ($C_2$-$C_6$)alkynyl having 2 to 6 carbon atoms and one or two triple bonds, and may be, but is not limited to ethynyl, propargyl, butynyl, i-butynyl, pentynyl, i-pentynyl or hexynyl.

In this specification, unless stated otherwise, the term "alkynylene" includes both straight and branched chain disubstituted alkynylene radicals. The term ($C_2$-$C_6$)alkynylene having 2 to 6 carbon atoms and one or two triple bonds, and may be, but is not limited to ethynylene, propargylene, butynylene, i-butynylene, pentynylene, i-pentynylene or hexynylene.

The term "aryl" refers to an optionally substituted monocyclic or bicyclic hydrocarbon ring system containing at least one unsaturated aromatic ring. Examples and suitable values of the term "aryl" are phenyl, naphthyl, 1,2,3,4-tetrahydronaphthyl, indyl, indenyl and the like.

In this specification, unless stated otherwise, the term "heteroaryl" refers to an optionally substituted monocyclic or bicyclic unsaturated, aromatic ring system containing at least one heteroatom selected independently from N, O or S. Examples of "heteroaryl" may be, but are not limited to benzimidazolyl, benzisothiazolyl benzisoxazolyl, benzofuryl, benzopyrazolyl, benzothiazolyl, benzothiophenyl, benzotriazolyl, benzoxazolyl, furazanyl, furyl, imidazolonyl, imidazolyl, imidazopyridazinyl, imidazopyridyl, indolyl, isoindolyl, isoquinolinyl, isothiazolyl, isoxazolyl, naphthyridinyl, oxadiazolyl, oxazolonyl, oxazolopyridazinyl, oxazolopyridyl, oxazolyl, phtalazinyl, pteridinyl, purinyl, pyrazinyl, pyrazolopyridinyl, pyrazolyl, pyridazinyl, pyridonyl, pyridyl, pyrimidyl, pyrrolyl, quinazolyl, quinolyl, quinoxalinyl, tetrahydrotriazolopyridyl, tetrahydrotriazolopyrimidinyl, tetrazolyl, thiadiazolyl, thiazolonyl, thiazolopyridazinyl, thiazolopyridyl, thiazolyl, thienyl, thionaphthyl, triazinyl and triazolyl.

In this specification, unless stated otherwise, the term "alkylene-aryl", "alkylene-heteroaryl" and "alkylene-cycloalkyl" refers respectively to a substituent that is attached via the alkyl radical to an aryl, heteroaryl or cycloalkyl radical, respectively. The term "($C_1$-$C_6$)alkylene-aryl" includes aryl-$C_1$-$C_6$-alkyl radicals such as benzyl, 1-phenylethyl, 2-phenylethyl, 1-phenylpropyl, 2-phenylpropyl, 3-phenylpropyl, 1-naphthylmethyl and 2-naphthylmethyl. The term "($C_1$-$C_6$) alkylene-heteroaryl" includes heteroaryl-$C_1$-$C_6$-alkyl radicals, wherein examples of heteroaryl are the same as those illustrated in the above definition, such as 2-furylmethyl, 3-furylmethyl, 2-thienylmethyl, 3-thienylmethyl, 1-imidazolylmethyl, 2-imidazolylmethyl, 3-imidazolylmethyl, 2-oxazolylmethyl, 3-oxazolylmethyl, 2-thiazolylmethyl, 3-thiazolylmethyl, 2-pyridinylmethyl, 3-pyridinylmethyl, 4-pyridinylmethyl, 1-quinolylmethyl and the like.

In this specification, unless stated otherwise, the term "heterocycle" refers to an optionally substituted, monocyclic, bicyclic or tricyclic saturated, partially saturated or unsaturated ring system containing at least one heteroatom selected independently from N, O and S. Bicyclic or tricyclic ring systems may be formed by annelation of two or more rings, by a bridging atom (e.g. O, S, N) or by a bridging group (e.g. alkylene). Examples of heterocyclic moieties include, but are not limited to: azetidinyl, dihydrofuranyl, dihydrothienyl, dioxolanyl, 1,1-dioxo-thiomorpholinyl, imidazolidinyl, imidazolinyl, isothiazolinyl, isoxazolidinyl, isoxazolinyl, morpholinyl, oxazolidinyl, oxazolinyl, oxetanyl, piperazinonyl, piperazinyl, piperidinonyl, piperidinyl, pyranyl, pyrrolidinonyl, pyrrolidinyl, pyrrolinyl, tetrahydrofuranyl, tetrahydropyranyl, tetrahydrothiopyranyl, thiazolidinyl, thiazolinyl, thiomorpholinyl, thiopyranyl, triazolinyl, and the corresponding benzannulated heterocycles (e.g. dihydrobenzofuranyl, dihydrobenzothiophenyl, dihydrobenzoxazinyl, dihydrofuropyridinyl, dihydroquinolinyl, dihydrothienopyridinyl, indolinyl, pyrrolopyridinyl, tetrahydroquinolinyl, tetrahydroquinoxalinyl, and the like).

In this specification, unless stated otherwise, a 5- or 6-membered ring containing one or more atoms independently selected from C, N, O and S, includes aromatic and heteroaromatic rings as well as carbocyclic and heterocyclic rings which may be saturated or unsaturated. Such rings include spirocyclic and bridged bicyclic systems. Examples of such rings may be, but are not limited to dihydrofuranyl, dihydrothienyl, dioxolanyl, 1,1-dioxo-thiomorpholinyl, furazanyl, furyl, imidazolidinyl, imidazolinyl, imidazolonyl, imidazolyl, isothiazolinyl, isothiazolyl, isoxazolidinyl, isoxazolinyl, isoxazolyl, morpholinyl, oxadiazolyl, oxazolidinyl, oxazolinyl, oxazolonyl, oxazolyl, phenyl, piperazinonyl, piperazinyl, piperidinonyl, piperidinyl, pyranyl, pyrazinyl, pyrazolyl, pyridazinyl, pyridonyl, pyridyl, pyrimidyl, pyrrolidinonyl, pyrrolidinyl, pyrrolinyl, pyrrolyl, tetrahydrofuranyl, tetrahydropyranyl, tetrahydrothiopyranyl, tetrazolyl, thiadiazolyl, thiazolidinyl, thiazolinyl, thiazolonyl, thiazolyl, thienyl, thiomorpholinyl, thiopyranyl, triazolinyl, triazinyl, triazolyl, cyclopentyl, cyclopentenyl, cyclohexyl and cyclohexenyl.

In this specification, unless stated otherwise, a 3- to 10-membered ring containing one or more atoms independently selected from C, N, O and S, includes aromatic and heteroaromatic rings as well as carbocyclic and heterocyclic rings which may be saturated or unsaturated. Examples of such rings may be, but are not limited to azetidinyl, benzimidazolyl, benzisothiazolyl benzisoxazolyl, benzofuryl, benzopyrazolyl, benzothiazolyl, benzothiophenyl, benzotriazolyl, benzoxazolyl, dihydrofuranyl, dihydrothienyl, dioxolanyl, 1,1-dioxo-thiomorpholinyl, furazanyl, furyl, imidazolidinyl, imidazolinyl, imidazolonyl, imidazolyl, imidazopyridazinyl, imidazopyridyl, indolyl, isoindolyl, isoquinolinyl, isothiazolinyl, isothiazolyl, isoxazolidinyl, isoxazolinyl, isoxazolyl, morpholinyl, naphthyl, naphthyridinyl, oxadiazolyl, oxazolidinyl, oxazolinyl, oxazolonyl, oxazolopyridazinyl, oxazolopyridyl, oxazolyl, oxetanyl, phenyl, piperazinonyl, piperazinyl, piperidinonyl, piperidinyl, phtalazinyl, pteridinyl, purinyl, pyranyl, pyrazinyl, pyrazolopyridinyl, pyrazolyl, pyridazinyl, pyridonyl, pyridyl, pyrimidyl, pyrrolidinonyl, pyrrolidinyl, pyrrolinyl, pyrrolyl, quinazolyl, quinolyl, quinoxalinyl, tetrahydrofuranyl, tetrahydropyranyl, tetrahydrothiopyranyl, tetrahydrotriazolopyridyl, tetrahydrotriazolopyrimidinyl, tetrazolyl, thiadiazolyl, thiazolidinyl, thiazolinyl, thiazolonyl, thiazolopyridazinyl, thiazolopyridyl, thiazolyl, thienyl, thiomorpholinyl, thionaphthyl, thiopyranyl, triazolinyl, triazinyl, triazolyl, cyclopropyl, cyclobutyl, cyclopentyl, cyclopentenyl, cyclohexyl, cyclohexenyl, cycloheptyl, cycloheptenyl, cyclooctyl and cyclooctenyl.

In this specification, unless stated otherwise, the term "halo" or "halogen" may be fluoro, chloro, bromo or iodo.

In this specification, unless stated otherwise, the term "haloalkyl" means an alkyl radical as defined above, substituted with one or more halo radicals. The term "($C_1$-$C_6$)haloalkyl" may include, but is not limited to, fluoromethyl, difluoromethyl, trifluoromethyl, fluoroethyl and difluoroethyl. The term "O-$C_1$-$C_6$-haloalkyl" may include, but is not limited

to, fluoromethoxy, difluoromethoxy, trifluoromethoxy and fluoroethoxy.

In this specification, unless stated otherwise, the term "cyanoalkyl" means an alkyl radical as defined above, substituted with one or more cyano.

In this specification, unless stated otherwise, the term "optionally substituted" refers to radicals further bearing one or more substituents which may be, acyl, $(C_1-C_6)$alkyl, -$(C_1-C_6)$haloalkyl, -$(C_3-C_7)$cycloalkyl, -$(C_1-C_6)$alkylene-$(C_3-C_7)$cycloalkyl, -$(C_3-C_7)$cycloalkyl-$(C_1-C_6)$alkylene, -$(C_0-C_6)$alkylene-$(C_3-C_7)$spiroalkyl-$(C_0-C_6)$alkylene, hydroxy, $(C_1-C_6)$alkylene-oxy, dimethylamino$(C_1-C_3)$alkyl, mercapto, aryl, heterocycle, heteroaryl, $(C_1-C_6)$alkylene-aryl, $(C_1-C_6)$ alkylene-heterocycle, $(C_1-C_6)$alkylene-heteroaryl, halogen, haloalkyl, trifluoromethyl, pentafluoroethyl, haloalkoxy, cyano, cyanomethyl, nitro, amino, amido, amidinyl, oxo, carboxyl, carboxamide, $(C_1-C_6)$alkylene-oxycarbonyl, carbamate, sulfonamide, ester or sulfonyl.

In this specification, unless stated otherwise, the term "independently" means that where more than one substituent is selected from a number of possible substituents, those substituents may be the same or different.

In this specification, unless stated otherwise, the term "solvate" refers to a complex of variable stoichiometry formed by a solute (e.g. a compound of Formula (I)) and a solvent. The solvent is a pharmaceutically acceptable solvent such as water; such solvent may not interfere with the biological activity of the solute.

In this specification, unless stated otherwise, the term "salt" refers to an acid addition or base addition salt of a compound of the invention. "Salts" include in particular "pharmaceutically acceptable salts".

The pharmaceutically acceptable salts of the invention can be synthesized from a basic or acidic moiety, by conventional chemical methods. When both a basic and an acid group are present in the same molecule, the compounds of the invention may also form internal salts, e.g., zwitterionic molecules.

In this specification, unless stated otherwise, certain compounds may exist in one or more particular geometric, optical, enantiomeric, diastereoisomeric, epimeric, stereoisomeric, tautomeric, conformational, or anomeric forms, including, but not limited to, *cis*- and *trans*-forms; *E*- and Z-forms; *endo*- and exo-forms, *R-, S-,* and meso-forms; *D*- and *L*-forms; d- and *l*-forms; (+) and (-) forms; keto-, enol-, and enolate-forms; $\alpha$- and $\beta$-forms; axial and equatorial forms; and combinations thereof, collectively referred to as "isomers" or "isomeric forms".

For example, the radical

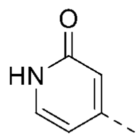

is a tautomer of

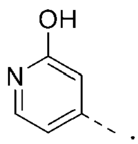

The term "isomer" includes compounds with one or more isotopic substitutions. For example, H may be in any isotopic form, including, but not limited to, $^1H$, $^2H$ (D), and $^3H$ (T); C may be in any isotopic form, including, but not limited to, $^{12}C$, $^{13}C$, $^{14}C$; O may be in any isotopic form, including, but not limited to, $^{16}O$ and $^{18}O$; and the like. F may be in any isotopic form, including, but not limited to, $^{19}F$ and $^{18}F$; and the like.

In this specification, unless stated otherwise, the term "negative allosteric modulator of mGlu7" or "allosteric modulator of mGlu7" refers also to a pharmaceutically acceptable acid or base addition salt thereof, a stereochemically isomeric form thereof or an N-oxide form thereof.

PHARMACEUTICAL COMPOSITIONS

Allosteric modulators of mGlu7 described herein, and the pharmaceutically acceptable salts, solvates and hydrates thereof can be used in pharmaceutical preparations in combination with a pharmaceutically acceptable carrier or diluent. Suitable pharmaceutically acceptable carriers include inert solid fillers or diluents and sterile aqueous or organic solutions. The allosteric modulators of mGlu7 will be present in such pharmaceutical compositions in amounts sufficient to provide the desired dosage amount in the range described herein. Techniques for formulation and administration of the compounds of the instant invention can be found in Remington: the Science and Practice of Pharmacy, 19th edition, Mack Publishing Co., Easton, PA (1995).

The amount of allosteric modulators of mGlu7, administered to the subject will depend on the type and severity of

the disease or condition and on the characteristics of the subject, such as general health, age, sex, body weight and tolerance to drugs. The skilled artisan will be able to determine appropriate dosages depending on these and other factors. Effective dosages for commonly used CNS drugs are well known to the skilled person. The total daily dose usually ranges from about 0.05 - 2000 mg.

The present invention relates to pharmaceutical compositions which provide from about 0.01 to 1000 mg of the active ingredient per unit dose. The compositions may be administered by any suitable route. For example, orally in the form of capsules and the like, parenterally in the form of solutions for injection, topically in the form of onguents or lotions, ocularly in the form of eye-drops, rectally in the form of suppositories, intranasally or transcutaneously in the form of a delivery system like patches,

For oral administration, the allosteric modulators of mGlu7 thereof can be combined with a suitable solid or liquid carrier or diluent to form capsules, tablets, pills, powders, syrups, solutions, suspensions and the like.

The tablets, pills, capsules, and the like contain from about 0.01 to about 99 weight percent of the active ingredient and a binder such as gum tragacanth, acacias, corn starch or gelatin; excipients such as dicalcium phosphate; a disintegrating agent such as corn starch, potato starch, alginic acid, a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

For parenteral administration the disclosed allosteric modulators of mGlu7, or salts thereof, can be combined with sterile aqueous or organic media to form injectable solutions or suspensions. For example, solutions in sesame or peanut oil, aqueous propylene glycol and the like can be used, as well as aqueous solutions of water-soluble pharmaceutically-acceptable salts of the compounds. Dispersions can also be prepared in glycerol, liquid polyethylene glycols and mixtures thereof in oils. Under ordinary conditions of storage and use, these preparations contain a preservative to prevent the growth of microorganisms.

In addition to the formulations described previously, the compounds may also be formulated as a depot preparation. Such long acting formulations may be administered for example, by subcutaneously implantation or by intramuscular injection. Thus, for example, the compounds may be formulated as an emulsion in an acceptable oil, or ion exchange resins, or as sparingly soluble derivatives, for example, as sparingly soluble salts.

Preferably disclosed allosteric modulators of mGlu7 or pharmaceutical formulations containing these compounds are in unit dosage form for administration to a mammal. The unit dosage form can be any unit dosage form known in the art including, for example, a capsule, an IV bag, a tablet, or a vial. The quantity of active ingredient in a unit dose of composition is an effective amount and may be varied according to the particular treatment involved. It may be appreciated that it may be necessary to make routine variations to the dosage depending on the age and condition of the patient. The dosage will also depend on the route of administration which may be by a variety of routes including oral, aerosol, rectal, transdermal, subcutaneous, intravenous, intramuscular, intraperitoneal and intranasal.

## METHODS OF SYNTHESIS

The compounds according to the invention, in particular the compounds according to the Formula (I) to (V), may be prepared by methods known in the art of organic synthesis as set forth in part by the following synthesis schemes. In all of the schemes described below, it is well understood that protecting groups for sensitive or reactive groups are employed where necessary in accordance with general principles of chemistry. Protecting groups are manipulated according to standard methods of organic synthesis (Green T.W. and Wuts P.G.M., (1991) Protecting Groups in Organic Synthesis, John Wiley & Sons). These groups are removed at a convenient stage of the compound synthesis using methods that are readily apparent to those skilled in the art. The selection of process as well as the reaction conditions and order of their execution shall be consistent with the preparation of compounds of Formula (I) to (V).

The compounds according to the invention may be represented as a mixture of enantiomers, which may be resolved into the individual pure R- or S-enantiomers. If for instance, a particular enantiomer is required, it may be prepared by asymmetric synthesis or by derivation with a chiral auxiliary, where the resulting diastereomeric mixture is separated and the auxiliary group cleaved to provide the pure desired enantiomers. Alternatively, where the molecule contains a basic functional group such as an amino or an acidic functional group such as carboxyl, this resolution may be conveniently performed by fractional crystallization from various solvents as the salts of an optical active acid or by other methods known in the literature (e.g. chiral column chromatography).

Resolution of the final product, an intermediate or a starting material may be performed by any suitable method known in the art (Eliel E. L., Wilen S. H. and Mander L .N. (1984) Stereochemistry of Organic Compounds, Wiley-Interscience).

Many of the heterocyclic compounds of the invention can be prepared using synthetic routes well known in the art (Katrizky A. R. and. Rees C. W. (1984) Comprehensive Heterocyclic Chemistry, Pergamon Press).

The product from the reaction can be isolated and purified by employing standard techniques, such as extraction, chromatography, recrystallization and distillation.

The compounds of the invention may be prepared by general route of synthesis as disclosed in the following methods.

In one embodiment of the present invention, compounds of Formula (I) may be prepared according to the synthetic sequence illustrated in Scheme 1. $\alpha$, $\gamma$-Diketo derivative **g2** may be prepared by reacting the corresponding $\beta$-ketoester derivative **g1** with haloacetyl derivatives in the presence of a base, such as, for example, sodium hydride and the like, in an appropriate solvent, such as, for example, THF and the like, at an appropriate temperature. Intermediate **g2** can then be converted into *N*-aryl pyrrole derivatives **g4** by reaction with a suitable aniline derivative **g3,** in the presence of a catalyst such as, for example an acid, such as, for example acetic acid, p-toluene sulfonic acid and the like, in an appropriate solvent such as, for example toluene, acetonitrile and the like, at an appropriate temperature (see for example Bioorg. Med. Chem. Lett. 2010, 20(1), 189; Bioorg. Med. Chem. Lett. 2008, 16(23), 10001).

Pyrrole derivative **g5** can then be prepared from the derivative **g4** by carbonylation reactions such as, for example but not limited to, Vilsmeier reactions, known in the art of organic synthesis, using adapted reagents in suitable solvents and at an appropriate temperature (see for example Tetrahedron 2006, 62, 6018).

Intermediate **g5** can be finally condensed with suitable phenylhydrazine derivative **g6** in the presence of a catalyst such as, for example an acid, such as, for example acetic acid, p-toluene sulfonic acid and the like, in a polar solvent such as EtOH, dioxane and the like, at an appropriate temperature, yielding final compound **g7** (see for example *B*ioorg. Med. Chem. 2010, 18(1), 202).

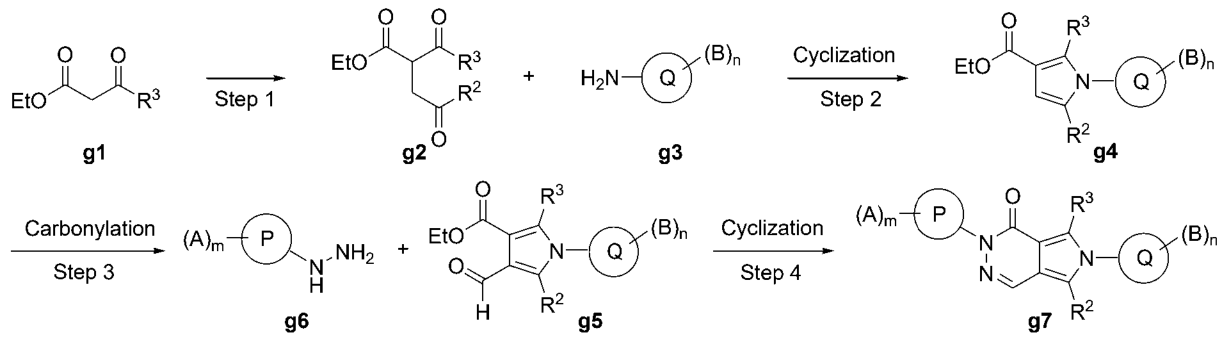


g1 g2 g3 g4 g6 g5 g7

**Scheme 1**

Similarly, final compounds **g7** may be prepared according to the synthetic sequence illustrated in Scheme 2. Intermediate **g5** can be first converted into the intermediate **g8** by reaction with hydrazine in the presence of a catalyst such as, for example an acid, such as, for example acetic acid, para-toluene sulfonic acid and the like, in a polar solvent such as EtOH, dioxane and the like, at an appropriate temperature (see for example Bioorg. Med. Chem. Lett. 2004, 14, 1295). Then final compound **g7** can be obtained by methods such as Chan-Lam coupling reactions, known in the art of organic synthesis, mediated by copper-complex catalysts such as $Cu(OAc)_2$ and the like, in the presence of a base such as triethylamine, pyridine and the like, in reaction-inert solvents such as DMF or dioxane and the like, at an appropriate temperature.

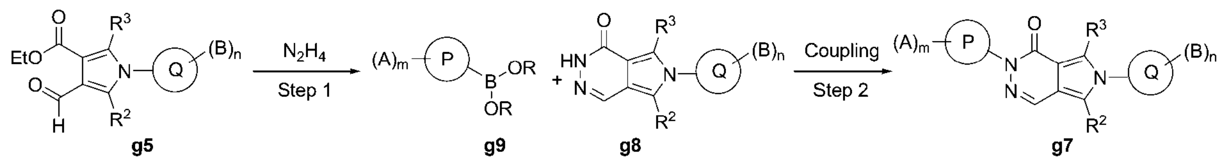


g5 g9 g8 g7

**Scheme 2**

In another specific aspect of Formula (I), intermediate derivatives **g5** used for the preparation of the final compounds **g7,** can be prepared according to Scheme 3. Intermediate derivatives **g10,** prepared according to Scheme 1 and wherein X represents any kind of functional group, such as but not limited to, halogen, cyano, ester, ether, carboxylic acid, carbonyl; may be converted to intermediate **g5** by suitable reactions known from people skilled in the art of organic synthesis, such as but not limited to, cross coupling reactions such as, Buchwald, Heck, Suzuki, Ullmann, Stille coupling

reactions, mediated by palladium-complex catalyst such as $Pd(PPh_3)_4$, $PdCl_2$(dppf) and the like, or copper-complex catalysts such as CuI, $Cu(OAc)_2$ and the like, in reaction-inert solvents such as DMF or dioxane and the like, at an appropriate temperature.

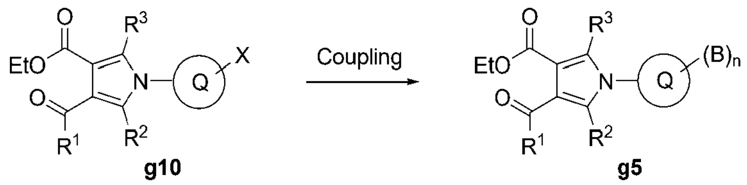


g10 g5

## Scheme 3

Similarly, final compounds **g7** may be prepared according to the synthetic sequence illustrated in Scheme 4. Intermediate **g11** prepared according to Schemes 1 or 2, and wherein X is a functional group, such as but not limited to, halogen, cyano, ester, ether, carboxylic acid, carbonyl; may be converted into final compound **g7** by suitable reactions as described for Scheme 3.

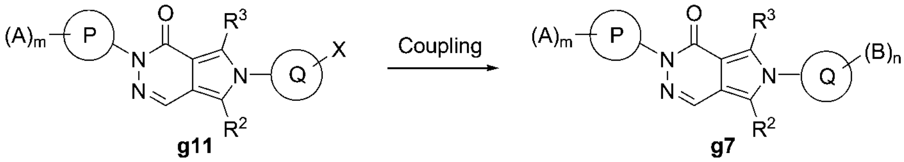


g11 g7

## Scheme 4

In another specific aspect of Formula (I), final compounds **g7** can be prepared according to Scheme 5. Intermediate **g12** prepared according to Schemes 1 or 2, and wherein X is a functional group, such as but not limited to, halogen, cyano, ester, ether, carboxylic acid, carbonyl; may be converted into final compound **g7** by suitable cross coupling reactions as described for Scheme 3.

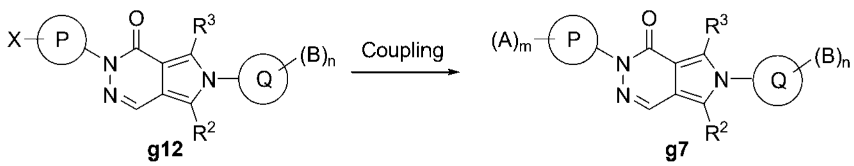


g12 g7

## Scheme 5

In another specific aspect of Formula (I), final compound **g14** can be prepared according to Scheme 6. Intermediate **g13** prepared according to Schemes 1 or 2, may be demethylated under acid conditions such as in the presence of HC1 to lead to final compound **g14.**

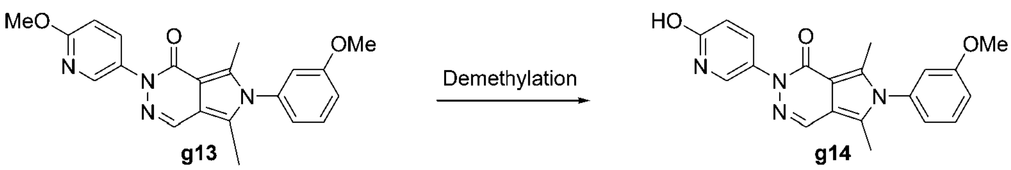


g13 g14

## Scheme 6

In another specific aspect of Formula (I), final compound **g20** can be prepared according to Scheme 7. Intermediate **g17** can be obtained by methods such as Ullmann coupling reactions, known in the art of organic synthesis, between pyridazin-3(2H)-one and 2-bromopyridines, mediated by copper-complex catalysts such as CuI and the like, in the presence of a base such as $N^1$,$N^2$-dimethylcyclohexane-1,2-diamine and the like, in reaction-inert solvents such as dioxane and the like, at an appropriate temperature. Intermediates **g17** can then be converted into 2,6-dihydro-1*H*-pyrrolo

[3,4-*d*]pyridazin-1-one derivatives **g19** by reaction with 1-(isocyanomethylsulfonyl)-4-methylbenzene **g18** in the presence of a base such as NaH, in an appropriate solvent such as THF, at an appropriate temperature. Then final compound **g20** can be obtained by method such as the Ullmann coupling as described for Step 1 between the intermediate **g19** and a bromoaryl such as **g20.**

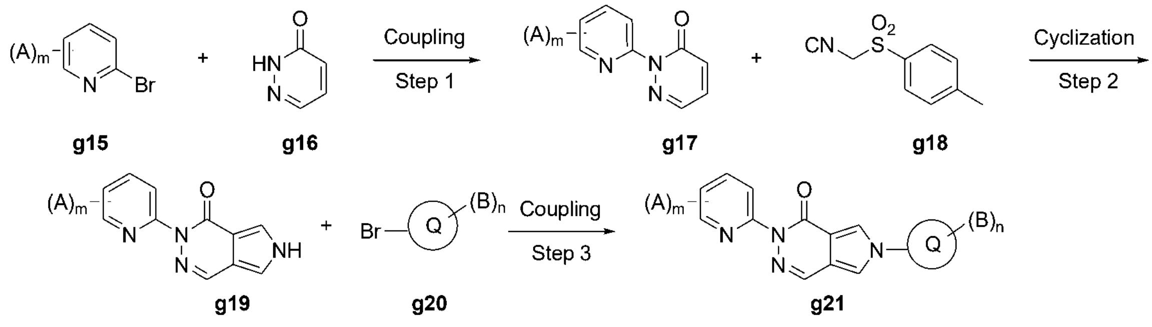


g15 g16 g17 g18

g19 g20 g21

**Scheme 7**

Pyrrole derivative **g5** can be condensed with suitable heteroarylhydrazine derivative **g6** in acidic condition, such as, for example acetic acid and the like, in a polar solvent such as EtOH, dioxane and the like, followed by cyclization in neat condition at an appropriate temperature, yielding final compound **g7.**

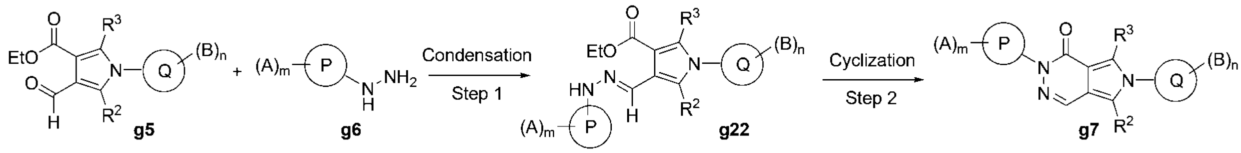


g5 g6 g22 g7

**Scheme 8**

Methyletheraryl **g23** can be cleaved in presence of a Lewis acid, such as $BBr_3$, to yield the corresponding hydroxyaryl **g24** which can be alkylated to lead to the ether compound **g25.** Subsequently **g25** can be cyclized with suitable heteroarylhydrazine derivative **g6** in acidic condition, yielding final compound **g26.**

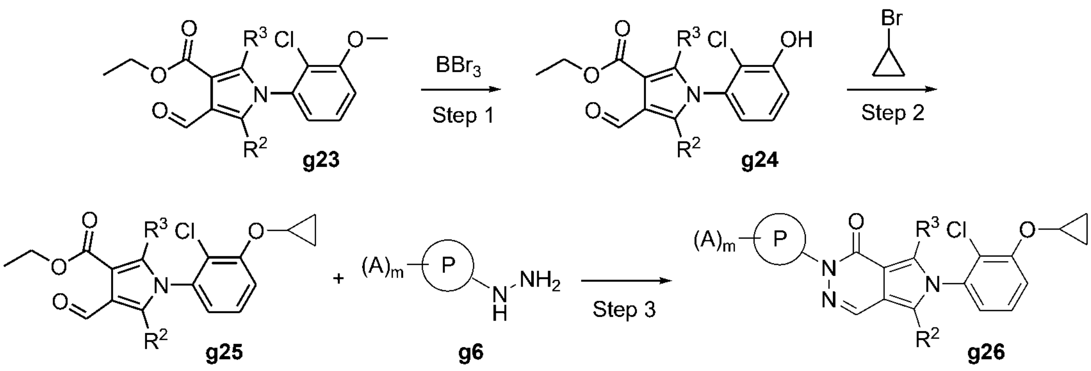


g23 g24

g25 g6 g26

**Scheme 9**

EXPERIMENTAL

Unless otherwise noted, all starting materials were obtained from commercial suppliers and used without further purification.

Specifically, the following abbreviations may be used in the examples and throughout the specification.

| | |
|---|---|
| ACN (Acetonitrile) | $MgSO_4$ (Magnesium sulfate) |

(continued)

| | |
|---|---|
| AcOH (Acetic acid) | min (Minute) |
| $BBr_3$ (Boron tribromide) | mL (Milliliter) |
| $Cs_2CO_3$ (Cesium carbonate) | μL (Microliter) |
| CuI (Copper (I) iodide) | mmol (Millimole) |
| $Cu(OAc)_2$ (Copper (II) acetate) | μmol (Micromole) |
| DAST (Diethylaminosulfur trifluoride) | m.p. (Melting point) |
| DCM (Dichloromethane) | $NH_4Cl$ (Ammonium chloride) |
| DME (1, 2-Dimethoxyethane) | $NH_4OH$ (Ammonium hydroxide) |
| DMF (Dimethylformamide) | $NaBH(OAc)_3$ (Sodium triacetoxyhydroborate) |
| EtOAc (Ethyl acetate) | $NaBH_4$ (Sodium borohydride) |
| EtOH (Ethanol) | NaCl (Sodium chloride) |
| $Et_2O$ (Diethyl ether) | NaH (Sodium hydride) |
| $Et_3N$ (Triethylamine) | $NaHCO_3$ (Sodium bicarbonate) |
| g (Gram) | NaI (Sodium iodide) |
| h (Hour) | NaOH (Sodium hydroxide) |
| $^1H$ (Proton) | $Na_2SO_4$ (Sodium sulfate) |
| HCl (Hydrochloric acid) | NMR (Nuclear Magnetic Resonance) |
| HPLC (High Performance Liquid Chromatography) | $PdCl_2$(dppf) [1,1'-Bis(diphenylphosphino)ferrocene] dichlor-opalladium(II) |
| Hz (Hertz) | $POCl_3$ (Phosphoryl chloride) |
| $K_2CO_3$ (Potassium carbonate) | rt (Room temperature) |
| $K_3PO_4$ (Potassium phosphate) | RT (Retention Time) |
| LCMS (Liquid Chromatography-Mass Spectrometry) | THF (Tetrahydrofuran) |
| $LiBH_4$ (Lithium borohydride) | TLC (Thin layer chromatography) |
| M (Molar) | UPLC-MS (Ultra Performance Liquid Chromatography-Mass Spectrometry) |
| MeOH (Methanol) | Xantphos (4,5-Bis(diphenylphosphino)-9,9-dimethylxanthene) |
| mg (Milligram) | |

All references to brine refer to a saturated aqueous solution of NaCl. Unless otherwise indicated, all temperatures are expressed in °C (degrees Centigrade). All reactions are conducted under an inert atmosphere at room temperature unless otherwise noted.

Most of the reactions were monitored by thin-layer chromatography on 0.25mm Merck silica gel plates (60F-254), visualized with UV light. Flash column chromatography was performed on prepacked silica gel cartridges (15-40 μM, Merck).

## EXAMPLES

### EXAMPLE 1: 6-(3-Methoxyphenyl)-5,7-dimethyl-2-phenyl-2,6-dihydro-1*H*-pyrrolo[3,4-*d*]pyridazin-1-one (Final Compound 1-26)

*Ethyl 2-acetyl-4-oxopentanoate*

According to Scheme 1 Step 1: To a stirred solution of ethyl-3-oxobutanoate (200 g, 1.54 mol) in THF (1 L) at 0°C

was added NaH (60% w/w, 67.6 g, 1.70 mol) portionwise over 2 h. After 30 min, 1-chloropropan-2-one (156 g, 1.70 mol) was added over 1 h and the reaction mixture was allowed to warm to rt overnight. $Et_2O$ (250 mL) and then brine (500 mL) were added and the organic layer was separated. The aqueous layer was extracted twice with $Et_2O$ (2x 250 mL). The organic layers were combined, dried over $Na_2SO_4$, filtered and evaporated under reduced pressure. The crude oil was distilled (126°C/14 mmHg) to afford the title compound as a pale yellow oil (140g, 49%).

*Ethyl 1-(3-methoxyphenyl)-2,5-dimethyl-1H-pyrrole-3-carboxylate*

According to Scheme 1 Step 2: To a solution of ethyl 2-acetyl-4-oxopentanoate (8.00 g, 43.0 mmol) in toluene (140 mL) was added 3-methoxyaniline (6.35 g, 51.6 mmol) and AcOH (0.09 g, 1.5 mmol). The reaction mixture was heated under reflux with a Dean-Stark apparatus overnight, then cooled to rt and concentrated under reduced pressure to afford the title compound as a brown oil (11.7 g, 100%) which was used in the next step without any further purification.

*Ethyl 4-formyl-1-(3-methoxyphenyl)-2,5-dimethyl-1H-pyrrole-3-carboxylate*

According to Scheme 1 Step 3: To a stirred solution of $POCl_3$ (1.76 g, 11.5 mmol) in DCM (13 mL) at 0°C was added dropwise a solution of DMF (0.84 g, 11.5 mmol) in DCM (10 mL) whilst maintaining the temperature at 0°C. Once the addition was complete, the reaction mixture was stirred at 0°C for 20 min and allowed to warm to rt. Then a solution of ethyl 1-(3-methoxyphenyl)-2,5-dimethyl-1*H*-pyrrole-3-carboxylate (2.85 g, 10.4 mmol) in DCM (13 mL) was added dropwise to the reaction mixture at 0°C and stirred for 1 h at this temperature, and then overnight at rt. Cold water was added and the pH adjusted to 10-11 using 6M aqueous NaOH. The resulting solution was extracted with DCM. The organic layer was washed with brine, dried over $MgSO_4$ and concentrated under reduced pressure to afford the title compound as a brown solid (3.62 g, 92%) which was used in the next step without any further purification.

*6-(3-Methoxyphenyl)-5,7-dimethyl-2-phenyl-2,6-dihydro-1H-pyrrolo[3,4-d]pyridazin-1-one*

According to Scheme 1 Step 4: To a stirred solution of ethyl 4-formyl-1-(3-methoxyphenyl)-2,5-dimethyl-1*H*-pyrrole-3-carboxylate (279 mg, 0.93 mmol) in dioxane (3 mL) was added phenylhydrazine (103 mg, 0.93 mmol) and AcOH (278 mg, 4.63 mmol). The reaction mixture was heated to 160°C under microwave irradiation for 20 min, cooled to rt, washed with $Et_2O$, then filtered and dried to afford the title compound as a white solid (146 mg, 46%).

M.p.: 213-215°C; UPLC-MS: RT = 1.09 min; MS m/z $ES^+$= 346; $^1$H-NMR (300MHz, DMSO-*d*$_6$) δ: 8.34 (1H, s), 7.56-7.42 (5H, m), 7.35-7.29 (1H, m), 7.18-7.15 (1H, dd), 7.05-6.98 (2H, m), 3.82 (3H, s), 2.40 (3H, s), 2.27 (3H, s).

**EXAMPLE 2: 2-(2-Methoxyphenyl)-5,7-dimethyl-6-*p*-tolyl-2,6-dihydro-1*H*-pyrrolo[3,4-*d*]pyridazin-1-one (Final compound 1-2)**

*5, 7-Dimethyl-6-p-tolyl-2, 6-dihydro-1H-pyrrolo[3, 4-d]pyridazin-1-one*

According to Scheme 2 Step 1: To a stirred solution of ethyl 4-formyl-2,5-dimethyl-1-*p*-tolyl-1*H*-pyrrole-3-carboxylate (1.66 g, 5.82 mmol) in EtOH (50 mL) was added hydrazine monohydrate (2.91 g, 58.2 mmol). The mixture was stirred at reflux overnight, cooled to rt, concentrated, filtered and dried to afford the title compound as a white solid (1.31 g, 89%).

*2-(2-Methoxyphenyl)-5,7-dimethyl-6-p-tolyl-2,6-dihydro-1H-pyrrolo[3,4-d]pyridazin-1-one*

According to Scheme 2 Step 2: To a stirred solution of 5,7-dimethyl-6-*p*-tolyl-2,6-dihydro-1*H*-pyrrolo[3,4-d] pyridazin-1-one (0.20 g, 0.79 mmol) in dioxane (3 mL) were added 1-iodo-2-methoxybenzene (185 mg, 0.79 mmol), CuI, trans-cyclohexane-1,2-diamine (18 mg, 158 μmol) and $K_3PO_4$ (503 mg, 2.37 mmol). The reaction mixture was heated at 110°C overnight, cooled to rt and extracted with EtOAc. The organic layers were washed with brine, dried over $Na_2SO_4$, filtered and concentrated under reduced pressure. The crude material was purified by flash chromatography over silica gel using DCM/MeOH (99: 1) as eluent to afford the title compound as a beige solid (97 mg, 34%).

M.p.: 202°C; LC-MS: RT = 2.83 min; MS m/z $ES^+$= 360; $^1$H-NMR (300MHz, $CDCl_3$) δ: 8.05 (1H, s), 7.51 (2H, d), 7.35 (2H, d), 7.15 (2H, d), 7.00 (2H, d), 3.85 (3H, s), 2.55 (6H, s), 2.30 (3H, s).

**EXAMPLE 3: 2-(2-Fluorophenyl)-5,7-dimethyl-6-phenyl-2,6-dihydro-1H-pyrrolo [3,4-d]pyridazin-1-one (Final compound 1-21)**

According to Scheme 2 Step 2: To a stirred solution of 5,7-dimethyl-6-phenyl-2,6-dihydro-1H-pyrrolo[3,4-d]

pyridazin-1-one (100 mg, 0.42 mmol) in DMF (2 mL) were added 1-fluoro-2-iodobenzene (111 mg, 0.50 mmol), CuI (40.0 mg, 0.21 mmol) and $K_2CO_3$ (116 mg, 0.84 mmol). The reaction mixture was heated at 140°C under microwave irradiation for 30 min, cooled to rt, poured onto water and extracted with DCM. The organic layers were combined, washed with brine, dried over $Na_2SO_4$, filtered and concentrated under reduced pressure. The crude material was purified by flash chromatography over silica gel using DCM/EtOAc (95:5) as eluent to afford the title compound as a beige solid (57 mg, 41%).

M.p.: 226°C; UPLC-MS: RT = 1.05 min; MS m/z $ES^+$= 334; $^1$H-NMR (300MHz, DMSO-$d_6$) δ: 8.34 (1H, s), 7.69-7.55 (3H, m), 7.50-7.42 (4H, m), 7.39-7.29 (2H, m), 2.38 (3H, s), 2.36 (3H, s), 2.25 (3H, s).

**EXAMPLE 4: 2-(4-Methoxyphenyl)-5,7-dimethyl-6-phenyl-2,6-dihydro-1H-pyrrolo[3,4-*d*]pyridazin-1-one (Final compound 1-13)**

According to Scheme 2 Step 2: To a stirred solution of 5,7-dimethyl-6-phenyl-2,6-dihydro-1*H*-pyrrolo[3,4-*d*] pyridazin-1-one (150 mg, 0.63 mmol) in DMF (6 mL) were added 4-methoxyphenylboronic acid (114 mg, 0.75 mmol), $Cu(OAc)_2$ (228 mg, 1.25 mmol) and $Et_3N$ (127 mg, 1.25 mmol). The mixture was stirred at 80°C for 6 h, cooled to rt, poured onto $NH_4OH$ and extracted with EtOAc. The organic layers were washed with brine, dried over $Na_2SO_4$, filtered and concentrated under reduced pressure. The crude material was purified by preparative HPLC to afford the title compound as a white solid (29 mg, 13%).

UPLC-MS: RT = 1.05 min; MS m/z $ES^+$= 346; $^1$H-NMR (300MHz, DMSO-$d_6$) δ: 8.32 (1H, s), 7.68-7.58 (3H, m), 7.46-7.37 (4H, m), 7.01 (2H, d), 3.80 (3H, s), 2.40 (3H, s), 2.21 (3H, s).

**EXAMPLE 5: 5,7-Dimethyl-2-phenyl-6-(3-(pyrrolidin-1-yl)phenyl)-2,6-dihydro-1H-pyrrolo[3,4-d]pyridazin-1-one (Final compound 1-101)**

*Ethyl 4-formyl-2,5-dimethyl-1-(3-(pyrrolidin-1-yl)phenyl)-1H-pyrrole-3-carboxylate*

According to Scheme 3: To a stirred solution of ethyl 1-(3-bromophenyl)-4-formyl-2,5-dimethyl-1H-pyrrole-3-carboxylate (1.00 g, 2.86 mmol) in DMF (14 mL) were added pyrrolidine (406 mg, 5.71 mmol), Xantphos (248 mg, 0.43 mmol), $PdCl_2$(dppf) (233 mg, 0.29 mmol) and $Cs_2CO_3$ (1.40 g, 4.28 mmol). The reaction mixture was stirred at 130°C in a sealed tube for 3 h, cooled to rt, diluted with DCM, filtered over Celite and concentrated under reduced pressure. The crude material was purified by flash chromatography over silica gel using DCM/MeOH (99:1) as eluent to afford the title compound as a yellow solid (513 mg, 53%).

*5, 7-Dimethyl-2-phenyl-6-(3-(pyrrolidin-1-yl)phenyl)-2,6-dihydro-1H-pyrrolo[3,4-d] pyridazin-1-one*

According to Scheme 1 Step 4: Prepared as per Final compound 1-26, from ethyl 4-formyl-2,5-dimethyl-1-(3-(pyrrolidin-1-yl)phenyl)-1*H*-pyrrole-3-carboxylate (120 mg, 0.35 mmol) and purified by preparative HPLC, yielding the title compound as a brown solid (513 mg, 53%).

M.p.: 228-229°C; UPLC-MS: RT = 1.29 min; MS m/z $ES^+$= 385; $^1$H-NMR (300MHz, DMSO-$d_6$) δ: 8.32 (1H, s), 7.52-7.29 (6H, m), 6.70 (1H, dd), 6.55 (1H, dd), 6.45-6.44 (1H, m), 4.45-4.44 (1H, m), 3.27-3.25 (4H, m), 2.41 (3H, s), 2.28 (3H, s), 1.97-1.95 (4H, m).

**EXAMPLE 6: 6-(3-Acetylphenyl)-5,7-dimethyl-2-phenyl-2,6-dihydro-1*H*-pyrrolo [3,4-d]pyridazin-1-one (Final compound 1-36)**

According to Scheme 4: To a stirred solution of 6-(3-bromophenyl)-5,7-dimethyl-2-phenyl-2,6-dihydro-1*H*-pyrrolo[3,4-*d*]pyridazin-1-one (Final compound 1-34, 120 mg, 0.30 mmol) in DMF (1.5 mL) was added 1-(vinyloxy)butane (152 mg, 1.52 mmol), $Et_3N$ (92 mg, 0.91 mmol) and $PdCl_2$(dppf) (25 mg, 30 μmol). The reaction mixture was stirred at 130°C under microwave irradiation under nitrogen atmosphere for 25 min. 1M HCl (1.5 mL) was added and the reaction mixture was stirred at rt for 10 min, and then diluted with DCM. The organic layers were combined, washed with brine, dried over $Na_2SO_4$, filtered and concentrated under reduced pressure. The crude material was purified by preparative HPLC to afford the title compound as a white solid (15 mg, 14%).

M.p.: 202-204°C; UPLC-MS: RT = 0.97 min; MS m/z $ES^+$= 358; $^1$H-NMR (300MHz, DMSO-$d_6$) δ: 8.36 (1H, s), 8.17-8.14 (1H, m), 8.00 (1H, s), 7.83-7.73 (2H, m), 7.53-7.43 (4H, m), 7.35-7.31 (1H, m), 2.65 (3H, s), 2.40 (3H, s), 2.27 (3H, s).

**EXAMPLE 7: 6-(3-(2-Methoxyethylamino)phenyl)-5,7-dimethyl-2-phenyl-2,6-dihydro-1*H*-pyrrolo[3,4-*d*]pyridazin-1-one (Final compound 1-43)**

According to Scheme 4: To a stirred solution of 6-(3-bromophenyl)-5,7-dimethyl-2-phenyl-2,6-dihydro-1*H*-pyrrolo[3,4-*d*]pyridazin-1-one (Final compound 1-34, 120 mg, 0.30 mmol) in DMF (1 mL) was added 2-methoxyethanamine (34 mg, 0.46 mmol), Xantphos (26 mg, 46 $\mu$mol), $PdCl_2$(dppf) (25 mg, 30 $\mu$mol) and $Cs_2CO_3$ (149 mg, 0.46 mmol). The mixture was stirred at 130°C in a sealed tube for 4 h, cooled to rt, diluted with DCM, filtered over Celite and concentrated under reduced pressure. The crude material was purified by preparative HPLC to afford the title compound as a brown solid (15 mg, 13%).

M.p.: 116-119°C; UPLC-MS: RT = 1.04 min; MS m/z $ES^+$= 389; $^1$H-NMR (300MHz, DMSO-*$d_6$*) $\delta$: 8.31 (1H, s), 7.52-7.44 (4H, m), 7.34-7.25 (2H, m), 6.80 (1H, d), 6.53-6.49 (2H, m), 6.07 (1H, t), 3.50 (2H, t), 3.33-3.22 (5H, m), 2.41 (3H, s), 2.27 (3H, s).

**EXAMPLE 8: 6-(3-Hydroxyphenyl)-5,7-dimethyl-2-phenyl-2,6-dihydro-1H-pyrrolo[3,4-d]pyridazin-1-one (Final compound 1-51)**

According to Scheme 4: To a stirred solution of 6-(3-methoxyphenyl)-5,7-dimethyl-2-phenyl-2,6-dihydro-1*H*-pyrrolo[3,4-*d*]pyridazin-1-one (Final compound 1-26, Example 1, 30 mg, 87 $\mu$mol) in DCM (1 mL) at 0°C, was added dropwise $BBr_3$ (0.17 mL, 0.17 mmol). The mixture was stirred at rt for 1 h, diluted with DCM and slowly hydrolysed with water. The organic layer was washed with water and $NH_4Cl$, dried over $MgSO_4$, filtered and concentrated under reduced pressure. The crude material was washed with $Et_2O$, filtered and dried under reduced pressure to afford the title compound as a beige solid (12 mg, 42%).

M.p.: 252-254°C; UPLC-MS: RT = 0.91 min; MS m/z $ES^+$= 332; $^1$H-NMR (300MHz, DMSO-*$d_6$*) $\delta$: 10.05 (1H, s), 8.32 (1H, s), 7.52-7.39 (5H, m), 7.34-7.29 (1H, m), 6.98 (1H, dd), 6.81 (1H, d), 6.75 (1H, s), 2.39 (3H, s), 2.25 (3H, s).

**EXAMPLE 9: 2-(4-(Aminomethyl)phenyl)-6-(3-methoxyphenyl)-5,7-dimethyl-2,6-dihydro-1*H*-pyrrolo[3,4-*d*]pyridazin-1-one (Final compound 1-88)**

*tert-Butyl 4-(6-(3-methoxyphenyl)-5,7-dimethyl-1-oxo-1H-pyrrolo[3,4-d]pyridazin-2 (6H)-yl)benzylcarbamate*

According to Scheme 2 Step 2: Prepared as per Final compound 1-13 from 6-(3-methoxyphenyl)-5,7-dimethyl-2,6-dihydro-1*H*-pyrrolo[3,4-*d*]pyridazin-1-one (140 mg, 0.52 mmol), 4-((*tert*-butoxycarbonylamino)methyl)phenylboronic acid (157 mg, 0.62 mmol), $Cu(OAc)_2$ (103 mg, 0.52 mmol) and $K_2CO_3$ (86 mg, 0.62 mmol), and purified by flash chromatography over silica gel using DCM/MeOH (99:1) as eluent to afford the title compound as a brown solid (400 mg, 89%).

*2-(4-(Aminomethyl)phenyl)-6-(3-methoxyphenyl)-5,7-dimethyl-2,6-dihydro-1H-pyrrolo [3,4-d]pyridazin-1-one*

According to Scheme 5: To a stirred solution of *tert*-butyl 4-(6-(3-methoxyphenyl)-5,7-dimethyl-1-oxo-1*H*-pyrrolo [3,4-*d*]pyridazin-2(6*H*)-yl)benzylcarbamate (100 mg, 0.21 mmol) in DCM (2 mL), was added trifluoroacetic acid (288 mg, 2.53 mmol) dropwise. The reaction mixture was stirred at rt for 4 h, diluted with DCM and water. The organic layer was washed with water and $NaHCO_3$, dried over $MgSO_4$, filtered and concentred under reduced pressure. The crude material was purified by flash chromatography over silica gel using DCM/MeOH (95:5) as eluent and recrystallized in $Et_2O$ to afford the title compound as a brown solid (7 mg, 9%).

M.p.: 145-155°C; UPLC-MS: RT = 0.72 min; MS m/z $ES^+$= 375; $^1$H-NMR (300MHz, DMSO-*$d_6$*) $\delta$: 8.33 (1H, s), 7.57-7.41 (5H, m), 7.16 (1H, dd), 7.03-6.98 (2H, m), 3.87 (2H, s), 3.82 (3H, s), 2.40 (3H, s), 2.27 (3H, s).

**EXAMPLE 10: 6-(3-Methoxyphenyl)-5,7-dimethyl-2-(4-((methylamino)methyl) phenyl)-2,6-dihydro-1H-pyrrolo [3,4-d]pyridazin-1-one (Final compound 1-109)**

*tert-Butyl 4-(6-(3-methoxyphenyl)-5,7-dimethyl-1-oxo-1H-pyrrolo[3,4-d[pyridazin-2 (6H)-yl)benzyl(methyl)carbamate*

According to Scheme 5: To a stirred solution of *tert*-butyl 4-(6-(3-methoxyphenyl)-5,7-dimethyl-1-oxo-1*H*-pyrrolo [3,4-d]pyridazin-2(6*H*)-yl)benzylcarbamate (235 mg, 0.49 mmol) in DMF (2 mL), was added iodomethane (211 mg, 1.49 mmol) and NaH (60% w/w, 59 mg, 1.49 mmol) portionwise. The reaction mixture was stirred at rt for 3 h and water was added cautiously. The reaction mixture was extracted with DCM and the combined organic layers were dried over $MgSO_4$, filtered and concentrated under reduced pressure to afford the title compound as a brown solid (300 mg, 99%), which was used in the next step without any further purification.

*6-(3-Methoxyphenyl)-5,7-dimethyl-2-(4-((methylamino)methyl)phenyl)-2,6-dihydro-1H-pyrrolo[3,4-d]pyridazin-1-one*

According to Scheme 5: Prepared as per Final compound 1-88 from *tert*-butyl 4-(6-(3-methoxyphenyl)-5,7-dimethyl-1-oxo-1*H*-pyrrolo[3,4-*d*]pyridazin-2(6*H*)-yl)benzyl (methyl)carbamate (300 mg, 0.61 mmol), purified by flash chromatography over silica gel using DCM/MeOH/$NH_4OH$ (95:5:0.1) as eluent and recrystallized in $Et_2O$, yielding the title compound as a light pink solid (34 mg, 14%).

M.p.: 150-154°C; UPLC-MS: RT = 0.73 min; MS m/z $ES^+$= 389; $^1H$-NMR (300MHz, DMSO-$d_6$) δ: 8.33 (1H, s), 7.56-7.39 (5H, m), 7.16 (1H, dd), 7.03-6.98 (2H, m), 3.82 (3H, s), 3.79 (2H, s), 2.40 (3H, s), 2.36 (3H, s), 2.27 (3H, s).

**EXAMPLE 11: 6-([1,1'-Biphenyl]-3-yl)-5,7-dimethyl-2-(pyridin-2-yl)-2,6-dihydro-1*H*-pyrrolo[3,4-*d*]pyridazin-1-one (Final compound 1-76)**

*6-(3-Bromophenyl)-5, 7-dimethyl-2-(pyridin-2-yl)-2,6-dihydro-1H-pyrrolo[3,4-d] pyridazin-1-one*

According to Scheme 1 Step 4: Prepared as per Final compound 1-26, from ethyl 1-(3-bromophenyl)-4-formyl-2,5-dimethyl-1*H*-pyrrole-3-carboxylate (2.00 g, 5.71 mmol), 2-hydrazinylpyridine (1.25 g, 11.4 mmol) and AcOH (1.72 g, 28.6 mmol), then purified by flash chromatography with DCM/MeOH/$NH_4OH$ (94:6:1) as eluent, yielding the title compound as a beige solid (1.45 g, 64%).

*6-([1,1'-Biphenyl]-3-yl)-5,7-dimethyl-2-(pyridin-2-yl)-2,6-dihydro-1H-pyrrolo[3,4-d] pyridazin-1-one*

According to Scheme 4: To a stirred solution of 6-(3-bromophenyl)-5,7-dimethyl-2-(pyridin-2-yl)-2,6-dihydro-1H-pyrrolo[3,4-d]pyridazin-1-one (80 mg, 0.20 mmol) in DME/water (8:2, 1.6 mL) were added phenylboronic acid (62 mg, 0.51 mmol), $K_3PO_4$ (107 mg, 0.51 mmol) and $PdCl_2$(dppf) (15 mg, 20 μmol). The mixture was stirred at 130°C under nitrogen atmosphere in a sealed tube for 45 min, cooled to rt, diluted with DCM, filtered through Celite, and concentrated under reduced pressure. The crude material was purified by flash chromatography over silica gel using DCM/MeOH (98:2) as eluent to afford the title compound as a grey solid (21 mg, 26%).

M.p.: 106-109°C; UPLC-MS: RT = 1.06 min; MS m/z $ES^+$= 393; $^1H$-NMR (300MHz, DMSO-$d_6$) δ: 8.58 (1H, dd), 8.33 (1H, s), 7.97-7.89 (2H, m), 7.82-7.69 (4H, m), 7.55-7.39 (6H, m), 2.43 (3H, s), 2.32 (3H, s).

**EXAMPLE 12: 6-(3-(Diethylamino)phenyl)-5,7-dimethyl-2-(pyridin-2-yl)-2,6-dihydro-1*H*-pyrrolo[3,4-*d*]pyridazin-1-one (Final compound 1-100)**

*6-(3-Aminophenyl)-5,7-dimethyl-2-(pyridin-2-yl)-2,6-dihydro-1H-pyrrolo[3,4-d] pyridazin-1-one*

According to Scheme 4: To a stirred solution of 5,7-dimethyl-6-(3-nitrophenyl)-2-(pyridin-2-yl)-2,6-dihydro-1*H*-pyrrolo[3,4-*d*]pyridazin-1-one (1.10 g, 3.04 mmol) in DCM (12 mL) was added zinc powder (2.00 g, 30.4 mmol) and AcOH (1.83 g, 30.4 mmol) dropwise. The reaction mixture was stirred at rt for 6 h, filtered through Celite and concentrated under reduced pressure. The crude residue was washed with $Et_2O$ and dried to afford the title compound as a brown solid (810 mg, 80%) which was used in the next step without any further purification.

*6-(3-(Diethylamino)phenyl)-5,7-dimethyl-2-(pyridin-2-yl)-2,6-dihydro-1H-pyrrolo[3,4-d]pyridazin-1-one*

According to Scheme 4: To a stirred solution of 6-(3-aminophenyl)-5,7-dimethyl-2-(pyridin-2-yl)-2,6-dihydro-1*H*-pyrrolo[3,4-*d*]pyridazin-1-one (100 mg, 0.30 mmol) in ACN (4 mL) at 0°C was added acetaldehyde (133 mg, 3.02 mmol) and AcOH (181 mg, 3.02 mmol). The reaction mixture was stirred at 0°C for 20 min then $NaBH(OAc)_3$ (192 mg, 0.91 mmol) was added. The mixture was stirred at rt for 1 h, poured onto water and extracted with DCM. The organic layer was washed with $NaHCO_3$ and $NH_4Cl$, dried over $MgSO_4$, filtered and concentrated under reduced pressure. The crude material was purified by flash chromatography over silica gel using DCM/MeOH (98:2) as eluent. The resulting solid was washed with $Et_2O$ to afford the title compound as an orange solid (26 mg, 22%).

M.p.< 85°C; UPLC-MS: RT = 0.98 min; MS m/z $ES^+$= 388; $^1H$-NMR (300MHz, DMSO-$d_6$) δ: 8.59-8.54 (1H, m), 8.29 (1H, s), 7.98-7.90 (1H, m), 7.51-7.46 (1H, m), 7.45-7.39 (1H, m), 7.39-7.31 (1H, m), 6.82 (1H, dd), 6.60-6.55 (1H, m), 6.52 (1H, dd), 3.37 (4H, q), 2.40 (3H, s), 2.28 (3H, s), 1.10 (6H, t).

**EXAMPLE 13: 2-(6-Hydroxypyridin-3-yl)-6-(3-methoxyphenyl)-5,7-dimethyl-2,6-dihydro-1*H*-pyrrolo[3,4-*d*]pyridazin-1-one (Final compound 1-131)**

According to scheme 6: To a solution of 6-(3-methoxyphenyl)-2-(6-methoxypyridin-3-yl)-5,7-dimethyl-2,6-dihydro-1*H*-pyrrolo[3,4-*d*]pyridazin-1-one (60 mg, 159 μmol) in THF (0.5 mL) was added dropwise 6M HCl (775 μL) and NaI (23.9 mg, 159 μmol). The mixture was stirred at 60°C for 5 h. The solvent was evaporated under reduced pressure. The residue was basified with an aqueous 1M $NaHCO_3$ solution and was extracted with DCM. The organic layer was dried over $MgSO_4$, filtered and concentrated under reduced pressure to afford a red liquid. The crude was purified by preparative HPLC to afford the title compound as an orange oil (19.4 mg, 34%).

M.p.: 207-209°C; UPLC-MS: RT = 0.79 min; MS m/z $ES^+$= 363; $^1$H-NMR (300MHz, $CDCl_3$) δ: 8.05 (1H, s), 7.95-7.88 (2H, m), 7.51-7.43 (1H, m), 7.07 (1H, dd), 6.80 (1H, dd), 6.75-6.66 (2H, m), 3.88 (3H, s), 2.51 (3H, s), 2.28 (3H, s).

**EXAMPLE 14: 2-(4-(2-Hydroxypropan-2-yl)phenyl)-6-(3-methoxyphenyl)-5,7-dimethyl-2,6-dihydro-1*H*-pyrrolo [3,4-*d*]pyridazin-1-one (Final compound 1-135)**

According to scheme 5: To a solution of 2-(4-acetylphenyl)-6-(3-methoxyphenyl)-5,7-dimethyl-2,6-dihydro-1*H*-pyrrolo[3,4-*d*]pyridazin-1-one (75 mg, 0.19 mmol) in THF (1.5 mL) at 0°C was added methylmagnesium bromide (0.19 mL, 0.58 mmol). The mixture was stirred at 0°C for 30 min. The mixture was then diluted with DCM and quenched with an aqueous solution of $NH_4Cl$. The mixture was washed with $NaHCO_3$ and then $NH_4Cl$. The organic layer was dried over $MgSO_4$, filtered and concentrated under reduced pressure to afford a yellow solid. The crude solid was purified by flash chromatography over silica gel using DCM/MeOH (98/2) as eluent then triturated with DCM/MeOH (1/9) and concentrated under reduced pressure to afford the title compound as a beige solid (16mg, 20%).

M.p.: 103-108°C; UPLC-MS: RT = 1.01 min; MS m/z $ES^+$= 404; $^1$H-NMR (300MHz, DMSO-$d_6$) δ: 8.32 (1H, s), 7.59-7.49 (3H, m), 7.42-7.39 (2H, m), 7.18-7.15 (2H, m), 7.04-6.98 (2H, m), 5.08 (1H, s), 3.82 (3H, s), 2.40 (1H, s), 2.26 (1H, s), 1.46 (6H, s).

**EXAMPLE 15: 2-(4-(1-Hydroxyethyl)phenyl)-6-(3-methoxyphenyl)-5,7-dimethyl-2,6-dihydro-1H-pyrrolo[3,4-d] pyridazin-1-one (Final compound 1-138)**

According to scheme 5: To a solution of 2-(4-acetylphenyl)-6-(3-methoxyphenyl)-5,7-dimethyl-2,6-dihydro-1*H*-pyrrolo[3,4-*d*]pyridazin-1-one (75 mg, 0.19 mmol) in THF (2 mL) at 0°C was added $NaBH_4$ (7 mg, 0.19 mmol). The mixture was stirred for 45 min at rt. The mixture was diluted with DCM, quenched with $NH_4Cl$ and washed with $NaHCO_3$. The organic layer was dried over $MgSO_4$, filtered and concentrated under reduced pressure to afford a beige solid. The crude solid was purified by flash chromatography over silica gel using DCM/MeOH (98/2) as eluent to afford a solid. The resulting solid was washed with $Et_2O$ to afford the title compound as a beige solid (40 mg, 53%).

M.p.: 192-195°C; UPLC-MS: RT = 0.97 min; MS m/z $ES^+$= 390; $^1$H-NMR (300MHz, DMSO-$d_6$) δ: 8.32 (1H, d), 7.58-7.50 (1H, m), 7.45-7.37 (4H, m), 7.16 (1H, dd), 7.04-6.97 (2H, m), 5.22 (1H, d), 4.81-4.73 (1H, m), 3.82 (3H, s), 2.40 (3H, s), 2.26 (3H, s), 1.35 (3H, d).

**EXAMPLE 16: 6-(3-(Azetidin-1-yl)phenyl)-5,7-dimethyl-2-(4-(pyrrolidin-1-yl) phenyl)-2,6-dihydro-1H-pyrrolo [3,4-d]pyridazin-1-one (Final compound 1-168)**

According to scheme 5: In a sealed tube, to a solution of 6-(3-(azetidin-1-yl)phenyl)-2-(4-bromophenyl)-5,7-dimethyl-2,6-dihydro-1*H*-pyrrolo[3,4-*d*]pyridazin-1-one (120 mg, 0.27 mmol) in DMF (5 mL) under argon were added pyrrolidine (56 mg, 0.80 mmol), Xantphos (15 mg, 27 μmol) and $Cs_2CO_3$ (0.13 g, 0.40 mmol). The mixture was degassed under argon for 10 min and $PdCl_2$(dppf) (10 mg, 13 μmol) was added to the mixture. The mixture was again degassed for 5 min and was then stirred at 130°C for 16 h. After addition of water (15 mL), the reaction mixture was extracted with EtOAc (4 x 25 mL). The organic layers were combined, washed with brine, dried over $Na_2SO_4$ and concentrated under reduced pressure. The crude material was purified by chromatography over silica using hexane/EtOAc (70/30) as eluent to afford the title compound as a yellow solid (22 mg, 19%).

UPLC-MS: RT = 3.61 min; MS m/z $ES^+$= 440; $^1$H-NMR (300MHz, $CDCl_3$) δ: 8.10 (1H, s), 7.40-7.30 (3H, m), 6.60 (2H, d), 6.55-6.45 (2H, m), 6.20-6.19 (1H, m), 3.90 (4H, t), 3.30 (4H, t), 2.50 (3H, s), 2.45-2.35 (2H, m), 2.30 (3H, s), 2.10-1.90 (4H, m).

**EXAMPLE 17: 6-(3-(Azetidin-1-yl)phenyl)-2-(4-(2-hydroxyethyl)phenyl)-5,7-dimethyl-2,6-dihydro-1*H*-pyrrolo [3,4-*d*]pyridazin-1-one (Final compound 1-169)**

According to scheme 5: Ethyl 2-(4-(6-(3-(azetidin-1-yl)phenyl)-5,7-dimethyl-1-oxo-1*H*-pyrrolo[3,4-*d*]pyridazin-2(6*H*)-yl)phenyl)acetate (100 mg, 0.22 mmol) was dissolved in $Et_2O$ (4 mL). To the solution, under argon atmosphere, was added $LiBH_4$ and then MeOH (0.1 mL) dropwise. After complete addition, the mixture was stirred at rt for 1 h and the reaction mixture was evaporated under reduced pressure. To the residue was added a saturated solution of $NH_4Cl$ and the reaction mixture was extracted with EtOAc. The organic layer was washed with brine, dried over $Na_2SO_4$ and concentrated

under reduced pressure to afford an oil. The crude oil was purified by chromatography over silica using DCM/MeOH as eluent to afford the title compound (20 mg, 22%).

UPLC-MS: RT = 2.96 min; MS m/z $ES^+$= 415; $^1$H-NMR (400MHz, $CDCl_3$) δ: 8.10 (1H, s), 7.50 (2H, d), 7.40-7.30 (3H, m), 6.60-6.50 (2H, m), 6.20-6.19 (1H, m), 4.00-3.80 (6H, m), 3.00-2.80 (2H, t), 2.50 (3H, s), 2.45-2.35 (2H, m), 2.30 (3H, s).

**EXAMPLE 18: 6-(3-(Azetidin-1-yl)phenyl)-2-(4-(2-hydroxypropan-2-yl)phenyl)-5,7-dimethyl-2,6-dihydro-1*H*-pyrrolo[3,4-*d*]pyridazin-1-one (Final compound 1-170)**

According to scheme 5: 2-(4-Acetylphenyl)-6-(3-(azetidin-1-yl)phenyl)-5,7-dimethyl-2,6-dihydro-1*H*-pyrrolo [3,4-*d*]pyridazin-1-one (200 mg, 0.48 mmol) was dissolved in dry THF (5 mL). After cooling the solution to -30°C, methylmagnesium iodide 3M in $Et_2O$ (0.242 mL, 0.727 mmol) was added dropwise to the solution and the reaction mixture was stirred at -30°C for 15 min. The mixture was then warmed to rt and stirred for 30 min. The reaction mixture was quenched with an aqueous solution of $NH_4Cl$ and the aqueous phase was extracted with EtOAc (3 x 25 mL). The organic layers were combined, washed with water (2 x 10 mL), dried over $Na_2SO_4$ and concentrated under reduced pressure. The crude product was purified by flash column chromatography over silica gel using hexane/EtOAc (75/25) to afford the title compound as a yellow solid (40 mg, 19%).

UPLC-MS: RT = 1.27 min; MS m/z $ES^+$= 422; $^1$H-NMR (400MHz, $CDCl_3$) δ: 8.07 (1H, s), 7.60 (4H, s), 7.37-7.33 (1H, m), 6.57-6.52 (2H, m), 6.20-6.19 (1H, m), 3.90 (4H, t), 2.50 (3H, s), 2.45-2.42 (2H, m), 2.30 (3H, s), 1.60 (6H, s).

**EXAMPLE 19: 6-(3-(Azetidin-1-yl)phenyl)-2-(pyridin-2-yl)-2,6-dihydro-1H-pyrrolo[3,4-d]pyridazin-1-one (Final compound 1-188)**

*2-(Pyridin-2-yl)pyridazin-3(2H)-one*

According to Scheme 7 Step 1: Prepared as per Final compound 1-2, Scheme 2 Step 2, from pyridazin-3(2*H*)-one (250 mg, 2.60 mmol), 2-bromopyridine (0.25 mL, 2.60 mmol), $K_3PO_4$ (1.10 g, 5.20 mmol), $N^1$,$N^2$-dimethylcyclohexane-1,2-diamine (111 mg, 0.78 mmol) and CuI (99 mg, 0.52 mmol) in dioxane (10 mL) to afford the title compound as an orange oil (300 mg, 67%).

*2-(Pyridin-2-yl)-2,6-dihydro-1H-pyrrolo[3,4-d]pyridazin-1-one*

According to Scheme 7 Step 2: To a solution of NaH (520 mg, 13.0 mmol) in THF (15 mL) at 0°C was added 2-(pyridin-2-yl)pyridazin-3(2*H*)-one (750 mg, 4.33 mmol) and a solution of 1-(isocyanomethylsulfonyl)-4-methylbenzene (930 mg, 4.76 mmol) in THF.

The reaction mixture was stirred for 12 h at rt. Then the solution was carefully quenched with water. After evaporation of the solvent, the resulting black solid was treated with DCM and MeOH, and the mixture was filtered. The solvents were removed under reduced pressure to afford a black solid which was purified by flash chromatography over silica gel using DCM/MeOH (95:5) as eluent to afford the title compound as an off white solid (340 mg, 37%).

*6-(3-(Azetidin-1-yl)phenyl)-2-(pyridin-2-yl)-2,6-dihydro-1H-pyrrolo[3,4-d]pyridazin-1-one*

According to Scheme 7 Step 3: Prepared as per the Final Compound 1-2, Scheme 2 Step 2, from 2-(pyridin-2-yl)-2,6-dihydro-1*H*-pyrrolo[3,4-*d*]pyridazin-1-one (100 mg, 0.47 mmol), 1-(3-bromophenyl)azetidine (100 mg, 0.47 mmol), $K_3PO_4$ (200 mg, 0.94 mmol), $N^1$,$N^2$-dimethylcyclohexane-1,2-diamine (20.1 mg, 141 μmol) and CuI (17.9 mg, 94 μmol) in dioxane (2 mL), to afford the title compound as an off-white solid (18 mg, 11%).

M.p.: 190-192°C; UPLC-MS: RT = 0.84 min; MS m/z $ES^+$= 345; $^1$H-NMR (300MHz, $CDCl_3$) δ: 8.60 (1H, s), 8.21 (1H, s), 7.91-7.62 (3H, m), 7.38 (1H, d), 7.28-7.18 (2H, m), 6.78-6.71 (1H, m), 6.43-6.37 (2H, m), 3.88 (4H, t), 2.35 (2H, q).

**EXAMPLE 20: 6-(3-(Azetidin-1-yl)phenyl)-2-(4-(2-fluoroethyl)phenyl)-5,7-dimethyl-2,6-dihydro-1H-pyrrolo[3,4-d]pyridazin-1-one (Final compound 1-189)**

According to Scheme 5: Under nitrogen, at -78°C, a solution of 6-(3-(azetidin-1-yl)phenyl)-2-(4-(2-hydroxyethyl) phenyl)-5,7-dimethyl-2,6-dihydro-1*H*-pyrrolo[3,4-d]pyridazin-1-one (200 mg, 0.48 mmol) in DCM (33 mL) was added dropwise to a solution of DAST reagent (128 μL, 0.96 mmol) in DCM (5 mL). The reaction mixture was stirred at -78°C for 1 h and for 30 min at rt. The mixture was poured on iced water and the aqueous layer was extracted three times with DCM/MeOH. The organic layers were combined, dried over $MgSO_4$ and concentrated under reduced pressure. The crude residue was purified by preparative HPLC to afford the title compound as a white solid (10 mg, 5%).

UPLC-MS: RT = 0.84 min; MS m/z ES+= 345; $^1$H-NMR (300MHz, $CDCl_3$) δ: 8.06 (1H, s), 7.54 (2H, d), 7.37-7.26 (3H, m), 6.56 (2H, t), 6.20 (1H, s), 4.41 (1H, t), 4.55 (1H, t), 3.93 (4H, t), 3.09 (1H, t), 3.02 (1H, t), 2.51 (3H, s), 2.41 (1H, t), 2.28 (3H, s).

**EXAMPLE 21: 5,7-Dimethyl-6-(1-methyl-1*H*-indol-6-yl)-2-(pyridin-2-yl)-2,6-dihydro-1*H*-pyrrolo[3,4-*d*]pyridazin-1-one (Final compound 1-96)**

According to Scheme 4: A mixture of 6-(1*H*-indol-6-yl)-5,7-dimethyl-2-(pyridin-2-yl)-2,6-dihydro-1*H*-pyrrolo [3,4-*d*]pyridazin-1-one (44 mg, 0.124 mmol) and NaH (4.46 mg, 186 μmol) in chloroform (0.5 mL) was stirred at rt for 30 min. Then iodomethane (9.25 μl, 149 μmol) was added dropwise and the solution was stirred overnight at rt. The reaction mixture was quenched with water and diluted with DCM and $NaHCO_3$ were added to the mixture. The organic layer was separated, dried over $MgSO_4$, filtered and evaporated under reduced pressure to afford a brown solid. The crude solid was purified by LC preparative to afford the title compound as a black oil (4.2 mg, 9%).

UPLC-MS: RT = 0.94 min; MS m/z ES+= 370; $^1$H-NMR (300MHz, $CDCl_3$) δ: 8.69-8.63 (1H, m), 8.16 (1H, s), 7.85-7.70 (3H, m), 7.29-7.18 (3H, m), 6.93 (1H, dd), 6.61 (1H, dd), 3.84 (3H, s), 2.50 (3H, s), 2.27 (3H, s).

**EXAMPLE 22: 6-(3-(Azetidin-1-yl)phenyl)-5,7-dimethyl-2-(pyridin-2-yl)-2,6-dihydro-1*H*-pyrrolo[3,4-*d*]pyridazin-1-one (Final compound 1-102)**

*(E)-Ethyl 1-(3-bromophenyl)-2,5-dimethyl-4-((2-(pyridin-2-yl)hydrazono)methyl)-1H-pyrrole-3-carboxylate*

According to Scheme 8, Step 1: To a solution of ethyl 1-(3-bromophenyl)-4-formyl-2,5-dimethyl-1*H*-pyrrole-3-carboxylate (12.0 g, 34.3 mmol) in dioxane (150 mL), 2-hydrazinylpyridine (4.11 g, 37.7 mmol) and acetic acid (14.4 g, 240 mmol, 13.8 mL) were added. The resulted mixture was refluxed for 2 h. Then, the solvent was evaporated under reduced pressure and EtOAc (200 mL) was added. The organic layer was washed with $NaHCO_3$(aq 15%) (75 mL), dried over $Na_2SO_4$, and concentrated under reduced pressure. The resulted solid was treated with hexane to obtain (*E*)-ethyl 1-(3-bromophenyl)-2,5-dimethyl-4-((2-(pyridin-2-yl)hydrazono)methyl)-*1*H-pyrrole-3-carboxylate (14 g, 88%).

LC-MS: RT = 1.29 min; [M+3H]+ m/z: 443; $^1$H NMR (500 MHz, DMSO-$d_6$) δ: 8.59 (1H, s), 8.09 (1H, d), 7.78 (1H, d), 7.70 (1H, s), 7.52 (2H, m), 7.39 (1H, d), 7.00 (1H, d), 6.65 (1H, t), 4.23 (2 H, q), 2.22 (3H, s), 2.20 (3H, s), 1.19 (3H, t).

*6-(3-Bromophenyl)-5,7-dimethyl-2-(pyridin-2-yl)-1H,2H,6H-pyrrolo[3,4-d]pyridazin-1-one*

According to Scheme 8, Step 2: (*E*)-Ethyl 1-(3-bromophenyl)-2,5-dimethyl-4-((2-(pyridin-2-yl)hydrazono) methyl)-1*H*-pyrrole-3-carboxylate (12.0 g, 27.2 mmol) was heated in an oil bath at 165-170 °C for 2 h to obtain 6-(3-bromophenyl)-5,7-dimethyl-2-(pyridin-2-yl)-1*H*,2*H*,6*H*-pyrrolo[3,4-*d*]pyridazin-1-one (10.0 g, 88.4%). The raw product was used without additional purification.

LC-MS: RT = 1.38 min, [M+3H]+ m/z: 397; $^1$H NMR (400 MHz, DMSO-$d_6$) δ: 8.57 (1H, d), 8.31 (1H, s), 7.94 (1H, t), 7.81 (2H, m), 7.59 (1H, t), 7.51 (2H, m), 7.42 (1H, t), 2.39 (3H, s), 2.26 (3H, s).

*6-(3-(Azetidin-1-yl)phenyl)-5,7-dimethyl-2-(pyridin-2-yl)-2,6-dihydro-1H-pyrrolo[3,4-d]pyridazin-1-one*

According to Scheme 4: The mixture of 6-(3-bromophenyl)-5,7-dimethyl-2-(pyridin-2-yl)-1*H*,2*H*,6*H*-pyrrolo [3,4-*d*]pyridazin-1-one (11.0 g, 27.8 mmol), azetidine hydrochloride (5.21 g, 55.7 mmol), Xantphos(1.61 g, 2.78 mmol), Pd(dppf)$Cl_2$*DCM (2.27 g, 2.78 mmol) and cesium carbonate (31.7 g, 97.4 mmol) in DMF (200 mL) was stirred at 130 °C for 5 h. Then the mixture was diluted with DCM (100 mL) and filtered through silica pad. The filtrate was concentrated under reduced pressure to afford 6-(3-(azetidin-1-yl)phenyl)-5,7-dimethyl-2-(pyridin-2-yl)-2,6-dihydro-1*H*-pyrrolo[3,4-*d*]pyridazin-1-one (11.0 g, 60% purity LC/MS, 17.8 mmol, 64% yield) as a black solid. The crude solid was dissolved in EtOAc (200 mL)m washed with water (50 mL), and Si-Thiol (1 g) was added. The obtained suspension was stirred for 8 h at room temperature; scavenger was filtered off, and the mother liquid was concentrated under reduced pressure. The resulted residue was purified by flash-chromatography (normal phase, $CHCl_3$/MTBE as eluent) to yield 4.4 g of a product with a purity of 95%. The following crystallization from acetonitrile, then from toluene and finally with acetonitrile yielded 1.7 g.

LC-MS: RT = 1.37 min, [M+H]+ m/z: 372; $^1$H NMR (500 MHz, DMSO-$d_6$) δ: 8.56 (1H, d), 8.28 (1H, s), 7.93 (1H, t), 7.48 (1H, d), 7.41 (1H, m), 7.37 (1H, t), 6.63 (1H, d), 6.57 (1H, d), 6.37 (1H, s), 3.85 (4H, t), 2.39 (3H, s), 2.32 (2H, t), 2.26 (3H, s).

**EXAMPLE 23: 6-(2-Chloro-3-cyclopropoxyphenyl)-5,7-dimethyl-2-(pyrimidin-2-yl)-2,6-dihydro-1H-pyrrolo[3,4-d]pyridazin-1-one (Final compound 1-215)**

*Ethyl 1-(2-chloro-3-hydroxy-phenyl)-4-formyl-2,5-dimethyl-pyrrole-3-carboxylate*

According to Scheme 9, Step 1: To the cold solution of ethyl 1-(2-chloro-3-methoxyphenyl)-4-formyl-2,5-dimethyl-pyrrole-3-carboxylate (77.0 mg, 0.23 mmol) in DCM (1 mL), $BBr_3$ (1M solution in DCM, 8 mL) was added. Reaction mixture was stirred on ice bath for 3 h and then poured into saturated solution of $NaHCO_3$. The product was extracted with DCM (3 x 30 mL). The combined organic extracts were washed with brine (45 mL), dried over $Na_2SO_4$, filtered and evaporated to give title compound (60 mg) as a light brown solid. Crude product used in next step without any further purification.

UPLC-MS/UV (2 min, low pH): RT = 1.04 min, m/z = 322 $[M^+H]^+$.

*Ethyl 1-[2-chloro-3-(cyclopropoxy)phenyl]-4-formyl-2,5-dimethyl-pyrrole-3-carboxylate*

According to Scheme 9, Step 2: Ethyl 1-(2-chloro-3-hydroxy-phenyl)-4-formyl-2,5-dimethyl-pyrrole-3-carboxylate (60.0 mg, 185 μmol), bromocyclopropane (67.0 mg, 554 μmol) and cesium carbonate (120 mg, 369 μmol) were suspended in DMF (0.6 mL). The reaction mixture was exposed to microwave irradiation at 170 °C for 6 h. Water (15 mL) was added and extracted with EtOAc (3 x 15 mL). Combined organic layers were washed with brine (15 mL), dried over $Na_2SO_4$, filtered and evaporated. The crude was purified by FCC (Interchym system, 4 g 15 μm $SiO_2$ column, weak eluent: cyclohexane, strong eluent: cyclohexane/EtOAc = 1/1, 0 - 60 % of strong eluent) to give title compound (26 mg, purity ~ 80 %) as a yellow oil.

UPLC-MS/UV (2 min, low pH): RT = 1.32 min, m/z = 362/364 $[M^+H]^+$.

*6-[2-Chloro-3-(cyclopropoxy)phenyl]-5,7-dimethyl-3-pyrimidin-2-yl-pyrrolo[3,4-d]pyridazin-4-one*

According to Scheme 9, Step 3: Ethyl 1-[2-chloro-3-(cyclopropoxy)phenyl]-4-formyl-2,5-dimethyl-pyrrole-3-carboxylate (26.0 mg, 71.9 μmol) and pyrimidin-2-yl-hydrazine (8.70 mg, 79.0 μmol) were dissolved in dioxane (300 μL) and acetic acid (41 μL, 719 μmol) was added. Reaction mixture was exposed to MW irradiation (Biotage) at 160 °C for 6h. Reaction mixture was basified with saturated $NaHCO_3$ (15 mL) and extracted with EtOAc (3 x 15 mL). Organic layer was washed with brine (20 mL), dried over $Na_2SO_4$, filtered and evaporated till dryness. Crude was purified by prep. HPLC to give title compound (14 mg) as a white solid.

M.p.: 228°C; UPLC-MS/UV (8 min, high pH): RT = 4.16 min, m/z = 408/410 $[M^+H]^+$; $^1H$ NMR (300 MHz, DMSO-d6) δ: 8.96 (2H, d), 8.31 (1H, s), 7.72 - 7.66 (1H, m), 7.65 - 7.57 (2H, m), 7.27 - 7.20 (1H, m), 4.12 - 4.03 (1H, m), 2.28 (3H, m), 2.18 (3H, s), 0.93 - 0.84 (2H, m), 0.83 - 0.74 (2H, m).

**EXAMPLE 24: 6-(3-(2*H*-1,2,3-Triazol-2-yl)phenyl)-5,7-dimethyl-2-(pyridin-2-yl)-2,6-dihydro-1*H*-pyrrolo[3,4-*d*]pyridazin-1-one (Final compound 1-199)**

*Ethyl 4-formyl-2,5-dimethyl-1-[3-(triazol-2-yl)phenyl]pyrrole-3-carboxylate*

According to Scheme 3: Ethyl 1-(3-bromophenyl)-4-formyl-2,5-dimethyl-pyrrole-3-carboxylate (440 mg, 1.26 mmol), 2*H*-triazole (130 mg, 1.88 mol), $K_3PO_4$ (800 mg, 3.77 mmol) and 2-(di-*tert*-butylphosphino)-2',4',6'-triisopropyl-3,6-dimethoxy-1,1'-biphenyl (60.9 mg, 0.13 mmol) were suspended in toluene (7 mL) under argon atmosphere. Suspension was bubbled with argon for 10 min and tBuBrettPhos Pd G3 (53.7 g, 0.06 mmol) was added. Vial was sealed and reaction mixture stirred at 130°C. After 4 h the reaction mixture was cooled to room temperature and EtOAc (20 mL) and water were added (50 mL). Layers were separated and product was extracted with EtOAc (2 x 30 mL). Organic layers were combined, washed with brine (40 mL), dried over $Na_2SO_4$, filtered and evaporated. The crude was purified using FCC (Interchym system, 12 g 15 μm $SiO_2$ column, weak eluent: cyclohexane, strong eluent: cyclohexane/EtOAc = 1/1, 0 - 50 % of strong eluent) to give title compound (276 mg) as a yellow oil.

UPLC-MS/UV (2 min, high pH): RT = 1.20 min, m/z = 339.0 $[M^+H]^+$.

*5,7-Dimethyl-3-(2-pyridyl)-6-[3-(triazol-2-yl)phenyl]pyrrolo[3,4-d]pyridazin-4-one*

According to Scheme 1 Step 4: Prepared as per Final compound 1-26, from ethyl 4-formyl-2,5-dimethyl-1-[3-(triazol-2-yl)phenyl]pyrrole-3-carboxylate and purified by preparative HPLC, yielding the title compound as a white solid.

M.p.: 173°C; UPLC-MS/UV(8 min, high pH): RT = 3.97 min; MS m/z $ES^+$= 384; $^1H$ NMR (500 MHz, DMSO-$d_6$) δ: 8.59 - 8.55 (1H, m), 8.33 (1H, s), 8.28 - 8.24 (1H, m), 8.21 (2H, s), 8.06 (1H, s), 7.95 (1H, dt), 7.85 (1H, t), 7.56 - 7.53 (1H, m), 7.51 (1H, d), 7.46 - 7.41 (1H, m), 2.43 (3H, s), 2.32 (3H, s).

The compounds in the following Table have been synthezised according to the same methods as previous

Examples 1 to 24, as denoted in the column denoted as "Exp. nr". The compounds denoted with the asterisk have been exemplified in the Examples.

**Table 1: Compounds prepared according to the Examples.**

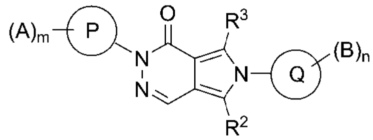

| Co.Nr. | Exp nr. | 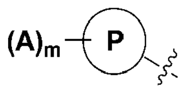 | 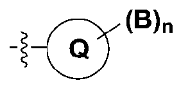 | $R_2$ | $R_3$ |
|---|---|---|---|---|---|
| 1-1 | 4 | 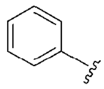 | 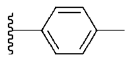 | Me | Me |
| 1-2 | 2* | 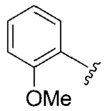 | 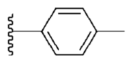 | Me | Me |
| 1-3 | 1 | 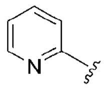 | 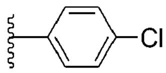 | Me | Me |
| 1-4 | 1 | 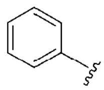 | 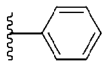 | Me | Me |
| 1-5 | 1 | 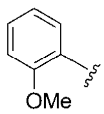 | 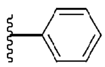 | Me | Me |
| 1-6 | 1 | 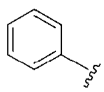 | 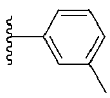 | Me | Me |
| 1-7 | 1 | 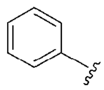 | 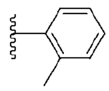 | Me | Me |
| 1-8 | 1 | 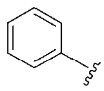 | 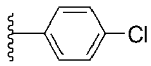 | Me | Me |
| 1-9 | 4 | 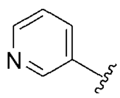 | 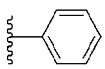 | Me | Me |
| 1-10 | 4 | 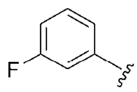 | 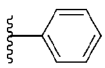 | Me | Me |
| 1-11 | 4 | 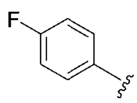 | 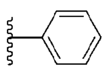 | Me | Me |
| 1-12 | 4 | 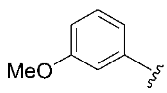 | 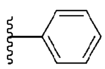 | Me | Me |

(continued)

| Co.Nr. | Exp nr. | 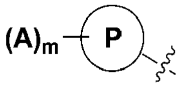 | 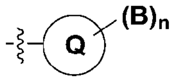 | $R_2$ | $R_3$ |
|---|---|---|---|---|---|
| 1-13 | 4* | 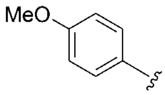 | 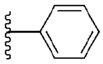 | Me | Me |
| 1-14 | 4 | 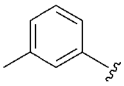 | 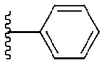 | Me | Me |
| 1-15 | 4 | 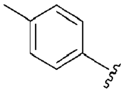 | 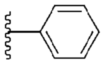 | Me | Me |
| 1-16 | 4 | 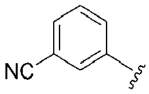 | 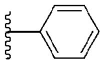 | Me | Me |
| 1-17 | 4 | 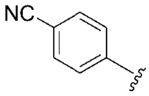 | 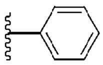 | Me | Me |
| 1-18 | 1 | 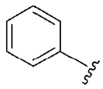 | 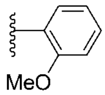 | Me | Me |
| 1-19 | 1 | 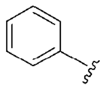 | 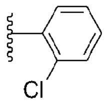 | Me | Me |
| 1-20 | 3 | 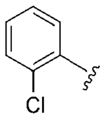 | 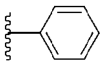 | Me | Me |
| 1-21 | 3* | 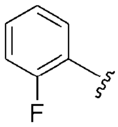 | 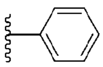 | Me | Me |
| 1-22 | 3 | 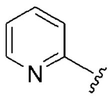 | 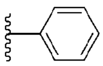 | Me | Me |
| 1-23 | 1 | 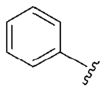 | 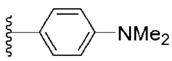 | Me | Me |
| 1-24 | 1 | 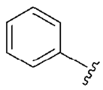 | 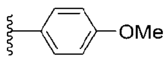 | Me | Me |
| 1-25 | 1 | 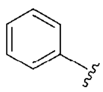 | 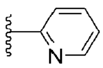 | Me | Me |

(continued)

| Co.Nr. | Exp nr. | 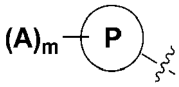 | 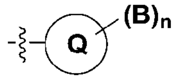 | $R_2$ | $R_3$ |
|---|---|---|---|---|---|
| 1-26 | 1* | 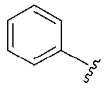 | 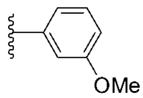 | Me | Me |
| 1-27 | 4 | 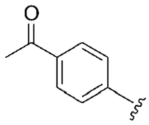 | 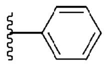 | Me | Me |
| 1-28 | 4 | 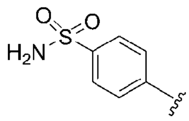 | 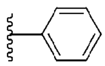 | Me | Me |
| 1-29 | 1 | 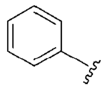 | 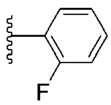 | Me | Me |
| 1-30 | 4 | 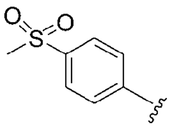 | 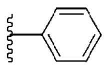 | Me | Me |
| 1-31 | 6 | 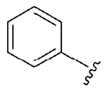 | 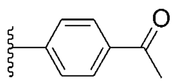 | Me | Me |
| 1-32 | 1 | 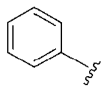 | 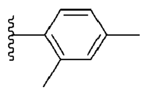 | Me | Me |
| 1-33 | 7 | 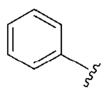 | 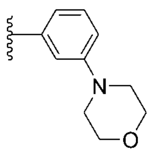 | Me | Me |
| 1-34 | 1 | 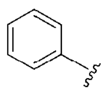 | 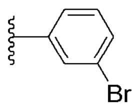 | Me | Me |
| 1-35 | 7 | 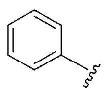 | 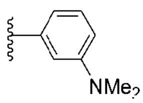 | Me | Me |
| 1-36 | 6* | 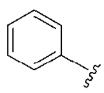 | 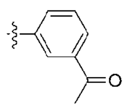 | Me | Me |
| 1-37 | 4 | 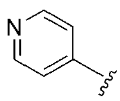 | 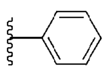 | Me | Me |
| 1-38 | 4 | 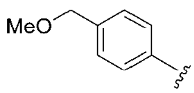 | 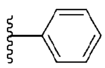 | Me | Me |

(continued)

| Co.Nr. | Exp nr. | 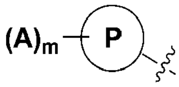 | 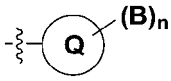 | $R_2$ | $R_3$ |
|---|---|---|---|---|---|
| 1-39 | 1 | 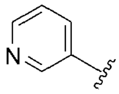 | 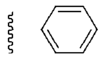 | Me | Me |
| 1-40 | 1 | 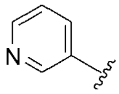 | 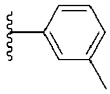 | Me | Me |
| 1-41 | 1 | 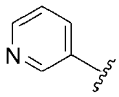 | 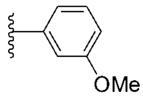 | Me | Me |
| 1-42 | 7 | 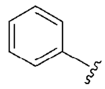 | 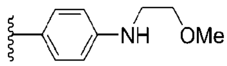 | Me | Me |
| 1-43 | 7* | 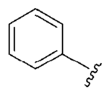 | 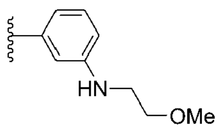 | Me | Me |
| 1-44 | 1 | 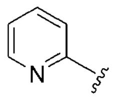 | 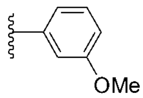 | Me | Me |
| 1-45 | 1 | 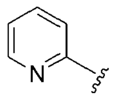 | 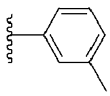 | Me | Me |
| 1-46 | 1 | 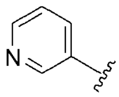 | 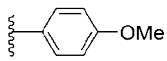 | Me | Me |
| 1-47 | 1 | 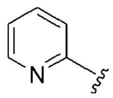 | 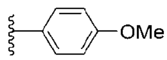 | Me | Me |
| 1-48 | 1 | 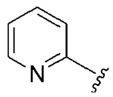 | 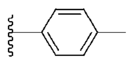 | Me | Me |
| 1-49 | 7 | 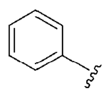 | 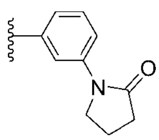 | Me | Me |
| 1-50 | 4 | 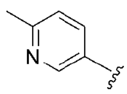 | 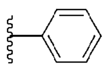 | Me | Me |
| 1-51 | 8* | 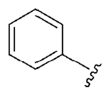 | 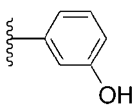 | Me | Me |

(continued)

| Co.Nr. | Exp nr. | 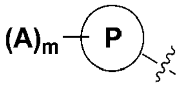 | 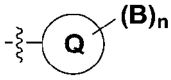 | $R_2$ | $R_3$ |
|---|---|---|---|---|---|
| 1-52 | 4 | 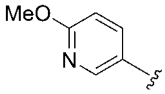 | 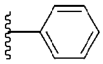 | Me | Me |
| 1-53 | 1 | 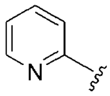 | 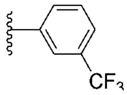 | Me | Me |
| 1-54 | 1 | 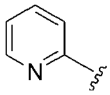 | 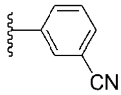 | Me | Me |
| 1-55 | 1 | 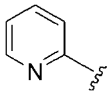 | 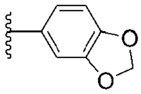 | Me | Me |
| 1-56 | 1 | 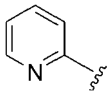 | 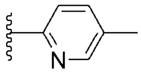 | Me | Me |
| 1-57 | 4 | 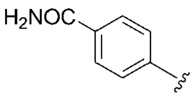 | 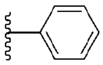 | Me | Me |
| 1-58 | 2 | 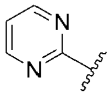 | 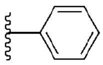 | Me | Me |
| 1-59 | 7 | 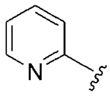 | 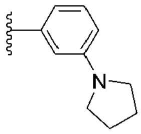 | Me | Me |
| 1-60 | 2 | 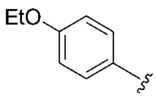 | 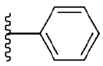 | Me | Me |
| 1-61 | 2 | 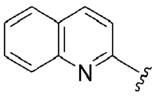 | 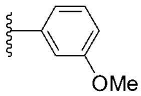 | Me | Me |
| 1-62 | 1 | 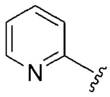 | 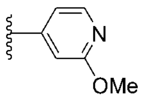 | Me | Me |
| 1-63 | 1 | 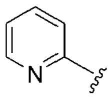 | 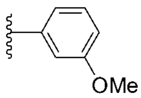 | Me | Me |
| 1-64 | 1 | 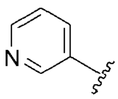 | 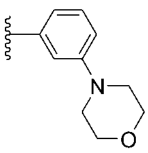 | Me | Me |

(continued)

| Co.Nr. | Exp nr. | 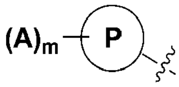 | 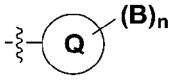 | $R_2$ | $R_3$ |
|---|---|---|---|---|---|
| 1-65 | 4 | 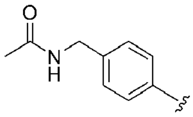 | 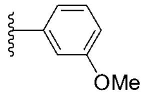 | Me | Me |
| 1-66 | 4 | 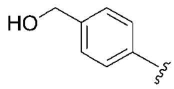 | 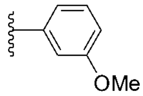 | Me | Me |
| 1-67 | 4 | 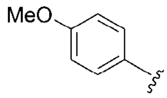 | 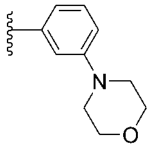 | Me | Me |
| 1-68 | 4 | 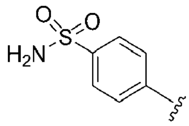 | 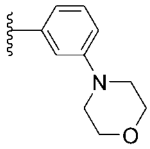 | Me | Me |
| 1-69 | 4 | 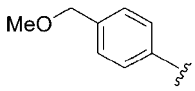 | 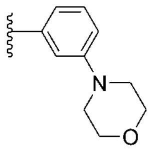 | Me | Me |
| 1-70 | 4 | 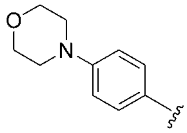 | 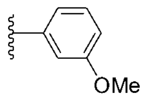 | Me | Me |
| 1-71 | 4 | 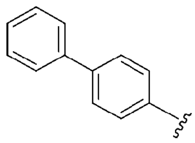 | 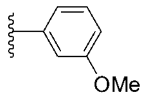 | Me | Me |
| 1-72 | 1 | 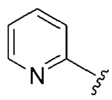 | 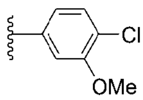 | Me | Me |
| 1-73 | 4 | 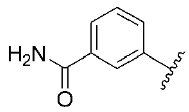 | 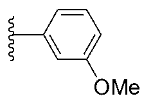 | Me | Me |
| 1-74 | 2 | 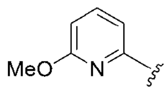 | 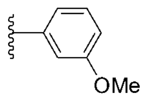 | Me | Me |
| 1-75 | 4 | 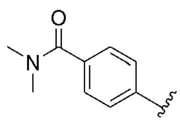 | 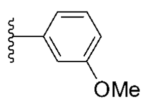 | Me | Me |

(continued)

| Co.Nr. | Exp nr. | 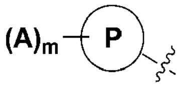 | 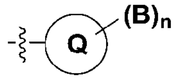 | $R_2$ | $R_3$ |
|---|---|---|---|---|---|
| 1-76 | 11* | 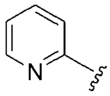 | 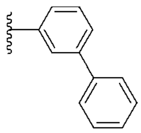 | Me | Me |
| 1-77 | 1 | 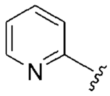 | 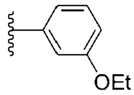 | Me | Me |
| 1-78 | 1 | 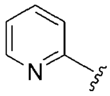 | 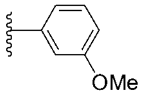 | Me | Et |
| 1-79 | 1 | 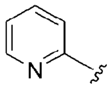 | 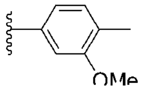 | Me | Me |
| 1-80 | 1 | 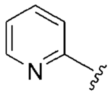 | 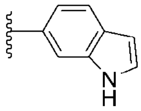 | Me | Me |
| 1-81 | 21 | 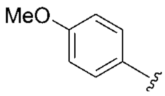 | 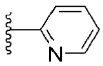 | Me | Me |
| 1-82 | 13 | 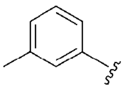 | 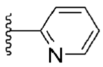 | Me | Me |
| 1-83 | 2 | 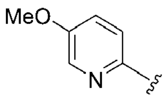 | 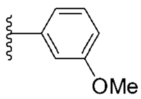 | Me | Me |
| 1-84 | 4 | 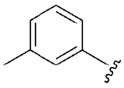 | 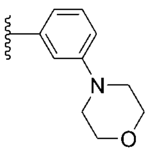 | Me | Me |
| 1-85 | 1 | 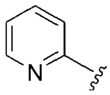 | 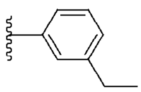 | Me | Me |
| 1-86 | 1 | 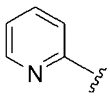 | 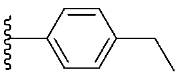 | Me | Me |
| 1-87 | 10 | 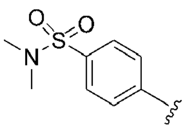 | 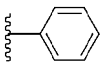 | Me | Me |
| 1-88 | 9* | 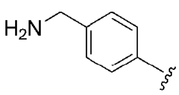 | 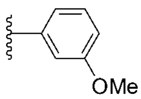 | Me | Me |

(continued)

| Co.Nr. | Exp nr. | 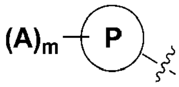 | 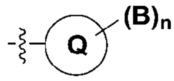 | R2 | R3 |
|---|---|---|---|---|---|
| 1-89 | 1 | 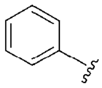 | 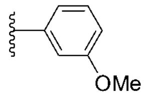 | Me | Et |
| 1-90 | 2 | 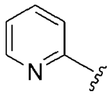 | 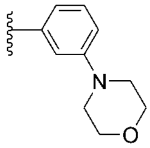 | Me | Me |
| 1-91 | 4 | 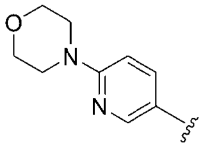 | 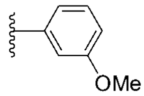 | Me | Me |
| 1-92 | 4 | 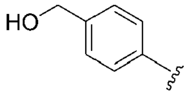 | 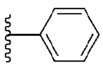 | Me | Me |
| 1-93 | 4 | 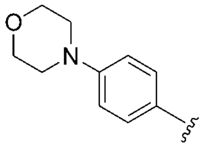 | 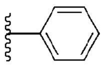 | Me | Me |
| 1-94 | 4 | 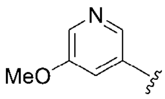 | 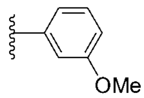 | Me | Me |
| 1-95 | 4 | 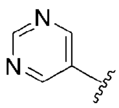 | 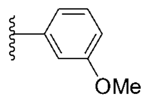 | Me | Me |
| 1-96 | 21* | 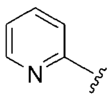 | 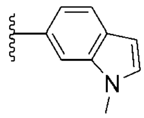 | Me | Me |
| 1-97 | 1 | 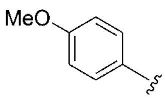 | 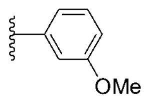 | Me | Me |
| 1-98 | 4 | 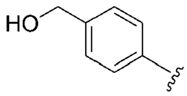 | 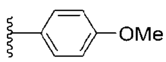 | Me | Me |
| 1-99 | 1 | 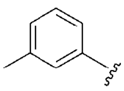 | 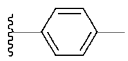 | Me | Me |
| 1-100 | 12* | 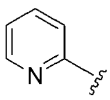 | 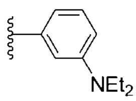 | Me | Me |

(continued)

| Co.Nr. | Exp nr. | 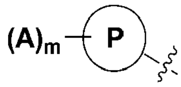 | 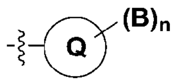 | $R_2$ | $R_3$ |
|---|---|---|---|---|---|
| 1-101 | 5* | 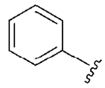 | 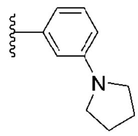 | Me | Me |
| 1-102 | 22* | 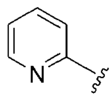 | 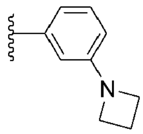 | Me | Me |
| 1-103 | 1 | 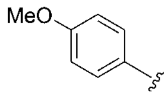 | 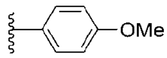 | Me | Me |
| 1-104 | 4 | 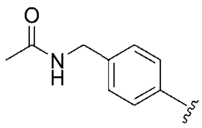 | 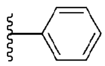 | Me | Me |
| 1-105 | 4 | 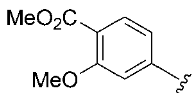 | 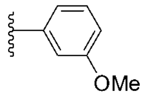 | Me | Me |
| 1-106 | 4 | 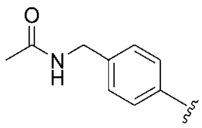 | 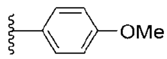 | Me | Me |
| 1-107 | 1 | 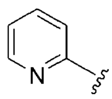 | 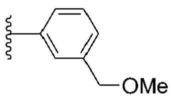 | Me | Me |
| 1-108 | 1 | 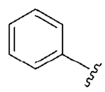 | 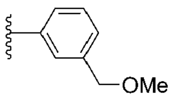 | Me | Me |
| 1-109 | 10* | 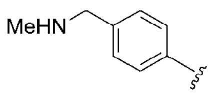 | 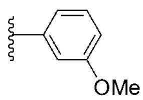 | Me | Me |
| 1-110 | 5 | 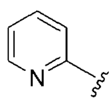 | 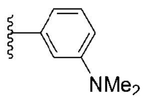 | Me | Me |
| 1-111 | 5 | 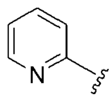 | 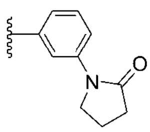 | Me | Me |
| 1-112 | 4 | 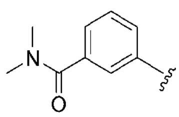 | 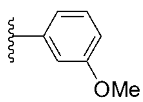 | Me | Me |

(continued)

| Co.Nr. | Exp nr. | 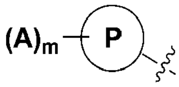 | 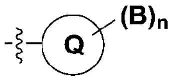 | $R_2$ | $R_3$ |
|---|---|---|---|---|---|
| **1-113** | **4** | 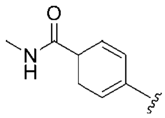 | 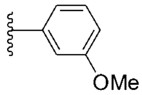 | Me | Me |
| **1-114** | **4** | 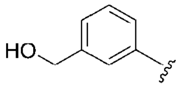 | 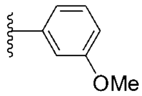 | Me | Me |
| **1-115** | **4** | 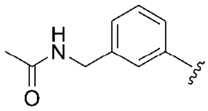 | 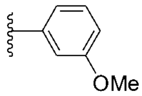 | Me | Me |
| **1-116** | **4** | 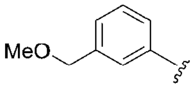 | 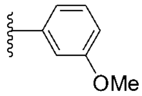 | Me | Me |
| **1-117** | **4** | 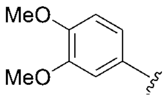 | 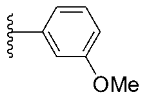 | Me | Me |
| **1-118** | **4** | 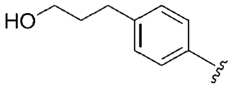 | 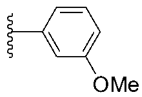 | Me | Me |
| **1-119** | **4** | 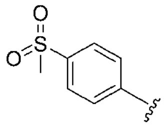 | 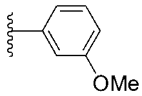 | Me | Me |
| **1-120** | **4** | 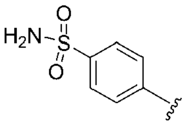 | 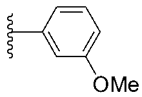 | Me | Me |
| **1-121** | **2** | 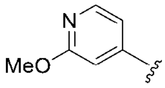 | 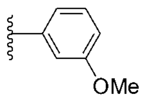 | Me | Me |
| **1-122** | **1** | 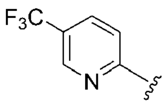 | 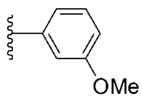 | Me | Me |
| **1-123** | **2** | 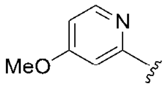 | 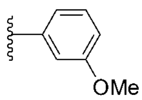 | Me | Me |
| **1-124** | **1** | 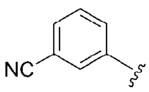 | 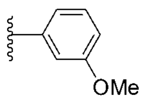 | Me | Me |
| **1-125** | **1** | 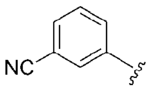 | 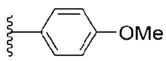 | Me | Me |

(continued)

| Co.Nr. | Exp nr. | 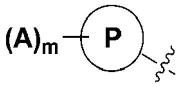 | 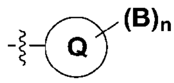 | $R_2$ | $R_3$ |
|---|---|---|---|---|---|
| 1-126 | 1 | 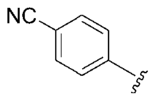 | 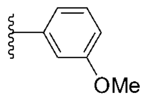 | Me | Me |
| 1-127 | 2 | 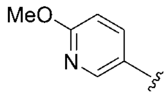 | 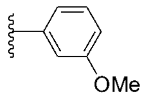 | Me | Me |
| 1-128 | 4 | 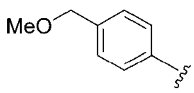 | 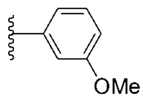 | Me | Me |
| 1-129 | 13 | 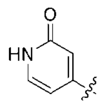 | 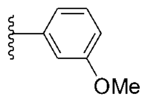 | Me | Me |
| 1-130 | 4 | 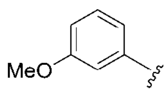 | 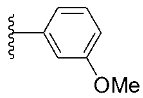 | Me | Me |
| 1-131 | 13* | 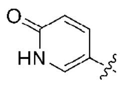 | 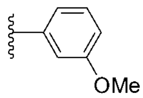 | Me | Me |
| 1-132 | 1 | 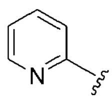 | 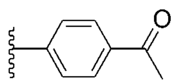 | Me | Me |
| 1-133 | 1 | 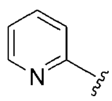 | 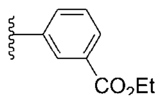 | Me | Me |
| 1-134 | 1 | 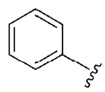 | 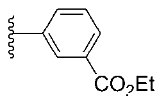 | Me | Me |
| 1-135 | 14* | 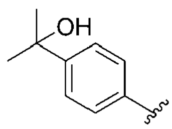 | 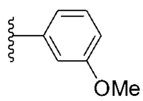 | Me | Me |
| 1-136 | 11 | 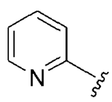 | 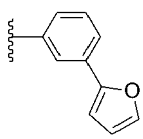 | Me | Me |
| 1-137 | 11 | 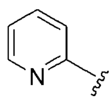 | 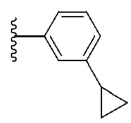 | Me | Me |
| 1-138 | 15* | 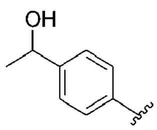 | 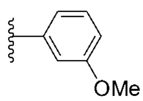 | Me | Me |

(continued)

| Co.Nr. | Exp nr. | 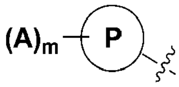 | 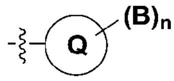 | $R_2$ | $R_3$ |
|---|---|---|---|---|---|
| 1-139 | 1 | 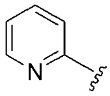 | 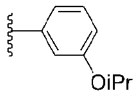 | Me | Me |
| 1-140 | 1 | 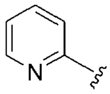 | 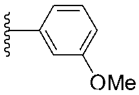 | Et | Me |
| 1-141 | 1 | 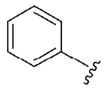 | 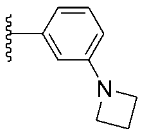 | Me | Me |
| 1-142 | 1 | 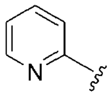 | 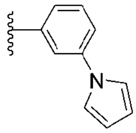 | Me | Me |
| 1-143 | 1 | 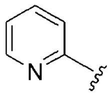 | 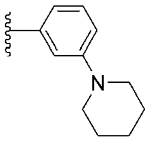 | Me | Me |
| 1-144 | 4 | 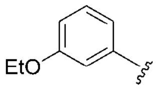 | 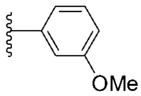 | Me | Me |
| 1-145 | 2 | 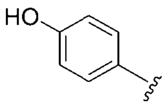 | 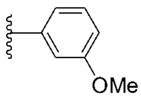 | Me | Me |
| 1-146 | 2 | 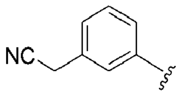 | 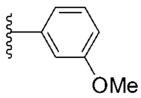 | Me | Me |
| 1-147 | 2 | 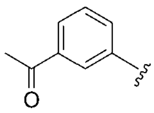 | 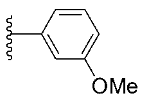 | Me | Me |
| 1-148 | 2 | 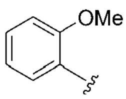 | 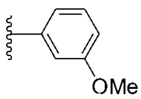 | Me | Me |
| 1-149 | 2 | 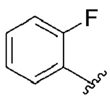 | 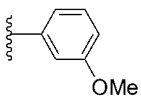 | Me | Me |
| 1-150 | 2 | 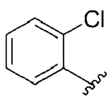 | 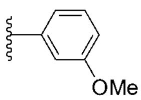 | Me | Me |

(continued)

| Co.Nr. | Exp nr. | 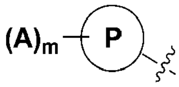 | 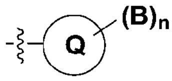 | $R_2$ | $R_3$ |
|---|---|---|---|---|---|
| 1-151 | 2 | 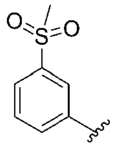 | 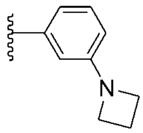 | Me | Me |
| 1-152 | 4 | 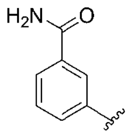 | 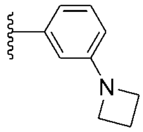 | Me | Me |
| 1-153 | 2 | 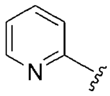 | 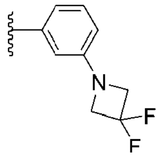 | Me | Me |
| 1-154 | 2 | 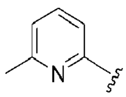 | 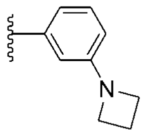 | Me | Me |
| 1-155 | 2 | 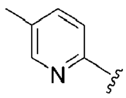 | 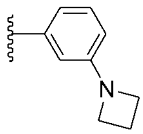 | Me | Me |
| 1-156 | 2 | 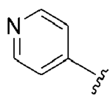 | 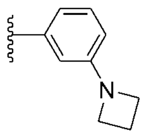 | Me | Me |
| 1-157 | 2 | 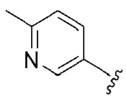 | 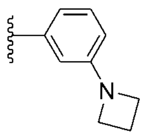 | Me | Me |
| 1-158 | 2 | 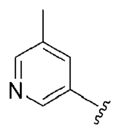 | 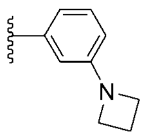 | Me | Me |
| 1-159 | 2 | 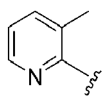 | 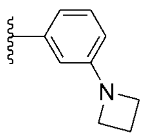 | Me | Me |
| 1-160 | 2 | 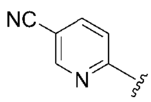 | 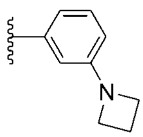 | Me | Me |

(continued)

| Co.Nr. | Exp nr. | 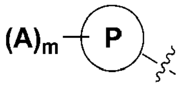 | 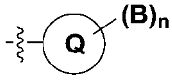 | $R_2$ | $R_3$ |
|---|---|---|---|---|---|
| **1-161** | **2** | 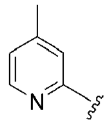 | 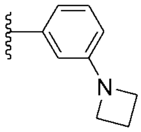 | Me | Me |
| **1-162** | **2** | 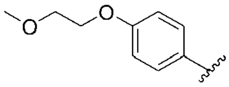 | 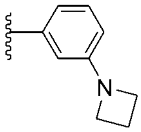 | Me | Me |
| **1-163** | **2** | 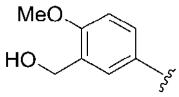 | 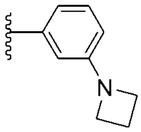 | Me | Me |
| **1-164** | **2** | 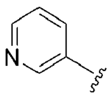 | 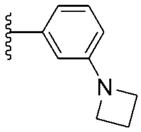 | Me | Me |
| **1-165** | **2** | 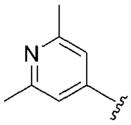 | 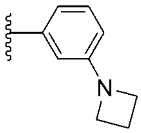 | Me | Me |
| **1-166** | **2** | 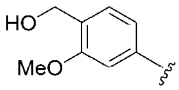 | 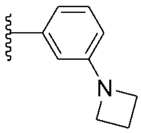 | Me | Me |
| **1-167** | **2** | 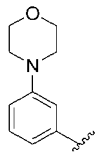 | 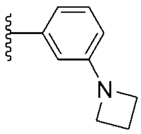 | Me | Me |
| **1-168** | **16*** | 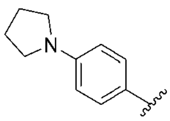 | 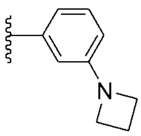 | Me | Me |
| **1-169** | **17*** | 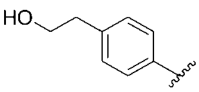 | 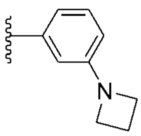 | Me | Me |
| **1-170** | **18*** | 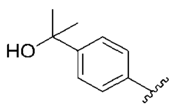 | 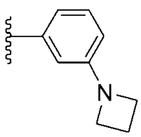 | Me | Me |

(continued)

| Co.Nr. | Exp nr. | 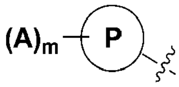 | 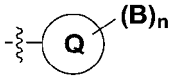 | $R_2$ | $R_3$ |
|---|---|---|---|---|---|
| 1-171 | 2 | 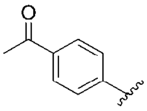 | 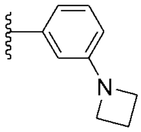 | Me | Me |
| 1-172 | 2 | 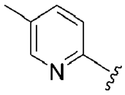 | 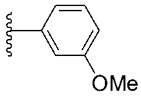 | Me | Me |
| 1-173 | 2 | 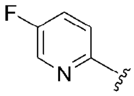 | 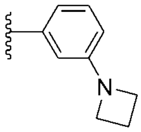 | Me | Me |
| 1-174 | 16 | 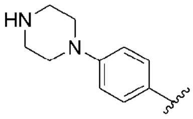 | 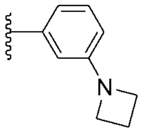 | Me | Me |
| 1-175 | 2 | 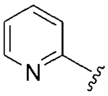 | 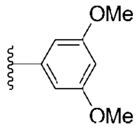 | Me | Me |
| 1-176 | 2 | 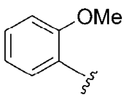 | 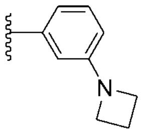 | Me | Me |
| 1-177 | 2 | 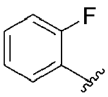 | 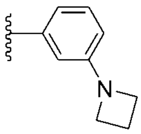 | Me | Me |
| 1-178 | 16 | 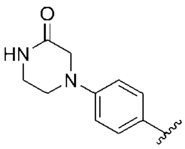 | 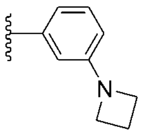 | Me | Me |
| 1-179 | 2 | 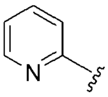 | 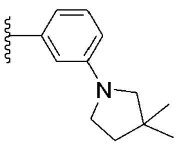 | Me | Me |
| 1-180 | 2 | 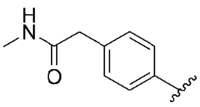 | 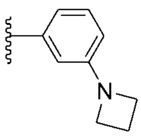 | Me | Me |

(continued)

| Co.Nr. | Exp nr. | 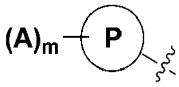 | 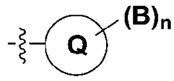 | $R_2$ | $R_3$ |
|---|---|---|---|---|---|
| **1-181** | **2** | 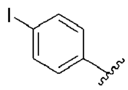 | 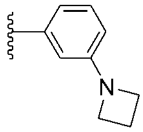 | Me | Me |
| **1-182** | **2** | 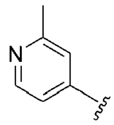 | 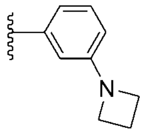 | Me | Me |
| **1-183** | **2** | 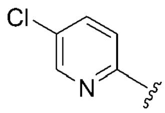 | 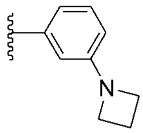 | Me | Me |
| **1-184** | **2** | 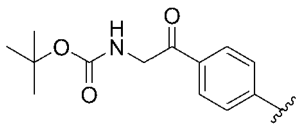 | 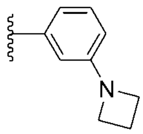 | Me | Me |
| **1-185** | **2** | 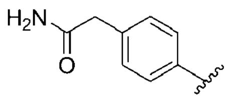 | 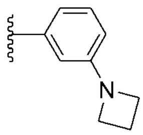 | Me | Me |
| **1-186** | **2** | 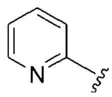 | 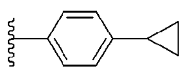 | Me | Me |
| **1-187** | **2** | 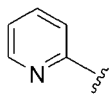 | 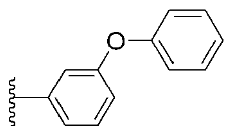 | Me | Me |
| **1-188** | **19*** | 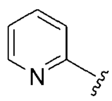 | 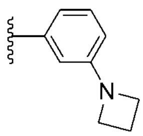 | H | H |
| **1-189** | **20*** | 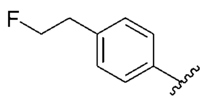 | 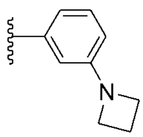 | Me | Me |
| **1-190** | **19** | 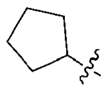 | 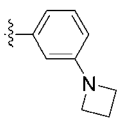 | Me | Me |
| **1-191** | **1** | 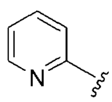 | 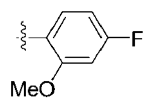 | Me | Me |

(continued)

| Co.Nr. | Exp nr. | 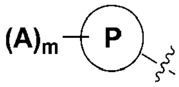 | 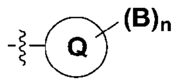 | $R_2$ | $R_3$ |
|---|---|---|---|---|---|
| 1-192 | 7 | 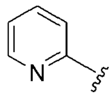 | 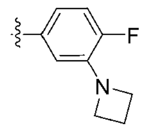 | Me | Me |
| 1-193 | 1 | 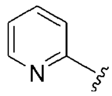 | 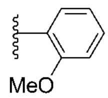 | Me | Me |
| 1-194 | 1 | 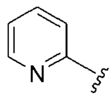 | 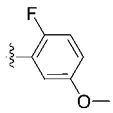 | Me | Me |
| 1-195 | 5 | 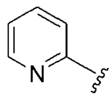 | 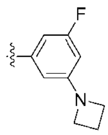 | Me | Me |
| 1-196 | 5 | 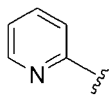 | 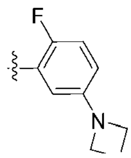 | Me | Me |
| 1-197 | 1 | 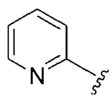 | 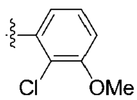 | Me | Me |
| 1-198 | 1 | 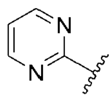 | 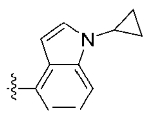 | Me | Me |
| 1-199 | 24* | 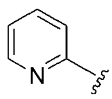 | 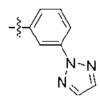 | Me | Me |
| 1-200 | 1 | 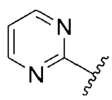 | 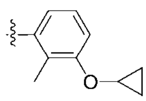 | Me | Me |
| 1-201 | 1 | 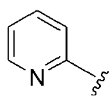 | 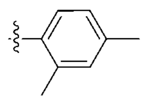 | Me | Me |
| 1-202 | 1 | 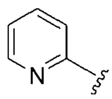 | 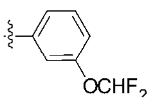 | Me | Me |
| 1-203 | 1 | 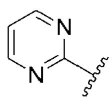 | 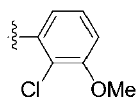 | Me | Me |

(continued)

| Co.Nr. | Exp nr. | 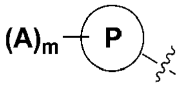 | 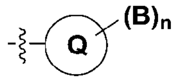 | $R_2$ | $R_3$ |
|---|---|---|---|---|---|
| 1-204 | 1 | 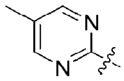 | 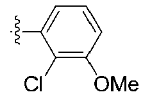 | Me | Me |
| 1-205 | 1 | 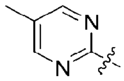 | 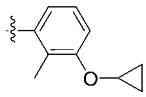 | Me | Me |
| 1-206 | 1 | 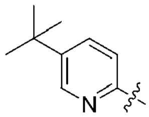 | 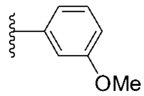 | Me | Me |
| 1-207 | 1 | 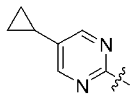 | 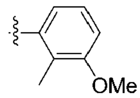 | Me | Me |
| 1-208 | 5 | 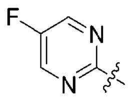 | 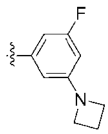 | Me | Me |
| 1-209 | 5 | 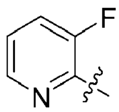 | 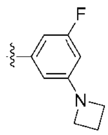 | Me | Me |
| 1-210 | 1 | 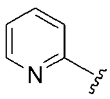 | 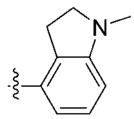 | Me | Me |
| 1-211 | 1 | 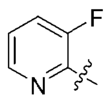 | 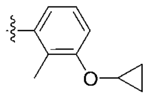 | Me | Me |
| 1-212 | 23 | 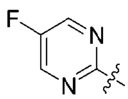 | 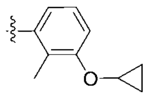 | Me | Me |
| 1-213 | 1 | 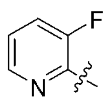 | 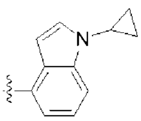 | Me | Me |
| 1-214 | 1 | 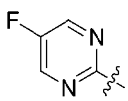 | 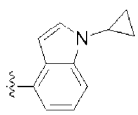 | Me | Me |
| 1-215 | 23* | 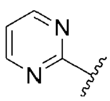 | 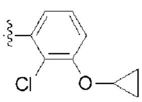 | Me | Me |

(continued)

| Co.Nr. | Exp nr. | 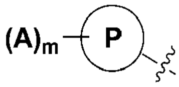 | 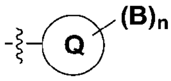 | $R_2$ | $R_3$ |
|---|---|---|---|---|---|
| 1-216 | 1 | 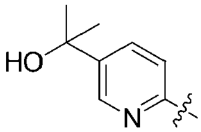 | 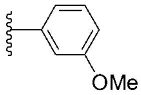 | Me | Me |
| 1-217 | 7 | 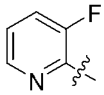 | 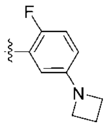 | Me | Me |
| 1-218 | 7 | 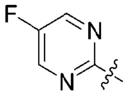 | 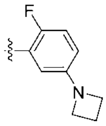 | Me | Me |
| 1-219 | 16 | 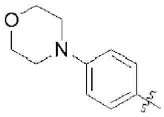 | 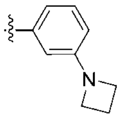 | Me | Me |
| 1-220 | 16 | 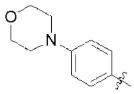 | 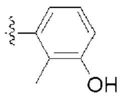 | Me | Me |
| 1-221 | 16 | 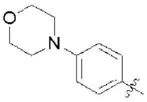 | 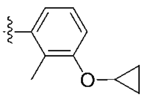 | Me | Me |
| 1-222 | 19 | 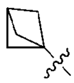 | 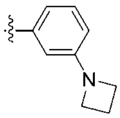 | Me | Me |
| 1-223 | 19 | 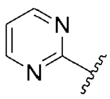 | 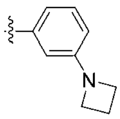 | Me | Me |
| 1-224 | 19 | 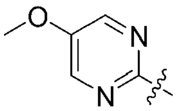 | 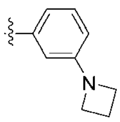 | Me | Me |
| 1-225 | 19 | 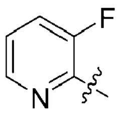 | 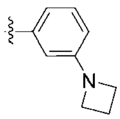 | Me | Me |
| 1-226 | 19 | 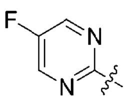 | 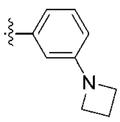 | Me | Me |
| 1-227 | 19 | 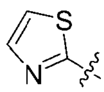 | 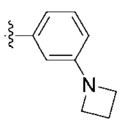 | Me | Me |

(continued)

| Co.Nr. | Exp nr. | 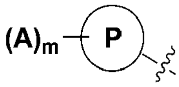 | 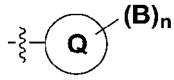 | $R_2$ | $R_3$ |
|---|---|---|---|---|---|
| 1-228 | 19 | 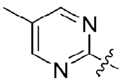 | 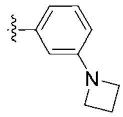 | Me | Me |
| 1-229 | 19 | 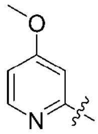 | 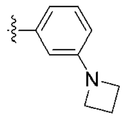 | Me | Me |
| 1-230 | 23 | 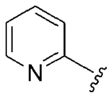 | 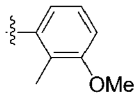 | Me | Me |
| 1-231 | 23 | 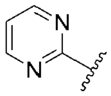 | 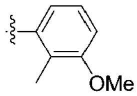 | Me | Me |
| 1-232 | 23 | 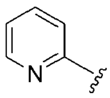 | 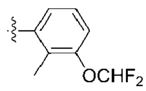 | Me | Me |
| 1-233 | 22 | 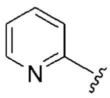 | 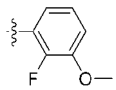 | Me | Me |
| 1-234 | 22 | 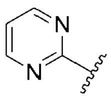 | 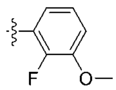 | Me | Me |
| 1-235 | 22 | 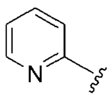 | 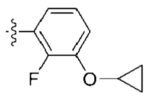 | Me | Me |
| 1-236 | 22 | 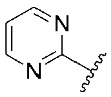 | 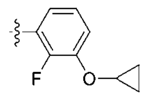 | Me | Me |
| 1-237 | 1 | 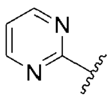 | 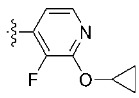 | H | Me |
| 1-238 | 1 | 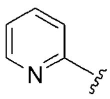 | 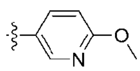 | Me | Me |
| 1-239 | 1 | 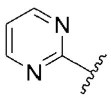 | 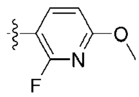 | Me | Me |
| 1-240 | 1 | 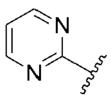 | 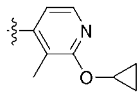 | H | Me |

(continued)

| Co.Nr. | Exp nr. | 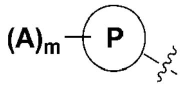 | 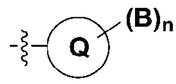 | $R_2$ | $R_3$ |
|---|---|---|---|---|---|
| **1-241** | **1** | 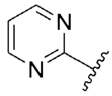 | 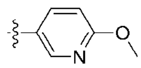 | Me | Me |
| **1-242** | **1** | 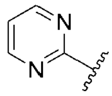 | 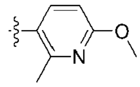 | Me | Me |
| **1-243** | **22** | 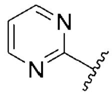 | 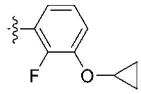 | H | Me |

**Physico-Chemical Data**

Melting points:

a) Melting point determination was performed on a Buchi B-540 apparatus.

b) DSC analysis

DSC data were collected on Mettler Toledo DSC 822$^e$ calorimeter equipped with a refrigerated cooling system. The sample was placed into aluminium DSC pans covered with a pin-hole punched lid. The sample cell was heated at a rate 10°C/min. over the temperature range 25 to 350°C. A nitrogen purge of 50 mL/min. was maintained over the sample. Melting points are expressed as integers at onset temperature of melting.

UPLC-MS methods:

Method 1:

UPLC-MS were recorded on Waters ACQUITY UPLC with the following conditions:
Reverse phase HPLC was carried out on BEH-$C_{18}$ cartridge (1.7 μm, 2.1 x 50 mm) from Waters, with a flow rate of 0.8 mL/min. The gradient conditions used are: 90% A (water + 0.1% of formic acid), 10% B (ACN + 0.1% of formic acid) to 100% B at 1.3 min, kept till 1.7 min and equilibrated to initial conditions at 1.8 min until 2.0 min. Injection volume 5 μL. ES MS detector was used, acquiring both in positive and negative ionization modes.

Method 2:

UPLC-MS were recorded on Waters ACQUITY UPLC with the following conditions:
Reverse phase HPLC was carried out on HSS T3 cartridge (1.8 μm, 2.1 x 50 mm) from Waters, with a flow rate of 0.8 mL/min. The gradient conditions used are: 98% A (water + 0.1% of formic acid), 2% B (ACN + 0.1% of formic acid) to 70% B at 1.3 min, then 90%B at 1.35 min, kept till 1.75 min and equilibrated to initial conditions at 1.8 min until 2.0 min. Injection volume 5 μL. ES MS detector was used, acquiring both in positive and negative ionization modes.

Method 5:

Waters Acquity UPLC coupled with SQD mass spectrometer system; High pH method (2 min)

Reverse phase HPLC was carried out on BEH-$C_{18}$ cartridge (1.7 μm, 2.1 x 50 mm) from Acquity, with a flow rate of 0.9 mL/min. The gradient conditions used are: 97% A (water + 0.05% of ammonia), 3% B (ACN + 0.03% of ammonia) to 97% B at 1.50 min, kept till 1.90 min and equilibrated to the initial conditions at 1.91 min until 2.0 min. Injection volume 2 μL.

ES MS detector was used, acquiring both in positive and negative ionization modes.

Method 6:

Waters Acquity UPLC coupled with SQD mass spectrometer system; Low pH method (2 min)

Reverse phase HPLC was carried out on BEH-$C_{18}$ cartridge (1.7 μm, 2.1 x 50 mm) from Acquity, with a flow rate of 0.9 mL/min. The gradient conditions used are: 97% A (water + 0.1% of formic acid, 3% B (ACN + 0.1% of formic acid) to 97% B at 1.50 min, kept till 1.90 min and equilibrated to the initial conditions at 1.91 min until 2.0 min. Injection volume 2 μL. ES MS detector was used, acquiring both in positive and negative ionization modes.

Method 7:

Waters Acquity UPLC coupled with SQD mass spectrometer system; High pH method (8 min)

Reverse phase HPLC was carried out on BEH-$C_{18}$ cartridge (1.7 μm, 2.1 x 100 mm) from Acquity, with a flow rate of 0.6 mL/min. The gradient conditions used are: 97% A (water + 0.05% of ammonia), 3% B (ACN + 0.05% of ammonia) to 97% B at 7.0 min, kept till 7.50 min and equilibrated to the initial conditions at 7.6 min until 8.0 min. Injection volume 2 μL. ES MS detector was used, acquiring both in positive and negative ionization modes.

Method 8:

Waters Acquity UPLC coupled with SQD mass spectrometer system; Low pH method (8 min)

Reverse phase HPLC was carried out on BEH-$C_{18}$ cartridge (1.7 μm, 2.1 x 100 mm) from Acquity, with a flow rate of 0.6 mL/min. The gradient conditions used are: 97% A (water + 0.1% of formic acid, 3% B (ACN + 0.1% of formic acid) to 97% B at 7.0 min, kept till 7.50 min and equilibrated to initial conditions at 7.6 min until 8.0 min. Injection volume 2 μL. ES MS detector was used, acquiring both in positive and negative ionization modes.

LC-MS methods:

Method 3:

LC-MS were recorded on a Waters Micromass ZQ 2996 system by the following conditions: Reverse phase HPLC was carried out on an Zorbax SB-$C_{18}$ cartridge (1.8 μm, 4.6 x 30 mm) from Agilent, with a flow rate of 1.5 mL/min. The gradient conditions used are: 90 % A (water + 0.05 % of formic acid), 10% B (ACN + 0.05 % of formic acid) to 100 % B at 3.5 min, kept till 3.7 min and equilibrated to initial conditions at 3.8 min until 4.5 min. Injection volume 5-20 μL. ES MS detector was used, acquiring both in positive and negative ionization modes. Cone voltage was 30 V for both positive and negative ionization modes.

Method 4:

LC-MS were recorded on an Agilent Model 1200 Series system by the following conditions: Reverse phase HPLC was carried out on an Atlantis d $C_{18}$ cartridge (3 μm, 50 x 3 mm) from Agilent, with a flow rate of 1.2 mL/min. The gradient conditions used are: 95 % A (water + 0.1 % of formic acid), 5% B (ACN + 0.1 % of formic acid) to 100 % B at 3.5 min, kept till 3.8 min and equilibrated to initial conditions at 3.9 min until 4.5 min. Injection volume 5 μL. ES MS detector was used, acquiring both in positive and negative ionization modes. Cone voltage was 30 V for both positive and negative ionization modes.

Method 9:

LC-MS were recorded on an Agilent Technologies 1260 Infinity LC/MSD system with DAD\ELSD Alltech 3300 and Agilent LC\MSD G6120B mass-spectrometer by the following conditions: Reverse phase UHPLC was carried out on a Poroshell 120 SB-$C_{18}$ cartridge (2.7 μm, 4.6 x 30 mm) with an UHPLC Guard Infinity Lab Poroshell 120 SB-$C_{18}$ cartridge (2.7 μm, 4.6 x 5 mm) from Agilent, with a flow rate at 3 mL/min and a temperature at 60°C. The gradient conditions used are: 1 % A (ACN:water (99:1%) + 0.1 % formic acid), 99 % B (water + 0.1 % formic acid) to 100 % A at 1.5 min, kept till 2.2 min and equilibrated to initial conditions at 2.21 min. Injection volume 0.5 μL. ES MS detector was used, acquiring both in positive and negative ionization modes.

Preparative HPLC purification:

Purifications were run on Agilent 1290 Infinity II series preparative HPLC system (1290 Infinity II Binary pump, 1260 Infinity II Diode Array Detector, LC/MSD mass detector), Waters SunFire ($C_{18}$, 19 x 100 mm, 5 μm) or Waters XBridge ($C_{18}$, 19 x 100 mm, 5 μm) columns using $H_2O$ + 0,1 % formic acid or $H_2O$ + 0,1 % ammonia (25% ammonia in water) and ACN as eluents. Gradients used covered the range from 10% ACN to 100 % ACN.

NMR:

$^1$H NMR spectra were recorded on a Bruker Avance (400MHz, 300MHz) or Varian 400MHz spectrometer. Chemical shifts are expressed in parts per million (ppm, δ units).

Splitting patterns describe apparent multiplicities and are designated as s (singlet), d (doublet), t (triplet), q (quadruplet), m (multiplet), br (broad).

NMR spectra were recorded on Bruker DPX 300 MHz equipped with a RT 5 mm BBI probe, Bruker AV400 MHz equipped with a RT 5 mm BBO probe, Bruker DRX 500 MHz equipped with a RT 5 mm SEI and TXI probes, Bruker Avance III 600 spectrometer equipped with a 5 mm RT BBI probe as well as Varian Unity Plus 400 MHz spectrometer. The samples were recorded at 25°C using DMSO-$d_6$, $CDCl_3$, pyridine-$d_5$ or toluene-$d_8$ as a solvent and TMS as the internal standard

**Table 2: Physico-chemical data.** (RT means retention time in minutes; $[MH]^+$ means the protonated mass of the compound (free base); nd = not determined).

| Co.nr. | Mp (°C) | $[MH]^+$ | RT (min) | MS method | $^1$H-NMR |
|---|---|---|---|---|---|
| **1-1** | 224[a)] | 330 | 2.72 | Method 3 | (300MHz, $CDCl_3$) δ: 8.09 (1H, s), 7.62 (2H, d), 7.50-7.42 (2H, m), 7.40-7.30 (3H, m), 7.13 (2H, d), 2.50 (6H, d), 2.30 (3H, s) |
| **1-2** | 202 [a)] | 360 | 2.83 | Method 3 | (300MHz, $CDCl_3$) δ: 8.05 (1H, s), 7.51 (2H, d), 7.35 (2H, d), 7.15 (2H, d), 7.00 (2H, d), 3.85 (3H, s), 2.55 (6H, s), 2.30 (3H, s) |
| **1-3** | 235 [a)] | 351-353 | 2.28 | Method 1 | (300MHz, $CDCl_3$) δ: 8.70 (1H, d), 8.20 (1H, s), 7.90-7.75 (2H, m), 7.70-7.55 (2H, m), 7.35 (1H, s), 7.30-7.20 (2H, m), 2.55 (3H, s), 2.27 (3H, s) |
| **1-4** | 239 [a)] | 316 | 2.63 | Method 3 | (300MHz, DMSO-$d_6$) δ: 8.32 (1H, s), 7.70-7.31 (10H, m), 2.40 (3H, s), 2.25 (3H, s) |
| **1-5** | 240 [a)] | 346 | 0.99 | Method 1 | (300MHz, $CDCl_3$) δ: 8.01 (1H, s), 7.53-7.44 (3H, m), 7.37-7.27 (2H, m), 7.20-7.15 (2H, m), 7.01-6.96 (3H, m), 3.75 (3H, s), 2.39 (3H, s), 2.20 (3H, s) |
| **1-6** | 172-175 [a)] | 330 | 1.15 | Method 1 | (300MHz, DMSO-$d_6$) δ: 8.34 (1H, s), 7.53-7.21 (9H, m), 2.42 (3H, s), 2.38 (3H, s), 2.25 (3H, s) |
| **1-7** | 130-134 [a)] | 330 | 1.12 | Method 1 | (300MHz, DMSO-$d_6$) δ: 8.35 (1H, s), 7.54-7.42 (7H, m), 7.35-7.30 (2H, m), 2.28 (3H, s), 2.15 (3H, s), 1.90 (3H, s) |
| **1-8** | 223-225 [a)] | 350-352 | 1.15 | Method 1 | (300MHz, DMSO-$d_6$) δ: 8.34 (1H, s), 7.70 (2H, d), 7.53-7.42 (6H, m), 7.35-7.29 (1H, m), 2.39 (3H, s), 2.25 (3H, s) |
| **1-9** | 234 [a)] | 317 | 0.80 | Method 1 | (300MHz, DMSO-$d_6$) δ: 8.78 (1H, d), 8.50 (1H, dd), 8.41 (1H, s), 8.00-7.96 (1H, m), 7.68-7.60 (3H, m), 7.53-7.44 (3H, m), 2.39 (3H, s), 2.25 (3H, s) |

| | | | | | |
|---|---|---|---|---|---|
| **1-10** | nd | 334 | 1.13 | Method 1 | (300MHz, DMSO-$d_6$) δ: 8.40 (1H, s), 7.67-7.60 (3H, m), 7.50-7.40 (5H, m), 7.21-7.15 (1H, m), 2.39 (3H, s), 2.21 (3H, s), 2.40 (3H, s), 2.21 (3H, s) |
| **1-11** | 214 [a)] | 334 | 1.08 | Method 1 | (300MHz, DMSO-$d_6$) δ: 8.40 (1H, s), 7.68-7.60 (2H, m), 7.58-7.51 (3H, m), 7.46-7.44 (2H, m), 7.32-7.24 (2H, m), 2.40 (3H, s), 2.21 (3H, s) |
| **1-12** | Nd | 346 | 1.07 | Method 1 | (300MHz, DMSO-$d_6$) δ: 8.35 (1H, s), 7.68-7.60 (3H, m), 7.47-7.43 (2H, m), 7.38-7.33 (1H, m), 7.11-7.06 (2H, m), 6.93-6.89 (1H, m), 3.80 (3H, s), 2.40 (3H, s), 2.20 (3H, s) |
| **1-13** | Nd | 346 | 1.05 | Method 1 | (300MHz, DMSO-$d_6$) δ: 8.32 (1H, s), 7.68-7.58 (3H, m), 7.46-7.37 (4H, m), 7.01 (2H, d), 3.80 (3H, s), 2.40 (3H, s), 2.21 (3H, s) |
| **1-14** | Nd | 330 | 1.13 | Method 1 | (300MHz, DMSO-$d_6$) δ: 8.31 (1H, s), 7.68-7.60 (3H, m), 7.47-7.44 (2H, m), 7.35-7.27 (3H, m), 7.15-7.13 (1H, m), 2.38 (3H, s), 2.36 (3H, s), 2.25 (3H, s) |
| **1-15** | Nd | 330 | 1.13 | Method 1 | (300MHz, DMSO-$d_6$) δ: 8.31 (1H, s), 7.67-7.59 (3H, m), 7.46-7.43 (2H, m), 7.39-7.35 (2H, m), 7.25-7.23 (2H, m), 2.38 (3H, s), 2.35 (3H, s), 2.24 (3H, s) |
| **1-16** | Nd | 341 | 1.10 | Method 1 | (300MHz, DMSO-$d_6$) δ: 8.42 (1H, s), 8.05 (1H, m), 7.93 (1H, m), 7.79 (1H, m), 7.69-7.60 (4H, m), 7.47-7.44 (2H, m), 2.39 (3H, s), 2.26 (3H, s) |
| **1-17** | 213 [a)] | 341 | 1.10 | Method 1 | (300MHz, DMSO-$d_6$) δ: 8.44 (1H, s), 7.93-7.82 (4H, m), 7.68-7.60 (3H, m), 7.47-7.44 (2H, m), 2.39 (3H, s), 2.25 (3H, s) |
| **1-18** | Nd | 346 | 1.05 | Method 1 | (300MHz, DMSO-$d_6$) δ: 8.31 (1H, s), 7.62-7.56 (1H, m), 7.52-7.29 (7H, m), 7.20-7.15 (1H, m), 3.78 (3H, s), 2.30 (3H, s), 2.17 (3H, |
| **1-19** | 162-164 [a)] | 350-352 | 1.11 | Method 1 | (300MHz, DMSO-$d_6$) δ: 8.36 (1H, s), 7.85-7.82 (1H, m), 7.71-7.62 (3H, m), 7.53-7.42 (4H, m), 7.36-7.30 (1H, m), 2.31 (3H, s), 2.19 (3H, s) |
| **1-20** | Nd | 350-352 | 1.07 | Method 1 | (300MHz, DMSO-$d_6$) δ: 8.32 (1H, s), 7.67-7.57 (4H, m), 7.47-7.42 (5H, m), 2.36 (3H, s), 2.25 (3H, s) |
| **1-21** | 226 [a)] | 334 | 1.05 | Method 1 | (300MHz, DMSO-$d_6$) δ: 8.34 (1H, s), 7.69-7.55 (3H, m), 7.50-7.42 (4H, m), 7.39-7.29 (2H, m), 2.38 (3H, s), 2.36 (3H, s), 2.25 (3H, |
| **1-22** | 180 [a)] | 317 | 0.83 | Method 1 | (300MHz, DMSO-$d_6$) δ: 8.56 (1H, d), 8.31 (1H, s), 7.97-7.90 (1H, m), 7.65-7.60 (3H, m), 7.51-7.40 (4H, m), 2.37 (3H, s), 2.26 (3H, s) |
| **1-23** | 211-213 [a)] | 359 | 1.15 | Method 1 | (300MHz, DMSO-$d_6$) δ: 8.30 (1H, s), 7.52-7.41 (4H, m), 7.34-7.28 (1H, m), 7.20-7.17 (2H, d), 6.87-6.84 (2H, d), 2.99 (6H, s), 2.37 (3H, s), 2.23 (3H, s) |
| **1-24** | 192-194 [a)] | 346 | 1.07 | Method 1 | (300MHz, DMSO-$d_6$) δ: 8.32 (1H, s), 7.52-7.41 (4H, m), 7.38-7.29 (3H, m), 7.18-7.12 (2H, d), 3.85 (3H, s), 2.42 (3H, s), 2.37 (3H, s), 2.24 (3H, s) |

| | | | | | |
|---|---|---|---|---|---|
| **1-25** | 199-201 [a)] | 317 | 0.86 | Method 1 | (300MHz, DMSO-$d_6$) δ: 8.73 (1H, d), 8.35 (1H, s), 8.19-8.13 (1H, m), 7.69-7.64 (2H, m), 7.54-7.42 (4H, m), 7.35-7.30 (1H, m), 2.45 (3H, s), 2.31 (3H, s) |
| **1-26** | 213-215 [a)] | 346 | 1.09 | Method 1 | (300MHz, DMSO-$d_6$) δ: 8.34 (1H, s), 7.56-7.42 (5H, m), 7.35-7.29 (1H, m), 7.18-7.15 (1H, dd), 7.05-6.98 (2H, m), 3.82 (3H, s), 2.40 (3H, s), 2.27 (3H, s) |
| **1-27** | 199-202 [a)] | 358 | 1.05 | Method 1 | (300MHz, DMSO-$d_6$) δ: 8.42 (1H, s), 8.05-8.01 (2H, m), 7.77-7.73 (2H, m), 7.68-7.60 (3H, m), 7.47-7.44 (2H, m), 2.61 (3H, s), 2.39 (3H, s), 2.25 (3H, s) |
| **1-28** | 327-329 [a)] | 395 | 0.89 | Method 1 | (300MHz, DMSO-$d_6$) δ: 8.42 (1H, s), 7.90-7.87 (2H, m), 7.79-7.75 (2H, m), 7.67-7.60 (3H, m), 7.47-7.42 (3H, m), 2.39 (3H, s), 2.26 |
| **1-29** | 199-201 [a)] | 334 | 1.05 | Method 1 | (300MHz, DMSO-$d_6$) δ: 8.35 (1H, s), 7.74-7.57 (3H, m), 7.53-7.42 (5H, m), 7.35-7.30 (1H, m), 2.37 (3H, s), 2.25 (3H, s) |
| **1-30** | 265-268 [a)] | 394 | 0.98 | Method 1 | (300MHz, DMSO-$d_6$) δ: 8.44 (1H, s), 7.99 (2H, d), 7.89 (2H, d), 7.68-7.60 (3H, m), 7.45 (2H, d), 3.26 (3H, s), 2.40 (3H, s), 2.26 (3H, s) |
| **1-31** | 215-217 [a)] | 358 | 0.99 | Method 1 | (300MHz, DMSO-$d_6$) δ: 8.34 (1H, s), 8.19 (2H, d), 7.64 (2H, d), 7.53-7.42 (4H, m), 7.35-7.30 (3H, m), 2.68 (3H, s), 2.41 (3H, s), 2.27 |
| **1-32** | 138-140 [a)] | 344 | 1.20 | Method 1 | (300MHz, DMSO-$d_6$) δ: 8.34 (1H, s), 8.53-7.42 (4H, m), 7.35-7.17 (4H, m), 2.39 (3H, s), 2.27 (3H, s), 2.15 (3H, s), 1.86 (3H, s) |
| **1-33** | 205-207 [a)] | 401 | 1.05 | Method 1 | (300MHz, DMSO-$d_6$) δ: 8.32 (1H, s), 7.52-7.41 (5H, m), 7.34-7.29 (1H, m), 7.13 (1H, dd), 6.96 (1H, s), 6.79 (1H, dd), 3.74 (4H, t), 3.20 (4H, t), 2.40 (3H, s), 2.26 (3H, s) |
| **1-34** | 199-201 [a)] | 394-396 | 1.16 | Method 1 | (300MHz, DMSO-$d_6$) δ: 8.35 (1H, s), 7.84-7.82 (2H, m), 7.63-7.57 (1H, m), 7.52-7.42 (5H, m), 7.35-7.29 (2H, m), 2.40 (3H, s), 2.26 |
| **1-35** | 199-201 [a)] | 359 | 1.15 | Method 1 | (300MHz, DMSO-$d_6$) δ: 8.32 (1H, s), 7.53-7.29 (6H, m), 6.90-6.87 (1H, m), 6.65-6.60 (2H, m), 2.95 (6H, s), 2.41 (3H, s), 2.28 (3H, |
| **1-36** | 202-204 [a)] | 358 | 0.97 | Method 1 | (300MHz, DMSO-$d_6$) δ: 8.36 (1H, s), 8.17-8.14 (1H, m), 8.00 (1H, s), 7.83-7.73 (2H, m), 7.53-7.43 (4H, m), 7.35-7.31 (1H, m), 2.65 (3H, s), 2.40 (3H, s), 2.27 (3H, s) |
| **1-37** | 232-233 [a)] | 317 | 0.75 | Method 1 | (300MHz, DMSO-$d_6$) δ: 8.60 (2H, d), 8.46 (1H, s), 7.77-7.75 (2H, m), 7.65-7.62 (3H, m), 7.47-7.45 (2H, m), 2.40 (3H, s), 2.25 (3H, s) |
| **1-38** | 167-168 [a)] | 360 | 1.05 | Method 1 | (300MHz, DMSO-$d_6$) δ: 8.34 (1H, s), 7.66-7.60 (3H, m), 7.51-7.37 (6H, m), 4.46 (2H, s), 2.38 (3H, s), 2.25 (3H, s) |
| **1-39** | 161-162 [a)] | 331 | 0.90 | Method 1 | (300MHz, DMSO-$d_6$) δ: 8.78 (1H, d), 8.50 (1H, dd), 8.40 (1H, s), 8.00-7.96 (1H, m), 7.50 (1H, dd), 7.38 (4H, dd), 2.43 (3H, s), 2.38 (3H, s), 2.25 (3H, s) |

| | | | | | |
|---|---|---|---|---|---|
| **1-40** | 156-158 a) | 331 | 0.89 | Method 1 | (300MHz, DMSO-$d_6$) δ: 8.78 (1H, d), 8.50 (1H, dd), 8.41 (1H, s), 7.98 (1H, d), 7.54-7.48 (1H, m), 7.43-7.40 (1H, m), 7.27-7.21 (2H, m), 2.42 (3H, s), 2.39 (3H, s), 2.25 (3H, s) |
| **1-41** | 134-136 a) | 347 | 0.83 | Method 1 | (300MHz, DMSO-$d_6$) δ: 8.78 (1H, d), 8.50 (1H, d), 8.41 (1H, s), 7.98 (1H, d), 7.57-7.48 (2H, m), 7.19-7.15 (1H, dd), 7.04-6.98 (2H, m), 3.82 (3H, s), 2.41 (3H, s), 2.27 (3H, s) |
| **1-42** | 207-209 a) | 389 | 1.02 | Method 1 | (300MHz, DMSO-$d_6$) δ: 8.29 (1H, s), 7.52-7.41 (4H, m), 7.33-7.29 (1H, m), 7.06 (2H, d), 6.74 (2H, d), 6.12 (1H, t), 3.53 (2H, t), 3.34-3.23 (5H, m), 2.37 (3H, s), 2.23 (3H, s) |
| **1-43** | 116-119 a) | 389 | 1.04 | Method 1 | (300MHz, DMSO-$d_6$) δ: 8.31 (1H, s), 7.52-7.44 (4H, m), 7.34-7.25 (2H, m), 6.80 (1H, d), 6.53-6.49 (2H, m), 6.07 (1H, t), 3.50 (2H, t), 3.33-3.22 (5H, m), 2.41 (3H, s), 2.27 (3H, s) |
| **1-44** | 145-147 a) | 347 | 0.85 | Method 1 | (300MHz, DMSO-$d_6$) δ: 8.56 (1H, d), 8.31 (1H, s), 7.97-7.91 (1H, m), 7.56-7.41 (3H, m), 7.16 (1H, dd), 7.05-6.99 (2H, m), 3.83 (3H, s), 2.39 (3H, s), 2.27 (3H, s) |
| **1-45** | 152-154 a) | 331 | 0.91 | Method 1 | (300MHz, DMSO-$d_6$) δ: 8.56 (1H, d), 8.31 (1H, s), 7.97-7.91 (1H, m), 7.54-7.40 (4H, m), 7.27-7.22 (2H, m), 2.42 (3H, s), 2.37 (3H, s), 2.25 (3H, s) |
| **1-46** | 160-164 a) | 347 | 0.82 | Method 1 | (300MHz, DMSO-$d_6$) δ: 8.78 (1H, d), 8.50 (1H, dd), 8.40 (1H, s), 8.00-7.96 (1H, m), 7.50 (1H, dd), 7.37 (2H, d), 7.16 (2H, d), 3.85 (3H, s), 2.38 (3H, s), 2.25 (3H, s) |
| **1-47** | 182-183 a) | 347 | 0.84 | Method 1 | (300MHz, DMSO-$d_6$) δ: 8.56 (1H, d), 8.30 (1H, s), 7.98-7.90 (1H, m), 7.50-7.36 (4H, m), 7.17-7.14 (2H, d), 3.86 (3H, s), 2.36 (3H, s), 2.24 (3H, s) |
| **1-48** | 231-233 a) | 331 | 0.92 | Method 1 | (300MHz, DMSO-$d_6$) δ: 8.60-8.54 (1H, m), 8.30 (1H, s), 8.00-7.90 (1H, m), 7.52-7.40 (4H, m), 7.36-7.30 (2H, m), 2.43 (3H, s), 2.36 (3H, s), 2.24 (3H, s) |
| **1-49** | 174-176 a) | 399 | 0.94 | Method 1 | (300MHz, DMSO-$d_6$) δ: 8.34 (1H, s), 7.89-7.82 (1H, m), 7.80-7.77 (1H, m), 7.67-7.59 (1H, m), 7.54-7.40 (4H, m), 7.36-7.28 (1H, m), 7.23-7.18 (1H, m), 3.89 (2H, t), 2.40 (3H, s), 2.27 (3H, s), 2.05 (2H, q) |
| **1-50** | 173-174 a) | 331 | 0.75 | Method 1 | (300MHz, DMSO-$d_6$) δ: 8.61 (1H, d), 8.39 (1H, s), 7.83 (1H, dd), 7.65-7.60 (3H, m), 7.46 (2H, d), 7.35 (1H, d), 2.50 (3H, s), 2.38 (3H, s), 2.25 (3H, s) |
| **1-51** | 252-254 a) | 332 | 0.91 | Method 1 | (300MHz, DMSO-$d_6$) δ: 10.05 (1H, s), 8.32 (1H, s), 7.52-7.39 (5H, m), 7.34-7.29 (1H, m), 6.98 (1H, dd), 6.81 (1H, d), 6.75 (1H, s), 2.39 (3H, s), 2.25 (3H, s) |
| **1-52** | 160-161 a) | 347 | 1.45 | Method 2 | (300MHz, DMSO-$d_6$) δ: 8.36 (1H, s), 8.30 (1H, d), 7.84 (1H, dd), 7.68-7.60 (3H, m), 7.45 (1H, dd), 6.91 (1H, d), 2.38 (3H, s), 2.25 (3H, s) |

| | | | | | |
|---|---|---|---|---|---|
| **1-53** | 161-163 [a)] | 385 | 0.96 | Method 1 | (300MHz, DMSO-$d_6$) δ: 8.57 (1H, dd), 8.34 (1H, s), 8.01-7.82 (5H, m), 7.50 (1H, d), 7.43 (1H, dd), 2.39 (3H, s), 2.27 (3H, s) |
| **1-54** | 215-216 [a)] | 342 | 0.76 | Method 1 | (300MHz, DMSO-$d_6$) δ: 8.57 (1H, dd), 8.33 (1H, s), 8.13-8.08 (2H, m), 7.98-7.92 (1H, m), 7.89-7.82 (2H, m), 7.49 (1H, d), 7.43 (1H, dd), 2.39 (3H, s), 2.27 (3H, s) |
| **1-55** | 214-217 [a)] | 361 | 0.81 | Method 1 | (300MHz, DMSO-$d_6$) δ: 8.57 (1H, dd), 8.29 (1H, s), 7.97-7.91 (1H, m), 7.48 (1H, d), 7.42 (1H, dd), 7.14-7.11 (2H, m), 6.91 (1H, dd), 6.18 (2H, s), 2.39 (3H, s), 2.27 (3H, s) |
| **1-56** | 184-187 [a)] | 332 | 0.72 | Method 1 | (300MHz, DMSO-$d_6$) δ: 8.56 (2H, brs), 8.31 (1H, s), 8.00-7.90 (2H, m), 7.57 (1H, d), 7.51 (1H, d), 7.46-7.40 (1H, m), 2.43 (3H, s), 2.42 (3H, s), 2.30 (3H, s) |
| **1-57** | 265-267 [a)] | 359 | 0.84 | Method 1 | (300MHz, DMSO-$d_6$) δ: 8.39 (1H, s), 8.03 (1H, brs), 7.94 (2H, d), 7.65-7.62 (5H, m), 7.47-7.44 (2H, m), 7.40 (1H, brs), 2.39 (3H, s), 2.25 (3H, s) |
| **1-58** | 236-238 [a)] | 318 | 0.58 | Method 1 | (300MHz, DMSO-$d_6$) δ: 9.00-8.95 (2H, m), 8.30 (1H, s), 7.64-7.56 (4H, m), 7.50-7.42 (2H, d), 2.39 (3H, s), 2.24 (3H, s) |
| **1-59** | 206-207 [a)] | 386 | 1.07 | Method 1 | (300MHz, DMSO-$d_6$) δ: 8.59-8.55 (1H, m), 8.29 (1H, s), 7.98-7.90 (1H, m), 7.52-7.46 (1H, m), 7.45-7.39 (1H, m), 7.38-7.34 (1H, m), 6.70 (1H, dd), 6.55 (1H, dd), 6.46-6.44 (1H, m), 3.26 (4H, dd), 2.41 (3H, s), 2.27 (3H, |
| **1-60** | 177-178 [a)] | 360 | 1.11 | Method 1 | (300MHz, DMSO-$d_6$) δ: 8.29 (1H, s), 7.66-7.60 (3H, m), 7.46-7.43 (2H, m), 7.39-7.36 (2H, m), 6.97 (2H, d), 4.06 (2H, q), 2.37 (3H, s), 2.24 (3H, s), 1.35 (3H, t) |
| **1-61** | 110 [a)] | 397 | 1.02 | Method 1 | (300MHz, DMSO-$d_6$) δ: 8.28-8.19 (3H, m), 7.90-7.80 (2H, m), 7.76-7.69 (1H, m), 7.60-7.53 (1H, m), 7.53-7.45 (1H, m), 7.09 (1H, dd), 6.84 (1H, d), 6.80-6.76 (1H, m), 3.88 (3H, s), 2.54 (3H, s), 2.30 (3H, s) |
| **1-62** | 196-197 [a)] | 348 | 0.75 | Method 1 | (300MHz, DMSO-$d_6$) δ: 8.60-8.52 (1H, m), 8.43 (1H, d), 8.33 (1H, s), 8.00-7.90 (1H, m), 7.52-7.47 (1H, m), 7.46-7.41 (1H, m), 7.17 (1H, dd), 7.11-7.08 (1H, d), 3.95 (3H, s), 2.43 (3H, s), 2.31 (3H, s) |
| **1-63** | 189-190 [a)] | 361 | 0.88 | Method 1 | (300MHz, DMSO-$d_6$) δ: 8.55 (1H, dd), 7.96-7.90 (1H, m), 7.57-7.40 (3H, m), 7.17 (1H, dd), 7.02-6.96 (2H, m), 3.83 (3H, s), 2.45 (3H, s), 2.38 (3H, s), 2.32 (3H, s) |
| **1-64** | 143-145 [a)] | 402 | 0.80 | Method 1 | (300MHz, DMSO-$d_6$) δ: 8.77 (1H, d), 8.50 (1H, dd), 8.40 (1H, s), 8.01-7.95 (1H, m), 7.54-7.46 (1H, m), 7.46-7.42 (1H, m), 7.16-7.10 (1H, m), 6.97-6.95 (1H, m), 6.82-6.77 (1H, m), 3.74 (4H, t), 3.20 (4H, t), 2.40 (3H, |
| **1-65** | 192-194 [a)] | 417 | 0.88 | Method 1 | (300MHz, DMSO-$d_6$) δ: 8.46-8.38 (1H, m), 8.33 (1H, s), 7.57-7.50 (1H, m), 7.46-7.42 (2H, m), 7.33-7.28 (2H, m), 7.17 (1H, ddd), 7.04-7.02 (1H, m), 7.01-6.94 (1H, m), 4.30 (2H, d), 3.83 (3H, s), 2.40 (3H, s), 2.27 (3H, s), 1.89 (3H, s) |

| | | | | | |
|---|---|---|---|---|---|
| **1-66** | 149-150 [a)] | 376 | 0.90 | Method 1 | (300MHz, DMSO-$d_6$) δ: 8.33 (1H, s), 7.58-7.50 (1H, m), 7.47-7.35 (4H, m), 7.19-7.13 (1H, ddd), 7.05-7.02 (1H, m), 7.02-6.97 (1H, m), 5.26 (1H, t), 4.54 (2H, d), 3.83 (3H, s), 2.40 (3H, s), 2.27 (3H, s) |
| **1-67** | 183-185 [a)] | 431 | 1.03 | Method 1 | (300MHz, DMSO-$d_6$) δ: 8.29 (1H, s), 7.49-7.41 (1H, m), 7.41-7.36 (2H, m), 7.16-7.10 (1H, m), 7.01-6.96 (2H, m), 6.95-6.93 (1H, m), 6.81-6.76 (1H, m), 3.80 (1H, s), 3.74 (4H, t), 3.20 (4H, t), 2.40 (3H, s), 2.26 (3H, s) |
| **1-68** | 211-213 [a)] | 480 | 0.88 | Method 1 | (300MHz, DMSO-$d_6$) δ: 8.40 (1H, s), 7.94-7.85 (2H, m), 7.80-7.74 (2H, m), 7.49-7.41 (3H, m), 7.17-7.10 (1H, ddd), 6.97-6.94 (1H, m), 6.81-6.76 (1H, ddd), 3.74 (4H, t), 3.20 (4H, t), 2.41 (3H, s), 2.27 (3H, s) |
| **1-69** | 167-168 [a)] | 445 | 1.03 | Method 1 | (300MHz, DMSO-$d_6$) δ: 8.33 (1H, s), 7.53-7.43 (3H,m), 7.44-7.35 (2H, m), 7.16-7.10 (1H, ddd), 6.97-6.94 (1H, m), 6.80 (1H, ddd), 4.46 (2H, s), 3.74 (4H, t), 3.32 (3H, s), 3.20 (4H, t), 2.41 (3H, s), 2.27 (3H, s) |
| **1-70** | Nd | 431 | 1.02 | Method 1 | (300MHz, DMSO-$d_6$) δ: 8.28 (1H, s), 7.57-7.50 (1H, m), 7.36-7.29 (2H, m), 7.19-7.14 (1H, ddd), 7.04-7.01 (1H, m), 7.01-6.95 (3H, m), 3.83 (3H, s), 3.79-3.74 (4H, m), 3.17-3.11 (4H, m), 2.40 (3H, s), 2.26 (3H, s) |
| **1-71** | Nd | 422 | 1.27 | Method 1 | (300MHz, DMSO-$d_6$) δ: 8.38 (1H, s), 7.77-7.68 (4H, m), 7.66-7.60 (2H, m), 7.55-7.45 (3H, m), 7.42-7.35 (1H, m), 7.20-7.14 (1H, ddd), 7.06-7.03 (1H, m), 7.02-6.97 (1H, m), 3.83 (3H, s), 2.42 (3H, s), 2.28 (3H, s) |
| **1-72** | 230 [a)] | 381-383 | 0.94) | Method 1 | (300MHz, $CDCl_3$) δ: 8.70 (1H, d), 8.20 (1H, s), 7.90-7.80 (2H, m), 7.60 (1H, d), 7.35-7.25 (2H, m), 6.90-6.80 (2H, m), 3.95 (3H, s), 2.55 (3H, s), 2.30 (3H, s) |
| **1-73** | 226-228 [a)] | 389 | 0.86 | Method 1 | (300MHz, DMSO-$d_6$) δ: 8.38 (1H, s), 8.07 (1H, brs), 8.05-8.02 (1H, m), 7.86-7.80 (1H, m), 7.71-7.64 (1H, m), 7.58-7.47 (2H, m), 7.45 (1H, s), 7.20-7.14 (1H, m), 7.06-7.03 (1H, m), 7.03-6.97 (1H, m), 3.80 (3H, s), 2.41 (3H, s), 2.28 (3H, s) |
| **1-74** | Nd | 377 | 0.99 | Method 1 | (300MHz, $CDCl_3$) δ: 8.10 (1H, s), 7.73-7.66 (1H, m), 7.51-7.44 (1H, m), 7.13 (1H, d), 7.07 (1H, dd), 6.84-6.79 (1H, m), 6.78-6.73 (2H, m), 3.89 (3H, s), 3.80 (3H, s), 2.43 (3H, s), 2.21 (3H, s) |
| **1-75** | 201-204 [a)] | 417 | 0.93 | Method 1 | (300MHz, DMSO-$d_6$) δ: 8.37 (1H, s), 7.62-7.46 (5H, m), 7.17 (1H, dd), 7.05-6.99 (2H, m), 3.83 (3H, s), 2.95 (6H, brs), 2.41 (3H, s), 2.27 (3H, s) |
| **1-76** | 106-109 [a)] | 393 | 1.06 | Method 1 | (300MHz, DMSO-$d_6$) δ: 8.58 (1H, dd), 8.33 (1H, s), 7.97-7.89 (2H, m), 7.82-7.69 (4H, m), 7.55-7.39 (6H, m), 2.43 (3H, s), 2.32 (3H, s) |
| **1-77** | Nd | 361 | 0.93 | Method 1 | (300MHz, DMSO-$d_6$) δ: 8.59-8.55 (1H, m), 8.31 (1H, s), 7.98-7.90 (1H, m), 7.56-7.46 (2H, m), 7.45-7.40 (1H, m), 7.18-7.12 (1H, m), 7.04-7.01 (1H, m), 7.00-6.96 (1H, m), 4.10 (2H, q), 2.39 (3H, s), 2.27 (3H, s), 1.35 (3H, t) |

| | | | | | |
|---|---|---|---|---|---|
| **1-78** | Nd | 361 | 0.91 | Method 1 | (300MHz, DMSO-$d_6$) δ: 8.56 (1H, dd), 8.31 (1H, s), 7.99-7.90 (1H, m), 7.58-7.47 (2H, m), 7.46-7.40 (1H, m), 7.18 (1H, dd), 7.07-7.04 (1H, m), 7.04-6.99 (1H, m), 3.83 (3H, s), 2.80 (2H, q), 2.25 (3H, s), 0.96 (3H, t) |
| **1-79** | 139-142 [a)] | 361 | 0.96 | Method 1 | (300MHz, DMSO-$d_6$) δ: 8.59-8.54 (1H, m), 8.31 (1H, s), 7.98-7.90 (1H, m), 7.51-7.46 (1H, m), 7.45-7.40 (1H, m), 7.39-7.35 (1H, m), 7.00 (1H, d), 6.91 (1H, dd), 3.83 (3H, s), 2.40 (3H, s), 2.27 (3H, s), 2.24 (3H, s) |
| **1-80** | 246-249 [a)] | 356 | 0.84 | Method 1 | (300MHz, DMSO-$d_6$) δ: 11.45 (1H, s), 8.58 (1H, dd), 8.31 (1H, s), 7.98-7.91 (1H, m), 7.75 (1H, d), 7.55-7.51 (2H, m), 7.46-7.40 (2H, m), 6.96 (1H, dd), 6.60-6.57 (1H, m), 2.38 (3H, s), 2.26 (3H, s) |
| **1-81** | 194 [a)] | 347 | 0.85 | Method 1 | (300MHz, $CDCl_3$) δ: 8.70-8.60 (1H, m), 8.00 (1H, s), 7.90-7.80 (1H, m), 7.50-7.40 (3H, m), 7.25 (1H, d), 6.95-6.85 (2H, m), 3.80 (3H, s), 2.50 (3H, s), 2.25 (3H, s) |
| **1-82** | Nd | 347 | 0.94 | Method 1 | (300MHz, $CDCl_3$) δ: 8.70-8.60 (1H, m), 8.00 (1H, s), 7.90-7.80 (1H, m), 7.50-7.40 (1H, m), 7.35-7.20 (3H, m), 7.10-7.00 (1H, m), 2.50 (3H, s), 2.35 (3H, s), 2.25 (3H, s) |
| **1-83** | Nd | 377 | 0.89 | Method 1 | (300MHz, $CDCl_3$) δ: 8.30 (1H, brs), 8.10 (1H, brs), 7.60 (1H, s), 7.50-7.43 (1H, m), 7.32 (1H, dd), 7.06 (1H, dd), 6.82 (1H, d), 6.76-6.74 (1H, m), 3.90 (3H, s), 3.86 (3H, s), 2.50 (3H, s), 2.26 (3H, s) |
| **1-84** | 158-160 [a)] | 415 | 1.10 | Method 1 | (300MHz, DMSO-$d_6$) δ: 8.31 (1H, brs), 7.49-7.41 (1H, m), 7.35-7.25 (3H, m), 7.16-7.08 (2H, m), 6.96-6.92 (1H, m), 6.86-6.76 (1H, m), 3.77-3.70 (4H, m), 3.24-3.15 (4H, m), 2.40 (3H, s), 2.36 (3H, s), 2.26 (3H,s) |
| **1-85** | 119-121 [a)] | 345 | 0.99 | Method 1 | (300MHz, DMSO-$d_6$) δ: 8.56 (1H, dd), 8.31 (1H, s), 7.97-7.91 (1H, m), 7.56-7.41 (4H, m), 7.30-7.24 (2H, m), 2.72 (2H, q), 2.38 (3H, s), 2.26 (3H, s), 1.23 (3H, t) |
| **1-86** | 192-194 [a)] | 345 | 0.99 | Method 1 | (300MHz, DMSO-$d_6$) δ: 8.56 (1H, dd), 8.30 (1H, s), 7.97-7.91 (1H, m), 7.50-7.40 (4H, m), 7.37-7.34 (2H, m), 2.73 (2H, q), 2.37 (3H, s), 2.25 (3H, s), 1.26 (3H, t) |
| **1-87** | 224-225 [a)] | 423 | 1.06 | Method 1 | (300MHz, DMSO-$d_6$) δ: 8.44 (1H, s), 7.91-7.80 (4H, m), 7.65-7.63 (3H, m), 7.48-7.45 (2H, m), 2.65 (6H, s), 2.40 (3H, s), 2.26 (3H, |
| **1-88** | 145-155 [a)] | 375 | 0.72 | Method 1 | (300MHz, DMSO-$d_6$) δ: 8.33 (1H, s), 7.57-7.41 (5H, m), 7.16 (1H, dd), 7.03-6.98 (2H, m), 3.87 (2H, s), 3.82 (3H, s), 2.40 (3H, s), |
| **1-89** | 170-172 [a)] | 360 | 1.16 | Method 1 | (300MHz, DMSO-$d_6$) δ: 8.33 (1H, s), 7.60-7.40 (5H, m), 7.36-7.27 (1H, m), 7.18 (1H, dd), 7.06-6.97 (2H, m), 3.83 (3H, s), 2.87-2.76 (2H, m), 2.24 (3H, s), 0.96 (3H, t) |
| **1-90** | Nd | 402 | 0.82 | Method 1 | (300MHz, $CDCl_3$) δ: 8.62 (1H, brs), 8.19 (1H, brs), 7.85-7.76 (2H, m), 7.47-7.40 (1H, m), 7.08-7.02 (1H, m), 7.05 (1H, dd), 6.74-6.68 (2H, m), 3.88 (4H, t), 3.22 (4H, t), 2.51 (3H, s), 2.26 (3H, s) |

| | | | | | |
|---|---|---|---|---|---|
| **1-91** | Nd | 432 | 0.88 | Method 1 | (300MHz, $CDCl_3$) δ: 8.42 (1H, d), 8.06 (1H, s), 7.76 (1H, dd), 7.50-7.43 (1H, m), 7.10-7.03 (1H, m), 6.84-6.78 (1H, dd), 6.75-6.73 (1H, m), 6.69 (1H, d), 3.86 (3H, s), 3.83 (4H, t), 3.53 (4H, t), 2.50 (3H, s), 2.26 (3H, s) |
| **1-92** | 233-235 [a)] | 346 | 0.87 | Method 1 | (300MHz, $CDCl_3$) δ: 8.06 (1H, s), 7.64-7.54 (4H, m), 7.46-7.41 (2H, d), 7.26-7.21 (3H, m), 4.72 (2H, brs), 2.49 (3H, s), 2.26 (3H, s) |
| **1-93** | 226-229 [a)] | 401 | 0.99 | Method 1 | (300MHz, $CDCl_3$) δ: 7.99 (1H, s), 7.56-7.46 (3H, m), 7.46-7.37 (2H, m), 7.19-7.14 (2H, m), 6.95-6.87 (2H, m), 3.81 (4H, t), 3.11 (4H, t), 2.42 (3H, s), 2.18 (3H, s) |
| **1-94** | Nd | 377 | 0.93 | Method 1 | (300MHz, $CDCl_3$) δ: 8.55 (1H, brs), 8.25 (1H, brs), 8.09 (1H, s), 7.61-7.56 (1H, m), 7.51-7.44 (1H, m), 7.10-7.05 (1H, dd), 6.84-6.79 (1H, m), 6.76-6.73 (1H, m), 3.89 (3H, s), 3.86 (3H, s), 2.50 (3H, s), 2.29 (3H, s) |
| **1-95** | Nd | 348 | 0.93 | Method 1 | (300MHz, $CDCl_3$) δ: 9.20 (2H, s), 9.09 (1H, s), 8.13 (1H, s), 7.52-7.45 (1H, m), 7.12-7.06 (1H, m), 6.85-6.79 (1H, m), 6.77-6.73 (1H, m), 3.87 (3H, s), 2.51 (3H, s), 2.28 (3H, s) |
| **1-96** | Nd | 370 | 0.94 | Method 1 | (300MHz, $CDCl_3$) δ: 8.69-8.63 (1H, m), 8.16 (1H, s), 7.85-7.70 (3H, m), 7.29-7.18 (3H, m), 6.93 (1H, dd), 6.61 (1H, dd), 3.84 (3H, s), 2.50 (3H, s), 2.27 (3H, s) |
| **1-97** | 169-170 [a)] | 376 | 1.09 | Method 1 | (300MHz, $CDCl_3$) δ: 8.05 (1H, s), 7.52-7.43 (3H, m), 7.07 (1H, dd), 7.00-6.94 (2H, m), 6.84-6.79 (1H, m), 6.76-6.74 (1H, m), 3.87 (3H, s), 3.84 (3H, s), 2.51 (3H, s), 2.27 (3H, s) |
| **1-98** | 231-232 [a)] | 376 | 0.91 | Method 1 | (300MHz, $CDCl_3$) δ: 8.09 (1H, s), 7.66-7.59 (2H, m), 7.50-7.43 (2H, m), 7.20-7.13 (2H, m), 7.11-7.04 (2H, m), 4.78-4.72 (2H, m), 3.92 (3H, s), 2.50 (3H, s), 2.27 (3H, s) |
| **1-99** | 195-197 [a)] | 344 | 1.24 | Method 1 | (300MHz, $CDCl_3$) δ: 8.08 (1H, s), 7.44-7.31 (5H, m), 7.18-7.09 (3H, m), 2.51 (3H, s), 2.49 (3H, s), 2.42 (3H, s), 2.27 (3H, s) |
| **1-100** | <85°C [a)] | 388 | 0.98 | Method 1 | (300MHz, DMSO-$d_6$) δ: 8.59-8.54 (1H, m), 8.29 (1H, s), 7.98-7.90 (1H, m), 7.51-7.46 (1H, m), 7.45-7.39 (1H, m), 7.39-7.31 (1H, m), 6.82 (1H, dd), 6.60-6.55 (1H, m), 6.52 (1H, dd), 3.37 (4H, q), 2.40 (3H, s), 2.28 (3H, |
| **1-101** | 228-229 [a)] | 385 | 1.29 | Method 1 | (300MHz, DMSO-$d_6$) δ: 8.32 (1H, s), 7.52-7.29 (6H, m), 6.70 (1H, dd), 6.55 (1H, dd), 6.45-6.44 (1H, m), 4.45-4.44 (1H, m), 3.27-3.25 (4H, m), 2.41 (3H, s), 2.28 (3H, s), 1.97- |
| **1-102** | 205-206 [a)] | 372 | 0.96 | Method 1 | (500 MHz, DMSO-$d_6$) δ: 8.56 (1H, d), 8.28 (1H, s), 7.93 (1H, t), 7.48 (1H, d), 7.41 (1H, m), 7.37 (1H, t), 6.63 (1H, d), 6.57 (1H, d), 6.37 (1H, s), 3.85 (4H, t), 2.39 (3H, s), 2.32 (2H, t), 2.26 (3H, s) |
| **1-103** | 199-200 [a)] | 376 | 1.08 | Method 1 | (300MHz, $CDCl_3$) δ: 8.07 (1H, s), 7.55-7.47 (2H, m), 7.19-7.13 (2H, m), 7.10-7.05 (2H, m), 7.01-6.95 (2H, m), 3.91 (3H, s), 3.86 (3H, s), 2.50 (3H, s), 2.27 (3H, s) |

| | | | | | |
|---|---|---|---|---|---|
| **1-104** | 249-251 a) | 387 | 0.89 | Method 1 | (300MHz, $CDCl_3$) δ: 8.09 (1H, s), 7.66-7.55 (6H, m), 7.42-7.35 (2H, m), 7.27-7.23 (2H, m), 5.79 (1H, s), 4.48 (1H, s), 4.46 (1H, s), 2.50 (3H, s), 2.28 (3H, s), 2.04 (3H, s) |
| **1-105** | 175-176 a) | 434 | 1.08 | Method 1 | (300MHz, $CDCl_3$) δ: 8.09 (1H, s), 7.92 (1H, d), 7.51-7.44 (1H, m), 7.40 (1H, d), 7.33 (1H, dd), 7.11-7.05 (1H, m), 6.84-6.78 (1H, m), 6.76-6.73 (1H, m), 3.96 (3H, s), 3.91 (3H, s), 3.87 (3H, s), 2.52 (3H, s), 2.27 (3H, s) |
| **1-106** | 213-214 a) | 417 | 0.88 | Method 1 | (300MHz, $CDCl_3$) δ: 8.08 (1H, s), 7.60-7.53 (2H, m), 7.40-7.34 (2H, m), 7.19-7.12 (2H, m), 7.11-7.03 (2H, m), 5.88 (1H, s), 4.49-4.42 (2H, d), 3.91 (3H, s), 2.49 (3H, s), 2.26 (3H, s), 2.03 (3H, s) |
| **1-107** | Nd | 361 | 0.84 | Method 1 | (300MHz, DMSO-$d_6$) δ: 8.56 (1H, dd), 8.31 (1H, s), 7.97-7.91 (1H, m), 7.65-7.37 (6H, m), 4.53 (2H, s), 3.30 (3H, s), 2.37 (3H, s), 2.26 (3H, s) |
| **1-108** | 141-142 a) | 360 | 1.06 | Method 1 | (300MHz, DMSO-$d_6$) δ: 8.34 (1H, s), 7.65-7.30 (9H, m), 4.52 (2H, s), 3.30 (3H, s), 2.38 (3H, s), 2.25 (3H, s), 2.18 (6H, s) |
| **1-109** | 150-154 a) | 389 | 0.73 | Method 1 | (300MHz, DMSO-$d_6$) δ: 8.33 (1H, s), 7.56-7.39 (5H, m), 7.16 (1H, dd), 7.03-6.98 (2H, m), 3.82 (3H, s), 3.79 (2H, s), 2.40 (3H, s), 2.36 (3H, s), 2.27 (3H, s) |
| **1-110** | 197 a) | 360 | 0.94 | Method 1 | (300MHz, $CDCl_3$) δ: 8.60 (1H, d), 8.05 (1H, s), 7.80-7.60 (2H, m), 7.40-7.30 (2H, m), 6.80-6.70 (1H, m), 6.50 (2H, dd), 2.90 (6H, s), 2.45 (3H, s), 2.20 (3H, s) |
| **1-111** | 120 a) | 400 | 0.75 | Method 1 | (300MHz, $CDCl_3$) δ: 8.60 (1H, d), 8.05 (1H, s), 7.90-7.45 (6H, m), 6.90 (1H, d), 3.85 (2H, t), 2.60 (2H, t), 2.45 (3H, s), 2.30-2.10 (5H, m) |
| **1-112** | 163 a) | 417 | 0.96 | Method 1 | (300MHz, $CDCl_3$) δ: 8.00 (1H, s), 7.70-7.60 (2H, m), 7.55-7.45 (2H, m), 7.35-7.30 (1H, m), 7.00 (1H, dd), 6.80-6.70 (2H, m), 3.80 (3H, s), 3.00 (6H, d), 2.45 (3H, s), 2.20 (3H, s) |
| **1-113** | 203 a) | 403 | 0.90 | Method 1 | (300MHz, $CDCl_3$) δ: 8.10 (1H, s), 7.85 (2H, d), 7.75 (2H, d), 7.55-7.45 (1H, m), 7.10 (1H, dd), 6.80-6.60 (2H, m), 6.30-6.20 (1H, m), 3.90 (3H, s), 3.10 (3H, d), 2.40 (6H, d) |
| **1-114** | 179 a) | 376 | 0.92 | Method 1 | (300MHz, $CDCl_3$) δ: 8.10 (1H, s), 7.65-7.35 (5H, m), 7.10 (1H, dd), 6.90-6.75 (2H, m), 4.75 (2H, s), 3.90 (3H, s), 2.50 (3H, s), 2.30 (3H, s) |
| **1-115** | 115 a) | 417 | 0.92 | Method 1 | (300MHz, $CDCl_3$) δ: 8.10 (1H, s), 7.60-7.25 (4H, m), 7.10 (2H, dd), 6.90-6.75 (2H, m), 5.90-5.80 (1H, m), 4.50 (2H, d), 3.90 (3H, s), 2.50 (3H, s), 2.30 (3H, s), 2.00 (3H, s) |
| **1-116** | 131 a) | 390 | 1.08 | Method 1 | (300MHz, $CDCl_3$) δ: 8.10 (1H, s), 7.60-7.45 (3H, m), 7.35-7.25 (2H, m), 7.10 (1H, dd), 6.85-6.75 (2H, m), 4.55 (2H, s), 3.90 (3H, s), 3.40 (3H, s), 2.55 (3H, s), 2.30 (3H, s) |
| **1-117** | 189 a) | 406 | 1.01 | Method 1 | (300MHz, $CDCl_3$) δ: 8.10 (1H, s), 7.50 (3H, t), 7.20-7.05 (3H, m), 6.90-6.75 (3H, m), 3.90 (9H, d), 2.50 (3H, s), 2.30 (3H, s) |

| | | | | | |
|---|---|---|---|---|---|
| **1-118** | 178 a) | 404 | 0.99 | Method 1 | (300MHz, $CDCl_3$) δ: 8.10 (1H, s), 7.60-7.45 (3H, m), 7.30 (2H, d), 7.10 (1H, dd), 6.85-6.75 (2H, m), 4.55 (2H, s), 3.90 (3H, s), 3.70 (2H, t), 2.55 (3H, s), 2.30 (3H, s), 2.00-1.90 (2H, |
| **1-119** | 123 a) | 424 | 1.00 | Method 1 | (300MHz, $CDCl_3$) δ: 8.12 (1H, s), 8.04-7.92 (4H, m), 7.53-7.45 (1H, m), 7.08 (1H, dd), 6.85-6.78 (1H, m), 6.77-6.73 (1H, m), 3.87 (3H, s), 3.03 (3H, s), 2.51 (3H, s), 2.29 (3H, s) |
| **1-120** | 235-237 a) | 425 | 0.92) | Method 1 | (300MHz, $CDCl_3$) δ: 8.12 (1H, s), 8.00-7.93 (2H, m), 7.88-7.82 (2H, m), 7.53-7.44 (1H, m), 7.09 (1H, dd), 6.85-6.78 (1H, m), 6.77-6.73 (1H, m), 5.01 (2H, s), 3.87 (3H, s), 2.51 (3H, s), 2.28 (3H, s) |
| **1-121** | 170-172 a) | 377 | 1.08 | Method 1 | (300MHz, $CDCl_3$) δ: 8.21 (1H, d), 8.11 (1H, s), 7.51-7.44 (1H, m), 7.42-7.36 (1H, m), 7.24 (1H, d), 7.11-7.05 (1H, m), 6.84-6.78 (1H, m), 6.76-6.73 (1H, m), 3.99 (3H, s), 3.88 (3H, s), 2.52 (3H, s), 2.28 (3H, s) |
| **1-122** | 186-187 a) | 415 | 1.11 | Method 1 | (300MHz, $CDCl_3$) δ: 8.11 (1H, s), 8.07-8.04 (1H, m), 8.02-7.96 (1H, m), 7.64-7.50 (2H, m), 7.20-7.13 (2H, m), 7.13-7.05 (2H, m), 3.92 (3H, s), 2.50 (3H, s), 2.28 (3H, s) |
| **1-123** | 95-97 a) | 377 | 0.81 | Method 1 | (300MHz, $CDCl_3$) δ: 8.74 (1H, s), 8.08 (1H, d), 7.95 (1H, d), 7.50-7.42 (1H, m), 7.07 (1H, dd), 6.80-6.75 (1H, m), 6.72-6.69 (1H, m), 3.90 (3H, t), 3.85 (3H, t), 2.34 (3H, t), 2.21 |
| **1-124** | 122-125 a) | 371 | 1.11 | Method 1 | (300MHz, $CDCl_3$) δ: 8.10 (1H, s), 8.04-8.02 (1H, m), 8.00-7.94 (1H, m), 7.60-7.45 (3H, m), 7.11-7.06 (1H, dd), 6.84-6.79 (1H, m), 6.76-6.74 (1H, m), 3.89 (3H, s), 2.53 (3H, s), 2.30 (3H, s) |
| **1-125** | 186-187 a) | 371 | 1.11 | Method 1 | (300MHz, $CDCl_3$) δ: 8.11 (1H, s), 8.07-8.04 (1H, m), 8.02-7.96 (1H, m), 7.64-7.50 (2H, m), 7.20-7.13 (2H, m), 7.13-7.05 (2H, m), 3.92 (3H, s), 2.50 (3H, s), 2.28 (3H, s) |
| **1-126** | 97-99 a) | 371 | 1.12 | Method 1 | (300MHz, $CDCl_3$) δ: 8.10 (1H, s), 7.90-7.85 (2H, m), 7.75-7.69 (2H, m), 7.52-7.42 (1H, m),7.09 (1H, dd), 6.84-6.79 (1H, m), 6.76-6.73 (1H, m), 3.87 (3H, s), 2.51 (3H, s), 2.28 (3H, |
| **1-127** | 156-157 a) | 377 | 1.04 | Method 1 | (300MHz, $CDCl_3$) δ: 8.42 (1H, d), 8.08 (1H, s), 7.85 (1H, dd), 7.52-7.45 (1H, m), 7.09 (1H, dd), 6.85-6.80 (2H, m), 6.77-6.75 (1H, m), 3.98 (3H, s), 3.88 (3H, s), 2.50 (3H, s), 2.28 (3H, s) |
| **1-128** | 167-169 a) | 390 | 1.09 | Method 1 | (300MHz, $CDCl_3$) δ: 8.07 (1H, s), 7.62-7.56 (2H, m), 7.50-7.43 (1H, m), 7.43-7.38 (2H, m), 7.07 (1H, dd), 6.82 (1H, dd), 6.76-6.74 (1H, m), 4.51 (2H, s), 3.86 (3H, s), 3.37 (3H, s), 2.51 (3H, s), 2.27 (3H, s) |
| **1-129** | 255.5-257 a) | 363 | 0.81 | Method 1 | (300MHz, $CDCl_3$) δ: 8.06 (1H, s), 7.50-7.43 (1H, m), 7.40 (1H, d), 7.12-7.04 (2H, m), 6.95 (1H, dd), 6.79 (1H, dd), 6.74-6.71 (1H, m), 3.86 (3H, s), 2.47 (3H, s), 2.24 (3H, s) |

| | | | | | |
|---|---|---|---|---|---|
| **1-130** | 188-190 a) | 376 | 1.1 | Method 1 | (300MHz, $CDCl_3$) δ: 8.06 (1H, s), 7.51-7.43 (1H, m), 7.39-7.31 (1H, m), 7.21-7.14 (2H, m), 7.10-7.05 (1H, m), 6.91-6.85 (1H, m), 6.85-6.79 (1H, m), 6.76-6.74 (1H, m), 3.86 (3H, s), 3.84 (3H, s), 2.51 (3H, s), 2.27 (3H, s) |
| **1-131** | 207-209 a) | 363 | 0.79 | Method 1 | (300MHz, $CDCl_3$) δ: 8.05 (1H, s), 7.95-7.88 (2H, m), 7.51-7.43 (1H, m), 7.07 (1H, dd), 6.80 (1H, dd), 6.75-6.66 (2H, m), 3.88 (3H, s), 2.51 (3H, s), 2.28 (3H, s) |
| **1-132** | 213-215 a) | 359 | 0.76 | Method 1 | (300MHz, DMSO-$d_6$) δ: 8.57 (1H, dd), 8.33 (1H, s), 8.20-8.17 (2H, m), 7.98-7.92 (1H, m), 7.67-7.64 (2H, m), 7.49 (1H, d), 7.44 (1H, dd), 2.68 (3H, s), 2.40 (3H, s), 2.28 (3H, s) |
| **1-133** | 86-90 a) | 389 | 0.91 | Method 1 | (300MHz, DMSO-$d_6$) δ: 8.57 (1H, dd), 8.33 (1H, s), 8.19-8.15 (1H, m), 7.98-7.92 (2H, m), 7.81-7.79 (2H, m), 7.52-7.48 (1H, m), 7.46-7.41 (1H, m), 4.35 (2H, q), 2.37 (3H, s), 2.26 (3H, s), 1.34 (3H, t) |
| **1-134** | 172-175 a) | 387 | 1.13 | Method 1 | (300MHz, DMSO-$d_6$) δ: 8.35 (1H, s), 8.20-8.15 (1H, m), 7.97-7.94 (1H, m), 7.81-7.76 (2H, m), 7.53-7.42 (4H, m), 7.35-7.30 (1H, m), 7.36-7.34 (2H, m), 2.38 (1H, s), 2.25 (1H, s), 1.34 (3H, t) |
| **1-135** | 103-108 a) | 404 | 1.01 | Method 1 | (300MHz, DMSO-$d_6$) δ: 8.32 (1H, s), 7.59-7.49 (3H, m), 7.42-7.39 (2H, m), 7.18-7.15 (2H, m), 7.04-6.98 (2H, m), 5.08 (1H, s), 3.82 (3H, s), 2.40 (1H, s), 2.26 (1H, s), 1.46 (6H, s) |
| **1-136** | 145-150 a) | 383 | 1.00 | Method 1 | (300MHz, DMSO-$d_6$) δ: 8.57 (1H, dd), 8.33 (1H, s), 7.98-7.90 (2H, m), 7.80 (2H, d), 7.71-7.66 (1H, m), 7.50 (1H, d), 7.45-7.35 (2H, m), 7.16 (1H, d), 6.65 (1H, dd), 2.42 (3H, s), 2.30 (3H, s) |
| **1-137** | 106-109 a) | 357 | 1.00 | Method 1 | (300MHz, DMSO-$d_6$) δ: 8.57 (1H, dd), 8.30 (1H, s), 7.97-7.91 (1H, m), 7.51-7.41 (3H, m), 7.28 (1H, d), 7.20-7.15 (2H, m), 2.37 (3H, s), 2.25 (3H, s), 2.05-1.99 (1H, m), 1.05-0.98 (2H, m), 0.86-0.74 (2H, m) |
| **1-138** | 192-195 a) | 390 | 0.97 | Method 1 | (300MHz, DMSO-$d_6$) δ: 8.32 (1H, d), 7.58-7.50 (1H, m), 7.45-7.37 (4H, m), 7.16 (1H, dd), 7.04-6.97 (2H, m), 5.22 (1H, d), 4.81-4.73 (1H, m), 3.82 (3H, s), 2.40 (3H, s), 2.26 (3H, s), 1.35 (3H, d) |
| **1-139** | 120-125 a) | 375 | 1.01 | Method 1 | (300MHz, DMSO-$d_6$) δ: 8.57 (1H, dd), 8.31 (1H, s), 8.00-7.91 (1H, m), 7.55-7.45 (2H, m), 7.45-7.41 (1H, m), 7.14-7.11 (1H, m), 7.02 (1H, brs), 6.94 (1H, brd), 4.76-4.68 (1H, m), 2.39 (3H, s), 2.27 (3H, s), 1.29 (6H, d) |
| **1-140** | 163-164 a) | 361 | 0.94 | Method 1 | (300MHz, DMSO-$d_6$) δ: 8.57 (1H, dd), 8.33 (1H, s), 7.97-7.92 (1H, m), 7.57-7.41 (3H, m), 7.18 (1H, dd), 7.07-7.00 (2H, m), 3.83 (3H, s), 2.72 (2H, q), 2.37 (3H, s), 1.00 (3H, t) |
| **1-141** | 220-222 a) | 371 | 1.20 | Method 1 | (300MHz, DMSO-$d_6$) δ: 8.32 (1H, s), 8.52-7.29 (6H, m), 6.63 (1H, dd), 6.57 (1H, dd), 6.38-6.36 (1H, m), 3.86 (4H, t), 2.40 (3H, s), 2.32 (2H, t), 2.26 (3H, s) |

| | | | | | |
|---|---|---|---|---|---|
| **1-142** | 180-181 [a)] | 382 | 0.98 | Method 1 | (300MHz, DMSO-$d_6$) δ: 8.57 (1H, dd), 8.34 (1H, s), 7.99-7.92 (1H, m), 7.85 (1H, dd), 7.77-7.80 (1H, m), 7.73-7.66 (1H, m), 7.57-7.54 (2H, m), 7.50 (1H, d), 7.45-7.41 (1H, m), 7.30 (1H, dd), 6.32-6.29 (1H, m), 2.43 (3H, s), 2.32 (3H, s) |
| **1-143** | 174-175 [a)] | 400 | 1.00 | Method 1 | (300MHz, DMSO-$d_6$) δ: 8.56 (1H, dd), 8.30 (1H, s), 7.91-7.99 (1H, m), 7.50-7.38 (3H, m), 7.10 (1H, dd), 6.90-6.93 (1H, m), 6.71 (1H, dd), 3.24 (4H, brt), 2.39 (3H, s), 2.27 (3H, s), 1.59 (6H, brs) |
| **1-144** | Nd | 390 | 3.32 | Method 4 | (400MHz, $CDCl_3$) δ: 8.00 (1H, s), 7.50-7.45 (1H, m), 7.38-7.33 (1H, m), 7.20-7.15 (2H, m), 7.10-7.13 (1H, m), 6.80 (2H, dd), 6.70 (1H, s), 4.10 (2H, q), 3.90 (3H, s), 2.51 (3H, s) |
| **1-145** | Nd | 362 | 2.72 | Method 4 | (400MHz, $CDCl_3$) δ: 8.10 (1H, s), 7.50-7.40 (1H, m), 7.40-7.30 (2H, m), 7.10-7.00 (1H, m), 6.90-6.70 (4H, m), 6.30 (1H, s), 3.90 (3H, s), 2.50 (3H, s), 2.30 (3H, s) |
| **1-146** | Nd | 385 | 3.06 | Method 4 | (400MHz, $CDCl_3$) δ: 8.10 (1H, s), 7.65-7.55 (2H, m), 7.50-7.40 (2H, m), 7.32-7.26 (1H, m), 7.10 (1H, d), 6.80 (1H, d), 6.75 (1H, s), 3.90 (3H, s), 3.80 (2H, s), 2.50 (3H, s), 2.30 |
| **1-147** | Nd | 388 | 3.08 | Method 4 | (400MHz, $CDCl_3$) δ: 8.20 (1H, s), 8.10 (1H, s), 7.90 (1H, d), 7.85 (1H, d), 7.60-7.40 (2H, m), 7.10 (1H, d), 6.85 (1H, d), 6.76 (1H, s), 3.90 (3H, s), 2.65 (3H, s), 2.50 (3H,s), 2.30 |
| **1-148** | Nd | 376 | 2.95 | Method 4 | (400MHz, $CDCl_3$) δ: 8.06 (1H, s), 7.49-7.45 (1H, m), 7.40-7.30 (2H, m), 7.10-7.00 (3H, m), 6.80 (1H, d), 6.70 (1H, s), 3.90-3.80 (6H, m), 2.50 (3H, s), 2.30 (3H, s) |
| **1-149** | Nd | 364 | 3.11 | Method 4 | (400MHz, $CDCl_3$) δ: 8.07 (1H, s), 7.50-7.40 (2H, m), 7.40-7.30 (1H, m), 7.30-7.20 (2H, m), 7.10 (1H, d), 6.80 (1H, d), 6.70 (1H, s), 3.90 (3H, s), 2.50 (3H, s), 2.30 (3H, s) |
| **1-150** | Nd | 380 | 3.13 | Method 4 | (300MHz, $CDCl_3$) δ: 8.08 (1H, s), 7.60-7.40 (3H, m), 7.40-7.30 (2H, m), 7.10 (1H, d), 6.85 (1H, d), 6.75 (1H, s), 3.90 (3H, s), 2.50 (3H, s), 2.30 (3H, s) |
| **1-151** | Nd | 449 | 3.18 | Method 4 | (300MHz, $CDCl_3$) δ: 8.31-8.30 (1H, m), 8.10 (1H, s), 8.05 (1H, d), 7.85 (1H, d), 7.65-7.60 (1H, m), 7.39-7.32 (1H, m), 6.58-6.54 (2H, m), 6.20-6.19 (1H, m), 3.90 (4H, t), 3.10 (3H, s), 2.50 (3H,s), 2.50-2.40 (2H, m), 2.30 (3H,s) |
| **1-152** | Nd | 414 | 2.81 | Method 4 | (300MHz, $CDCl_3$) δ: 8.15-8.10 (1H, m), 8.00-7.90 (1H, m), 7.90-7.80 (1H, m), 7.80-7.70 (1H, m), 7.60-7.50 (2H, m), 7.40-7.30 (1H, m), 7.05-7.00 (1H, m), 6.65-6.50 (2H, m), 6.20-6.18 (1H, m), 4.00-3.90 (4H, m), 2.50 (3H, s), 2.45-2.42 (2H, m), 2.30 (3H, s) |
| **1-153** | Nd | 408 | 2.83 | Method 4 | (300MHz, $CDCl_3$) δ: 8.70-8.60 (1H, brs), 8.10 (1H, s), 7.85-7.65 (2H, m), 7.50-7.40 (2H, m), 6.75-6.60 (2H, m), 6.40 (1H, s), 4.30 (4H, t), 2.50 (3H, s), 2.30 (3H, s) |

| | | | | | |
|---|---|---|---|---|---|
| **1-154** | Nd | 386 | 2.85 | Method 4 | (300MHz, $CDCl_3$) δ: 8.10 (1H, s), 7.70-7.60 (1H, m), 7.45 (1H, d), 7.37-7.34 (1H, m), 7.13 (1H, d), 6.60-6.53 (2H, m), 6.20-6.19 (1H, m), 3.90 (4H, t), 2.60 (3H, s), 2.50 (3H, s), 2.43-2.40 (2H, m), 2.30 (3H, s) |
| **1-155** | Nd | 386 | 2.9 | Method 4 | (300MHz, $CDCl_3$) δ: 8.46 (1H, s), 8.11 (1H, s), 7.60 (2H, s), 7.37-7.33 (1H, m), 6.60-6.50 (2H, m), 6.20-6.19 (1H, m), 3.90 (4H, t), 2.50 (3H, s), 2.45-2.40 (2H, m), 2.40 (3H, s), 2.30 |
| **1-156** | Nd | 372 | 2.45 | Method 4 | (300MHz, $CDCl_3$) δ: 8.60 (2H, s), 8.10 (1H, brs), 7.80 (2H, s), 7.37-7.32 (1H, m), 6.60-6.50 (2H, m), 6.20-6.19 (1H, m), 3.90 (4H, t), 2.50 (3H, s), 2.50-2.40 (2H, m), 2.30 (3H, s) |
| **1-157** | Nd | 386 | 2.52 | Method 4 | (300MHz, $CDCl_3$) δ: 8.80 (1H, s), 8.10 (1H, s), 7.90 (1H, d), 7.37-7.32 (1H, m), 7.20 (1H, d), 6.56-6.51 (2H, m), 6.20-6.19 (1H, m), 3.90 (4H, t), 2.60 (3H, s), 2.50 (3H, s), 2.40-2.30 (2H, m), 2.30 (3H, s) |
| **1-158** | Nd | 386 | 2.78 | Method 4 | (300MHz, $CDCl_3$) δ: 8.75 (1H, brs), 8.40 (1H, brs), 8.10 (1H, s), 7.85 (1H, s), 7.39-7.34 (1H, m), 6.58-6.53 (2H, m), 6.20-6.19 (1H, s), 3.94 (4H, t), 2.50 (3H, s), 2.50-2.45 (2H, m), 2.40 (3H, s), 2.30 (3H, s) |
| **1-159** | Nd | 386 | 2.91 | Method 4 | (300MHz, $CDCl_3$) δ: 8.50-8.48 (1H, m), 8.10 (1H, s), 7.60 (1H, d), 7.37-7.32 (1H, m), 7.27-7.24 (1H, m), 6.50 (2H, d), 6.20-6.19 (1H, m), 3.90 (4H, t), 2.50 (3H, s), 2.50-2.40 (2H, m), 2.30 (6H, s) |
| **1-160** | Nd | 397 | 3.07 | Method 4 | (300MHz, $CDCl_3$) δ: 8.90 (1H, s), 8.20 (1H, s), 8.00 (2H, s), 7.38-7.33 (1H, m), 6.60-6.50 (2H, m), 6.20-6.19 (1H, m), 3.90 (4H, t), 2.50 (3H, s), 2.45-2.40 (2H, m), 2.30 (3H, s) |
| **1-161** | Nd | 386 | 2.81 | Method 4 | (300MHz, $CDCl_3$) δ: 8.50 (1H, d), 8.10 (1H, s), 7.50 (1H, s), 7.34-7.31 (1H, m), 7.10 (1H, d), 6.60-6.50 (2H, m), 6.22-6.20 (1H, m), 3.90 (4H, t), 2.50 (3H, s), 2.50-2.40 (5H, m), 2.30 |
| **1-162** | Nd | 445 | 3.24 | Method 4 | (300MHz, $CDCl_3$) δ: 8.04 (1H, s), 7.48 (2H, d), 7.36-7.31 (1H, m), 6.99 (2H, d), 6.55-6.50 (2H, m), 6.20-6.18 (1H, m), 4.16 (2H, t), 3.92 (4H, t), 3.76 (2H, t), 3.46 (3H, s), 2.50 (3H, s), 2.45 (2H, t), 2.30 (3H, s) |
| **1-163** | Nd | 431 | 2.93 | Method 4 | (300MHz, $CDCl_3$) δ: 8.04 (1H, s), 7.50-7.47 (2H, m), 7.36-7.31 (1H, m), 6.95 (1H, d), 6.55-6.50 (2H, m), 6.20-6.19 (1H, m), 4.70 (2H, s), 6.95-6.90 (7H, m), 2.50 (3H, s), 2.50-2.40 (2H, m), 2.30 (3H, s) |
| **1-164** | Nd | 372 | 2.77 | Method 4 | (300MHz, $CDCl_3$) δ: 8.70 (1H, s), 8.50 (1H, s), 8.10 (1H, s), 8.00 (1H, d), 7.30-7.20 (2H, m), 6.57-6.51 (2H, m), 6.20-6.19 (1H, m), 3.90 (4H, t), 2.50 (3H, s), 2.46-2.41 (2H, m), 2.30 |
| **1-165** | Nd | 400 | 2.47 | Method 4 | (300MHz, $CDCl_3$) δ: 8.10 (1H, s), 7.40 (2H, s), 7.40-7.30 (1H, m), 6.60-6.50 (2H, m), 6.20-6.19 (1H, m), 3.90 (4H, t), 2.60 (6H, s), 2.50 (3H, s), 2.45-2.40 (2H, m), 2.30 (3H, s) |

| | | | | | |
|---|---|---|---|---|---|
| **1-166** | Nd | 431 | 2.96 | Method 4 | (300MHz, $CDCl_3$) δ: 8.06 (1H, s), 7.40-7.30 (2H, m), 7.20-7.17 (2H, m), 6.57-6.51 (2H, m), 6.20-6.19 (1H, m), 4.70 (2H, d), 4.00-3.90 (7H, m), 2.50 (3H, s), 2.45-2.38 (2H, m), 2.30 (3H, s) |
| **1-167** | Nd | 456 | 3.25 | Method 4 | (300MHz, $CDCl_3$) δ: 8.05 (1H, s), 7.36-7.31 (2H, m), 7.15-7.07 (2H, m), 6.89-6.86 (1H, m), 6.56-6.51 (2H, m), 6.20-6.19 (1H, m), 2.90 (4H, t), 3.90-3.80 (4H, m), 3.21-3.18 (4H, m), 2.50 (3H, s), 2.45-2.40 (2H, m), 2.30 (3H, s) |
| **1-168** | Nd | 440 | 3.61 | Method 4 | (300MHz, $CDCl_3$) δ: 8.10 (1H, s), 7.40-7.30 (3H, m), 6.60 (2H, d), 6.55-6.45 (2H, m), 6.20-6.19 (1H, m), 3.90 (4H, t), 3.30 (4H, t), 2.50 (3H, s), 2.45-2.35 (2H, m), 2.30 (3H, s), 2.10-1.90 (4H, m) |
| **1-169** | Nd | 415 | 2.96 | Method 4 | (400MHz, $CDCl_3$) δ: 8.10 (1H, s), 7.50 (2H, d), 7.40-7.30 (3H, m), 6.60-6.50 (2H, m), 6.20-6.19 (1H, m), 4.00-3.80 (6H, m), 3.00-2.80 (2H, t), 2.50 (3H, s), 2.45-2.35 (2H, m), 2.30 (3H, s) |
| **1-170** | Nd | 429 | 3.15 | Method 4 | (400MHz, $CDCl_3$) δ: 8.07 (1H, s), 7.60 (4H, s), 7.37-7.33 (1H, m), 6.57-6.52 (2H, m), 6.20-6.19 (1H, m), 3.90 (4H, t), 2.50 (3H, s), 2.45-2.42 (2H, m), 2.30 (3H, s), 1.60 (6H, s) |
| **1-171** | Nd | 413 | 3.37 | Method 4 | (300MHz, $CDCl_3$) δ: 8.10 (1H, s), 8.05 (2H, d), 7.80 (2H, d), 7.37-7.32 (1H, m), 6.57-6.51 (2H, m), 6.20-6.19 (1H, m), 3.90 (4H, t), 2.63 (3H, s), 2.50 (3H, s), 2.40 (2H, t), 2.30 (3H, s) |
| **1-172** | Nd | 361 | 2.69 | Method 4 | (300MHz, $CDCl_3$) δ: 8.50 (1H, brs), 8.10 (1H, brs), 7.60 (2H, s), 7.60-7.43 (1H, m), 7.10 (1H, d), 6.80 (1H, d), 6.75 (1H, s), 3.90 (3H, s), 2.50 (3H, s), 2.40 (3H, s), 2.30 (3H, s) |
| **1-173** | Nd | 390 | 3.00 | Method 4 | (300MHz, $CDCl_3$) δ: 8.65 (1H, s), 8.34-8.29 (1H, m), 8.19-8.18 (1H, m), 7.45-7.44 (1H, m), 7.35-7.29 (1H, m), 6.55-6.45 (2H, dd), 6.13 (1H, s), 3.95-3.90 (4H, m), 2.46-2.41 (2H, m), 2.35 (3H, s), 2.22 (3H, s) |
| **1-174** | Nd | 455 | 2.35 | Method 4 | (300MHz, $CDCl_3$) δ: 8.04 (1H, s), 7.44 (2H, d), 7.40-7.30 (1H, m), 6.98 (2H, d), 6.54-6.53 (2H, m), 6.20-6.19 (1H, m), 3.93 (4H, t), 3.19-3.16 (4H, m), 3.07-3.05 (4H, m), 2.51 (3H, s), 2.45-2.30 (2H, m), 2.27 (3H, s), 1.95 (1H, brs) |
| **1-175** | Nd | 377 | 2.70 | Method 4 | (300MHz, $CDCl_3$) δ: 8.67 (1H, brs), 8.15 (1H, brs), 7.83-7.60 (2H, m), 7.26-7.25 (1H, m), 6.61-6.60 (1H, m), 6.37-6.36 (2H, m), 3.80 (6H, s), 2.53 (3H, s), 2.30 (3H, s) |
| **1-176** | Nd | 401 | 3.16 | Method 4 | (300MHz, $CDCl_3$) δ: 8.05 (1H, s), 7.38-7.33 (3H, m), 7.04-7.02 (2H, m), 6.55-6.52 (2H, m), 6.20-6.19 (1H, m), 3.92 (4H, t), 3.83 (3H, s), 2.50 (3H, s), 2.45-2.40 (2H, m), 2.28 (3H, |
| **1-177** | Nd | 389 | 3.33 | Method 4 | (300MHz, $CDCl_3$) δ: 8.06 (1H, s), 7.50-7.40 (1H, m), 7.37-7.31 (2H, m), 7.24-7.20 (2H, m), 6.60-6.50 (2H, m), 6.20-6.19 (1H, m), 3.93 (4H, t), 2.51 (3H, s), 2.45-2.40 (2H, m), 2.28 |

| | | | | | |
|---|---|---|---|---|---|
| **1-178** | Nd | 469 | 2.84 | Method 4 | (300MHz, $CDCl_3$) δ: 8.05 (1H, s), 7.50 (2H, d), 7.36-7.31 (1H, m), 6.95 (2H, d), 6.56-6.51 (2H, m), 6.20-6.19 (1H, m), 6.05 (1H, brs), 3.95-3.90 (6H, m), 3.52-3.47 (4H, m), 2.51 (3H, s), 2.47-2.40 (2H, m), 2.28 (3H, s) |
| **1-179** | Nd | 414 | 3.36 | Method 4 | (300MHz, $CDCl_3$) δ: 8.67 (1H, brs), 8.16 (1H, brs), 7.85-7.74 (3H, m), 7.40-7.35 (1H, m), 6.66-6.60 (1H, m), 6.50 (1H, d), 6.25-6.20 (1H, m), 3.40 (2H, t), 3.10 (2H, s), 2.55 (3H, s), 2.32 (3H, s), 1.85 (2H, t), 1.19 (6H, s) |
| **1-180** | Nd | 442 | 2.85 | Method 4 | (300MHz, $CDCl_3$) δ: 8.08 (1H, s), 7.60 (2H, d), 7.37-7.31 (3H, m), 6.57-6.51 (2H, m), 6.20-6.19 (1H, m), 7.40 (1H, brs), 3.93 (4H, t), 3.64 (2H, s), 2.75-2.74 (3H, m), 2.52 (3H, s), 2.48-2.38 (2H, m), 2.29 (3H, s) |
| **1-181** | Nd | 497 | 3.85 | Method 4 | (300MHz, $CDCl_3$) δ: 8.10 (1H, s), 7.76-7.73 (2H, m), 7.43-7.40 (2H, m), 7.37-7.31 (1H, m), 6.57-6.50 (2H, m), 6.19-6.18 (1H, m), 3.92 (4H, t), 2.50 (3H, s), 3.45-3.40 (2H, m), 2.28 |
| **1-182** | Nd | 386 | 2.43 | Method 4 | (300MHz, $CDCl_3$) δ: 8.55-8.54 (1H, m), 8.12 (1H, s), 7.68 (1H, s), 7.60-7.59 (1H, m), 7.36-7.30 (1H, m), 6.59-6.51 (2H, m), 6.21-6.20 (1H, m), 3.95 (4H, t), 2.63 (3H, s), 2.53 (3H, s), 2.45-2.40 (2H, m), 2.29 (3H, s) |
| **1-183** | Nd | 406 | 3.19 | Method 4 | (300MHz, $CDCl_3$) δ: 8.60-8.59 (1H, m), 8.14 (1H, s), 7.76-7.75 (2H, m), 7.38-7.33 (1H, m), 6.55-6.52 (2H, m), 6.21-6.20 (1H, m), 3.94 (4H, t), 2.52 (3H, s), 2.50-2.40 (2H, m), 2.29 |
| **1-184** | Nd | 472; 528 | 3.56 | Method 4 | (300MHz, $CDCl_3$) δ: 8.05-8.04 (1H, m), 8.02 (2H, d), 7.86 (2H, d), 7.36-7.32 (1H, m), 6.57-6.51 (2H, m), 6.20-6.18 (1H, m), 5.60 (1H, brs), 4.75-4.65 (2H, m), 3.93 (4H, t), 2.52 (3H, s), 2.45-2.39 (2H, m), 2.29 (3H, s), 1.49 (9H, |
| **1-185** | Nd | 428 | 2.75 | Method 4 | (300MHz, $CDCl_3$) δ: 8.08 (1H, s), 7.65 (2H, d), 7.37-7.32 (3H, m), 6.57-6.51 (2H, m), 6.20-6.19 (1H, m), 5.43-5.33 (2H, m), 3.96-3.91 (4H, t), 3.65 (2H, s), 2.52 (3H, s), 2.46-2.41 (2H, m), 2.29 (3H, s) |
| **1-186** | Nd | 357 | 2.88 | Method 4 | nd |
| **1-187** | Nd | 409 | 3.05 | Method 4 | (300MHz, $CDCl_3$) δ: 8.67 (1H, brs), 8.14 (1H, s), 7.84-7.78 (1H, m), 7.78-7.73 (1H, m), 7.55-7.52 (1H, m), 7.40-7.30 (2H, m), 7.30-7.25 (2H, m), 7.25-7.15 (2H, m), 7.10 (2H, d), 7.00 (1H, d), 6.82-6.80 (1H, m), 2.53 (3H, s), 2.30 |
| **1-188** | 190-192 [a)] | 345 | 0.84 | Method 1 | (300MHz, $CDCl_3$) δ: 8.60 (1H, s), 8.21 (1H, s), 7.91-7.62 (3H, m), 7.38 (1H, d), 7.28-7.18 (2H, m), 6.78-6.71 (1H, m), 6.43-6.37 (2H, m), 3.88 (4H, t), 2.35 (2H, q) |
| **1-189** | Nd | 417 | 1.24 | Method 1 | (300MHz, $CDCl_3$) δ: 8.06 (1H, s), 7.54 (2H, d), 7.37-7.26 (3H, m), 6.56 (2H, t), 6.20 (1H, s), 4.41 (1H, t), 4.55 (1H, t), 3.93 (4H, t), 3.09 (1H, t), 3.02 (1H, t), 2.51 (3H, s), 2.41 (1H, t), 2.28 (3H, s) |

| | | | | | |
|---|---|---|---|---|---|
| **1-190** | Nd | 362 363 | 4.47 | Method 9 | (400 MHz, DMSO-$d_6$) δ 1.60 (m, 2H), 1.79 (m, 4H), 1.85 (m, 2H), 2.21 (s, 3H), 2.32 (m, 2H), 2.38 (s, 3H), 3.84 (t, 4H), 5.37 (m, 1H), 6.32 (s, 1H), 6.57 (m, 2H), 7.36 (t, 1H), 8.20 |
| **1-191** | 195 [b)] | 365 | 3.94 | Method 5 | (500 MHz, DMSO-$d_6$) δ: 8.58 - 8.54 (1H, m), 8.28 (1H, s), 7.94 (1H, dt), 7.50 (1H, d), 7.47 (1H, dd), 7.42 (1H, dd), 7.29 (1H, dd), 7.01 (1H, dt), 3.82 (3H, s), 2.29 (3H, s), 2.17 (3H, |
| **1-192** | Nd | 390 | 4.56 | Method 5 | (500 MHz, DMSO-d6) δ: 8.58 - 8.54 (1H, m), 8.29 (1H, s), 7.94 (1H, dt), 7.48 (1H, d), 7.44 - 7.40 (1H, m), 7.26 (1H, dd), 6.73 - 6.69 (1H, m), 6.55 (1H, dd), 4.01 - 3.93 (4H, m), 2.40 (3H, s), 2.35 - 2.29 (2H, m), 2.27 (3H, s) |
| **1-193** | Nd | 347 | 3.82 | Method 5 | (500 MHz, $CDCl_3$) δ: 8.65 (1H, dd), 8.15 (1H, s), 7.80 (1H, dt), 7.72 (1H, d), 7.52 (1H, dt), 7.26 - 7.24 (1H, m), 7.19 (1H, dd), 7.14 - 7.11 (2H, m), 3.81 (3H, s), 2.44 (3H, s), 2.21 (3H, |
| **1-194** | Nd | 365 | 3.97 | Method 5 | (500 MHz, $CDCl_3$) δ: 8.66 (1H, d), 8.15 (1H, s), 7.83 – 7.79 (1H, m), 7.72 (1H, d), 7.28 - 7.25 (2H, m), 7.08 - 7.05 (1H, m), 6.80 - 6.78 (1H, m), 3.85 (3H, s), 2.52 (3H, s), 2.29 (3H, |
| **1-195** | Nd | 390 | 4.60 | Method 5 | (500 MHz, DMSO-$d_6$) δ: 8.58 - 8.55 (1H, m), 8.30 (1H, s), 7.96 - 7.91 (1H, m), 7.48 (1H, d), 7.45 - 7.40 (1H, m), 6.58 (1H, d), 6.40 (1H, d), 6.25 (1H, s), 3.89 (4H, t), 2.42 (3H, s), 2.37 - 2.31 (2H, m), 2.29 (3H, s) |
| **1-196** | 233 [b)] | 390 | 4.30 | Method 5 | (500 MHz, DMSO-$d_6$) δ: 8.56 (1H, dd), 8.31 (1H, s), 7.94 (1H, dt), 7.50 (1H, d), 7.44 - 7.41 (1H, m), 7.36 (1H, t), 6.63 - 6.60 (1H, m), 6.54-6.52 (1H, m), 3.84 (4H, t), 2.38 (3H, s), 2.34 - 2.28 (2H, m), 2.27 (3H, s) |
| **1-197** | Nd | 381 383 | 3.97 | Method 6 | (300 MHz, DMSO-$d_6$) δ: 8.57 - 8.55 (1H, m), 8.31 (1H, s), 7.94 (1H, dt), 7.58 (1H, t), 7.50 (1H, d), 7.44 - 7.40 (2H, m), 7.19 (1H, dd), 3.96 (3H, s), 2.29 (3H, s), 2.18 (3H, s) |
| **1-198** | 118 [b)] | 397 | 4.30 | Method 5 | (500 MHz, DMSO-$d_6$) δ: 8.98 (2H, d), 8.32 (1H, d), 7.80 (1H, d), 7.62 (1H, t), 7.48 (1H, d), 7.40 (1H, t), 7.15 (1H, d), 5.97 (1H, d), 3.59 - 3.52 (1H, m), 2.29 (3H, s), 2.19 (3H, s), 1.14 - 1.08 (2H, m), 1.07 - 1.02 (2H, m) |
| **1-199** | 173 [b)] | 384 | 3.97 | Method 5 | (500 MHz, DMSO-$d_6$) δ: 8.59 - 8.55 (1H, m), 8.33 (1H, s), 8.28 - 8.24 (1H, m), 8.21 (2H, s), 8.06 (1H, s), 7.95 (1H, dt), 7.85 (1H, t), 7.56 - 7.53 (1H, m), 7.51 (1H, d), 7.46 - 7.41 (1H, m), 2.43 (3H, s), 2.32 (3H, s) |
| **1-200** | Nd | 388 | 4.39 | Method 5 | (500 MHz, DMSO-$d_6$) δ: 8.95 (2H, d), 8.30 (1H, s), 7.60 (1H, t), 7.52 - 7.41 (2H, m), 7.96 (1H, dd), 3.97 - 3.92 (1H, m), 2.35 (3H, s), 2.15 (3H, s), 1.60 (3H, s), 0.88 - 0.81 (2H, m), 0.77 - 0.70 (2H, m) |
| **1-201** | 243 [b)] | 345 | 4.41 | Method 5 | (500 MHz, DMSO-$d_6$) δ: 8.58 - 8.55 (1H, m), 8.31 (1H, s), 7.97 - 7.92 (1H, m), 7.51 (1H, d), 7.45 - 7.41 (1H, m), 7.34 (1H, s), 7.27 - 7.24 (1H, m), 7.20 (1H, d), 2.40 (3H, s), 2.26 (3H, s), 2.16 (3H, s), 1.86 (3H, s) |

| | | | | | |
|---|---|---|---|---|---|
| **1-202** | 135 b) | 383 | 4.11 | Method 5 | (500 MHz, DMSO-$d_6$) δ: 8.58 - 8.55 (1H, m), 8.32 (1H, s), 7.95 (1H, dt), 7.70 (1H, t), 7.49 (1H, d), 7.45 - 7.40 (2H, m), 7.39 (1H, t), 7.38 (1H, s), 7.36 (1H, d), 2.40 (3H, s), 2.28 (3H, s) |
| **1-203** | 151 b) | 382<br>384 | 3.65 | Method 5 | (300 MHz, DMSO-$d_6$) δ: 8.96 (2H, d), 8.32 (1H, s), 7.62 - 7.56 (2H, m), 7.43 (1H, dd), 7.21 (1H, dd), 3.96 (3H, s), 2.28 (3H, s), 2.19 (3H, s) |
| **1-204** | 218 b) | 396<br>398 | 3.75 | Method5 | (300 MHz, DMSO-$d_6$) δ: 8.78 (2H, s), 8.29 (1H, s), 7.58 (1H, t), 7.43 (1H, dd), 7.20 (1H, dd), 3.96 (3H, s), 2.36 (3H, s), 2.27 (3H, s), 2.18 (3H, s) |
| **1-205** | 158 b) | 402 | 4.45 | Method 5 | (300 MHz, DMSO-$d_6$) δ: 8.78 (2H, s), 8.28 (1H, s), 7.51 – 7.41 (2H, m), 6.95 (1H, dd), 3.98-3.92 (1H, m), 2.36 (3H, s), 2.24 (3H, s), 2.15 (3H, s), 1.63 (3H, s), 0.88 - 0.83 (2H, m), 0.79 - 0.70 (2H, m) |
| **1-206** | 187 b) | 403 | 5.06 | Method 5 | (300 MHz, DMSO-$d_6$) δ: 8.58 (1H, d), 8.28 (1H, s), 7.93 (1H, dd), 7.53 (1H, t), 7.39 (1H, d), 7.15 (1H, dd), 7.04 – 6.97 (2H, m), 3.82 (3H, s), 2.38 (3H, s), 2.26 (3H, s), 1.36 (9H, s) |
| **1-207** | 253 b) | 402 | 4.43 | Method 5 | (500 MHz, DMSO-$d_6$) δ: 8.70 (2H, s), 8.30 (1H, s), 7.43 (1H, t), 7.21 (1H, d), 6.94 (1H, d), 3.89 (3H, s), 2.25 (3H, s), 2.16 (3H, s), 2.10 - 2.02 (1H, m), 1.70 (3H, s), 1.13 – 1.07 (2H, m), 0.97 - 0.90 (2H, m) |
| **1-208** | Nd | 409 | 4.61 | Method 5 | (500 MHz, DMSO-$d_6$) δ: 9.05 (2H, m), 8.30 (1H, m), 6.62 - 6.57 (1H, m), 6.42 - 6.38 (1H, m), 6.27 (1H, s), 3.88 (4H, t), 2.40 (3H, s), 2.37 - 2.31 (2H, m), 2.30 (3H, s) |
| **1-209** | 154 b) | 408 | 4.78 | Method 5 | (DMSO-d6, 300 MHz) δ: 8.47 - 8.42 (1H, m), 8.34 (1H, s), 7.98 - 7.89 (1H, m), 7.65 - 7.58 (1H, m), 6.64 - 6.58 (1H, m), 6.44 - 6.35 (1H, m), 6.30 - 6.25 (1H, m), 3.88 (4H, t), 2.41 (3H, s), 2.37 - 2.24 (5H, m) |
| **1-210** | Nd | 372 | 4.17 | Method 6 | (300 MHz, DMSO-$d_6$) δ: 8.57 - 8.53 (1H, m), 8.28 (1H, s), 7.93 (1H, dt), 7.49 (1H, d), 7.44 - 7.38 (1H, m), 7.24 (1H, t), 6.66 (1H, d), 6.52 (1H, d), 3.38 - 3.30 (2H, m), 2.78 (3H, s), 2.56 (2H, t), 2.32 (3H, s), 2.21 (3H, s) |
| **1-211** | 199 b) | 405 | 4.96 | Method 5 | (300 MHz, DMSO-$d_6$) δ: 8.47 - 8.43 (1H, m), 8.36 (1H, s), 7.99 - 7.90 (1H, m), 7.65 - 7.57 (1H, m), 7.53 - 7.41 (2H, m), 7.00 – 6.93 (1H, m), 4.00 - 3.92 (1H, m), 2.26 (3H, s), 2.16 (3H, s), 1.66 (3H, s), 0.89 - 0.80 (2H, m), 0.78 - 0.69 (2H, m) |
| **1-212** | 188 b) | 406 | 4.61 | Method 5 | (300 MHz, DMSO-$d_6$) δ: 9.06 (2H, s), 8.32 (1H, s), 7.54 - 7.40 (2H, m), 6.97 (1H, dd), 4.00 - 3.92 (1H, m), 2.25 (3H, s), 2.17 (3H, s), 1.65 (3H, s), 0.90 - 0.82 (2H, m), 0.80 - 0.68 |

| | | | | | |
|---|---|---|---|---|---|
| **1-213** | 207 [b)] | 414 | 4.88 | Method 5 | (300 MHz, DMSO-$d_6$) δ: 8.46 - 8.44 (1H, m), 8.36 (1H, s), 7.97 - 7.91 (1H, m), 7.79 (1H, d), 7.64 - 7.58 (1H, m), 7.47 (1H, d), 7.39 (1H, t), 7.14 (1H, d), 5.98 (1H, dd), 3.60 - 3.52 (1H, m), 2.28 (3H, s), 2.18 (3H, s), 1.14 - 1.08 (2H, m), 1.05 - 1.00 (2H, m) |
| **1-214** | Nd | 415 | 4.56 | Method 5 | (300 MHz, DMSO-$d_6$) δ: 9.06 (2H, s), 8.33 (1H, s), 7.79 (1H, d), 7.47 (1H, d), 7.39 (1H, t), 7.14 (1H, d), 5.96 (1H, d), 3.59 - 3.52 (1H, m), 2.27 (3H, s), 2.17 (3H, s), 1.14 - 1.07 (2H, m), 1.05 - 1.00 (2H, m) |
| **1-215** | 228 [b)] | 408<br>410 | 4.16 | Method 5 | (300 MHz, DMSO-$d_6$) δ: 8.96 (2H, d), 8.31 (1H, s), 7.72 - 7.66 (1H, m), 7.65 - 7.57 (2H, m), 7.27 - 7.20 (1H, m), 4.12 - 4.03 (1H, m), 2.28 (3H, m), 2.18 (3H, s), 0.93 - 0.84 (2H, m) |
| **1-216** | 177 [b)] | 405 | 3.88 | Method 6 | (600 MHz, $CDCl_3$) δ: 8.70 (1H, s), 8.13 (1H, s), 7.92 (1H, dd), 7.63 (1H, d), 7.47 (1H, t), 7.07 (1H, dd), 6.84 - 6.81 (1H, m), 6.76 (1H, s), 3.86 (3H, s), 2.51 (3H, s), 2.48 (1H, bs), 2.27 (3H, s), 1.61 (6H, s) |
| **1-217** | 225 [b)] | 408 | 4.61 | Method 6 | (500 MHz, DMSO-$d_6$) δ: 8.48 – 8.42 (1H, m), 8.36 (1H, s), 7.96 - 7.92 (1H, m), 7.63 - 7.60 (1H, m), 7.36 (1H, t), 6.63 - 6.60 (1H, m), 5.56 - 6.54 (1H, m), 3.83 (4H, t), 2.38 (3H, s), 2.34 – 2.29 (2H, m), 2.79 (3H, s) |
| **1-218** | Nd | 409 | 4.38 | Method 6 | (600 MHz, $CDCl_3$) δ: 8.73 (2H, bs), 8.10 (1H, s), 7.15 (1H, t), 6.55 - 6.49 (1H, m), 6.21 (1H, dd), 3.90 (4H, t), 2.50 (3H, s), 2.42 (2H, quin), 2.28 (3H, s) |
| **1-219** | 229 [b)] | 456 | 5.15 | Method 6 | (600 MHz, DMSO-$d_6$) δ: 8.26 (1H, s), 7.37 (1H, t), 7.34 - 7.31 (2H, m), 7.00 - 6.96 (2H, m), 6.62 (1H, dq), 6.57 (1H, dq), 6.35 (1H, t), 3.85 (4H, t), 3.76 (4H, t), 3.14 (4H, t), 2.39 (3H, s), 2.32 (2H, quin), 2.25 (3H, s) |
| **1-220** | 276.8 [b)] | 431 | 4.13 | Method 6 | (500 MHz, DMSO-$d_6$) δ: 9.99 (1H, s), 8.27 (1H, s), 7.33 (2H, d), 7.23 (1H, t), 7.04 - 6.95 (3H, m), 6.72 (1H, d), 3.79 - 3.71 (4H, m), 3.17 - 3.10 (4H, m), 2.27 (3H, s), 2.14 (3H, s), 1.65 (3H, s) |
| **1-221** | Nd | 471 | 5.41 | Method 7 | (500 MHz, DMSO-$d_6$): 8.28 (1H, s), 7.49 (1H, d), 7.44 (1H, t), 7.36 - 7.31 (2H, m), 7.01 - 6.96 (2H, m), 6.93 (1H, d), 3.97 – 3. 94 (1H, m), 3.76 (4H, t), 3.14 (4H, t), 2.26 (3H, s), 2.14 (3H, s), 1.64 (3H, s), 0.88 - 0.82 (2H, m), 0.76 - 0.69 (2H, m) |
| **1-222** | Nd | 360<br>361 | 1.57 | Method 8 | (400 MHz, DMSO-$d_6$) δ 2.20 (s, 3H), 2.25 (s, 6H), 2.31 (m, 3H), 2.35 (s, 3H), 3.84 (t, 4H), 6.32 (m, 1H), 6.57 (m, 2H), 7.35 (t, 1H), 8.11 (s, 1H) |
| **1-223** | Nd | 372<br>373 | 1.30 | Method 8 | (400 MHz, $CDCl_3$) δ 2.20 (s, 3H), 2.36 (s, 3H), 2.41 (m, 2H), 3.91 (t, 4H), 6.14 (s, 1H), 6.46 (d, 1H), 6.53 (d, 1H), 7.01 (m, 1H), 7.30 (t, 1H), 8.65 (d, 2H), 8.82 (s, 1H) |

| | | | | | |
|---|---|---|---|---|---|
| **1-224** | Nd | 403 | 3.33 | Method 8 | (400 MHz, $CDCl_3$) δ 2.25 (s, 3H), 2.40 (p, 2H), 2.47 (s, 3H), 3.91 (t, 4H), 3.95 (s, 3H), 6.18 (m, 1H), 6.51 (m, 2H), 7.31 (t, 1H), 8.05 (s, 1H), 8.50 (s, 2H) |
| **1-225** | Nd | 389<br>390 | 1.43 | Method 8 | (400 MHz, $CDCl_3$) δ 2.26 (s, 3H), 2.42 (m, 2H), 2.48 (s, 3H), 3.92 (t, 4H), 6.21 (s, 1H), 6.54 (t, 2H), 7.33 (m, 2H), 7.56 (d, 1H), 8.10 (s, 1H), 8.44 (d, 1H) |
| **1-226** | Nd | 390<br>391 | 1.33 | Method 8 | (400 MHz, $CDCl_3$) δ 2.25 (s, 3H), 2.41 (p, 2H), 2.48 (s, 3H), 3.92 (t, 4H), 6.18 (s, 1H), 6.52 (m, 2H), 7.32 (t, 1H), 8.07 (s, 1H), 8.71 (s, 2H) |
| **1-227** | Nd | 377<br>378 | 1.48 | Method 8 | (400 MHz, $CDCl_3$) δ 2.20 (s, 3H), 2.39 (s, 3H), 2.42 (m, 2H), 3.90 (t, 4H), 6.11 (s, 1H), 6.44 (d, 1H), 6.52 (d, 1H), 6.97 (d, 1H), 7.30 (t, 1H), 7.48 (d, 1H) |
| **1-228** | Nd | 387 | 1.19 | Method 8 | (400 MHz, $CDCl_3$) δ 2.19 (s, 3H), 2.26 (s, 3H), 2.35 (s, 3H), 2.42 (dd, 2H), 3.91 (t, 4H), 6.14 (s, 1H), 6.46 (d, 1H), 6.53 (d, 1H), 7.30 (t, 1H), 8.47 (s, 2H), 8.73 (s, 1H) |
| **1-229** | Nd | 402 | 1.37 | Method 8 | ($CDCl_3$, 400 MHz): δ (ppm) 2.19 (s, 3H), 2.36 (s, 3H), 2.41 (m, 2H), 3.91 (m, 7H), 6.13 (s, 1H), 6.45 (d, 1H), 6.51 (d, 1H), 6.63 (d, 1H), 7.29 (t, 1H), 8.04 (m, 2H), 9.34 (s, 1H) |
| **1-230** | Nd | 461 | 3.98 | Method 8 | (500 MHz, CDCl3): δ = 8.65 (ddd, J = 4.9, 2.0, 0.9 Hz, 1H), 8.15 (s, 1H), 7.80 (ddd, J = 8.1, 7.4, 2.0 Hz, 1H), 7.72 (dt, J = 8.1, 1.0 Hz, 1H), 7.33 (ddd, J = 8.5, 7.8, 0.7 Hz, 1H), 7.27 – 7.24 (m, 1H), 7.00 (dd, J = 8.4, 1.0 Hz, 1H), 6.77 (dd, J = 7.9, 1.1 Hz, 1H), 3.92 (s, 3H), 2.40 (s, 3H), 2.17 (s, 3H), 1.76 (s, 3H). |
| **1-231** | Nd | 462 | 3.98 | Method 8 | (500 MHz, DMSO-D6): δ = 8.50 (s, 1H), 8.43 (d, J = 4.8 Hz, 2H), 7.40 – 7.35 (m, 1H), 7.16 (dd, J = 8.5, 1.1 Hz, 1H), 6.86 (dd, J = 8.0, 1.0 Hz, 1H), 6.79 (t, J = 4.8 Hz, 1H), 3.87 (s, 3H), 2.16 (s, 3H), 2.04 (s, 3H), 1.70 (s, 3H). |
| **1-232** | Nd | 397 | 4.09 | Method 8 | (500 MHz, DMSO-d6): δ = 8.57 (ddd, J = 4.9, 2.0, 0.8 Hz, 1H), 8.34 (s, 1H), 7.95 (td, J = 7.7, 2.0 Hz, 1H), 7.58 – 7.51 (m, 2H), 7.46 – 7.41 (m, 2H), 7.36 (t, J = 73.5 Hz, 1H), 7.30 (dd, J = 7.9, 1.1 Hz, 1H), 2.28 (s, 3H), 2.18 (s, 3H), 1.79 (s, 3H). |
| **1-233** | Nd | 365 | 1.23 | Method 9 | Nd |
| **1-234** | Nd | 366 | 1.17 | Method 9 | (600 MHz, $CDCl_3$): δ = 2.27 (s, 3H), 2.48 (s, 3H), 3.99 (s, 3H), 6.83 (dd, 1H), 7.16 (m, 1H), 7.29 (m, 2H), 8.10 (s, 1H), 8.89 (m, 2H). |

| | | | | | |
|---|---|---|---|---|---|
| **1-235** | Nd | 391 | 1.38 | Method 9 | (400 MHz, DMSO-d6): δ = 0.76 – 0.83 (m, 2H), 0.84 – 0.91 (m, 2H), 2.25 (s, 3H), 2.36 (s, 3H), 4.04 – 4.12 (m, 1H), 7.16 (t, 1H), 7.39 – 7.47 (m, 2H), 7.51 (d, 1H), 7.69 (t, 1H), 7.95 (td, 1H), 8.32 (s, 1H), 8.57 (d, 1H). |
| **1-236** | Nd | 392 | 1.32 | Method 9 | (400 MHz, DMSO-d6) δ = 0.76 – 0.83 (m, 2H), 0.83 – 0.92 (m, 2H), 2.26 (s, 3H), 2.35 (s, 3H), 4.03 – 4.12 (m, 1H), 7.18 (t, 1H), 7.43 (t, 1H), 7.62 (t, 1H), 7.69 (t, 1H), 8.33 (s, 1H), 8.97 (d, 2H). |

## PHARMACOLOGY

The compounds provided in the present invention are negative allosteric modulators of mGlu7. As such, these compounds are expected to have their effect at mGlu7 by virtue of their ability to block the function of the receptor after binding to a site that is not the orthosteric glutamate recognition site.

Some of the compounds of Formula (I) have been tested according to the following methods.

### Example A

***mGlu7 assay on HEK-expressing human mGlu7***

*Transfection and Cell culture*

The cDNA encoding the human metabotropic glutamate 7 receptor (hmGlu7), (accession number NM_181874.2, NCBI Nucleotide database browser), was subcloned into an expression vector containing also the hygromycin resistance gene. In parallel, the cDNA encoding a G protein allowing redirection of the activation signal to intracellular calcium flux was subcloned into a different expression vector containing also the Puromycin resistance gene. Transfection of both these vectors into HEK293 cells with PolyFect reagent (Qiagen) according to supplier's protocol, and hygromycin and puromycin treatment allowed selection of antibiotic resistant cells which had integrated stably one or more copies of the plasmids. Positive cellular clones expressing hmGlu7 were identified in a functional assay measuring changes in calcium fluxes in response to glutamate and L-AP4 or known mGlu7 orthosteric antagonists.

HEK-293 cells expressing hmGlu7 were maintained in media containing DMEM, Fetal Bovine Serum (10%), Glutamax™ (2 mM), penicillin (100 units/mL), streptomycin (100 μg/mL), geneticin (100 μg/mL) and hygromycin-B (40 μg/mL) and puromycin (1 μg/mL) at 37°C with 5% $CO_2$ in a humidified atmosphere.

*Fluorescent cell based- $Ca^{2+}$ mobilization assay*

Human mGlu7 HEK-293 cells were plated out 24 hours prior to a fluorescent cell-based calcium mobilization assay using $FLIPR^{384}$ assay (Molecular Device, Sunnyvale, CA, USA) in black-walled, clear-bottomed, poly-L-ornithine-coated 384-well plates at a density of 25,000 cells/well in a glutamine/glutamate free DMEM medium containing fetal bovine serum (10%), penicillin (100 units/mL), streptomycin (100 μg/mL) and doxycyline (1 μg/ml) at 37°C with 5% $CO_2$ in a humidified atmosphere.

On the day of the assay, the medium was aspirated and the cells were loaded with a 3 μM solution of Fluo4-AM (LuBioScience, Lucerne, Switzerland) in 0.03% pluronic acid. After 1 hour at 37°C/5% $CO_2$, the non incorporated dye was removed by washing cell plate with the assay buffer. All assays were performed in a pH 7.4 buffered-solution containing 20 mM HEPES, 143 mM NaCl, 6 mM KCl, 1 mM $MgSO_4$, 1 mM $CaCl_2$, 0.125 mM sulfinpyrazone and 0.1% glucose.

After 10 s of basal fluorescence recording, various concentrations of the compounds of the invention were added to the cells. Changes in fluorescence levels were first monitored for 180 s in order to detect any agonist activity of the compounds. Then the cells were stimulated by an $EC_{80}$ L-AP4 concentration for an additional 110 s in order to measure inhibiting activities of the compounds of the invention. $EC_{80}$ L-AP4 concentration is the concentration giving 80% of the maximal glutamate response.

The concentration-response curves of L-AP4 or representative compounds of the present invention were generated using the Prism GraphPad software (Graph Pad Inc, San Diego, USA). The curves were fitted to a four-

parameter logistic equation:

$$(Y=Bottom + (Top-Bottom)/(1+10^{\wedge}((LogIC_{50}-X)*Hill\ Slope)$$

allowing determination of $IC_{50}$ values.

The compounds of this application have $IC_{50}$ values less than 10 $\mu$M.

The Table 3 below represents the mean $IC_{50}$ obtained from at least three independent experiments of selected molecules performed in duplicate.

**Table 3: Activity data for selected compounds**

| Co.nr. | $Ca^{2+}$ Flux* | Co.nr. | $Ca^{2+}$ Flux* | Co.nr. | $Ca^{2+}$ Flux* |
|---|---|---|---|---|---|
| 1-1 | +++ | 1-80 | + | 1-159 | ++ |
| 1-2 | ++ | 1-81 | + | 1-160 | +++ |
| 1-3 | ++ | 1-82 | + | 1-161 | +++ |
| 1-4 | ++ | 1-83 | ++ | 1-162 | +++ |
| 1-5 | + | 1-84 | + | 1-163 | +++ |
| 1-6 | +++ | 1-85 | ++ | 1-164 | +++ |
| 1-7 | ++ | 1-86 | ++ | 1-165 | +++ |
| 1-8 | ++ | 1-87 | ++ | 1-166 | +++ |
| 1-9 | ++ | 1-88 | ++ | 1-167 | ++ |
| 1-10 | ++ | 1-89 | ++ | 1-168 | ++ |
| 1-11 | +++ | 1-90 | + | 1-169 | +++ |
| 1-12 | +++ | 1-91 | ++ | 1-170 | +++ |
| 1-13 | ++ | 1-92 | ++ | 1-171 | +++ |
| 1-14 | +++ | 1-93 | ++ | 1-172 | ++ |
| 1-15 | +++ | 1-94 | ++ | 1-173 | +++ |
| 1-16 | ++ | 1-95 | ++ | 1-174 | +++ |
| 1-17 | ++ | 1-96 | + | 1-175 | ++ |
| 1-18 | ++ | 1-97 | +++ | 1-176 | ++ |
| 1-19 | ++ | 1-98 | ++ | 1-177 | +++ |
| 1-20 | ++ | 1-99 | ++ | 1-178 | +++ |
| 1-21 | ++ | 1-100 | ++ | 1-179 | ++ |
| 1-22 | + | 1-101 | ++ | 1-180 | +++ |
| 1-23 | ++ | 1-102 | +++ | 1-181 | + |
| 1-24 | ++ | 1-103 | ++ | 1-182 | +++ |
| 1-25 | + | 1-104 | ++ | 1-183 | +++ |
| 1-26 | +++ | 1-105 | ++ | 1-184 | ++ |
| 1-27 | ++ | 1-106 | ++ | 1-185 | +++ |
| 1-28 | +++ | 1-107 | + | 1-186 | ++ |
| 1-29 | ++ | 1-108 | ++ | 1-187 | + |
| 1-30 | ++ | 1-109 | ++ | 1-188 | + |
| 1-31 | +++ | 1-110 | ++ | 1-189 | +++ |
| 1-32 | ++ | 1-111 | + | 1-190 | ++ |

(continued)

| Co.nr. | $Ca^{2+}$ Flux* | Co.nr. | $Ca^{2+}$ Flux* | Co.nr. | $Ca^{2+}$ Flux* |
|---|---|---|---|---|---|
| 1-33 | ++ | 1-112 | ++ | 1-191 | + |
| 1-34 | + | 1-113 | ++ | 1-192 | ++ |
| 1-35 | +++ | 1-114 | ++ | 1-193 | + |
| 1-36 | ++ | 1-115 | ++ | 1-194 | ++ |
| 1-37 | ++ | 1-116 | +++ | 1-195 | +++ |
| 1-38 | +++ | 1-117 | ++ | 1-196 | +++ |
| 1-39 | ++ | 1-118 | +++ | 1-197 | ++ |
| 1-40 | ++ | 1-119 | +++ | 1-198 | +++ |
| 1-41 | ++ | 1-120 | +++ | 1-199 | ++ |
| 1-42 | + | 1-121 | +++ | 1-200 | +++ |
| 1-43 | ++ | 1-122 | ++ | 1-201 | ++ |
| 1-44 | ++ | 1-123 | + | 1-202 | +++ |
| 1-45 | ++ | 1-124 | ++ | 1-203 | ++ |
| 1-46 | ++ | 1-125 | ++ | 1-204 | ++ |
| 1-47 | ++ | 1-126 | ++ | 1-205 | +++ |
| 1-48 | ++ | 1-127 | ++ | 1-206 | ++ |
| 1-49 | ++ | 1-128 | ++ | 1-207 | ++ |
| 1-50 | ++ | 1-129 | ++ | 1-208 | +++ |
| 1-51 | + | 1-130 | +++ | 1-209 | +++ |
| 1-52 | ++ | 1-131 | ++ | 1-210 | ++ |
| 1-53 | ++ | 1-132 | + | 1-211 | +++ |
| 1-54 | + | 1-133 | ++ | 1-212 | +++ |
| 1-55 | ++ | 1-134 | ++ | 1-213 | +++ |
| 1-56 | + | 1-135 | +++ | 1-214 | +++ |
| 1-57 | ++ | 1-136 | +++ | 1-215 | +++ |
| 1-58 | + | 1-137 | ++ | 1-216 | +++ |
| 1-59 | +++ | 1-138 | +++ | 1-217 | +++ |
| 1-60 | ++ | 1-139 | ++ | 1-218 | ++ |
| 1-61 | ++ | 1-140 | + | 1-219 | ++ |
| 1-62 | + | 1-141 | +++ | 1-220 | + |
| 1-63 | + | 1-142 | +++ | 1-221 | + |
| 1-64 | + | 1-143 | +++ | 1-222 | ++ |
| 1-65 | +++ | 1-144 | +++ | 1-223 | ++ |
| 1-66 | +++ | 1-145 | +++ | 1-224 | +++ |
| 1-67 | ++ | 1-146 | +++ | 1-225 | +++ |
| 1-68 | ++ | 1-147 | ++ | 1-226 | +++ |
| 1-69 | ++ | 1-148 | ++ | 1-227 | + |
| 1-70 | ++ | 1-149 | +++ | 1-228 | +++ |
| 1-71 | ++ | 1-150 | ++ | 1-229 | ++ |

(continued)

| Co.nr. | $Ca^{2+}$ Flux* | Co.nr. | $Ca^{2+}$ Flux* | Co.nr. | $Ca^{2+}$ Flux* |
|---|---|---|---|---|---|
| **1-72** | + | **1-151** | +++ | **1-230** | ++ |
| **1-73** | ++ | **1-152** | ++ | **1-231** | + |
| **1-74** | + | **1-153** | ++ | **1-232** | ++ |
| **1-75** | ++ | **1-154** | ++ | **1-233** | ++ |
| **1-76** | ++ | **1-155** | +++ | **1-234** | ++ |
| **1-77** | ++ | **1-156** | +++ | **1-235** | ++ |
| **1-78** | ++ | **1-157** | +++ | **1-236** | +++ |
| **1-79** | ++ | **1-158** | +++ | **1-241** | + |

***Table legend:**
(+): 1 μM < $IC_{50}$ <10 μM
(++): 100 nM < $IC_{50}$ <1 μM
(+++): $IC_{50}$ < 100 nM

The results shown in Table 3 demonstrate that the compounds described in the present invention are negative allosteric modulators of human mGlu7 receptors.

**Example B**

**Water associated zero maze:**

The procedure was performed as described previously by Ritov and Richter-Levin, 2014 with minor modifications. The apparatus consists of annular platform with two opposite, enclosed quadrants (with walls 35 cm height) and two open quadrants (with borders 5 mm height). The plastic tank that holds this platform is filled up with water (22 + 2°C, 50 cm deep), arising to 10 cm below the platform level. Thus, the annular platform and the plastic tank comprise one unified arena. For the tests, rats were first habituated to the room for 4 min and then were placed into one of the open quadrants facing a closed part of the apparatus. Rats were allowed to explore the arena for a 5 mins session. During this time rats behavior was tracked, recorded and analyzed by the Etho-Vision system (Noldus Information Technology, Wageningen, Netherlands). Behavioral measures that were analyzed include the time spent in the open quadrants, distance traveled in the open quadrants, distance travelled in the closed quadrants and total freezing behavior. The impact of exposure to various stressors and/or compounds were assessed using these parameters. Pre-treatment time and route of administration of the different tested compounds were defined based of their pharmacokinetic properties.

**Example C**

**Elevated plus maze:**

The elevated plus maze (EPM) test was conducted using Sprague-Dawley male rats. The EPM is made of plastic that has two open arms (50 cm × 10 cm) and two closed arms of the same size with walls 40 cm high, elevated 86 cm above the ground. Both arms are made of black Plexiglas. The average illumination level on the open arms was 187 LUX and 100 LUX on the closed arms. At the beginning of the experiment, rats were brought into a holding room directly next to the testing room and allowed to habituate to the environment for 30 min. At the commencement of testing, rats were placed in the center of the maze, facing one of the open arms and observed for 5 min. During this time rats behavior was tracked, recorded and analyzed by the Etho-Vision system (Noldus Information Technology, Wageningen, Netherlands). Behavioral measures that were analyzed include the time spent in the open arms, number of entries in the open arms as well as the distance travelled. Pre-treatment time and route of administration of the different tested compounds were defined based of their pharmacokinetic properties.

**Example D**

**Fear conditioning model of post-traumatic stress disorder in the rat:**

The fear-conditioning arena (30 cm × 20 cm × 25 cm, Med Associates) is made of Plexiglas in different contexts. The system is placed in a sound-proof ventilated box. The arena floor consists of grid floor (19 parallel 0.48 cm diameter stainless steel rods, 1.6 cm apart) above a stainless steel waste pan. All rods were wired to a shock generator and scrambler. A speaker was mounted in the chamber wall to provide the source of the auditory stimuli. Fear conditioning procedure was performed over two days. The first day (training), rats were placed in the training context (context A) and after a 120 s acclimation period, they received five pairings of the CS and US. The CS tone (78 dB, 2 kHz, 5 ms rise/fall time) was presented for 30 s and co-terminated with a brief US footshock (O.5 s, 0.66 mA). The inter-tone interval (tone onset to next tone onset) ranged from 60s. The conditioning chambers were cleaned between subjects with 70% ethanol.
The time-spent freezing during delivery of the CS tone was scored (CS freezing). The second day (test day), animals were placed in a new context (context B) and were exposed to the CS (120s) after 60s of acclimation. Time-spent in freezing was measured during both acclimation and CS. Tested compounds were administrated prior and/or after training phase as well as testing phase. Pre-treatment time and route of administration of the different tested compounds were adjusted based of their pharmacokinetic properties.

**Example E**

**Noise-induced hearing loss (NIHL) model in the mice:**

Young adult males CBA/CaJ mice were used to assess the effect of tested compound on NIHL. Animals were exposed to octave band noise (8-16khz) at a sound pressure level of 110dB over 2 hours. Tested compounds were administrated prior and/or after noise exposure. Hearing function were measured using using auditory brainstrem response (ABR) audiograms or Distorsion Product of Autoacoustic Emissions (DPOAE) at different timepoint 24 hours, 2 and 4 weeks post acoustic trauma. Pre-treatment time and route of administration of the different tested compounds were adjusted based of their pharmacokinetic properties. The experimental groups were compared to the vehicle treated group through the measure of, for example, ABR Threshold, or ABR Threshold shift.

**Example F**

**Colorectal Distension test of visceral pain in rat.**

Male stress-sensitive Wistar Kyoto rats (250-300 g) were used in this study. Animals were fasted overnight (16 h) and on the day of testing, were anaesthetised using isoflurane. 6 cm latex balloon was inserted into the colorectal cavity, 1 cm from the anus. The animals were allowed to recover for 20 min before colorectal distension commenced. The paradigm used is an ascending phasic distension from 0 mmHg to 80 mmHg over 8 min using a computer-driven electronic barostat. The parameters measured were the threshold pressure (mmHg) that evoked visually identifiable visceral pain behaviour, and the total number of pain behaviours. Postures defined as visceral pain behaviours were abdominal retractions and/or abdominal withdrawal reflex.
Tested compounds were administrated prior colorectal distension. Pre-treatment time and route of administration of the different tested compounds were adjusted based of their pharmacokinetic properties.

<u>FORMULATION EXAMPLES</u>

Typical examples of recipes for the formulation of the invention are as follows:

1. <u>Tablets</u>

| | |
|---|---|
| Active ingredient | 5 to 50 mg |
| Di-calcium phosphate | 20 mg |
| Lactose | 30 mg |
| Talcum | 10 mg |
| Magnesium stearate | 5 mg |
| Potato starch | ad 200 mg |

In this Example, active ingredient can be replaced by the same amount of any of the compounds according to the present invention, in particular by the same amount of any of the exemplified compounds.

2. Suspension

An aqueous suspension is prepared for oral administration so that each 1 milliliter contains 1 to 5 mg of one of the active compounds, 50 mg of sodium carboxymethyl cellulose, 1 mg of sodium benzoate, 500 mg of sorbitol and water ad 1 mL.

3. Injectable

A parenteral composition is prepared by stirring 1.5 % by weight of active ingredient of the invention in 10% by volume propylene glycol and water.

4. Ointment

| | |
|---|---|
| Active ingredient | 5 to 1000 mg |
| Stearyl alcohol | 3 g |
| Lanoline | 5 g |
| White petroleum | 15 g |
| Water | ad 100 g |

In this Example, active ingredient can be replaced with the same amount of any of the compounds according to the present invention, in particular by the same amount of any of the exemplified compounds.

Reasonable variations are not to be regarded as a departure from the scope of the invention. It will be obvious that the described invention may be varied in many ways by those skilled in the art.

The invention includes the subject matter described in the following numbered paragraphs.

1. A compound having the Formula (I):

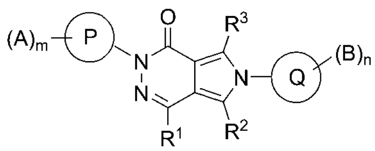

(I)

a pharmaceutically acceptable acid or base addition salt thereof, a stereochemically isomeric form thereof or an N-oxide form thereof, wherein:

$R^1$ is selected from the group of hydrogen, $-CH_3$ and $-CF_3$;

$R^2$ and $R^3$ are each independently selected from the group of hydrogen, $-(C_1-C_6)$alkyl and $-(C_1-C_6)$haloalkyl and $-CF_3$;

P represents a cycloalkyl, aryl or heteroaryl of formula:

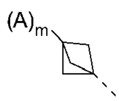 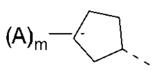 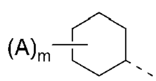

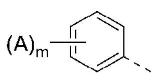 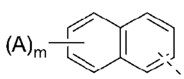 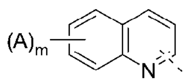 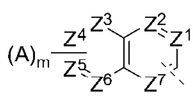

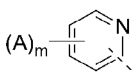 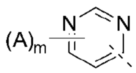 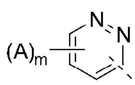 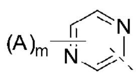 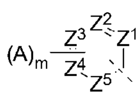

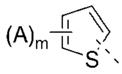 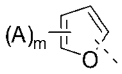 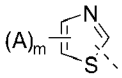 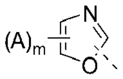 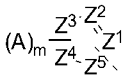

wherein each cycloalkyl, aryl or heteroaryl ring is optionally substituted with m radicals A, wherein m is an integer equal to zero, 1, 2, 3 or 4;

wherein $Z^1$, $Z^2$, $Z^3$, $Z^4$, $Z^5$, $Z^6$ and $Z^7$ are each independently selected from C, N, O or S; provided that at least one of $Z^1$, $Z^2$, $Z^3$, $Z^4$, $Z^5$, $Z^6$ and $Z^7$ is N;

the or each $(A)_m$ is independently selected from the group of hydrogen, halogen, - CN, -OH, -$NO_2$, -$CF_3$, -SH, -$NH_2$ and an optionally substituted radical selected from the group of -($C_1$-$C_6$)alkyl, -($C_1$-$C_6$)haloalkyl, -($C_2$-$C_6$)alkynyl, -($C_2$-$C_6$)alkenyl, -($C_3$-$C_7$)cycloalkyl, -($C_1$-$C_6$)alkylene-($C_3$-$C_7$)cycloalkyl, -($C_3$-$C_8$)cycloalkenyl, -($C_1$-$C_6$)cyanoalkyl, -($C_1$-$C_6$)alkylene-heteroaryl, -($C_1$-$C_6$)alkylene-aryl, aryl, heteroaryl, -($C_1$-$C_6$)alkylene-heterocycle, heterocycle, -($C_0$-$C_6$)alkylene-$OR^4$, -O-($C_2$-$C_6$)alkylene-$OR^4$, -$NR^4$($C_2$-$C_6$)alkylene-$OR^5$, -($C_3$-$C_6$)alkynylene-$OR^4$, -($C_3$-$C_6$)alkynylene-$NR^4R^5$, -($C_3$-$C_6$)alkenylene-$OR^4$, -($C_3$-$C_6$)alkenylene-$NR^4R^5$, -($C_0$-$C_6$)alkylene-S-$R^4$, -O-($C_2$-$C_6$)alkylene-S-$R^4$, -$NR^4$-($C_2$-$C_6$)alkylene-S-$R^5$, -($C_0$-$C_6$)alkylene-S(=O)-$R^4$, -O-($C_1$-$C_6$)alkylene-S(=O)-$R^4$, -$NR^4$-($C_1$-$C_6$)alkylene-S(=O)-$R^5$, -($C_0$-$C_6$)alkylene-$S(=O)_2$-$R^4$, -O-($C_1$-$C_6$)alkylene-$S(=O)_2$-$R^4$, -$NR^4$-($C_1$-$C_6$)alkylene-$S(=O)_2$-$R^5$, -($C_0$-$C_6$)alkylene-$NR^4R^5$, -O-($C_2$-$C_6$)alkylene-$NR^4R^5$, -$NR^4$-($C_2$-$C_6$)alkylene-$NR^5R^6$, -($C_0$-$C_6$)alkylene-$S(=O)_2NR^4R^5$, -O-($C_1$-$C_6$)alkylene-$S(=O)_2NR^4R^5$, -$NR^4$-($C_1$-$C_6$)alkylene-$S(=O)_2NR^5R^6$, -($C_0$-$C_6$)alkylene-$NR^4$-$S(=O)_2R^5$, -O-($C_2$-$C_6$)alkylene-$NR^4$-$S(=O)_2R^5$, -$NR^4$-($C_2$-$C_6$)alkylene-$NR^5$-$S(=O)_2R^6$, -($C_0$-$C_6$)alkylene-C(=O)-$NR^4R^5$, -O-($C_1$-$C_6$)alkylene-C(=O)-$NR^4R^5$, -$NR^4$-($C_1$-$C_6$)alkylene-C(=O)-$NR^5R^6$, - ($C_0$-$C_6$)alkylene-$NR^4$C(=O)-$R^5$, -O-($C_2$-$C_6$)alkylene-$NR^4$C(=O)-$R^5$, -$NR^4$-($C_2$-$C_6$)alkylene-$NR^5$C(=O)-$R^6$, -($C_0$-$C_6$)alkylene-OC(=O)-$R^4$, -O-($C_2$-$C_6$)alkylene-OC(=O)-$R^4$, -$NR^4$-($C_2$-$C_6$)alkylene-OC(=O)-$R^5$, -($C_0$-$C_6$)alkylene-C(=O)-$OR^4$, -O-($C_1$-$C_6$)alkylene-C(=O)-$OR^4$, -$NR^4$-($C_1$-$C_6$)alkylene-C(=O)-$OR^5$, -($C_0$-$C_6$)alkylene-C(=O)-$R^4$, -O-($C_1$-$C_6$)alkylene-C(=O)-$R^4$, -$NR^4$-($C_1$-$C_6$)alkylene-C(=O)-$R^5$, -($C_0$-$C_6$)alkylene-$NR^4$-C(=O)-$OR^5$, -C(=O)-($C_1$-$C_6$)alkylene-$NR^4$-C(=O)-$OR^5$, -($C_0$-$C_6$)alkylene-O-C(=O)-$NR^4R^5$, -($C_0$-$C_6$)alkylene-$NR^4$-C(=O)-$NR^5R^6$, -O-($C_2$-$C_6$)alkylene-$NR^4$-C(=O)-$NR^5R^6$, -$NR^4$-($C_2$-$C_6$)alkylene-$NR^5$-C(=O)-$NR^6R^7$, -($C_0$-$C_6$)alkylene-$NR^4$-C(=S)-$NR^5R^6$ and -($C_0$-$C_6$)alkylene-$NR^4$-C(=$NR^5$)-$NR^6R^7$;

$R^4$, $R^5$, $R^6$ and $R^7$ are each independently hydrogen or an optionally substituted radical selected from the group of -($C_1$-$C_6$)haloalkyl, -($C_1$-$C_6$)alkyl, -($C_1$-$C_6$)cyanoalkyl, -($C_3$-$C_7$)cycloalkyl, -($C_1$-$C_6$)alkylene-($C_3$-$C_7$)cycloalkyl, heteroaryl, -($C_1$-$C_6$)alkylene-heteroaryl, aryl, -($C_1$-$C_6$)alkylene-heterocycle, heterocycle, -($C_1$-$C_6$)alkylene-aryl, -($C_0$-$C_6$)alkylene-O-($C_0$-$C_6$)alkyl and -($C_0$-$C_6$)alkylene-N-(($C_0$-$C_6$)alkyl$)_2$;

Q is aryl or heteroaryl which is optionally substituted with n radicals B, wherein n is an integer equal to zero, 1, 2, 3, 4 or 5;

the or each $(B)_n$ is independently selected from the group of hydrogen, halogen, - CN, -OH, -$NO_2$, -$CF_3$, -SH, -$NH_2$ and an optionally substituted radical selected from the group of -($C_1$-$C_6$)alkyl, -($C_1$-$C_6$)haloalkyl, -($C_2$-$C_6$)alkynyl, -($C_2$-$C_6$)alkenyl, - ($C_3$-$C_7$)cycloalkyl, -($C_1$-$C_6$)alkylene-($C_3$-$C_7$)cycloalkyl, -($C_3$-$C_8$)cycloalkenyl, -($C_1$-$C_6$)cyanoalkyl, -($C_1$-$C_6$)alkylene-heteroaryl, -($C_1$-$C_6$)alkylene-aryl, aryl, heteroaryl, -($C_1$-$C_6$)alkylene-heterocycle, heterocycle, -($C_0$-$C_6$)alkylene-$OR^8$, -O-($C_2$-$C_6$)alkylene-$OR^8$, -$NR^8$($C_2$-$C_6$)alkylene-$OR^9$, -($C_3$-$C_6$)alkynylene-$OR^8$, -($C_3$-$C_6$)alkynylene-$NR^8R^9$, -($C_3$-$C_6$)alkenylene-$OR^8$, -($C_3$-$C_6$)alkenylene-$NR^8R^9$, -($C_0$-$C_6$)alkylene-S-$R^8$, -O-($C_2$-$C_6$)alkylene-S-$R^8$, -$NR^8$-($C_2$-$C_6$)alkylene-S-$R^9$, -($C_0$-$C_6$)alkylene-S(=O)-$R^8$, -O-($C_1$-$C_6$)alkylene-S(=O)-$R^8$, -$NR^8$-($C_1$-$C_6$)alkyleneS(=O)-$R^9$, -($C_0$-$C_6$)alkylene-$S(=O)_2$-$R^8$, -O-($C_1$-$C_6$)alkylene-$S(=O)_2$-$R^8$, -$NR^8$-($C_1$-$C_6$)alkylene-$S(=O)_2$-$R^9$, -($C_0$-$C_6$)alkylene-$NR^8R^9$, -O-($C_2$-$C_6$)alkylene-$NR^8R^9$, - $NR^8$-($C_2$-$C_6$)alkylene-$NR^9R^{10}$, -($C_0$-$C_6$)alkylene-$S(=O)_2NR^8R^9$, -O-($C_1$-$C_6$)alkylene-$S(=O)_2NR^8R^9$, -$NR^8$-($C_1$-$C_6$)alkylene-$S(=O)_2NR^9R^{10}$, -($C_0$-$C_6$)alkylene-$NR^8$-$S(=O)_2R^9$, -O-($C_2$-$C_6$)alkylene-$NR^8$-$S(=O)_2R^9$, -$NR^8$-($C_2$-$C_6$)alkylene-$NR^9$-$S(=O)_2R^{10}$, -($C_0$-$C_6$)alkylene-C(=O)-$NR^8R^9$, -O-($C_1$-$C_6$)alkylene-C(=O)-$NR^8R^9$, -$NR^8$-($C_1$-$C_6$)alkylene-C(=O)-$NR^9R^{10}$, -($C_0$-$C_6$)alkylene-$NR^8$C(=O)-$R^9$, -O-($C_2$-$C_6$)alkylene-$NR^8$C(=O)-$R^9$, -$NR^8$-($C_2$-$C_6$)alkylene-$NR^9$C(=O)-$R^{10}$, -($C_0$-$C_6$)alkylene-OC(=O)-$R^8$, -O-($C_2$-$C_6$)alkylene-OC(=O)-$R^8$, - $NR^8$-($C_2$-$C_6$)alkylene-OC(=O)-$R^9$, -($C_0$-$C_6$)alkylene-C(=O)-$OR^8$, -O-($C_1$-$C_6$)alkylene-C(=O)-$OR^8$, -$NR^8$-($C_1$-$C_6$)alkylene-C(=O)-$OR^9$, -($C_0$-$C_6$)alkylene-C(=O)-$R^8$, -O-($C_1$-$C_6$)alkylene-C(=O)-$R^8$, -$NR^8$-($C_1$-$C_6$)alkylene-C(=O)-$R^9$, -($C_0$-$C_6$)alkylene-$NR^8$-C(=O)-$OR^9$, -($C_0$-$C_6$)alkylene-O-C(=O)-$NR^8R^9$, -($C_0$-$C_6$)alkylene-$NR^8$-C(=O)-$NR^9R^{10}$, -O-($C_2$-$C_6$)alkylene-$NR^8$-C(=O)-$NR^9R^{10}$, -$NR^8$-($C_2$-$C_6$)alkylene-$NR^9$-C(=O)-$NR^{10}R^{11}$, -($C_0$-$C_6$)alkylene-$NR^8$-C(=S)-$NR^9R^{10}$ and - ($C_0$-$C_6$)alkylene-$NR^8$-C(=$NR^9$)-$NR^{10}R^{11}$;

$R^8$, $R^9$, $R^{10}$ and $R^{11}$ are each independently hydrogen or an optionally substituted radical selected from the group of -($C_1$-$C_6$)haloalkyl, -($C_1$-$C_6$)alkyl, -($C_1$-$C_6$)cyanoalkyl, -($C_3$-$C_7$)cycloalkyl, -($C_1$-$C_6$)alkylene-($C_3$-$C_7$)cycloalkyl, heteroaryl, -($C_1$-$C_6$)alkylene-heteroaryl, aryl, -($C_1$-$C_6$)alkylene-heterocycle, heterocycle, -($C_1$-$C_6$)alkylene-aryl, -($C_0$-$C_6$)alkylene-O-($C_0$-$C_6$)alkyl and -($C_0$-$C_6$)alkylene-N-(($C_0$-$C_6$)alkyl)$_2$;

wherein optionally any two radicals A are combined with the intervening atoms to form a 3 to 10 membered bicyclic heterocycle, aryl or heteroaryl ring, wherein each ring is optionally further substituted with 1 to 5 radicals independently selected from the group of halogen, -CN, nitro, -($C_1$-$C_6$)alkyl, -($C_0$-$C_6$)alkylene-O-($C_0$-$C_6$)alkyl and -($C_0$-$C_6$)alkylene-N-(($C_0$-$C_6$)alkyl)$_2$;

wherein optionally two of the substituents $R^4$, $R^5$, $R^6$ or $R^7$ are combined with the intervening atoms to form a 3 to 10 membered heterocycle, aryl or heteroaryl ring, wherein each ring is optionally further substituted with 1 to 5 radicals independently selected from the group of halogen, cyano, nitro, -($C_1$-$C_6$)alkyl, -($C_0$-$C_6$)alkylene-O-($C_0$-$C_6$)alkyl and -($C_0$-$C_6$)alkylene-N-(($C_0$-$C_6$)alkyl)$_2$;

wherein optionally two substituents from $R^8$, $R^9$, $R^{10}$ or $R^{11}$ are combined with the intervening atoms to form a 3 to 10 membered heterocycle, aryl or heteroaryl ring, wherein each ring is optionally further substituted with 1 to 5 radicals independently selected from the group of halogen, cyano, nitro, -($C_1$-$C_6$)alkyl, -($C_0$-$C_6$)alkylene-O-($C_0$-$C_6$)alkyl and -($C_0$-$C_6$)alkylene-N-(($C_0$-$C_6$)alkyl)$_2$;

wherein optionally any two radicals B are combined with the intervening atoms to form a 3 to 10 membered bicyclic heterocycle, aryl or heteroaryl ring, wherein each ring is optionally further substituted with 1 to 5 radicals independently selected from the group of halogen, -CN, nitro, -($C_1$-$C_6$)alkyl, -($C_0$-$C_6$)alkylene-O-($C_0$-$C_6$)alkyl and -($C_0$-$C_6$)alkylene-N-(($C_0$-$C_6$)alkyl)$_2$.

2. The compound according to paragraph 1 having the Formula (I) wherein:
Q represents an aryl or heteroaryl group of formula:

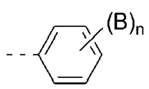 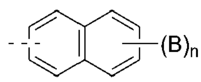 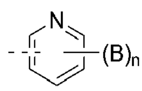 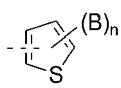 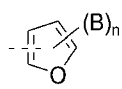 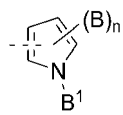

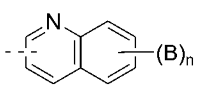 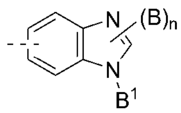 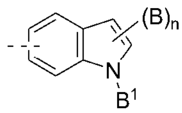 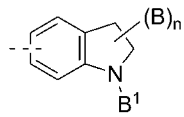 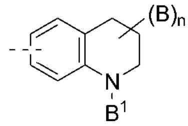

wherein each radical is optionally substituted with n radicals B, wherein n is an integer equal to zero, 1, 2, 3, 4 or 5; and wherein $B^1$ is a radical B.

3. The compound according to paragraph 2 having the Formula (I), wherein Q represents an aryl or heteroaryl group of formula:

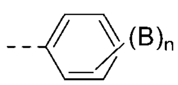 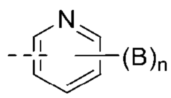 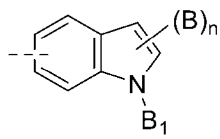 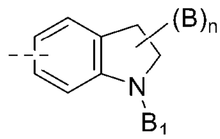

wherein each radical is optionally substituted with n radicals B, wherein n is an integer equal to zero, 1, 2, 3, 4 or 5, and wherein $B^1$ is a radical B.

4. The compound according to paragraph 2 or 3 having the Formula (I), wherein $B^1$ is -hydrogen, ($C_1$-$C_6$)alkyl or -($C_3$-$C_7$)cycloalkyl.

5. The compound according to any preceding paragraph having the Formula (I) wherein P represents a cycloalkyl, aryl or heteroaryl of formula:

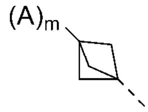 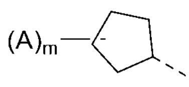 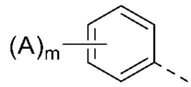

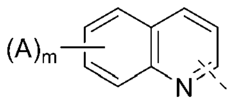 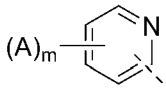 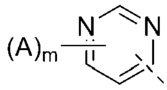 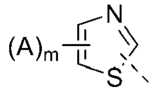 ,

wherein each radical is optionally substituted with m radicals A, wherein m is an integer equal to zero, 1, 2, 3 or 4.

6. The compound according to any preceding paragraph having the Formula (I) wherein:
the cycloalkyl, heterocycle, aryl and heteroaryl ring systems of $(A)_m$ are selected from the group of azetidinyl, benzimidazolyl, benzisothiazolyl benzisoxazolyl, benzofuryl, benzopyrazolyl, benzothiazolyl, benzothiophenyl, benzotriazolyl, benzoxazolyl, dihydrofuranyl, dihydrothienyl, dioxolanyl, 1,1-dioxo-thiomorpholinyl, furazanyl, furyl, imidazolidinyl, imidazolinyl, imidazolonyl, imidazolyl, imidazopyridazinyl, imidazopyridyl, indolyl, isoindolyl, isoquinolinyl, isothiazolinyl, isothiazolyl, isoxazolidinyl, isoxazolinyl, isoxazolyl, morpholinyl, naphthyl, naphthyridinyl, oxadiazolyl, oxazolidinyl, oxazolinyl, oxazolonyl, oxazolopyridazinyl, oxazolopyridyl, oxazolyl, oxetanyl, phenyl, piperazinonyl, piperazinyl, piperidinonyl, piperidinyl, phtalazinyl, pteridinyl, purinyl, pyranyl, pyrazinyl, pyrazolopyridinyl, pyrazolyl, pyridazinyl, pyridonyl, pyridyl, pyrimidyl, pyrrolidinonyl, pyrrolidinyl, pyrrolinyl, pyrrolyl, quinazolyl, quinolyl, quinoxalinyl, tetrahydrofuranyl, tetrahydropyranyl, tetrahydrothiopyranyl, tetrahydrotriazolopyridyl, tetrahydrotriazolopyrimidinyl, tetrazolyl, thiadiazolyl, thiazolidinyl, thiazolinyl, thiazolonyl, thiazolopyridazinyl, thiazolopyridyl, thiazolyl, thienyl, thiomorpholinyl, thionaphthyl, thiopyranyl, triazolinyl, triazinyl, triazolyl, cyclopropyl, cyclobutyl, cyclopentyl, cyclopentenyl, cyclohexyl, cyclohexenyl, cycloheptyl, cycloheptenyl, cyclooctyl and cyclooctenyl and each ring of said ring system is optionally substituted independently with 1 to 4 substituents $R^4$, $R^5$, $R^6$ or $R^7$.

7. The compound according to any preceding paragraph having the Formula (I) wherein:
the cycloalkyl, heterocycle, aryl and heteroaryl ring systems of $(B)_n$ are selected from the group of azetidinyl, benzimidazolyl, benzisothiazolyl benzisoxazolyl, benzofuryl, benzopyrazolyl, benzothiazolyl, benzothiophenyl, benzotriazolyl, benzoxazolyl, dihydrofuranyl, dihydrothienyl, dioxolanyl, 1,1-dioxo-thiomorpholinyl, furazanyl, furyl, imidazolidinyl, imidazolinyl, imidazolonyl, imidazolyl, imidazopyridazinyl, imidazopyridyl, indolyl, isoindolyl, isoquinolinyl, isothiazolinyl, isothiazolyl, isoxazolidinyl, isoxazolinyl, isoxazolyl, morpholinyl, naphthyl, naphthyridinyl, oxadiazolyl, oxazolidinyl, oxazolinyl, oxazolonyl, oxazolopyridazinyl, oxazolopyridyl, oxazolyl, oxetanyl, phenyl, piperazinonyl, piperazinyl, piperidinonyl, piperidinyl, phtalazinyl, pteridinyl, purinyl, pyranyl, pyrazinyl, pyrazolopyridinyl, pyrazolyl, pyridazinyl, pyridonyl, pyridyl, pyrimidyl, pyrrolidinonyl, pyrrolidinyl, pyrrolinyl, pyrrolyl, quinazolyl, quinolyl, quinoxalinyl, tetrahydrofuranyl, tetrahydropyranyl, tetrahydrothiopyranyl, tetrahydrotriazolopyridyl, tetrahydrotriazolopyrimidinyl, tetrazolyl, thiadiazolyl, thiazolidinyl, thiazolinyl, thiazolonyl, thiazolopyridazinyl, thiazolopyridyl, thiazolyl, thienyl, thiomorpholinyl, thionaphthyl, thiopyranyl, triazolinyl, triazinyl, triazolyl, cyclopropyl, cyclobutyl, cyclopentyl, cyclopentenyl, cyclohexyl, cyclohexenyl, cycloheptyl, cycloheptenyl, cyclooctyl and cyclooctenyl and each ring of said ring system is optionally substituted independently with 1 to 4 substituents $R^8$, $R^9$, $R^{10}$ or $R^{11}$.

8. The compound according to any preceding paragraph having the Formula (I) wherein:
the cycloalkyl, heterocycle, aryl and heteroaryl ring systems of $R^4$, $R^5$, $R^6$, $R^7$, $R^8$, $R^9$, $R^{10}$ or $R^{11}$ are selected from the group of azetidinyl, benzimidazolyl, benzisothiazolyl benzisoxazolyl, benzofuryl, benzopyrazolyl, benzothiazolyl, benzothiophenyl, benzotriazolyl, benzoxazolyl, dihydrofuranyl, dihydrothienyl, dioxolanyl, 1,1-dioxo-thiomorpholinyl, furazanyl, furyl, imidazolidinyl, imidazolinyl, imidazolonyl, imidazolyl, imidazopyridazinyl, imidazopyridyl, indolyl, isoindolyl, isoquinolinyl, isothiazolinyl, isothiazolyl, isoxazolidinyl, isoxazolinyl, isoxazolyl, morpholinyl, naphthyl, naphthyridinyl, oxadiazolyl, oxazolidinyl, oxazolinyl, oxazolonyl, oxazolopyridazinyl, oxazolopyridyl, oxazolyl, oxetanyl, phenyl, piperazinonyl, piperazinyl, piperidinonyl, piperidinyl, phtalazinyl, pteridinyl, purinyl, pyranyl, pyrazinyl, pyrazolopyridinyl, pyrazolyl, pyridazinyl, pyridonyl, pyridyl, pyrimidyl, pyrrolidinonyl, pyrrolidinyl, pyrrolinyl, pyrrolyl, quinazolyl, quinolyl, quinoxalinyl, tetrahydrofuranyl, tetrahydropyranyl, tetrahydrothiopyranyl, tetrahydrotriazolopyridyl, tetrahydrotriazolopyrimidinyl, tetrazolyl, thiadiazolyl, thiazolidinyl, thiazolinyl, thiazolonyl, thiazolopyridazinyl, thiazolopyridyl, thiazolyl, thienyl, thiomorpholinyl, thionaphthyl, thiopyranyl, triazolinyl, triazinyl, triazolyl, cyclopropyl, cyclobutyl, cyclopentyl, cyclopentenyl, cyclohexyl, cyclohexenyl, cycloheptyl, cycloheptenyl, cyclooctyl and cyclooctenyl and each ring of said ring system is optionally substituted with 1-5 radicals independently selected from hydrogen, halogen, -CN, nitro, $-(C_1-C_6)$alkyl, $-(C_0-C_6)$alkylene-O-$(C_0-C_6)$alkyl and $-(C_0-C_6)$alkylene-N-$((C_0-C_6)$alkyl$)_2$.

9. The compound according to any preceding paragraph having the Formula (I), wherein $R^2$ and $R^3$ are each independently selected from the group of hydrogen, methyl and ethyl.

10. The compound according to any preceding paragraph having the Formula (I), wherein $R^4$ and $R^5$ are each independently hydrogen or $-(C_1-C_6)$alkyl.

11. The compound according to any preceding paragraph having the Formula (I), wherein the or each $(A)_m$ is independently selected from the group of hydrogen, halogen, -CN, -OH, $-CF_3$ and an optionally substituted radical selected from the group of $-(C_1-C_6)$alkyl, $-(C_1-C_6)$haloalkyl, $-(C_3-C_7)$cycloalkyl, $-(C_1-C_6)$cyanoalkyl, aryl, heterocycle, $-(C_0-C_6)$alkylene-$OR^4$, -O-$(C_2-C_6)$alkylene-$OR^4$, $-(C_0-C_6)$alkylene-$S(=O)_2-R^4$, $-(C_0-C_6)$alkylene-$NR^4R^5$, $-(C_0-C_6)$alkylene-$S(=O)_2NR^4R^5$, $-(C_0-C_6)$alkylene-C(=O)-$NR^4R^5$, $-(C_0-C_6)$alkylene-$NR^4C(=O)-R^5$, $-(C_0-C_6)$alkylene-C(=O)-$OR^4$, $-(C_0-C_6)$alkylene-C(=O)-$R^4$ and -C(=O)-$(C_1-C_6)$alkylene-$NR^4$-C(=O)-$OR^5$.

12. The compound according to any preceding paragraph having the Formula (I), wherein the or each $(B)_n$ is independently selected from the group of hydrogen, halogen, -CN, $-CF_3$, and an optionally substituted radical selected from the group of $-(C_1-C_6)$alkyl, $-(C_3-C_7)$cycloalkyl, aryl, heteroaryl, heterocycle, $-(C_0-C_6)$alkylene-$OR^8$, $-NR^8(C_2-C_6)$alkylene-$OR^9$, $-(C_0-C_6)$alkylene-$NR^8R^9$, $-(C_0-C_6)$alkylene-C(=O)-$OR^8$ and $-(C_0-C_6)$alkylene-C(=O)-$R^8$.

13. The compound according to any preceding paragraph having the Formula (I), wherein $R^8$ and $R^9$ are each independently selected from the group of hydrogen, - $(C_1-C_6)$haloalkyl, $-(C_1-C_6)$alkyl, $-(C_3-C_7)$cycloalkyl and aryl.

14. The compound according to any preceding paragraph having the Formula (II):

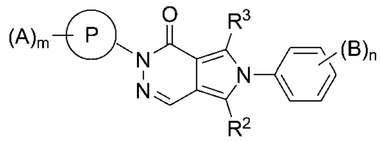

(II)

a pharmaceutically acceptable acid or base addition salt thereof, a stereochemically isomeric form thereof or an N-oxide form thereof.

15. The compound according to paragraph 14 having the Formula (III):

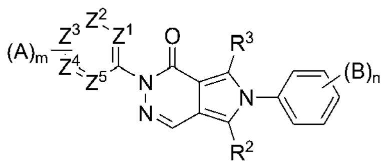

(III)

a pharmaceutically acceptable acid or base addition salt thereof, a stereochemically isomeric form thereof or an *N*-oxide form thereof wherein:
$Z^1$, $Z^2$, $Z^3$, $Z^4$ and $Z^5$ are each independently selected from C or N.

16. The compound according to paragraph 14 having the Formula (IV):

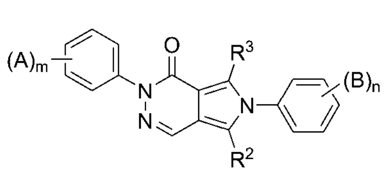

(IV)

a pharmaceutically acceptable acid or base addition salt thereof, a stereochemically isomeric form thereof or an N-oxide form thereof.

17. The compound according to paragraph 16 having the Formula (IV) wherein:

$R^2$ and $R^3$ are independently selected from the group of methyl and ethyl;

the or each $(A)_m$ is independently selected from the group of hydrogen, halogen, - CN, -OH, $-CF_3$ and an optionally substituted radical selected from the group of - $(C_1-C_6)$alkyl, $-(C_1-C_6)$haloalkyl, $-(C_3-C_7)$cycloalkyl, $-(C_1-C_6)$ cyanoalkyl, aryl, heterocycle, $-(C_0-C_6)$alkylene-$OR^4$, -O-$(C_2-C_6)$alkylene-$OR^4$, $-(C_0-C_6)$alkyleneS$(=O)_2$-$R^4$, $-(C_0-C_6)$alkylene-$NR^4R^5$, $-(C_0-C_6)$alkylene-S$(=O)_2NR^4R^5$, $-(C_0-C_6)$alkylene-C(=O)-$NR^4R^5$, $-(C_0-C_6)$alkylene-$NR^4$C(=O)-$R^5$, $-(C_0-C_6)$alkylene-C(=O)-$OR^4$, $-(C_0-C_6)$alkylene-C(=O)-$R^4$ and -C(=O)-$(C_1-C_6)$alkylene-$NR^4$-C(=O)-$OR^5$;

$R^4$ and $R^5$ are each independently hydrogen or $-(C_1-C_6)$alkyl;

the or each $(B)_n$ is independently selected from the group of hydrogen, halogen and an optionally substituted radical selected from the group of $-(C_1-C_6)$alkyl, heterocycle, $-(C_0-C_6)$alkylene-$OR^8$, $-NR^8(C_2-C_6)$alkylene-$OR^9$, $-(C_0-C_6)$alkylene-$NR^8R^9$, $-(C_0-C_6)$alkylene-C(=O)-$OR^8$ and $-(C_0-C_6)$alkylene-C(=O)-$R^8$; and

$R^8$ and $R^9$ are each independently hydrogen and $-(C_1-C_6)$alkyl.

18. The compound according to paragraph 16 having the Formula (IV) wherein:
one or both of $R^2$ and $R^3$ are methyl.

19. The compound according to paragraph 14 having the Formula (V):

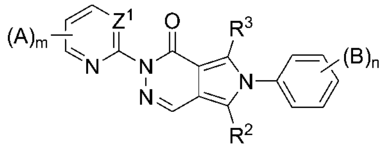

(V)

a pharmaceutically acceptable acid or base addition salt thereof, a stereochemically isomeric form thereof or an N-oxide form thereof wherein:

$Z^1$ is selected from C or N;

$R^2$ and $R^3$ are independently selected from the group of hydrogen, methyl and ethyl;

the or each $(A)_m$ is independently selected from the group of hydrogen, halogen, - CN, -OH, $-CF_3$ and an optionally substituted radical selected from the group of - $(C_1-C_6)$alkyl, $-(C_3-C_7)$cycloalkyl, heterocycle and $-(C_0-C_6)$ alkylene-$OR^4$;

$R^4$ is selected from hydrogen or $-(C_1-C_6)$alkyl;

the or each $(B)_n$ is independently selected from the group of hydrogen, halogen, - CN, $-CF_3$ and an optionally substituted radical selected from the group of $-(C_1-C_6)$alkyl, $-(C_3-C_7)$cycloalkyl, aryl, heteroaryl, heterocycle, $-(C_0-C_6)$alkylene-$OR^8$, - $(C_0-C_6)$alkylene-$NR^8R^9$, $-(C_0-C_6)$alkylene-C(=O)-$OR^8$ and $-(C_0-C_6)$alkylene-C(=O)-$R^8$; and

$R^8$ and $R^9$ are each independently hydrogen, $-(C_1-C_6)$haloalkyl, $-(C_1-C_6)$alkyl, $-(C_3-C_7)$cycloalkyl, or aryl.

20. The compound according to paragraph 19 having the Formula (V) wherein:
one or both of $R^2$ and $R^3$ are methyl.

21. The compound according to paragraph 1 to 20, wherein the compound can exist as optical isomers, and wherein the compound is either a racemic mixture or one or both of the individual optical isomers.

22. The compound according to paragraph 1 to 21, wherein said compound is one or more selected from:

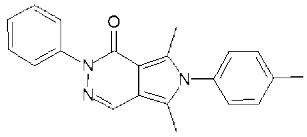
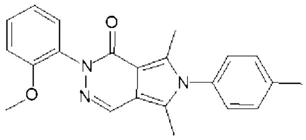
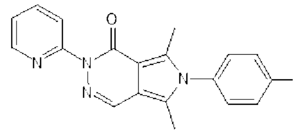

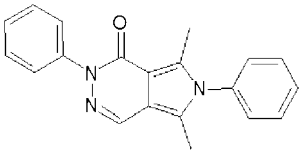
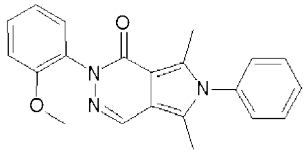
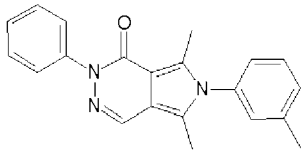

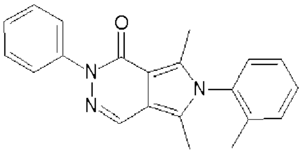
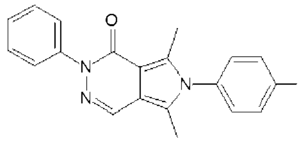
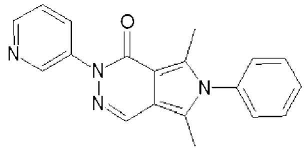

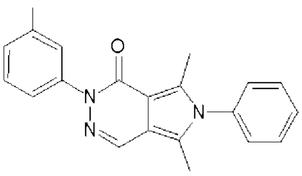
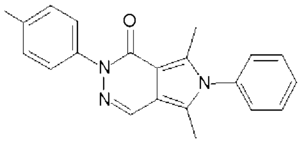
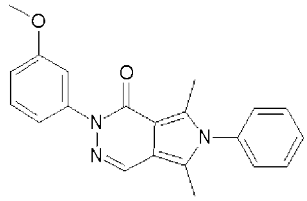

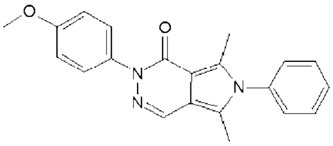
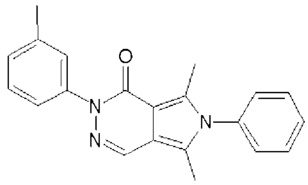
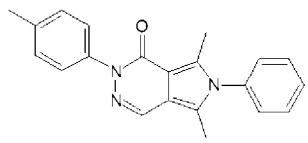

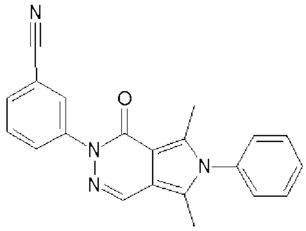
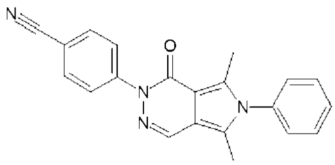
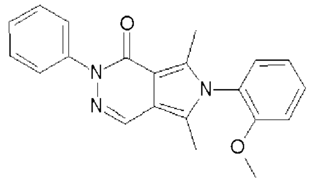

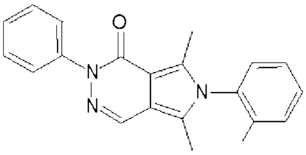
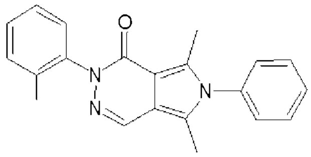
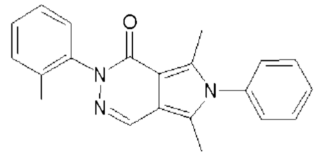

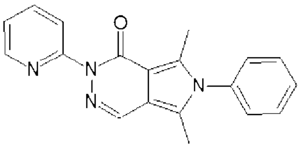
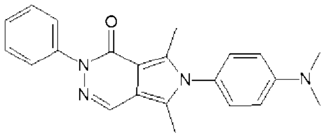
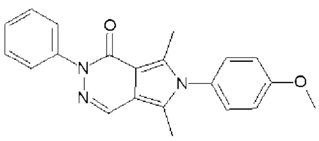

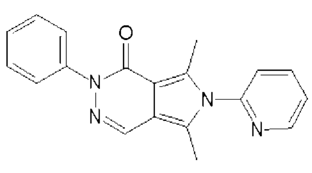
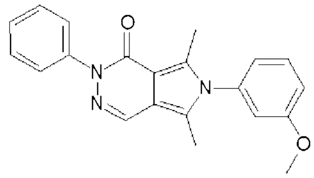
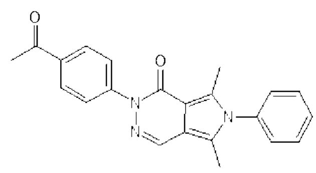
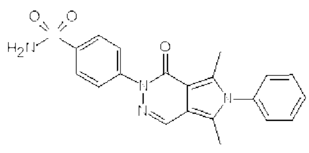
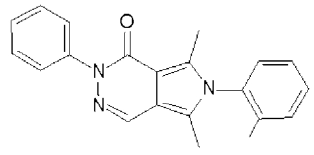
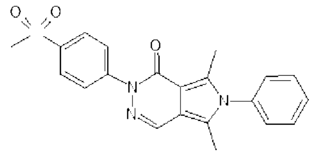
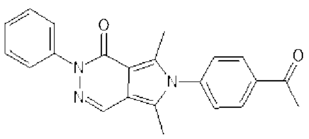
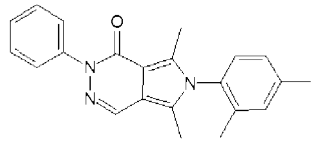
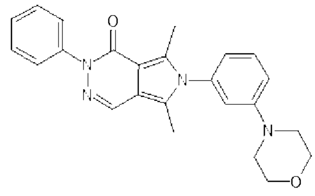
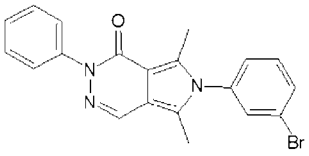
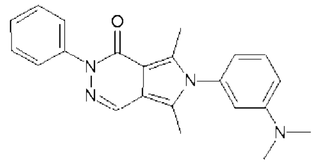
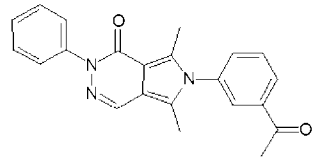
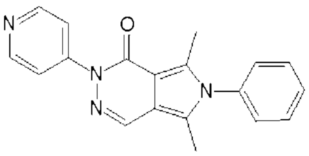
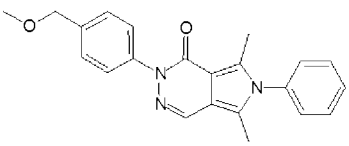
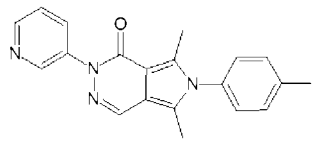
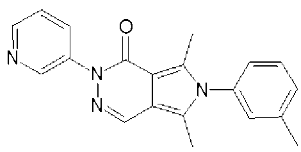
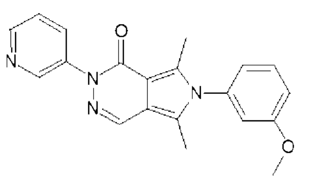
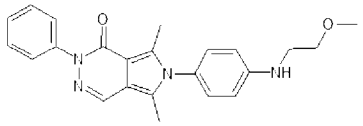
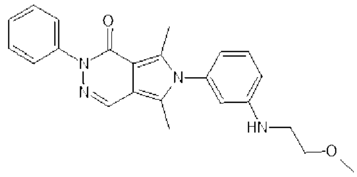
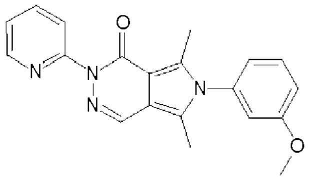
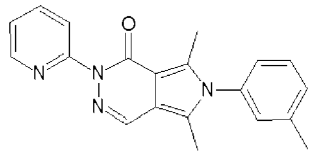
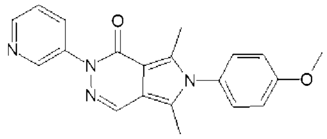
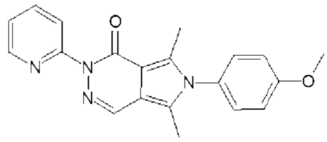
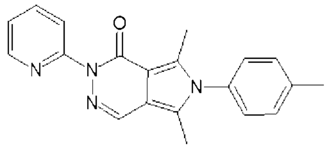

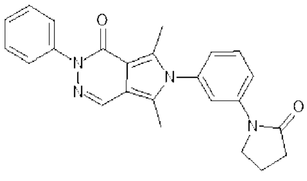
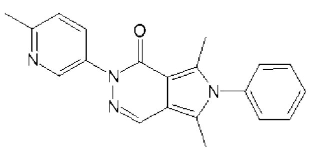
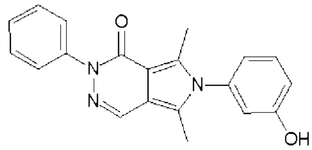
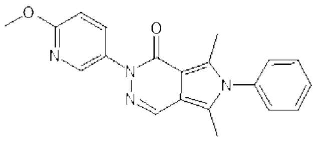
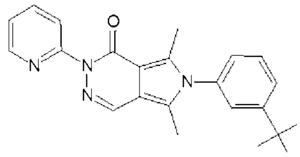
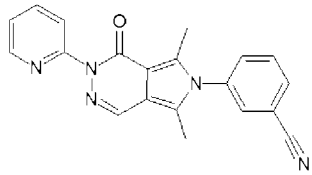
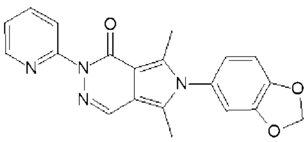
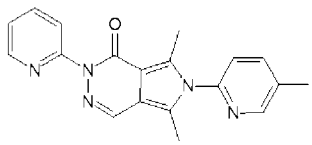
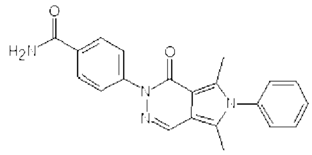
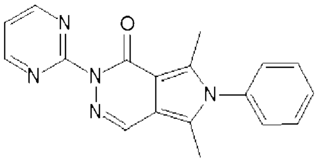
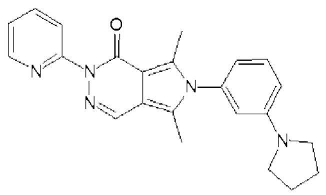
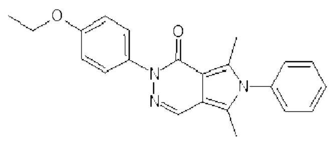
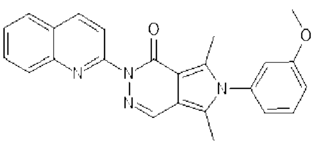
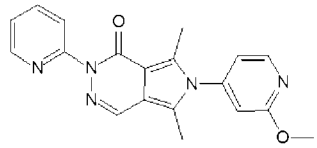
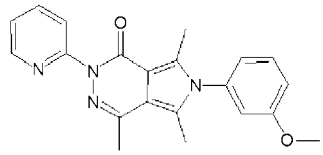
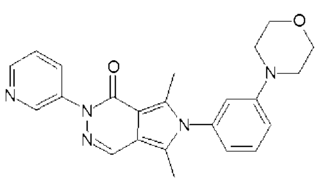
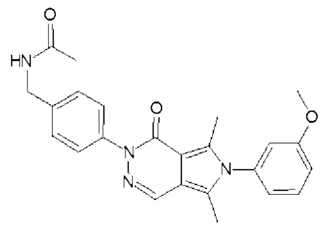
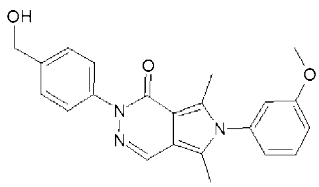
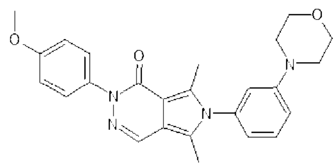
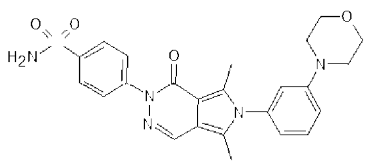
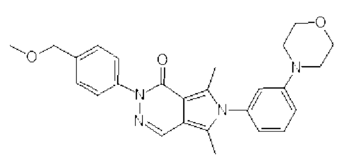
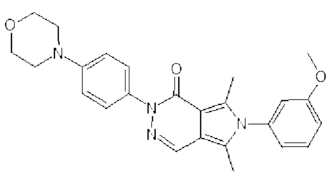
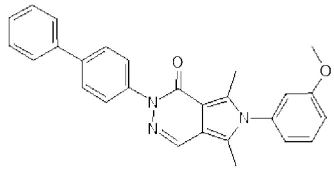
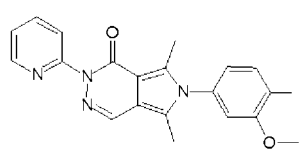

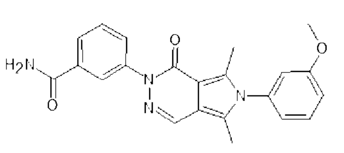
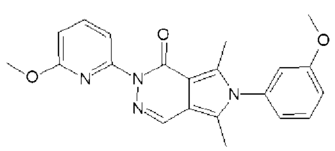
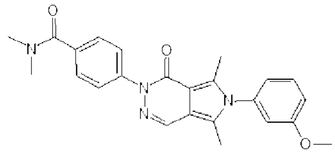

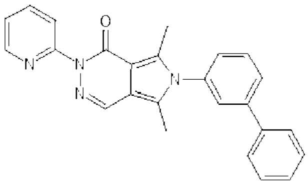
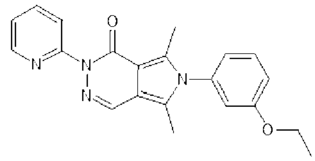
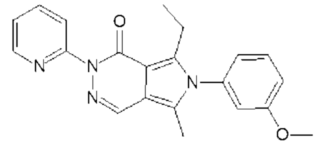

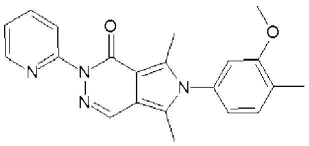
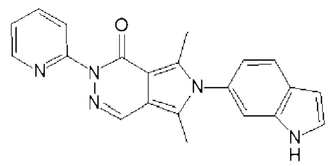
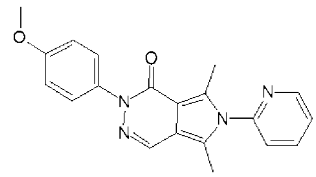

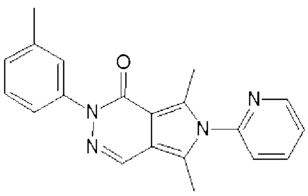
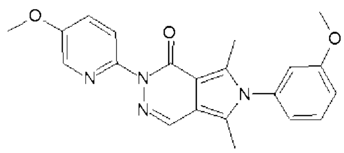
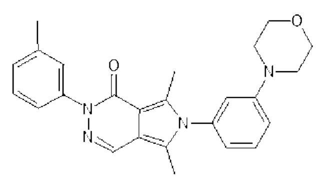

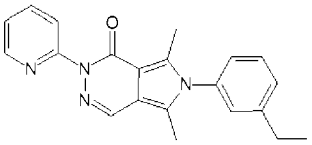
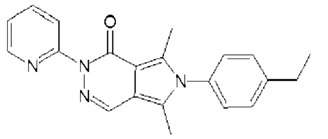
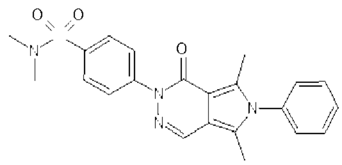

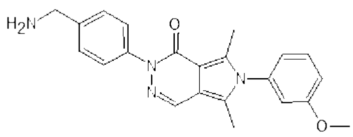
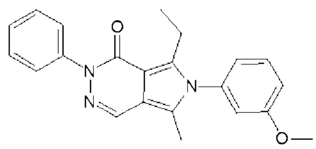
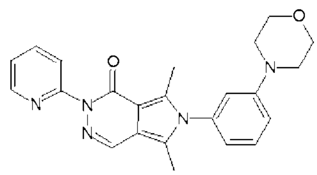

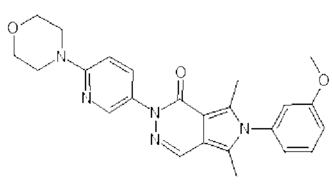
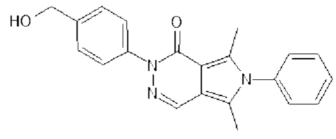
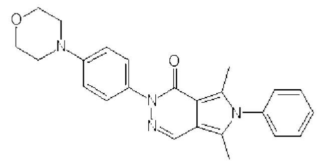

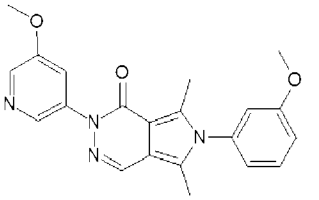
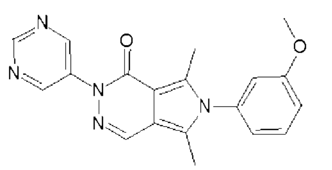
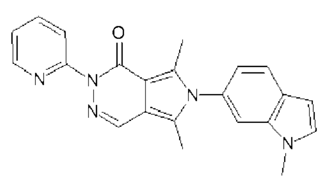

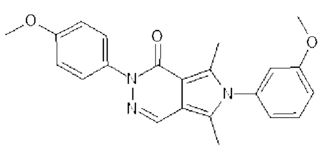
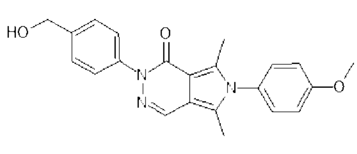
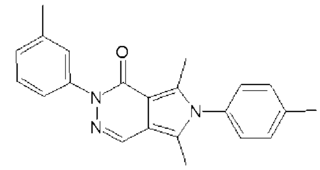

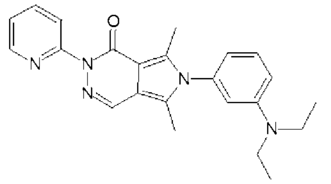
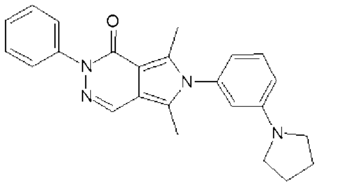
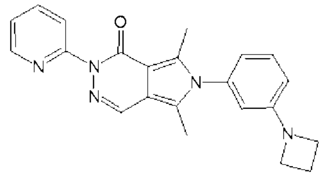

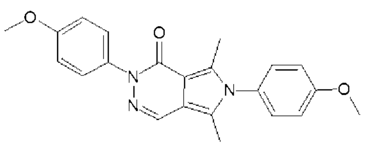
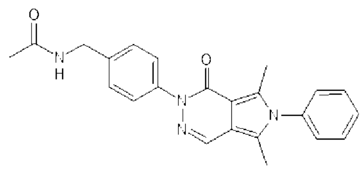
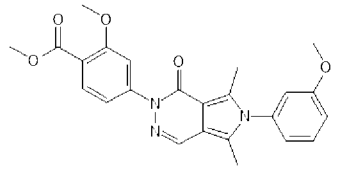

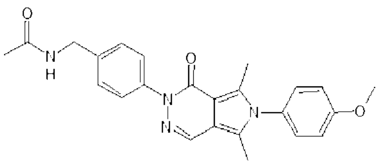
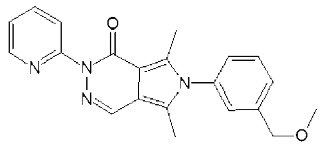
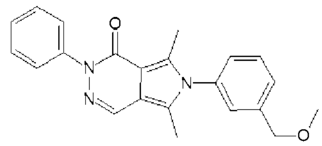

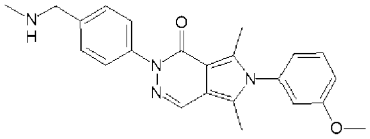
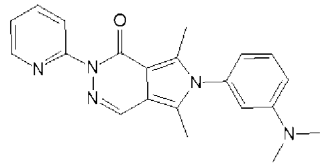
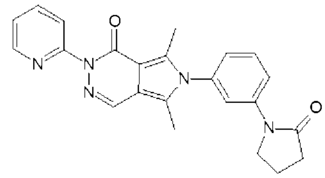

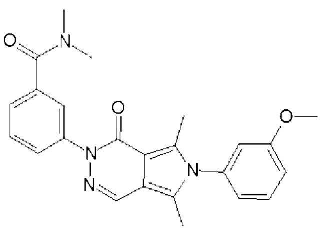
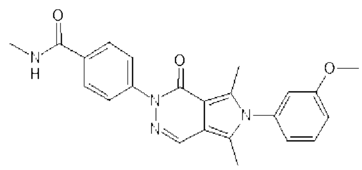
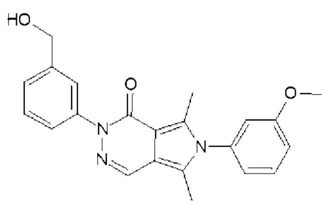

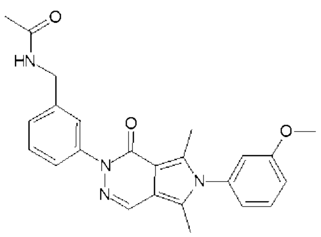
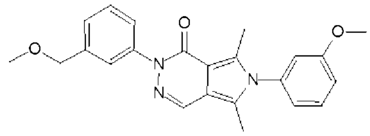
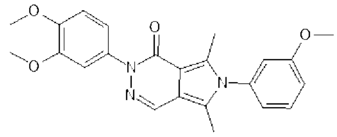

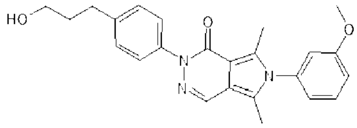
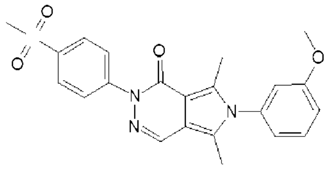
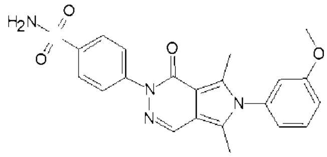

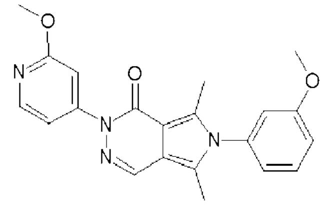
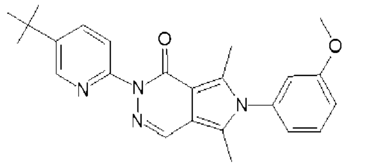
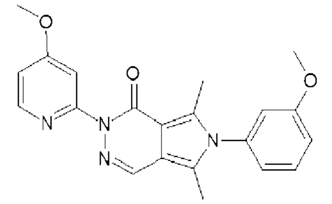

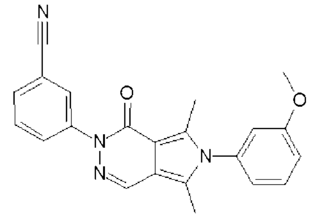
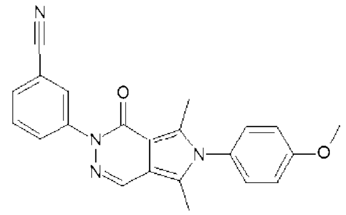
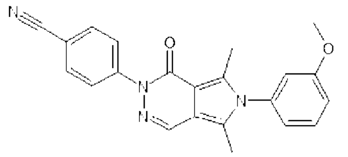

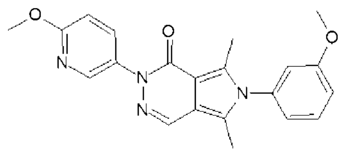
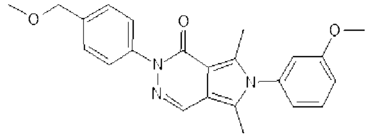
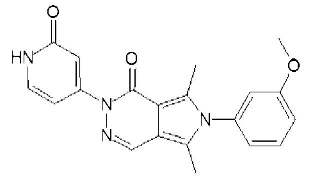

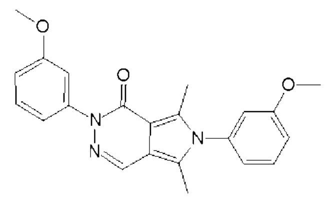
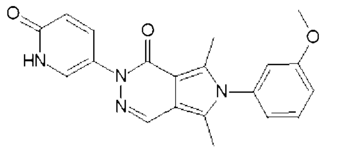
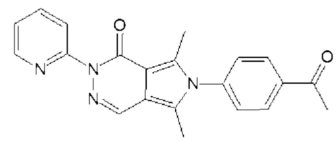

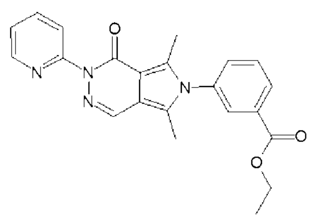
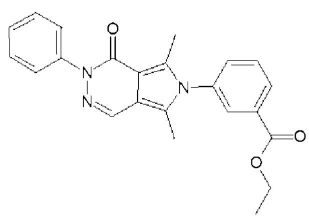
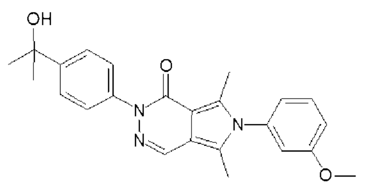

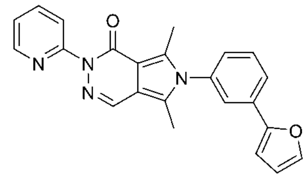
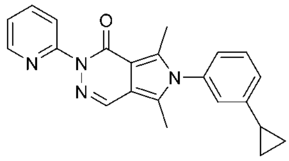
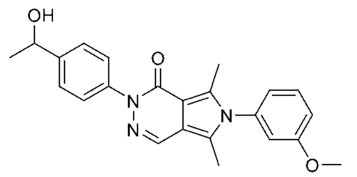

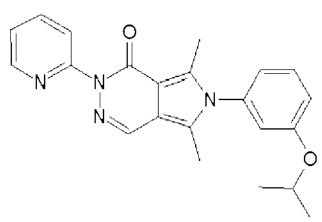
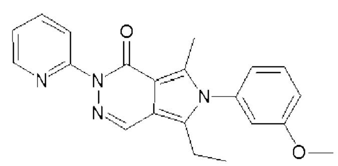
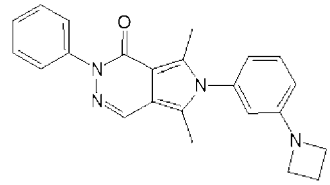

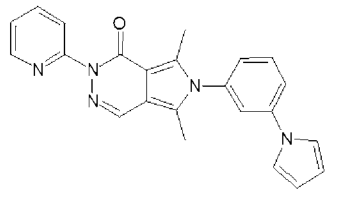
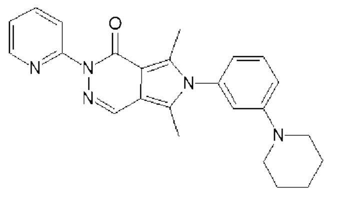
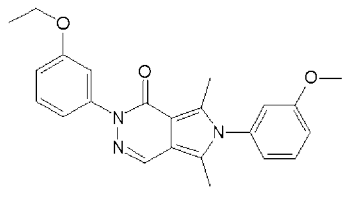

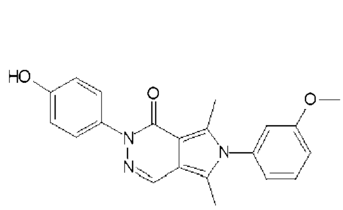
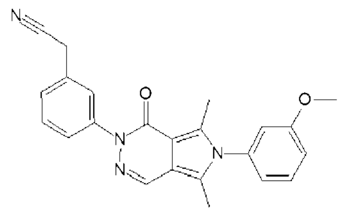
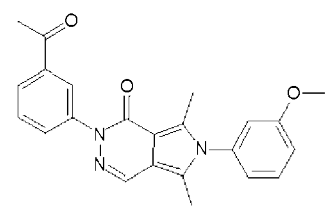

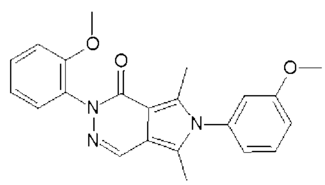
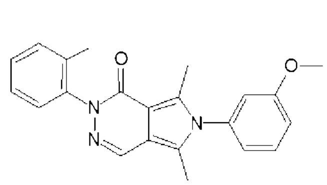
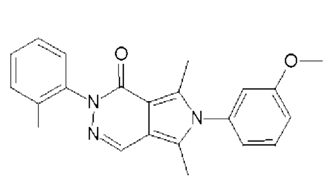

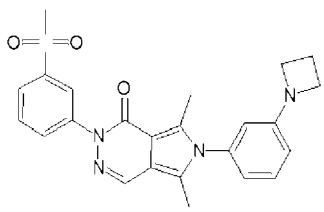
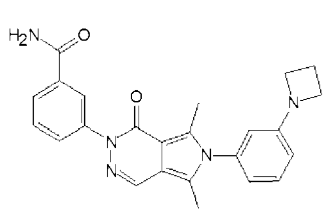
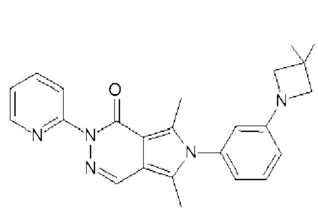

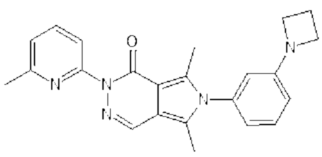
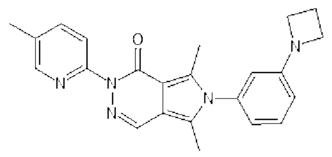
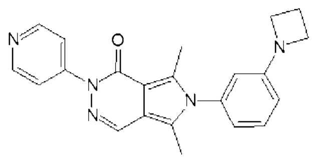

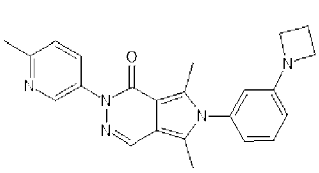
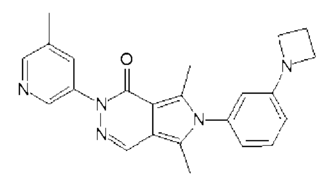
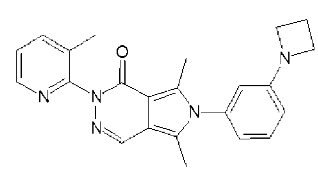

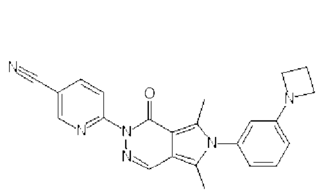
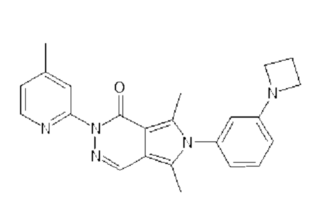
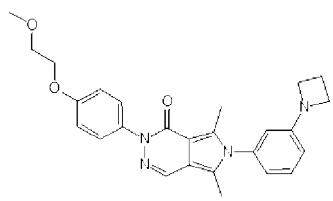

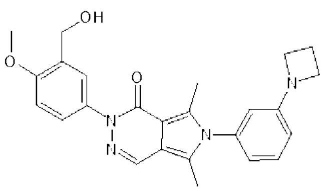
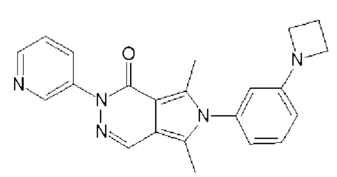
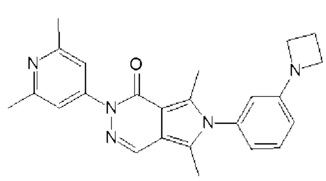

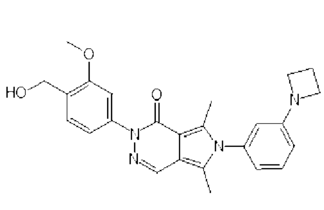
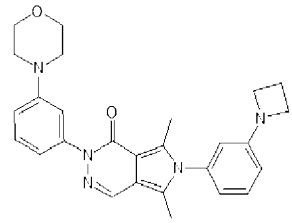
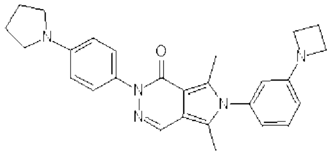

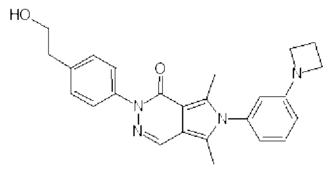
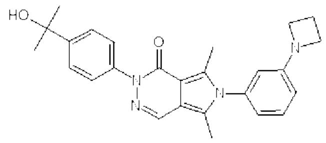
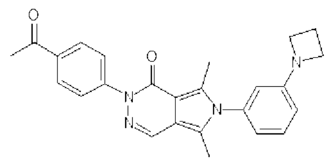

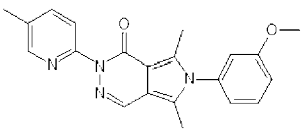
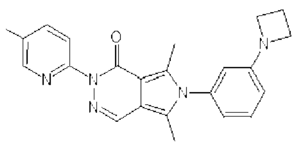
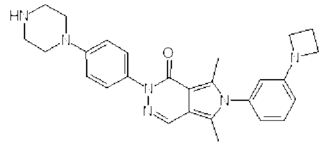

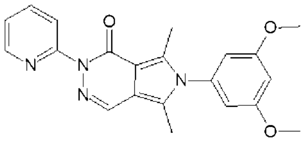
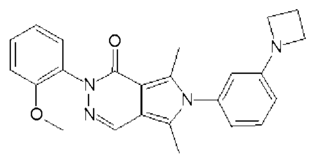
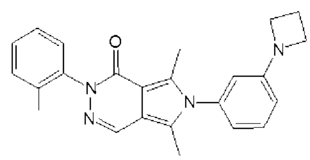

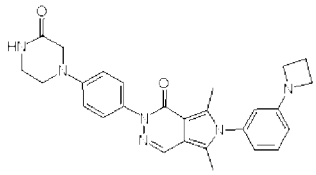
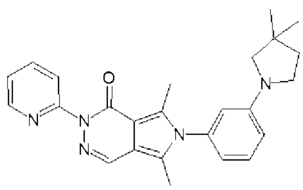
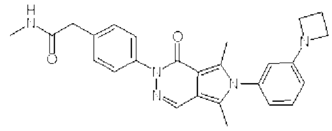

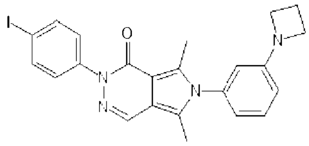
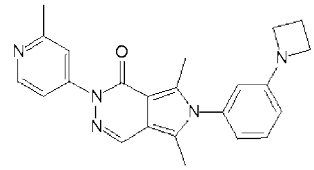
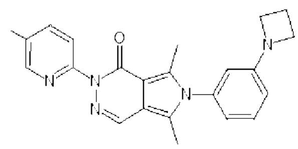

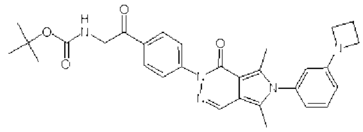
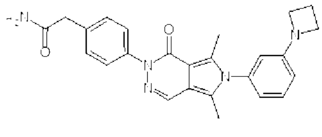
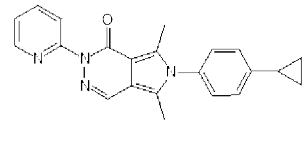

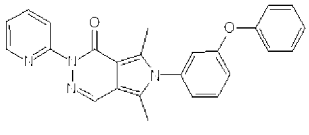
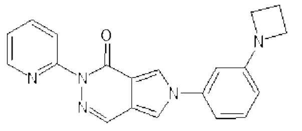
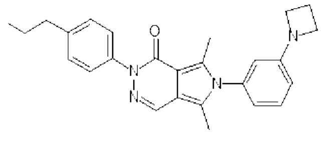

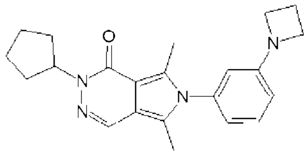
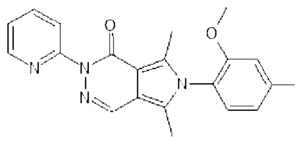
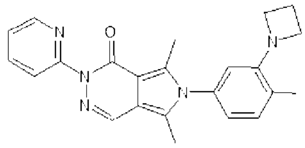
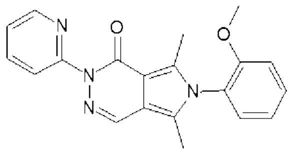
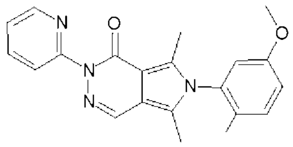
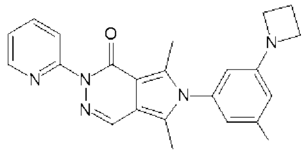
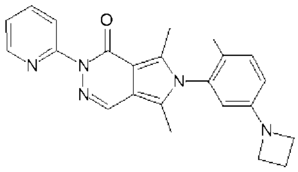
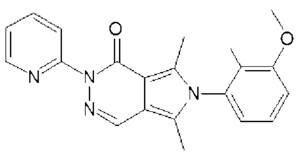
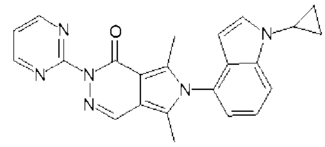
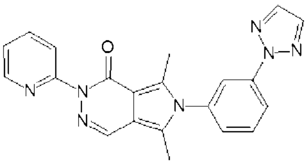
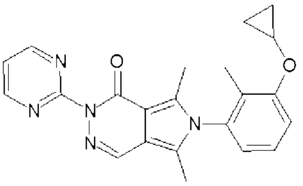
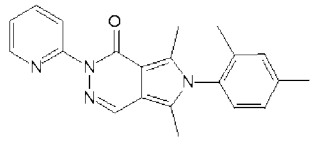
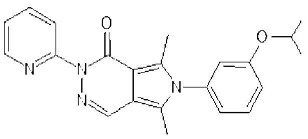
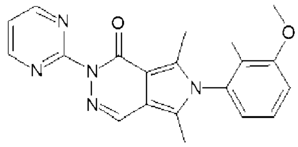
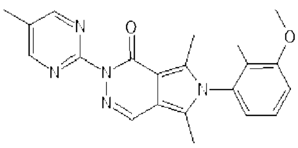
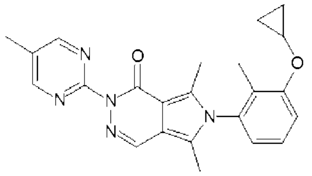
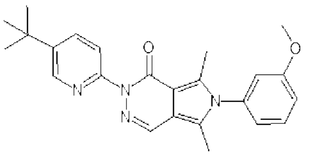
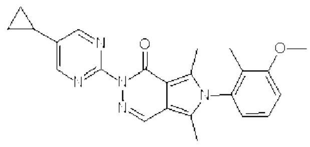
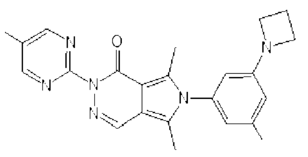
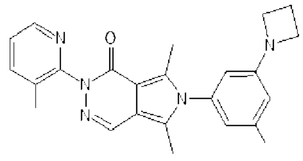
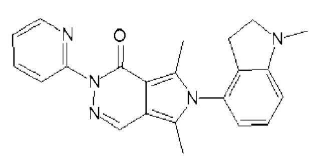
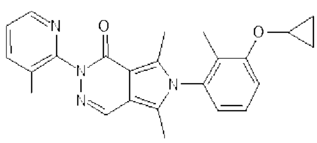
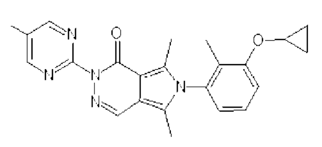
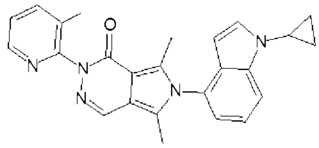
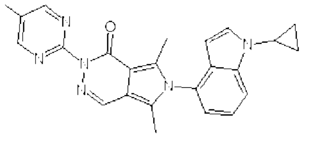
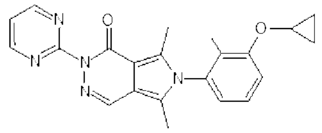
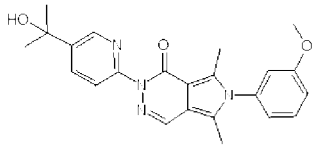

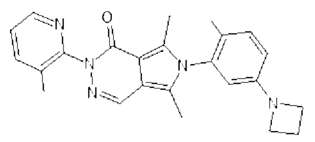
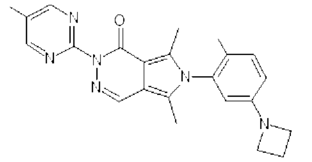
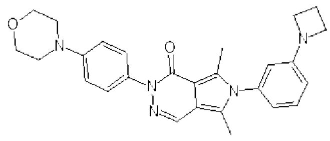
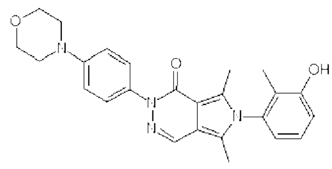
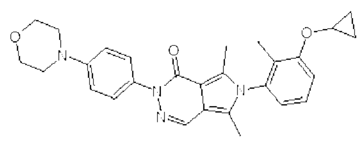
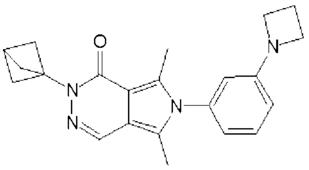
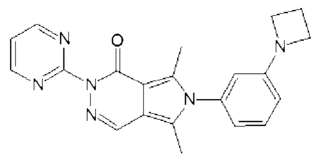
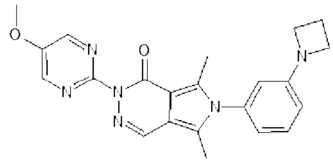
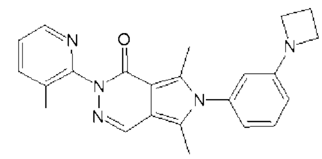
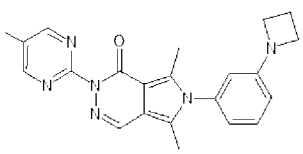
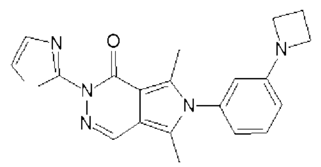
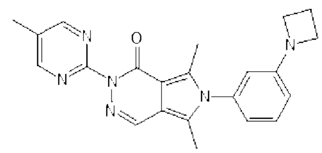
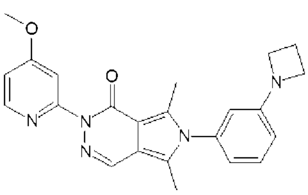
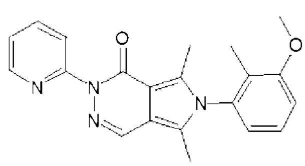
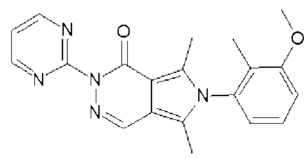
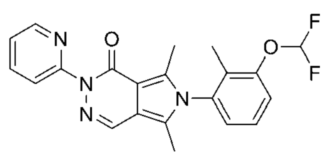
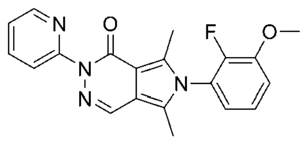
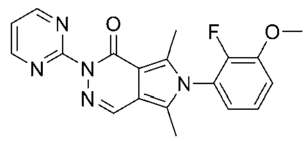
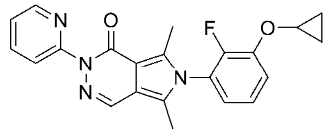
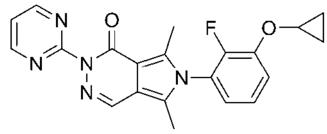
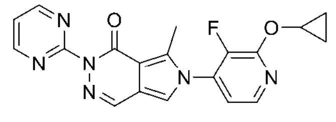
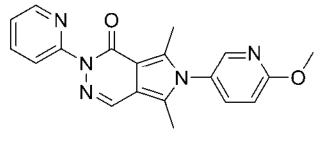
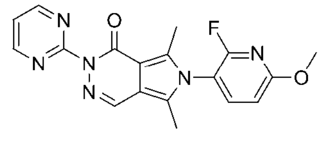
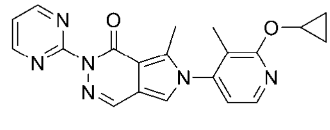
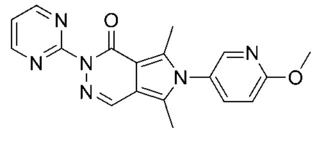
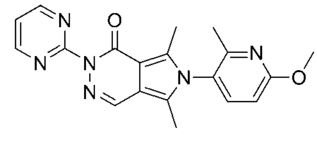
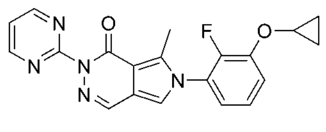

and a pharmaceutically acceptable acid or base addition salt thereof, a stereochemically isomeric form thereof or an

N-oxide form thereof.

23. The compound according to paragraph 22, wherein said compound is one or more selected from

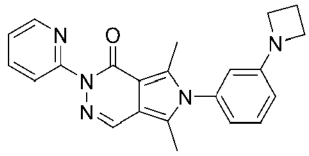,
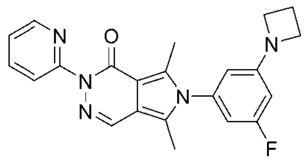,
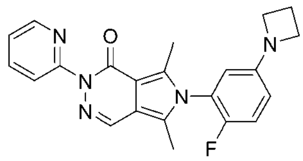,

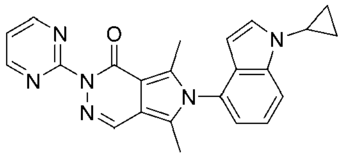,
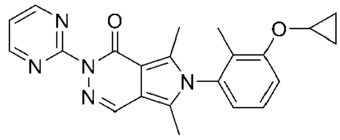,
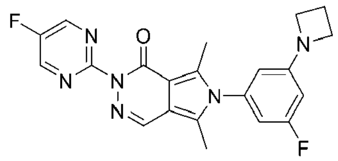,

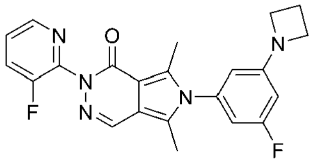,
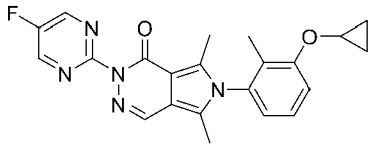,
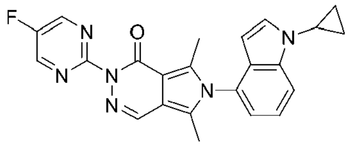,

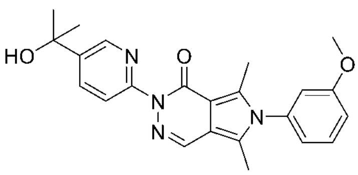,
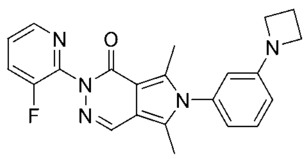,
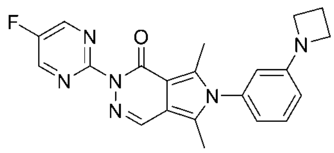.

and

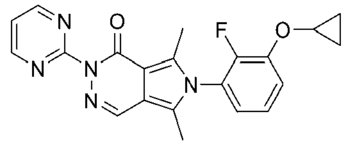,

and a pharmaceutically acceptable acid or base addition salt thereof, a stereochemically isomeric form thereof or an N-oxide form thereof.

24. The compound according to paragraph 23, wherein said compound is one or more selected from

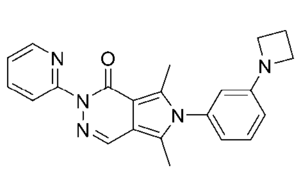,
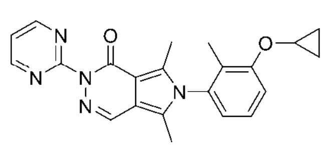,
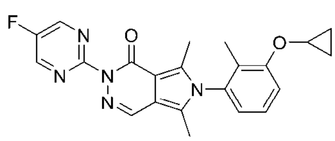

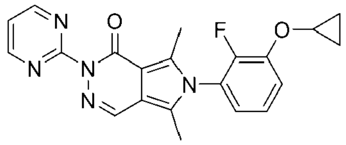

,

and a pharmaceutically acceptable acid or base addition salt thereof, a stereochemically isomeric form thereof or an N-oxide form thereof.

25. A pharmaceutical composition comprising a therapeutically effective amount of a compound according to any one of paragraphs 1 to 24 and a pharmaceutically acceptable carrier and/or excipient.

26. A method of treating or preventing a condition in a mammal, comprising administering to a mammal in need of such treatment or prevention, an effective amount of a compound/composition according to paragraphs 1 to 25.

27. The method according to paragraph 26 wherein the treatment or prevention is affected or facilitated by the modulatory effect of a mGlu7 allosteric modulator, such as a mGlu7 negative allosteric modulator.

28. The method of treating, preventing, ameliorating, controlling or reducing the risk of various neurological and psychiatric disorders associated with glutamate dysfunction in a mammal, comprising administering to a mammal in need of such treatment or prevention, an effective amount of a compound/composition according to any one of paragraphs 1 to 25.

29. The method according to paragraph 28, wherein the treatment or prevention is affected or facilitated by the modulatory effect of a mGlu7 negative allosteric modulator.

30. The method according to any one of paragraphs 26 or 27 wherein the condition is one or more of a central nervous system disorder, an otic disease or disorder or a pain disorder.

31. The method according to paragraph 30, wherein the central nervous disorder is an anxiety disorder such as agoraphobia, generalized anxiety disorder (GAD), obsessive-compulsive disorder (OCD), panic disorder, or post-traumatic stress disorder (PTSD).

32. The method according to paragraph 30, wherein the otic disease and disorder is one or more of an inner ear impairment, age-related hearing impairment (presbycusis), Meniere's disease, sudden hearing loss, noise induced hearing loss, otitis media, autoimmune inner ear disease, acute tinnitus, chronic tinnitus, drug-induced hearing loss, hidden hearing loss, cisplatin-induced hearing loss, aminoglycosides-induced hearing loss, ototoxicity, central auditory processing disorder or vestibular disorder.

33. A method according to paragraph 30, wherein the pain disorder is one or more of neuropathic pain, inflammatory pain, visceral pain, acute pain, chronic pain, severe pain, intractable pain, post-traumatic pain, post-operative pain, headache pain or cancer pain.

34. A compound or composition according to any one of paragraphs 1 to 25 for use as a medicament.

35. A compound or composition according to any one of paragraphs 1 to 25 for use in a method of treatment or prevention as defined in any one of paragraphs 26, 27, 30, 31, 32 or 33.

36. A compound or composition according to any one of paragraphs 1 to 25 for a use in a method as defined in paragraph 28 or 29.

37. Use of a compound according to any one of paragraphs 1 to 25 in the manufacture of a medicament for the treatment or prevention as defined in any one of paragraphs 26, 27, 30, 31, 32 or 33.

38. Use of a compound according to any one of paragraphs 1 to 25 in the manufacture of a medicament for a treatment or prevention as defined in paragraphs 28 or 29.

## Claims

**1.** A compound having the Formula (I):

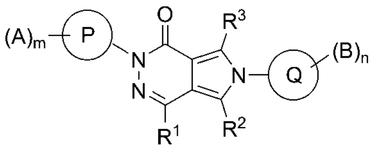

(I)

a pharmaceutically acceptable acid or base addition salt thereof, a stereochemically isomeric form thereof or an *N*-oxide form thereof, wherein:

$R^1$ is selected from the group of hydrogen, $-CH_3$ and $-CF_3$;
$R^2$ and $R^3$ are each independently selected from the group of hydrogen, $-(C_1-C_6)$alkyl and $-(C_1-C_6)$haloalkyl and $-CF_3$;
P represents a cycloalkyl, aryl or heteroaryl of formula:

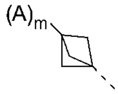 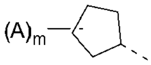 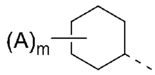

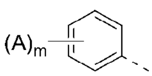 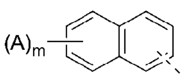 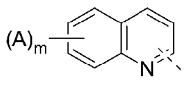 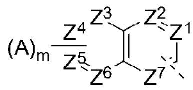

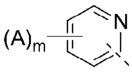 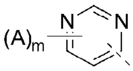 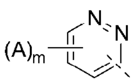 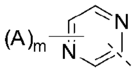 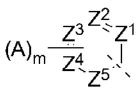

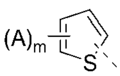 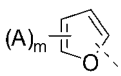 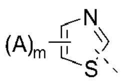 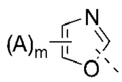 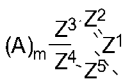

wherein each cycloalkyl, aryl or heteroaryl ring is optionally substituted with m radicals A, wherein m is an integer equal to zero, 1, 2, 3 or 4;
wherein $Z^1$, $Z^2$, $Z^3$, $Z^4$, $Z^5$, $Z^6$ and $Z^7$ are each independently selected from C, N, O or S; provided that at least one of $Z^1$, $Z^2$, $Z^3$, $Z^4$, $Z^5$, $Z^6$ and $Z^7$ is N;
the or each $(A)_m$ is independently selected from the group of hydrogen, halogen, - CN, -OH, $-NO_2$, $-CF_3$, -SH, $-NH_2$ and an optionally substituted radical selected from the group of $-(C_1-C_6)$alkyl, $-(C_1-C_6)$haloalkyl, $-(C_2-C_6)$alkynyl, $-(C_2-C_6)$alkenyl, $-(C_3-C_7)$cycloalkyl, $-(C_1-C_6)$alkylene-$(C_3-C_7)$cycloalkyl, $-(C_3-C_8)$cycloalkenyl, $-(C_1-C_6)$cyanoalkyl, $-(C_1-C_6)$alkylene-heteroaryl, $-(C_1-C_6)$alkylene-aryl, aryl, heteroaryl, $-(C_1-C_6)$alkylene-heterocycle, heterocycle, $-(C_0-C_6)$alkylene-$OR^4$, $-O-(C_2-C_6)$alkylene-$OR^4$, $-NR^4(C_2-C_6)$alkylene-$OR^5$, $-(C_3-C_6)$alkynylene-$OR^4$, $-(C_3-C_6)$alkynylene-$NR^4R^5$, $-(C_3-C_6)$alkenylene-$OR^4$, $-(C_3-C_6)$alkenylene-$NR^4R^5$, $-(C_0-C_6)$alkylene-S-$R^4$, $-O-(C_2-C_6)$alkylene-S-$R^4$, $-NR^4-(C_2-C_6)$alkylene-S-$R^5$, $-(C_0-C_6)$alkylene-S(=O)-$R^4$, $-O-(C_1-C_6)$alkylene-S(=O)-$R^4$, $-NR^4-(C_1-C_6)$alkylene-S(=O)-$R^5$, $-(C_0-C_6)$alkylene-$S(=O)_2$-$R^4$, $-O-(C_1-C_6)$alkylene-$S(=O)_2$-$R^4$, $-NR^4-(C_1-C_6)$alkylene-$S(=O)_2$-$R^5$, $-(C_0-C_6)$alkylene-$NR^4R^5$, $-O-(C_2-C_6)$alkylene-$NR^4R^5$, $-NR^4-(C_2-C_6)$alkylene-$NR^5R^6$, $-(C_0-C_6)$alkylene-$S(=O)_2NR^4R^5$, $-O-(C_1-C_6)$alkylene-$S(=O)_2NR^4R^5$, $-NR^4-(C_1-C_6)$alkylene-$S(=O)_2NR^5R^6$, $-(C_0-C_6)$alkylene-$NR^4$-$S(=O)_2R^5$, $-O-(C_2-C_6)$alkylene-$NR^4$-$S(=O)_2R^5$, $-NR^4-(C_{2-}C_6)$alkylene-$NR^5$-$S(=O)_2R^6$, $-(C_0-C_6)$alkylene-C(=O)-$NR^4R^5$, $-O-(C_1-C_6)$alkylene-C(=O)-$NR^4R^5$, $-NR^4-(C_1-C_6)$alkylene-C(=O)-$NR^5R^6$, - $(C_0-C_6)$alkylene-$NR^4C(=O)$-$R^5$, $-O-(C_2-C_6)$alkylene-$NR^4C(=O)$-$R^5$, $-NR^4-(C_2-C_6)$alkylene-$NR^5C(=O)$-$R^6$, $-(C_0-C_6)$alkylene-OC(=O)-$R^4$, $-O-(C_2-C_6)$alkylene-OC(=O)-$R^4$, $-NR^4-(C_2-C_6)$alkylene-OC(=O)-$R^5$, $-(C_0-C_6)$alkylene-C(=O)-$OR^4$, $-O-(C_1-C_6)$alkylene-C(=O)-$OR^4$, - $NR^4$-(C $_i$-$C_6$)alkylene-C(=0)-$OR^5$, $-(C_0-C_6)$alkylene-C(=O)-$R^4$, $-O-(C_1-C_6)$alkylene-C(=O)-$R^4$, $-NR^4-(C_1-C_6)$alkylene-C(=O)-$R^5$, $-(C_0-C_6)$alkylene-$NR^4$-C(=O)-$OR^5$, $-C(=O)-(C_1-C_6)$alkylene-$NR^4$-C(=O)-$OR^5$, $-(C_0-C_6)$alkylene-O-C(=O)-$NR^4R^5$, $-(C_0-C_6)$alkylene-$NR^4$-C(=O)-$NR^5R^6$, $-O-(C_2-C_6)$alkylene-$NR^4$-C(=O)-$NR^5R^6$, $-NR^4-(C_2-C_6)$alkylene-$NR^5$-C(=O)-$NR^6R^7$, $-(C_0-C_6)$alkylene-$NR^4$-C(=S)-$NR^5R^6$ and $-(C_0-C_6)$alkylene-$NR^4$-C(=$NR^5$)-$NR^6R^7$;

$R^4$, $R^5$, $R^6$ and $R^7$ are each independently hydrogen or an optionally substituted radical selected from the group of -($C_1$-$C_6$)haloalkyl, -($C_1$-$C_6$)alkyl, -($C_1$-$C_6$)cyanoalkyl, -($C_3$-$C_7$)cycloalkyl, -($C_1$-$C_6$)alkylene-($C_3$-$C_7$)cycloalkyl, heteroaryl, -($C_1$-$C_6$)alkylene-heteroaryl, aryl, -($C_1$-$C_6$)alkylene-heterocycle, heterocycle, -($C_1$-$C_6$)alkylene-aryl, -($C_0$-$C_6$)alkylene-O-($C_0$-$C_6$)alkyl and -($C_0$-$C_6$)alkylene-N-(($C_0$-$C_6$)alkyl)$_2$;
Q is aryl or heteroaryl which is optionally substituted with n radicals B, wherein n is an integer equal to zero, 1, 2, 3, 4 or 5;
the or each $(B)_n$ is independently selected from the group of hydrogen, halogen, - CN, -OH, -$NO_2$, -$CF_3$, -SH, -$NH_2$ and an optionally substituted radical selected from the group of -($C_1$-$C_6$)alkyl, -($C_1$-$C_6$)haloalkyl, -($C_2$-$C_6$)alkynyl, -($C_2$-$C_6$)alkenyl, - ($C_3$-$C_7$)cycloalkyl, -($C_1$-$C_6$)alkylene-($C_3$-$C_7$)cycloalkyl, -($C_3$-$C_8$)cycloalkenyl, -($C_1$-$C_6$)cyanoalkyl, -($C_1$-$C_6$)alkylene-heteroaryl, -($C_1$-$C_6$)alkylene-aryl, aryl, heteroaryl, -($C_1$-$C_6$)alkylene-heterocycle, heterocycle, -($C_0$-$C_6$)alkylene-$OR^8$, -O-($C_2$-$C_6$)alkylene-$OR^8$, -$NR^8$($C_2$-$C_6$)alkylene-$OR^9$, -($C_3$-$C_6$)alkynylene-$OR^8$, -($C_3$-$C_6$)alkynylene-$NR^8R^9$, -($C_3$-$C_6$)alkenylene-$OR^8$, -($C_3$-$C_6$)alkenylene-$NR^8R^9$, -($C_0$-$C_6$)alkylene-S-$R^8$, -O-($C_2$-$C_6$)alkylene-S-$R^8$, -$NR^8$-($C_2$-$C_6$)alkylene-S-$R^9$, -($C_0$-$C_6$)alkylene-S(=O)-$R^8$, -O-($C_1$-$C_6$)alkylene-S(=O)-$R^8$, -$NR^8$-($C_1$-$C_6$)alkyleneS(=O)-$R^9$, -($C_0$-$C_6$)alkylene-S(=O)$_2$-$R^8$, -O-($C_1$-$C_6$)alkylene-S(=O)$_2$-$R^8$, -$NR^8$-($C_1$-$C_6$)alkylene-S(=O)$_2$-$R^9$, -($C_0$-$C_6$)alkylene-$NR^8R^9$, -O-($C_2$-$C_6$)alkylene-$NR^8R^9$, - $NR^8$-($C_2$-$C_6$)alkylene-$NR^9R^{10}$, -($C_0$-$C_6$)alkylene-S(=O)$_2NR^8R^9$, -O-($C_1$-$C_6$)alkylene-S(=O)$_2NR^8R^9$, -$NR^8$-($C_1$-$C_6$)alkylene-S(=O)$_2NR^9R^{10}$, -($C_0$-$C_6$)alkylene-$NR^8$-S(=O)$_2R^9$, -O-($C_2$-$C_6$)alkylene-$NR^8$-S(=O)$_2R^9$, -$NR^8$-($C_2$-$C_6$)alkylene-$NR^9$-S(=O)$_2R^{10}$, -($C_0$-$C_6$)alkylene-C(=O)-$NR^8R^9$, -O-($C_1$-$C_6$)alkylene-C(=O)-$NR^8R^9$, -$NR^8$-($C_1$-$C_6$)alkylene-C(=O)-$NR^9R^{10}$, -($C_0$-$C_6$)alkylene-$NR^8$C(=O)-$R^9$, -O-($C_2$-$C_6$)alkylene-$NR^8$C(=O)-$R^9$, -$NR^8$-($C_2$-$C_6$)alkylene-$NR^9$C(=O)-$R^{10}$, -($C_0$-$C_6$)alkylene-OC(=O)-$R^8$, -O-($C_2$-$C_6$)alkylene-OC(=O)-$R^8$, - $NR^8$-($C_2$-$C_6$)alkylene-OC(=O)-$R^9$, -($C_0$-$C_6$)alkylene-C(=O)-$OR^8$, -O-($C_1$-$C_6$)alkylene-C(=O)-$OR^8$, -$NR^8$-($C_1$-$C_6$)alkylene-C(=O)-$OR^9$, -($C_0$-$C_6$)alkylene-C(=O)-$R^8$, -O-($C_1$-$C_6$)alkylene-C(=O)-$R^8$, -$NR^8$-($C_1$-$C_6$)alkylene-C(=O)-$R^9$, -($C_0$-$C_6$)alkylene-$NR^8$-C(=O)-$OR^9$, -($C_0$-$C_6$)alkylene-O-C(=O)-$NR^8R^9$, -($C_0$-$C_6$)alkylene-$NR^8$-C(=O)-$NR^9R^{10}$, -O-($C_2$-$C_6$)alkylene-$NR^8$-C(=O)-$NR^9R^{10}$, -$NR^8$-($C_2$-$C_6$)alkylene-$NR^9$-C(=O)-$NR^{10}R^{11}$, -($C_0$-$C_6$)alkylene-$NR^8$-C(=S)-$NR^9R^{10}$ and - ($C_0$-$C_6$)alkylene-$NR^8$-C(=$NR^9$)-$NR^{10}R^{11}$;
$R^8$, $R^9$, $R^{10}$ and $R^{11}$ are each independently hydrogen or an optionally substituted radical selected from the group of -($C_1$-$C_6$)haloalkyl, -($C_1$-$C_6$)alkyl, -($C_1$-$C_6$)cyanoalkyl, -($C_3$-$C_7$)cycloalkyl, -($C_1$-$C_6$)alkylene-($C_3$-$C_7$)cycloalkyl, heteroaryl, -($C_1$-$C_6$)alkylene-heteroaryl, aryl, -($C_1$-$C_6$)alkylene-heterocycle, heterocycle, -($C_1$-$C_6$)alkylene-aryl, -($C_0$-$C_6$)alkylene-O-($C_0$-$C_6$)alkyl and -($C_0$-$C_6$)alkylene-N-(($C_0$-$C_6$)alkyl)$_2$;
whererin optionally any two radicals A are combined with the intervening atoms to form a 3 to 10 membered bicyclic heterocycle, aryl or heteroaryl ring, wherein each ring is optionally further substituted with 1 to 5 radicals independently selected from the group of halogen, -CN, nitro, -($C_1$-$C_6$)alkyl, -($C_0$-$C_6$)alkylene-O-($C_0$-$C_6$)alkyl and -($C_0$-$C_6$)alkylene-N-(($C_0$-$C_6$)alkyl)$_2$;
wherein optionally two of the substituents $R^4$, $R^5$, $R^6$ or $R^7$ are combined with the intervening atoms to form a 3 to 10 membered heterocycle, aryl or heteroaryl ring, wherein each ring is optionally further substituted with 1 to 5 radicals independently selected from the group of halogen, cyano, nitro, -($C_1$-$C_6$)alkyl, -($C_0$-$C_6$)alkylene-O-($C_0$-$C_6$)alkyl and -($C_0$-$C_6$)alkylene-N-(($C_0$-$C_6$)alkyl)$_2$;
wherein optionally two substituents from $R^8$, $R^9$, $R^{10}$ or $R^{11}$ are combined with the intervening atoms to form a 3 to 10 membered heterocycle, aryl or heteroaryl ring, wherein each ring is optionally further substituted with 1 to 5 radicals independently selected from the group of halogen, cyano, nitro, -($C_1$-$C_6$)alkyl, -($C_0$-$C_6$)alkylene-O-($C_0$-$C_6$)alkyl and -($C_0$-$C_6$)alkylene-N-(($C_0$-$C_6$)alkyl)$_2$;
wherein optionally any two radicals B are combined with the intervening atoms to form a 3 to 10 membered bicyclic heterocycle, aryl or heteroaryl ring, wherein each ring is optionally further substituted with 1 to 5 radicals independently selected from the group of halogen, -CN, nitro, -($C_1$-$C_6$)alkyl, -($C_0$-$C_6$)alkylene-O-($C_0$-$C_6$)alkyl and -($C_0$-$C_6$)alkylene-N-(($C_0$-$C_6$)alkyl)$_2$.

**2.** The compound according to claim 1 having the Formula (I) wherein:
Q represents an aryl or heteroaryl group of formula:

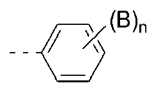 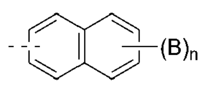 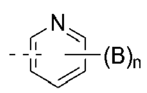 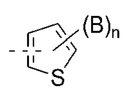 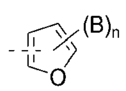 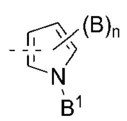

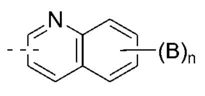 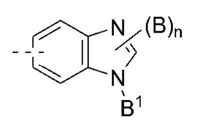 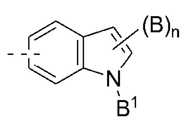 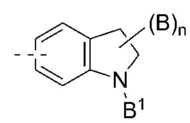 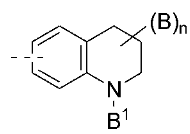

wherein each radical is optionally substituted with n radicals B, wherein n is an integer equal to zero, 1, 2, 3, 4 or 5; and wherein $B^1$ is a radical B.

**3.** The compound according to claim 2 having the Formula (I), wherein Q represents an aryl or heteroaryl group of formula:

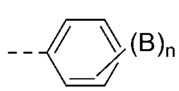 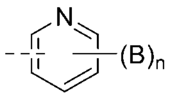 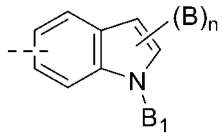 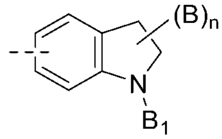,

wherein each radical is optionally substituted with n radicals B, wherein n is an integer equal to zero, 1, 2, 3, 4 or 5, and wherein $B^1$ is a radical B; and/or wherein $B^1$ is -hydrogen, $(C_1\text{-}C_6)$alkyl or $-(C_3\text{-}C_7)$cycloalkyl.

**4.** The compound according to any preceding claim having the Formula (I) wherein P represents a cycloalkyl, aryl or heteroaryl of formula:

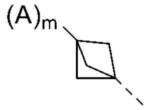 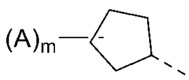 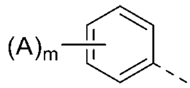

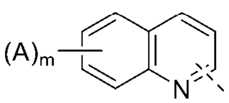 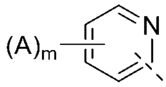 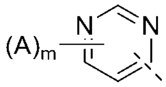 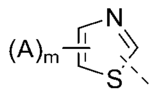,

wherein each radical is optionally substituted with m radicals A, wherein m is an integer equal to zero, 1, 2, 3 or 4.

**5.** The compound according to any preceding claim having the Formula (1) wherein:

the cycloalkyl, heterocycle, aryl and heteroaryl ring systems of $(A)_m$ are selected from the group of azetidinyl, benzimidazolyl, benzisothiazolyl benzisoxazolyl, benzofuryl, benzopyrazolyl, benzothiazolyl, benzothiophenyl, benzotriazolyl, benzoxazolyl, dihydrofuranyl, dihydrothienyl, dioxolanyl, 1,1-dioxo-thiomorpholinyl, furazanyl, furyl, imidazolidinyl, imidazolinyl, imidazolonyl, imidazolyl, imidazopyridazinyl, imidazopyridyl, indolyl, isoindolyl, isoquinolinyl, isothiazolinyl, isothiazolyl, isoxazolidinyl, isoxazolinyl, isoxazolyl, morpholinyl, naphthyl, naphthyridinyl, oxadiazolyl, oxazolidinyl, oxazolinyl, oxazolonyl, oxazolopyridazinyl, oxazolopyridyl, oxazolyl, oxetanyl, phenyl, piperazinonyl, piperazinyl, piperidinonyl, piperidinyl, phtalazinyl, pteridinyl, purinyl, pyranyl, pyrazinyl, pyrazolopyridinyl, pyrazolyl, pyridazinyl, pyridonyl, pyridyl, pyrimidyl, pyrrolidinonyl, pyrrolidinyl, pyrrolinyl, pyrrolyl, quinazolyl, quinolyl, quinoxalinyl, tetrahydrofuranyl, tetrahydropyranyl, tetrahydrothiopyranyl, tetrahydrotriazolopyridyl, tetrahydrotriazolopyrimidinyl, tetrazolyl, thiadiazolyl, thiazolidinyl, thiazolinyl, thiazolonyl, thiazolopyridazinyl, thiazolopyridyl, thiazolyl, thienyl, thiomorpholinyl, thionaphthyl, thiopyranyl, triazolinyl, triazinyl, triazolyl, cyclopropyl, cyclobutyl, cyclopentyl, cyclopentenyl, cyclohexyl, cyclohexenyl, cycloheptyl, cycloheptenyl, cyclooctyl and cyclooctenyl and each ring of said ring system is optionally substituted independently with 1 to 4 substituents $R^4$, $R^5$, $R^6$ or $R^7$; and/or wherein the cycloalkyl, heterocycle, aryl and heteroaryl ring systems of $(B)_n$ are selected from the group of azetidinyl, benzimidazolyl, benzisothiazolyl benzisoxazolyl, benzofuryl, benzopyrazolyl, benzothiazolyl, benzothiophenyl, benzotriazolyl, benzoxazolyl, dihydrofuranyl, dihydrothienyl, dioxolanyl, 1,1-dioxo-thiomorpholinyl, furazanyl, furyl, imidazolidinyl, imidazolinyl, imidazolonyl, imidazolyl, imidazopyridazinyl, imidazopyridyl, indolyl, isoindolyl, isoquinolinyl, isothiazolinyl, isothiazolyl, isoxazolidinyl, isoxazolinyl, isoxazolyl, morpholinyl, naphthyl, naphthyridinyl, oxadiazolyl, oxazolidinyl, oxazolinyl, oxazolonyl, oxazolopyridazinyl, oxazolopyridyl, oxazolyl, oxetanyl, phenyl, piperazinonyl, piperazinyl, piperidinonyl, piperidinyl, phtalazinyl, pteridinyl, purinyl, pyranyl, pyrazinyl, pyrazolopyridinyl, pyrazolyl, pyridazinyl, pyridonyl, pyridyl, pyrimidyl, pyrrolidinonyl, pyrrolidinyl, pyrrolinyl, pyrrolyl, quinazolyl, quinolyl, quinoxalinyl, tetrahydrofuranyl, tetrahydropyranyl, tetrahydrothiopyranyl, tetrahydrotriazolopyridyl, tetrahydrotriazolopyrimidinyl, tetrazolyl, thiadiazolyl, thiazolidinyl, thiazolinyl, thiazolonyl, thiazolopyridazinyl, thiazolopyridyl, thiazolyl, thienyl, thiomorpholinyl, thionaphthyl, thiopyranyl, triazolinyl, triazinyl, triazolyl, cyclopropyl, cyclobutyl, cyclopentyl, cyclopentenyl, cyclohexyl, cyclohexenyl, cycloheptyl, cycloheptenyl, cyclooctyl and cyclooctenyl and each ring of said ring system is optionally substituted independently with 1 to 4 substituents $R^8$, $R^9$, $R^{10}$ or $R^{11}$;

and/or wherein the cycloalkyl, heterocycle, aryl and heteroaryl ring systems of $R^4$, $R^5$, $R^6$, $R^7$, $R^8$, $R^9$, $R^{10}$ or $R^{11}$ are selected from the group of azetidinyl, benzimidazolyl, benzisothiazolyl benzisoxazolyl, benzofuryl, benzopyrazolyl, benzothiazolyl, benzothiophenyl, benzotriazolyl, benzoxazolyl, dihydrofuranyl, dihydrothienyl, dioxolanyl, 1,1-dioxo-thiomorpholinyl, furazanyl, furyl, imidazolidinyl, imidazolinyl, imidazolonyl, imidazolyl, imidazopyridazinyl, imidazopyridyl, indolyl, isoindolyl, isoquinolinyl, isothiazolinyl, isothiazolyl, isoxazolidinyl, isoxazolinyl, isoxazolyl, morpholinyl, naphthyl, naphthyridinyl, oxadiazolyl, oxazolidinyl, oxazolinyl, oxazolonyl, oxazolopyridazinyl, oxazolopyridyl, oxazolyl, oxetanyl, phenyl, piperazinonyl, piperazinyl, piperidinonyl, piperidinyl, phtalazinyl, pteridinyl, purinyl, pyranyl, pyrazinyl, pyrazolopyridinyl, pyrazolyl, pyridazinyl, pyridonyl, pyridyl, pyrimidyl, pyrrolidinonyl, pyrrolidinyl, pyrrolinyl, pyrrolyl, quinazolyl, quinolyl, quinoxalinyl, tetrahydrofuranyl, tetrahydropyranyl, tetrahydrothiopyranyl, tetrahydrotriazolopyridyl, tetrahydrotriazolopyrimidinyl, tetrazolyl, thiadiazolyl, thiazolidinyl, thiazolinyl, thiazolonyl, thiazolopyridazinyl, thiazolopyridyl, thiazolyl, thienyl, thiomorpholinyl, thionaphthyl, thiopyranyl, triazolinyl, triazinyl, triazolyl, cyclopropyl, cyclobutyl, cyclopentyl, cyclopentenyl, cyclohexyl, cyclohexenyl, cycloheptyl, cycloheptenyl, cyclooctyl and cyclooctenyl and each ring of said ring system is optionally substituted with 1-5 radicals independently selected from hydrogen, halogen, -CN, nitro, $-(C_1-C_6)$alkyl, $-(C_0-C_6)$alkylene-O-$(C_0-C_6)$alkyl and $-(C_0-C_6)$alkylene-N-$((C_0-C_6)$alkyl$)_2$; and/or wherein $R^2$ and $R^3$ are each independently selected from the group of hydrogen, methyl and ethyl; and/or
wherein $R^4$ and $R^5$ are each independently hydrogen or $-(C_1-C_6)$alkyl; and/or
wherein the or each $(A)_m$ is independently selected from the group of hydrogen, halogen, -CN, -OH, $-CF_3$ and an optionally substituted radical selected from the group of $-(C_1-C_6)$alkyl, $-(C_1-C_6)$haloalkyl, $-(C_3-C_7)$cycloalkyl, $-(C_1-C_6)$cyanoalkyl, aryl, heterocycle, $-(C_0-C_6)$alkylene-$OR^4$, -O-$(C_2-C_6)$alkylene-$OR^4$, $-(C_0-C_6)$alkylene-$S(=O)_2$-$R^4$, $-(C_0-C_6)$alkylene-$NR^4R^5$, $-(C_0-C_6)$alkylene-$S(=O)_2NR^4R^5$, $-(C_0-C_6)$alkylene-C(=O)-$NR^4R^5$, $-(C_0-C_6)$alkylene-$NR^4C(=O)$-$R^5$, $-(C_0-C_6)$alkylene-C(=O)-$OR^4$, $-(C_0-C_6)$alkylene-C(=O)-$R^4$ and -C(=O)-$(C_1-C_6)$alkylene-$NR^4$-C(=O)-$OR^5$; and/or
wherein the or each $(B)_n$ is independently selected from the group of hydrogen, halogen, -CN, $-CF_3$, and an optionally substituted radical selected from the group of $-(C_1-C_6)$alkyl, $-(C_3-C_7)$cycloalkyl, aryl, heteroaryl, heterocycle, $-(C_0-C_6)$alkylene-$OR^8$, $-NR^8(C_2-C_6)$alkylene-$OR^9$, $-(C_0-C_6)$alkylene-$NR^8R^9$, $-(C_0-C_6)$alkylene-C(=O)-$OR^8$ and $-(C_0-C_6)$alkylene-C(=O)-$R^8$; and/or wherein $R^8$ and $R^9$ are each independently selected from the group of hydrogen, $-(C_1-C_6)$haloalkyl, $-(C_1-C_6)$alkyl, $-(C_3-C_7)$cycloalkyl and aryl.

**6.** The compound according to any preceding claim having the Formula (II):

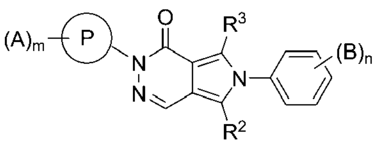

(II)

a pharmaceutically acceptable acid or base addition salt thereof, a stereochemically isomeric form thereof or an N-oxide form thereof;
or having the Formula (III):

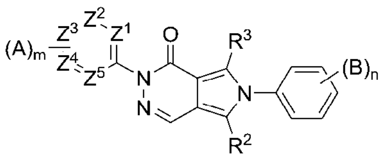

(III)

a pharmaceutically acceptable acid or base addition salt thereof, a stereochemically isomeric form thereof or an N-oxide form thereof wherein:
$Z^1$, $Z^2$, $Z^3$, $Z^4$ and $Z^5$ are each independently selected from C or N;
or having the Formula (IV):

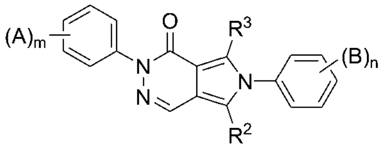

(IV)

a pharmaceutically acceptable acid or base addition salt thereof, a stereochemically isomeric form thereof or an N-oxide form thereof.

**7.** The compound according to claim 6 having the Formula (IV) wherein:

$R^2$ and $R^3$ are independently selected from the group of methyl and ethyl;
the or each $(A)_m$ is independently selected from the group of hydrogen, halogen, - CN, -OH, $-CF_3$ and an optionally substituted radical selected from the group of - $(C_1-C_6)$alkyl, $-(C_1-C_6)$haloalkyl, $-(C_3-C_7)$cycloalkyl, $-(C_1-C_6)$cyanoalkyl, aryl, heterocycle, $-(C_0-C_6)$alkylene-$OR^4$, -O-$(C_2-C_6)$alkylene-$OR^4$, $-(C_0-C_6)$alkyleneS$(=O)_2$-$R^4$, $-(C_0-C_6)$alkylene-$NR^4R^5$, $-(C_0-C_6)$alkylene-S$(=O)_2NR^4R^5$, $-(C_0-C_6)$alkylene-C(=O)-$NR^4R^5$, $-(C_0-C_6)$alkylene-$NR^4$C(=O)-$R^5$, $-(C_0-C_6)$alkylene-C(=O)-$OR^4$, $-(C_0-C_6)$alkylene-C(=O)-$R^4$ and -C(=O)-$(C_1-C_6)$alkylene-$NR^4$-C(=O)-$OR^5$;
$R^4$ and $R^5$ are each independently hydrogen or $-(C_1-C_6)$alkyl;
the or each $(B)_n$ is independently selected from the group of hydrogen, halogen and an optionally substituted radical selected from the group of $-(C_1-C_6)$alkyl, heterocycle, $-(C_0-C_6)$alkylene-$OR^8$, $-NR^8(C_2-C_6)$alkylene-$OR^9$, $-(C_0-C_6)$alkylene-$NR^8R^9$, $-(C_0-C_6)$alkylene-C(=O)-$OR^8$ and $-(C_0-C_6)$alkylene-C(=O)-$R^8$; and
$R^8$ and $R^9$ are each independently hydrogen and $-(C_1-C_6)$alkyl; or wherein one or both of $R^2$ and $R^3$ are methyl.

**8.** The compound according to claim 6 having the Formula (V):

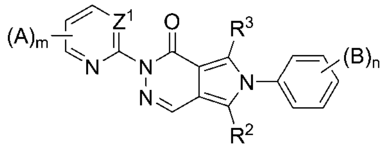

(V)

a pharmaceutically acceptable acid or base addition salt thereof, a stereochemically isomeric form thereof or an N-oxide form thereof wherein:

$Z^1$ is selected from C or N;
$R^2$ and $R^3$ are independently selected from the group of hydrogen, methyl and ethyl;
the or each $(A)_m$ is independently selected from the group of hydrogen, halogen, - CN, -OH, $-CF_3$ and an optionally substituted radical selected from the group of - $(C_1-C_6)$alkyl, $-(C_3-C_7)$cycloalkyl, heterocycle and $-(C_0-C_6)$alkylene-$OR^4$;
$R^4$ is selected from hydrogen or $-(C_1-C_6)$alkyl;
the or each $(B)_n$ is independently selected from the group of hydrogen, halogen, - CN, $-CF_3$ and an optionally substituted radical selected from the group of $-(C_1-C_6)$alkyl, $-(C_3-C_7)$cycloalkyl, aryl, heteroaryl, heterocycle, $-(C_0-C_6)$alkylene-$OR^8$, - $(C_0-C_6)$alkylene-$NR^8R^9$, $-(C_0-C_6)$alkylene-C(=O)-$OR^8$ and $-(C_0-C_6)$alkylene-C(=O)-$R^8$; and
$R^8$ and $R^9$ are each independently hydrogen, $-(C_1-C_6)$haloalkyl, $-(C_1-C_6)$alkyl, $-(C_3-C_7)$cycloalkyl, or aryl for example wherein:
one or both of $R^2$ and $R^3$ are methyl.

**9.** The compound according to claims 1 to 8, wherein the compound can exist as optical isomers, and wherein the compound is either a racemic mixture or one or both of the individual optical isomers.

**10.** The compound according to claims 1 to 9, wherein said compound is one or more selected from:

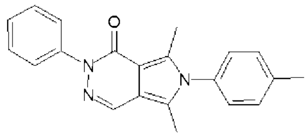 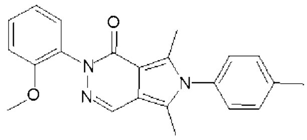 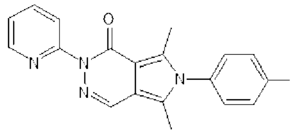

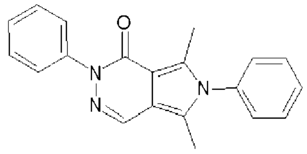
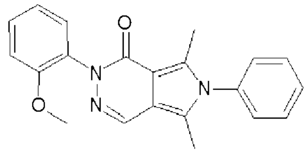
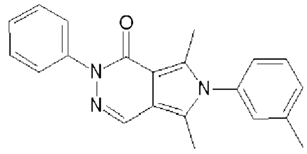
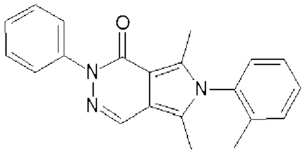
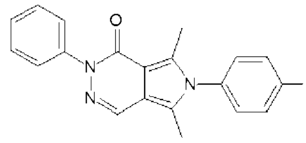
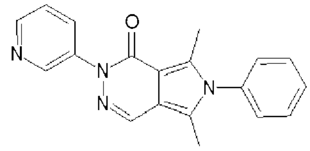
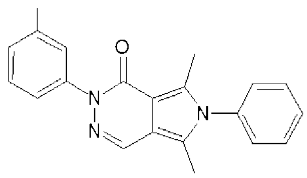
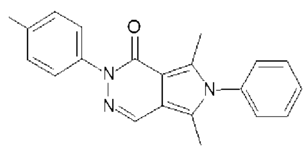
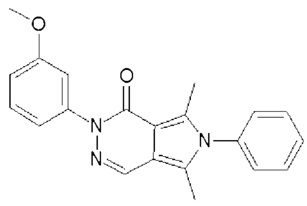
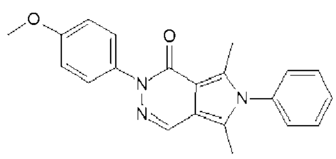
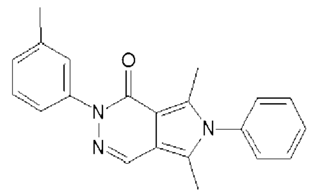
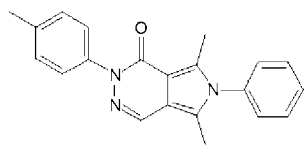
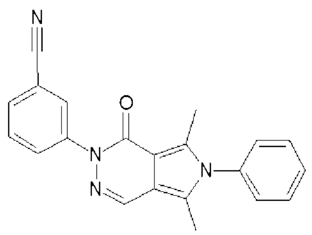
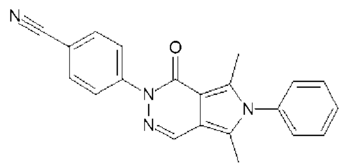
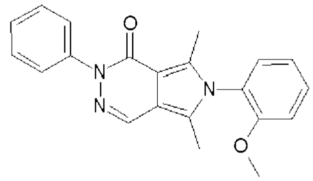
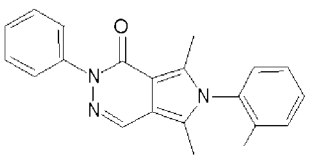
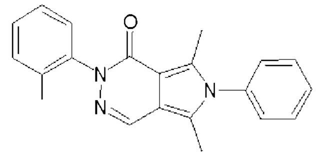
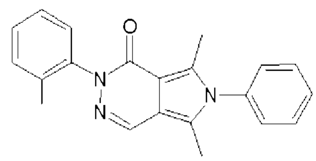
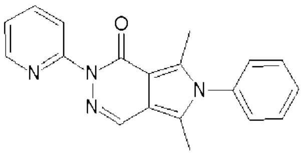
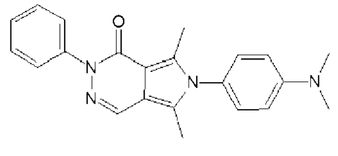
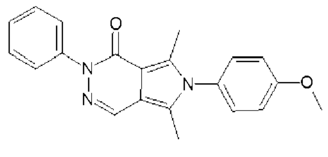
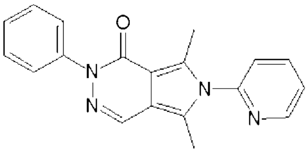
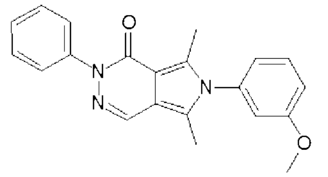
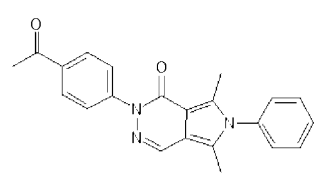

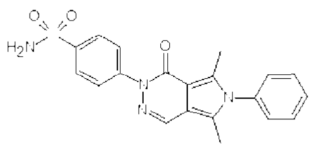
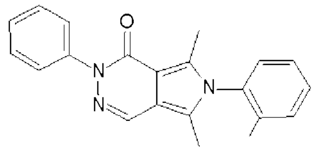
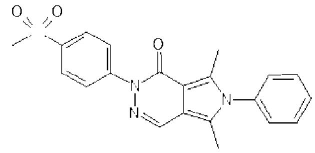

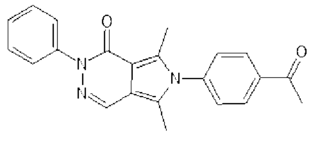
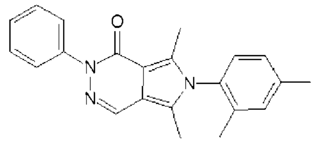
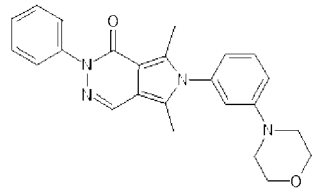

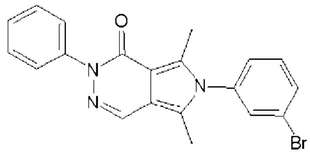
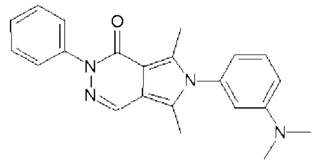
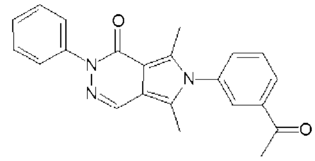

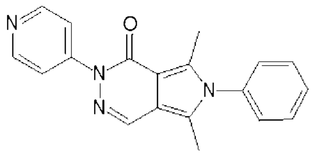
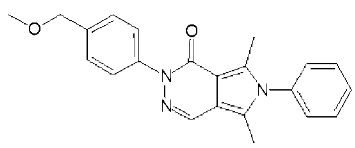
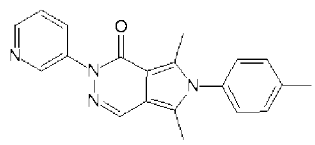

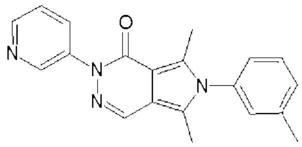
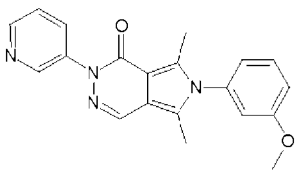
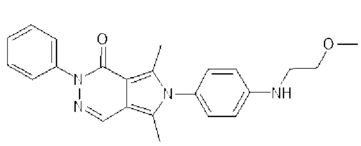

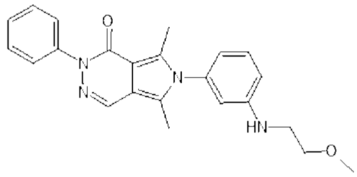
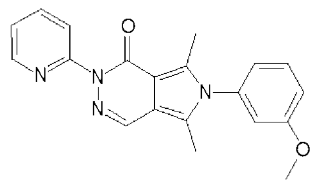
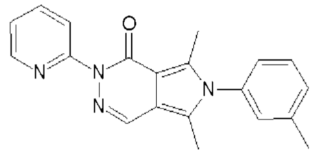

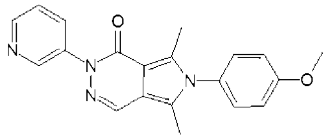
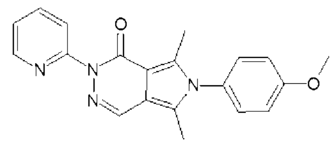
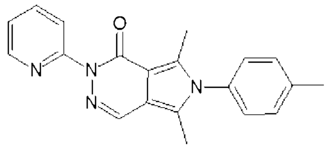

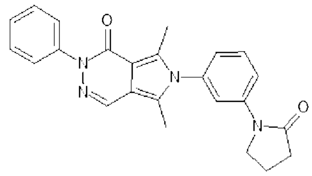
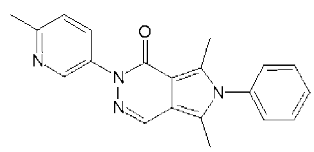
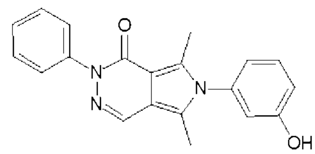

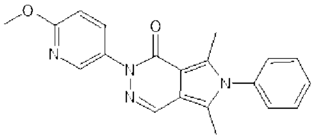
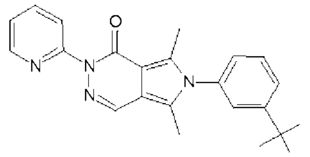
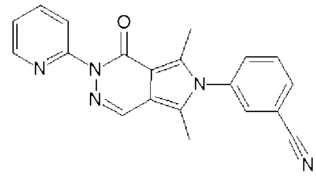

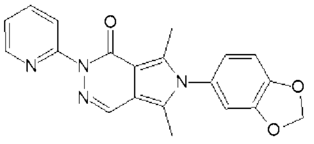
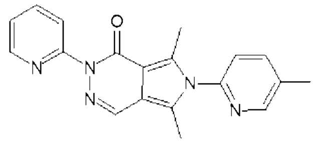
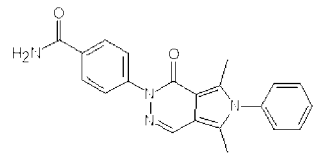

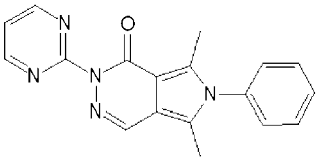
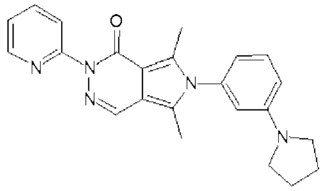
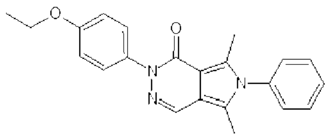

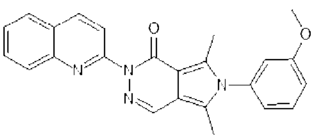
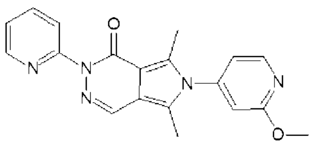
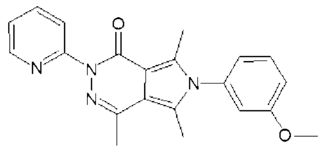

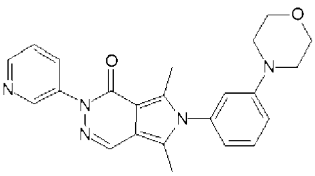
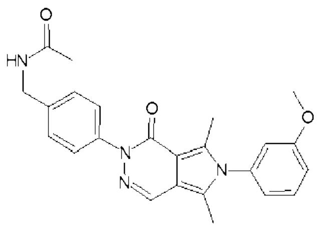
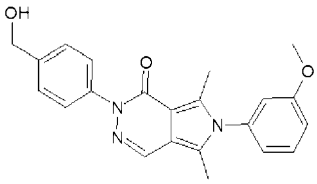

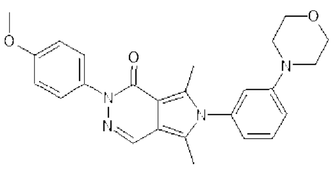
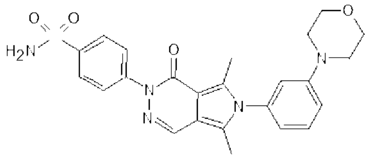
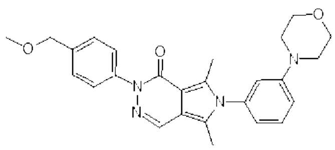

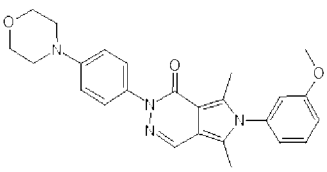
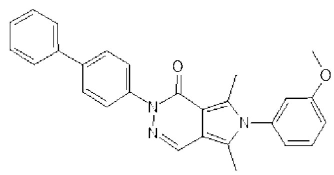
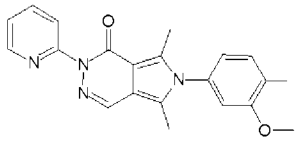

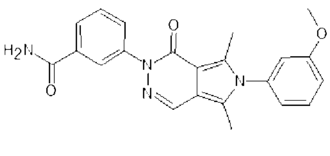
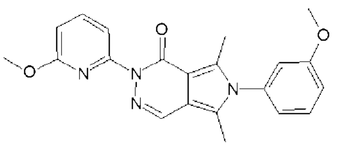
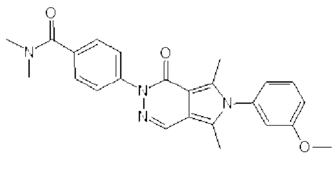

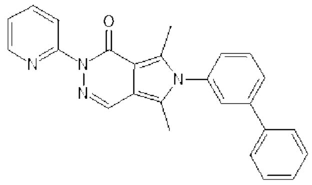
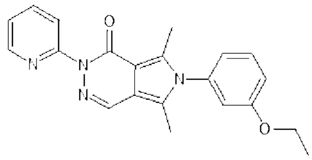
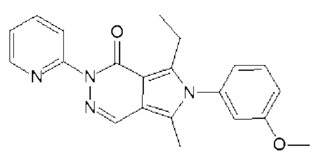
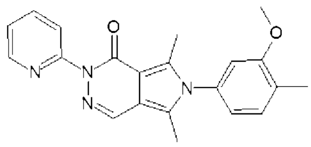
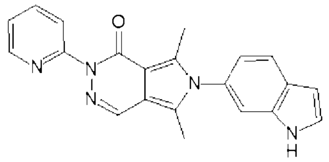
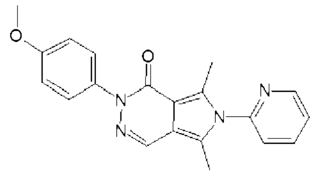
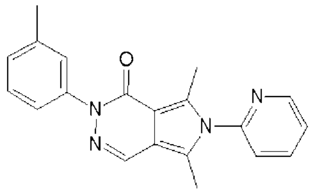
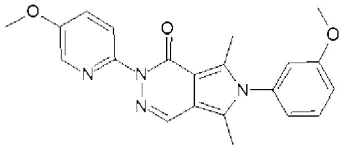
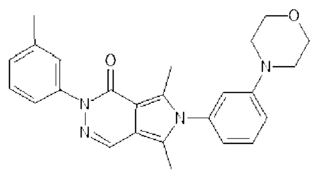
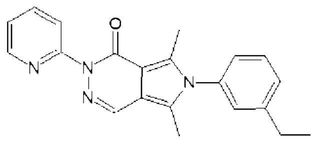
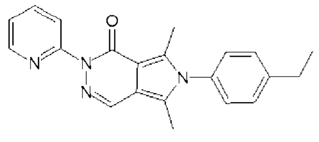
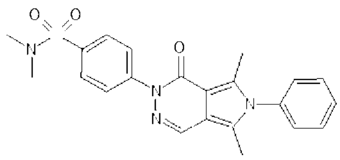
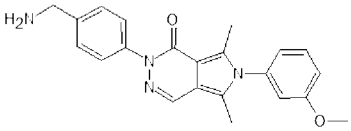
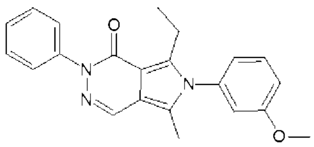
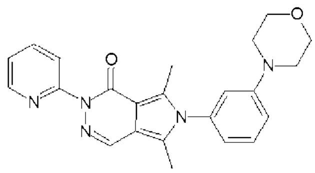
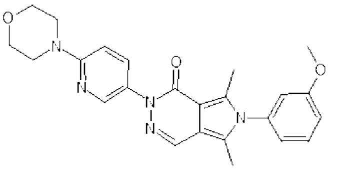
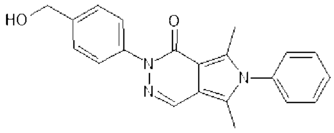
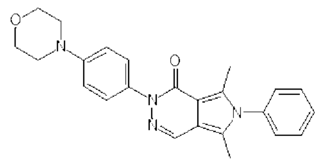
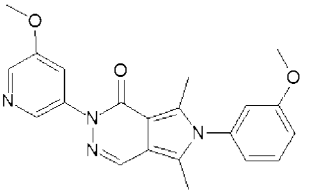
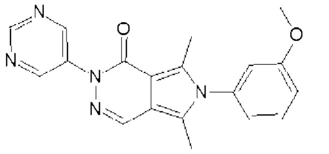
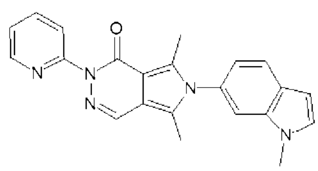
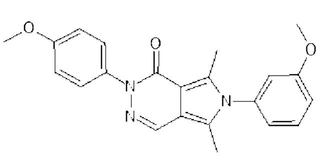
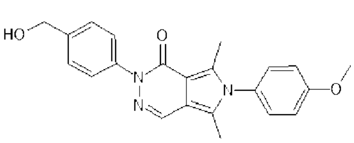
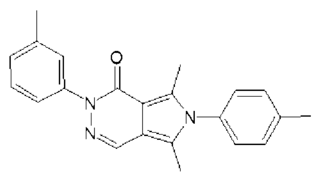

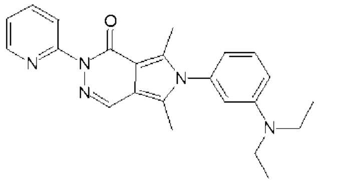 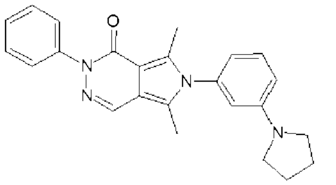 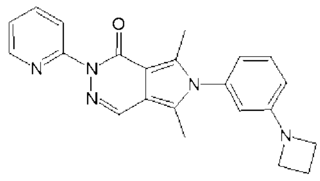

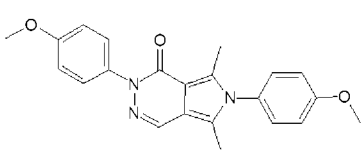 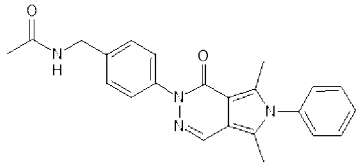 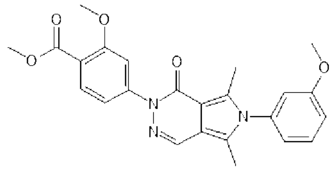

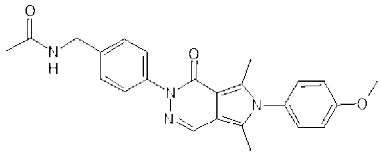 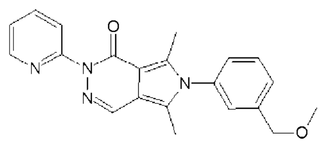 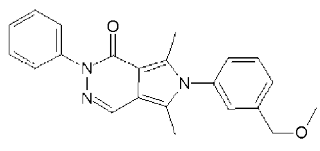

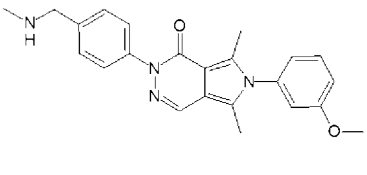 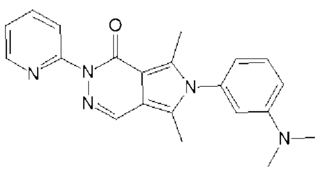 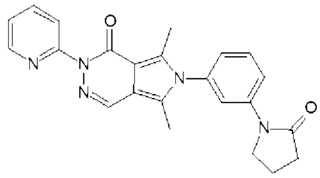

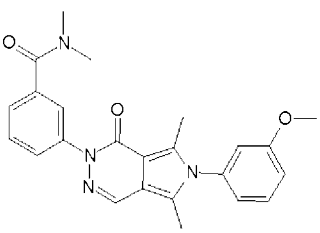 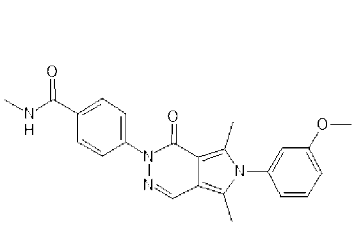 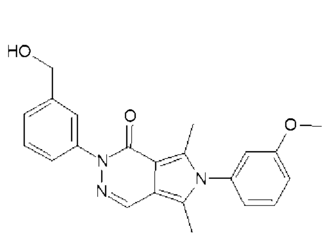

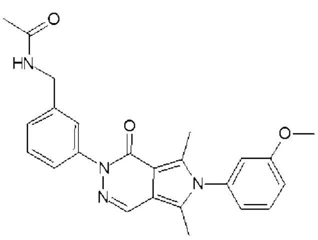 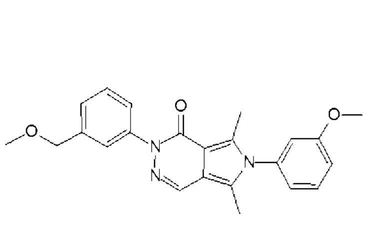 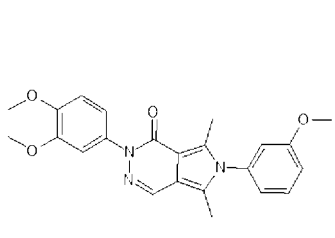

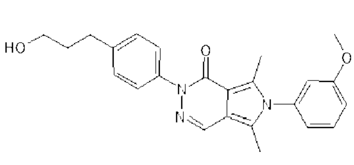 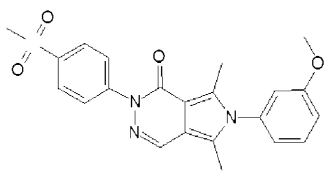 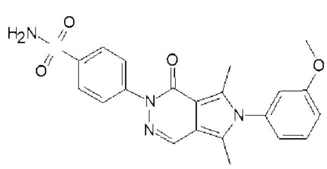

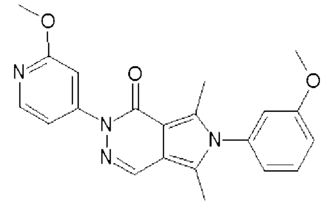
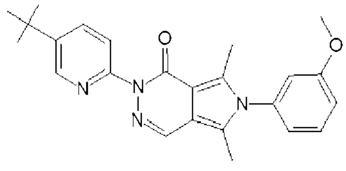
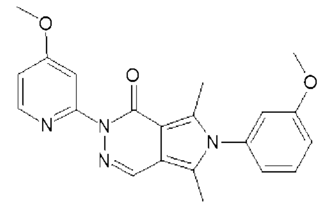
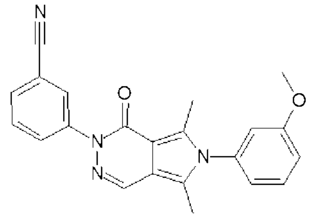
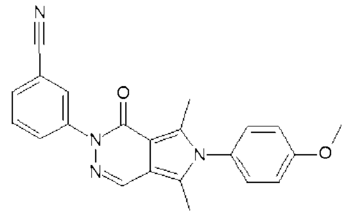
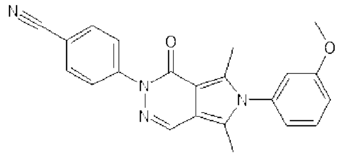
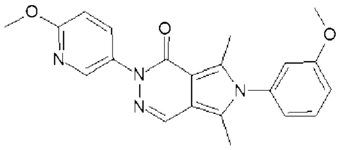
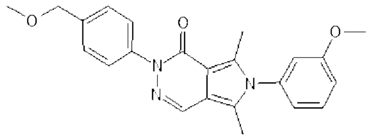
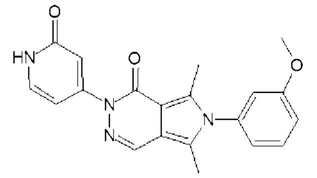
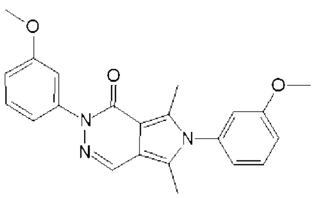
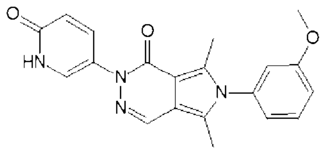
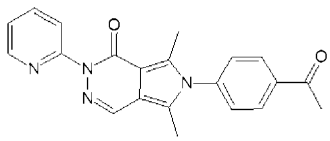
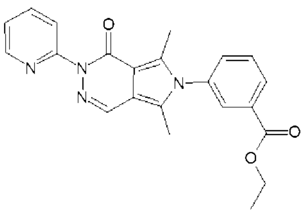
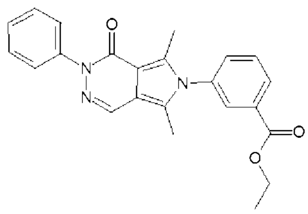
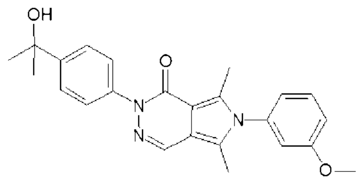
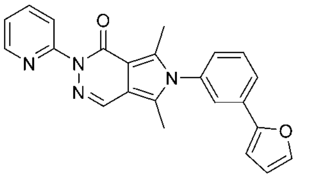
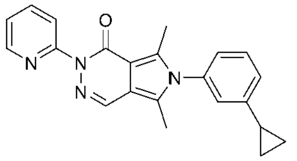
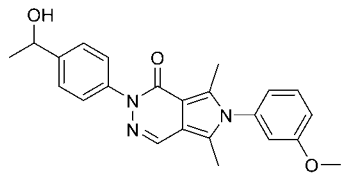
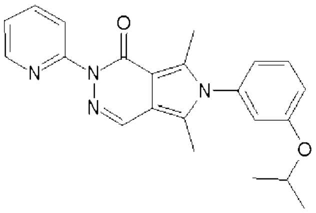
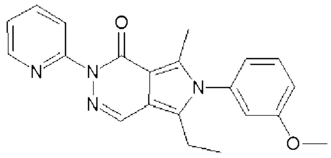
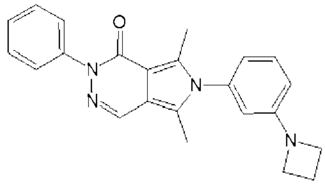

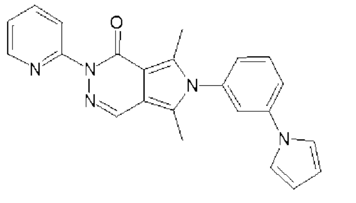
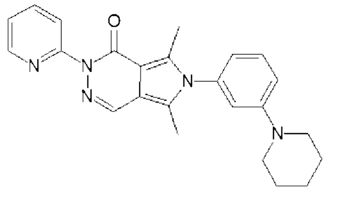
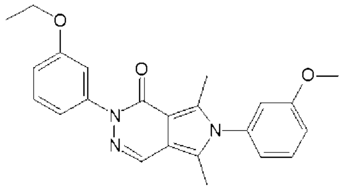

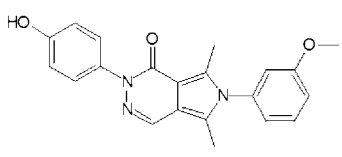
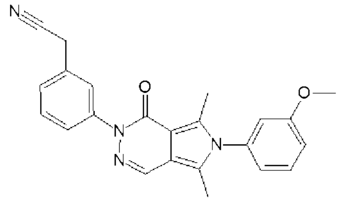
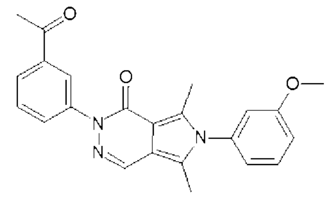

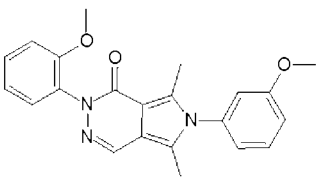
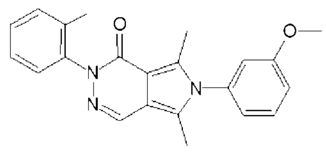
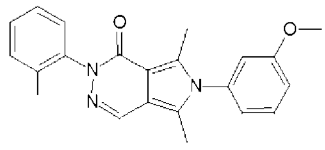

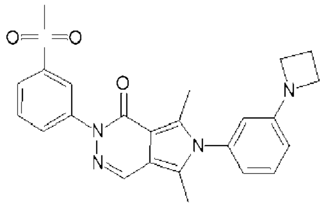
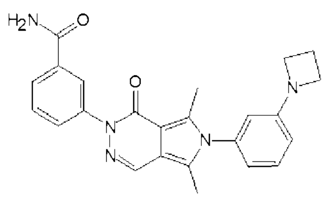
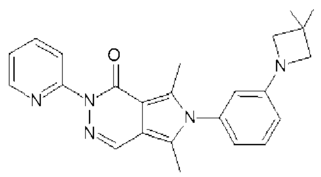

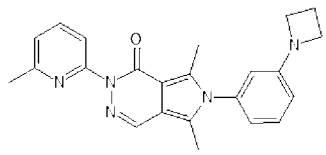
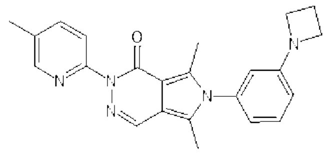
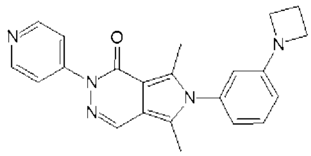

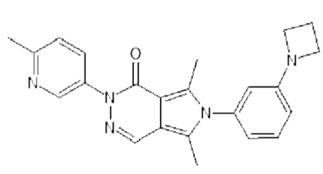
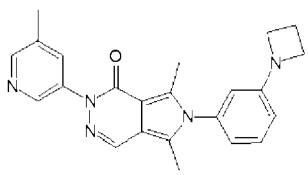
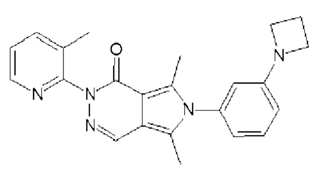

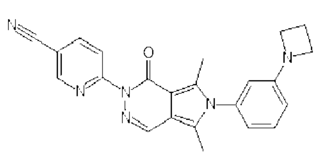
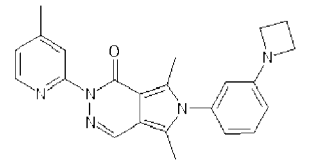
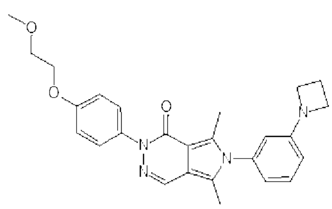

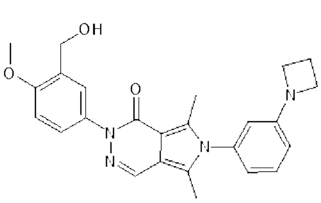
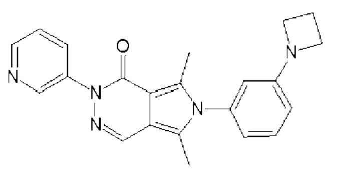
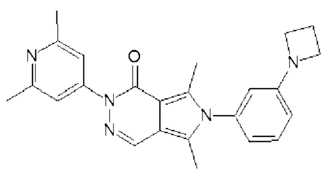

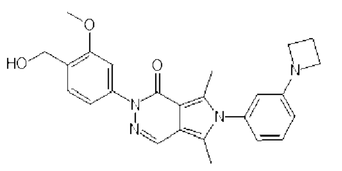
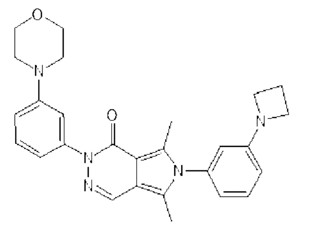
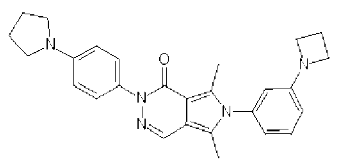
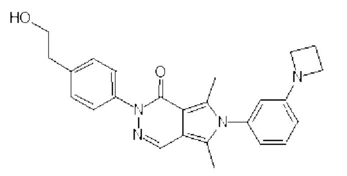
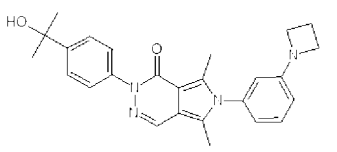
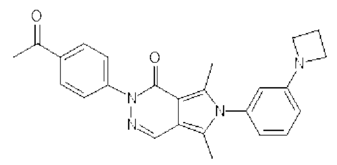
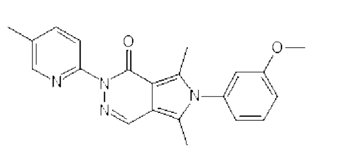
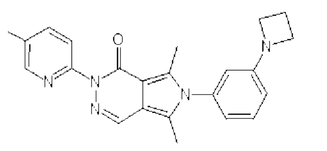
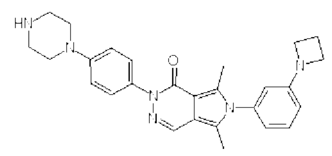
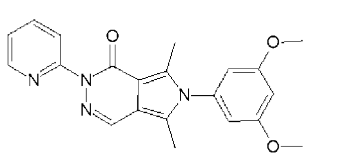
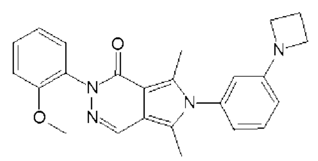
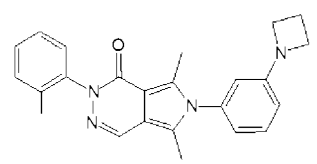
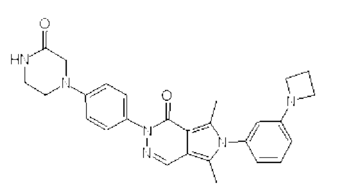
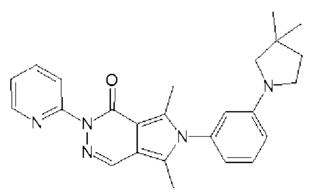
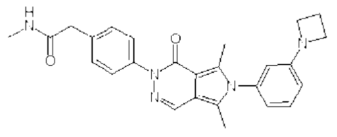
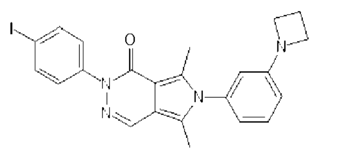
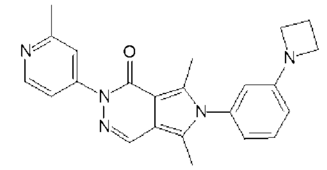
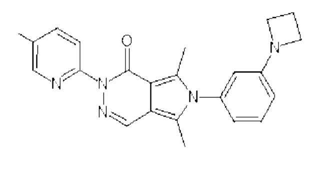
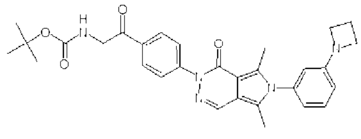
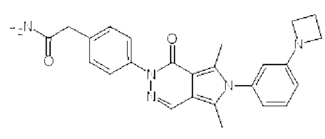
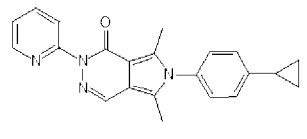
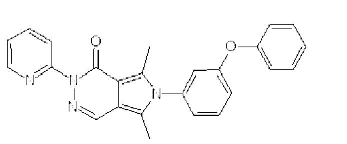
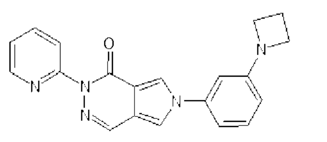
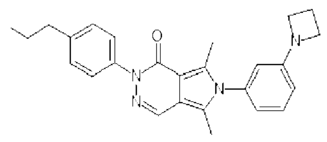

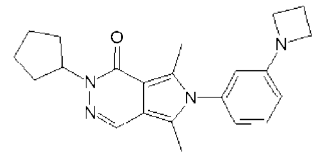
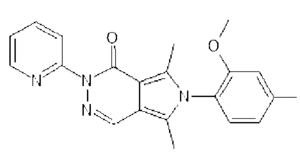
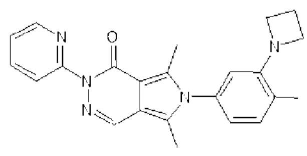

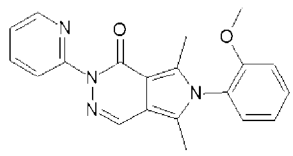
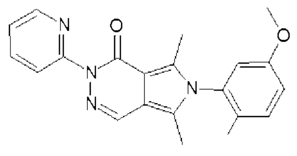
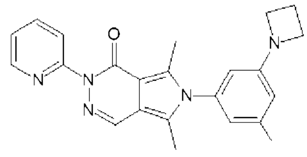

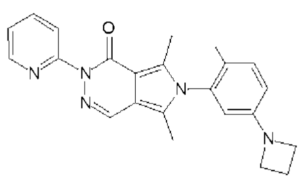
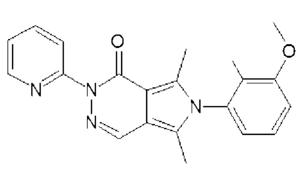
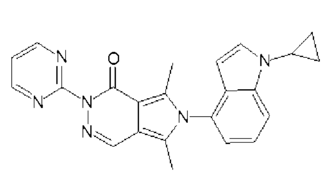

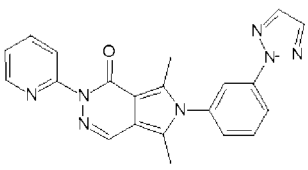
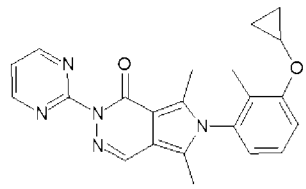
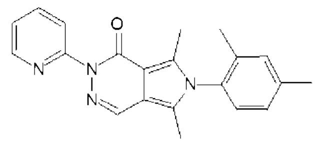

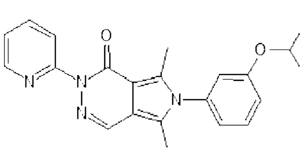
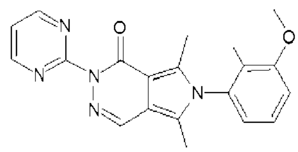
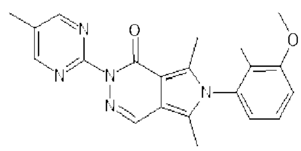

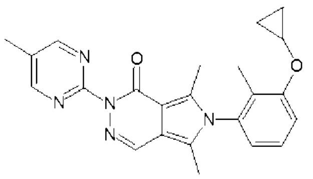
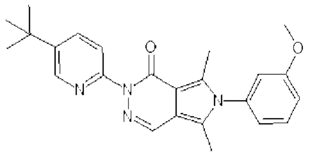
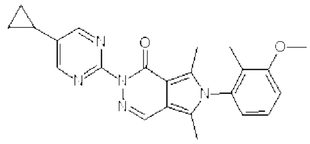

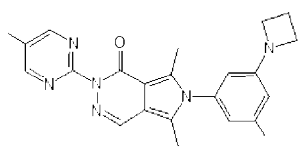
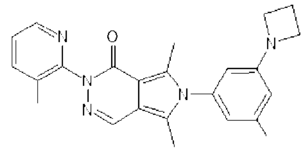
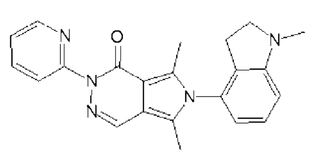

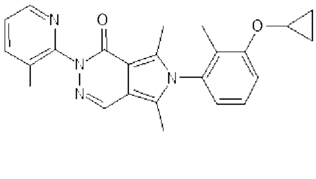
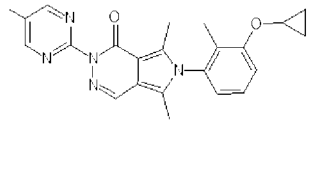
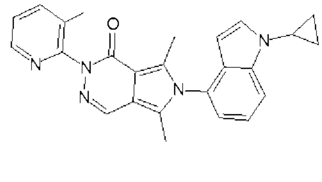

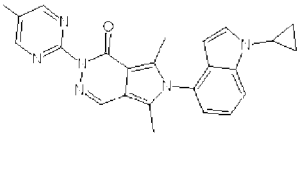
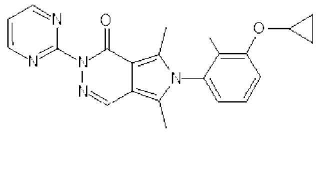
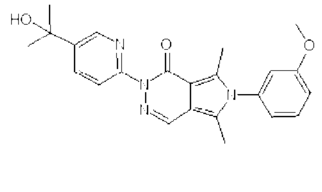

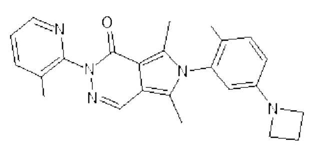
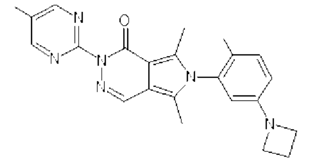
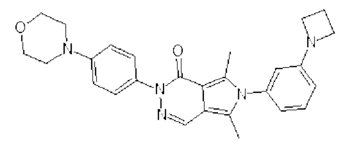

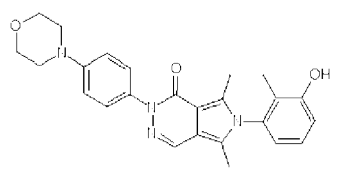
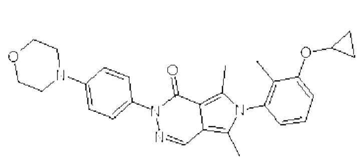
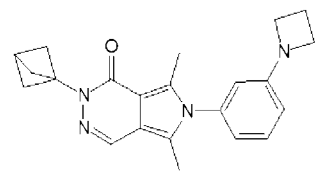

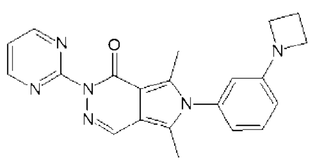
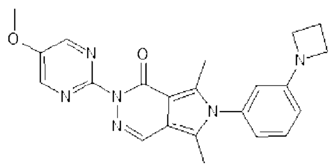
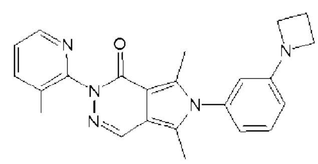

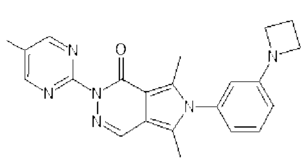
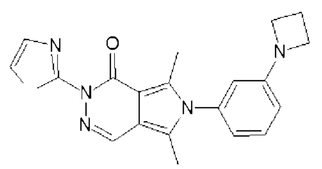
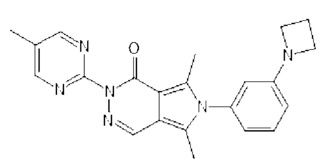

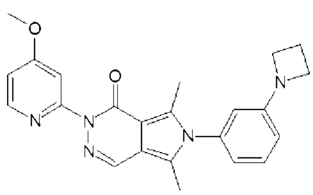
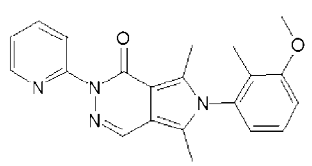
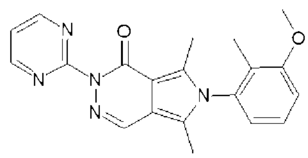

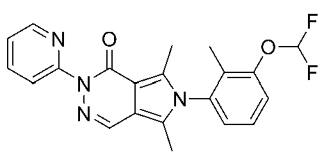
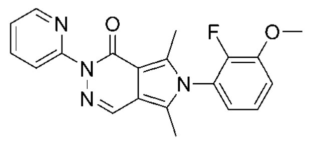
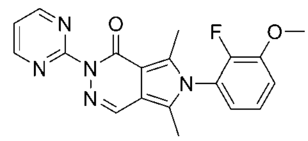

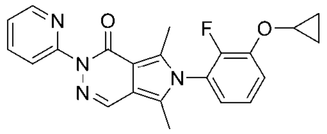
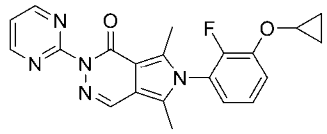
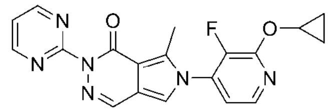

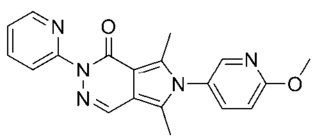
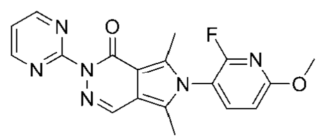
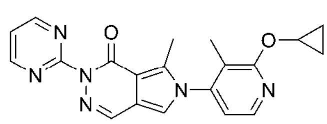

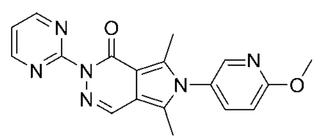
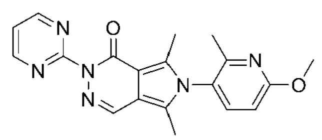
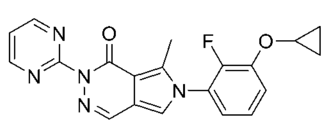

and a pharmaceutically acceptable acid or base addition salt thereof, a stereochemically isomeric form thereof or an

N-oxide form thereof; for example wherein said compound is one or more selected from

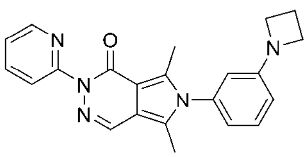,
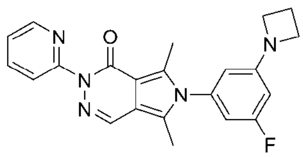,
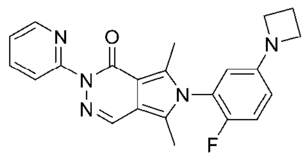,
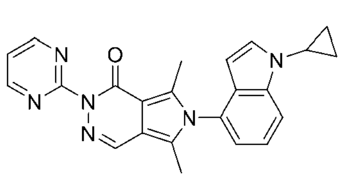,
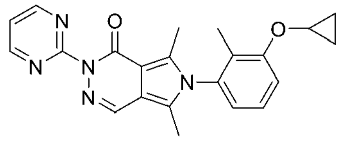,
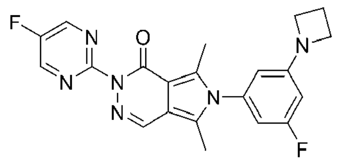,
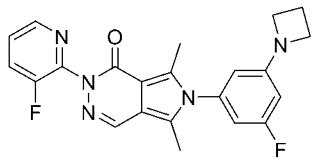,
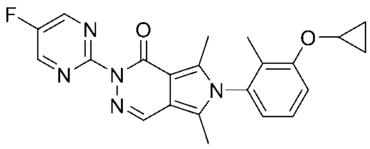,
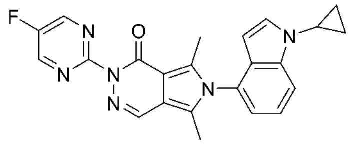,
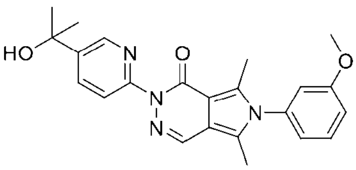,
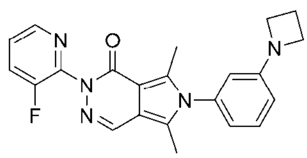,
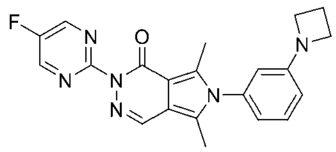
and
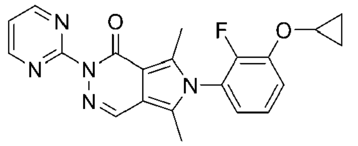,

and a pharmaceutically acceptable acid or base addition salt thereof, a stereochemically isomeric form thereof or an N-oxide form thereof ; for example wherein said compound is one or more selected from

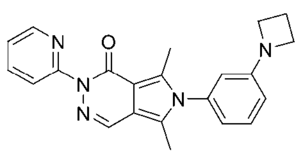,
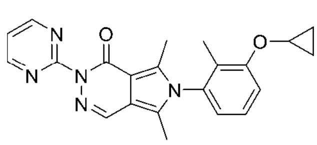,
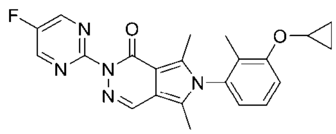

and

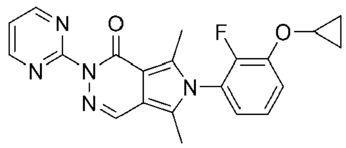,

and a pharmaceutically acceptable acid or base addition salt thereof, a stereochemically isomeric form thereof or an

N-oxide form thereof.

**11.** A pharmaceutical composition comprising a therapeutically effective amount of a compound according to any one of claims 1 to 10 and a pharmaceutically acceptable carrier and/or excipient.

**12.** A compound or composition according to any one of claims 1 to 11 for use in the treatment or prevention of a condition in a mammal affected or facilitated by the modulatory effect of a mGlu7 allosteric modulator, such as a mGlu7 negative allosteric modulator.

**13.** A compound or composition according to any one of claims 1 to 11 for use in treating, preventing, ameliorating, controlling or reducing the risk of various neurological and psychiatric disorders associated with glutamate dysfunction in a mammal; for example wherein the treatment or prevention is affected or facilitated by the modulatory effect of a mGlu7 negative allosteric modulator.

**14.** A compound or composition for use according to claim 13 wherein the condition is one or more of a central nervous system disorder, an otic disease or disorder or a pain disorder;

for example wherein the central nervous disorder is an anxiety disorder such as agoraphobia, generalized anxiety disorder (GAD), obsessive-compulsive disorder (OCD), panic disorder, or post-traumatic stress disorder (PTSD);
for example wherein the otic disease and disorder is one or more of an inner ear impairment, age-related hearing impairment (presbycusis), Meniere's disease, sudden hearing loss, noise induced hearing loss, otitis media, autoimmune inner ear disease, acute tinnitus, chronic tinnitus, drug-induced hearing loss, hidden hearing loss, cisplatin-induced hearing loss, aminoglycosides-induced hearing loss, ototoxicity, central auditory processing disorder or vestibular disorder;
for example wherein the pain disorder is one or more of neuropathic pain, inflammatory pain, visceral pain, acute pain, chronic pain, severe pain, intractable pain, post-traumatic pain, post-operative pain, headache pain or cancer pain.

**15.** A compound or composition according to any one of claims 1 to 11 for use as a medicament.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019063569 A, Duvey
- WO 2008131439 A, Friedman
- WO 9929695 A, Bantick


### Non-patent literature cited in the description


- **NAKANISHI et al.** *Brain Res. Rev.*, 1998, vol. 26, 230-235
- **NISWENDER ; CONN**. *Ann. Rev. Pharmacol. Toxicol.*, 2010, vol. 50, 295-322
- **SCHOEPP et al.** *Neuropharmacology*, 1999, vol. 38, 1431-1476
- **OHISHI et al.** *J. Comp. Neurol.*, 1995, vol. 360 (4), 555-570
- **KINZIE et al.** *Neuroscience*, 1995, vol. 69 (1), 167-176
- **CORTI et al.** *Eur. J. Neurosci*, 1998, vol. 10 (12), 3629-3641
- **DALEZIOS et al.** *Cereb. Cortex*, 2002, vol. 12 (9), 961-974
- **CARTMELL ; SCHOEPP**. *J. Neurochem.*, 2000, vol. 75, 889-907
- **SOMOGYI et al.** *Eur. J. Neurosci.*, 2003, vol. 17 (12), 2503-2520
- **HELLYER et al.** *Curr. Opin. Pharmacol.*, 2017, vol. 32, 49-55
- **STANSLEY ; CONN**. *Trends Pharmacol. Sci.*, 2019, vol. 40 (4), 240-52
- **DOGRA ; CONN**. *Mol.*, 2022, vol. 101 (5), 275-285
- **VASQUEZ-VILLA ; TRABANCO**. *Med. Chem. Comm.*, 2019, vol. 10, 193-9
- **MITSUKAWA et al.** *Proc. Natl. Acad. Sci. USA*, 2005, vol. 102, 18712-18717
- **GEE et al.** *J. Biol. Chem. 18*, 2014, vol. 289 (16), 10975-10987
- **SUZUKI et al.** *J. Pharmacol. Exp. Ther.*, 2007, vol. 323, 147-156
- **KALINICHEV et al.** *J. Pharmacol. Exp. Ther.*, 2013, vol. 344 (3), 624-636
- **REED et al.** *ACS Med. Chem. Lett.*, 2017 (12), 1326-1330
- **PALLAZO et al.** *Curr. Neuropharmacol.*, 2016, vol. 14 (5), 504-513
- **PETERLIK et al.** *Curr Neuropharmacol.*, 2016, vol. 14 (5), 514-539
- **KINOSHITA et al.** *J. Comp. Neurol.*, 1998, vol. 393 (3), 332-352
- **MAKOFF et al.** *Brain Res. Mol. Brain Res.*, 1996, vol. 40 (1), 165-170
- **CRYAN et al.** *Eur. J. Neuroscience*, 2003, vol. 17, 2409-2417
- **MITSUKAWA et al.** *PNAS*, 2005, vol. 102 (51), 18712-18717
- **MITSUKAWA et al.** *Neuropsychopharm*, 2006, vol. 31 (6), 1112-1122
- **MASUGI et al.** *J. Neurosc.*, 1999, vol. 19 (3), 955-963
- **GODDYN et al.** *Neurobiol. Learn. Mem.*, 2008, vol. 90 (1), 103-111
- **SUKOFF RIZZO et al.** *J. Pharmacol. Exp. Ther.*, 2011, vol. 338 (1), 345-352
- **PELKEY et al.** *Neuropharmacology*, 2007, vol. 52 (1), 108-117
- **PALAZZO et al.** *Pain*, 2015, vol. 156 (6), 1060-1073
- **GEE et al.** *J. Biol. Chem.*, 2014, vol. 289 (16), 10975-10987
- **PALAZZO et al.** *Neuropharmacol.*, 2008, vol. 55 (4), 537-545
- **MARABESE et al.** *J. Neurophysiol.*, 2007, vol. 98, 43-53
- **DOLAN et al.** *Behav. Pharmacol.*, 2009, vol. 20 (7), 596-604
- **OSIKOWICZ et al.** *Pain*, 2008, vol. 139 (1), 117-126
- **MOLONEY et al.** *Neurobiol. Stress*, 2015, vol. 2, 28-33
- **VAN LAER et al.** *Eur. J. Hum. Genet.*, 2010, vol. 18 (6), 685-693
- **FRIEDMAN et al.** *Hum. Mol. Genet.*, 2009, vol. 18 (4), 785-796
- **NEWMAN et al.** *Hear Res.*, 2012, vol. 294, 125-132
- **LUO et al.** *PLoS One*, 2013, vol. 8 (10), e77153
- **HAIDER et al.** *Front. Aging Neurosci.*, 2017, vol. 9, 346
- **MATYAS et al.** *Pathol. Oncol. Res.*, 2019, vol. 25 (4), 1645-52

- **CHANG et al.** *J. Int. Adv. Otol.*, 2018, vol. 14 (2), 170-175
- **LU et al.** *BMC Med. Genet.*, 2018, vol. 19 (1), 4
- **FLOR et al.** *Neuropharmacol*, 1997, vol. 36, 153-159
- **OKAMATO et al.** *J. Biol. Chem.*, 1994, vol. 269, 1231-1236
- **FERRAGUTI F** ; **SHIGEMOTO R.** *Cell Tissue Res.*, 2006, vol. 326, 483-504
- **B. A. STEAMS et al.** *Bioorg. & Med. Chem. Let*, 2004, vol. 14, 1295-1298
- Remington: the Science and Practice of Pharmacy. Mack Publishing Co., 1995
- **GREEN T.W.** ; **WUTS P.G.M.** Protecting Groups in Organic Synthesis. John Wiley & Sons, 1991
- **ELIEL E. L** ; **WILEN S. H.** ; **MANDER L .N.** Stereochemistry of Organic Compounds. Wiley-Interscience, 1984
- **KATRIZKY A. R.** ; **REES C. W.** Comprehensive Heterocyclic Chemistry. Pergamon Press, 1984
- *Bioorg. Med. Chem. Lett.*, 2010, vol. 20 (1), 189
- *Bioorg. Med. Chem. Lett.*, 2008, vol. 16 (23), 10001
- *Tetrahedron*, 2006, vol. 62, 6018
- *ioorg. Med. Chem.*, 2010, vol. 18 (1), 202
- *Bioorg. Med. Chem. Lett.*, 2004, vol. 14, 1295